(19) Europäisches Patentamt
European Patent Office
Office européen des brevets

(11) EP 4 768 487 A1

(12) **EUROPEAN PATENT APPLICATION**
published in accordance with Art. 153(4) EPC

(43) Date of publication:
**01.07.2026 Bulletin 2026/27**

(21) Application number: **24870954.5**

(22) Date of filing: **27.09.2024**

(51) International Patent Classification (IPC):
***C07D 491/052*** (2006.01) ***C07D 401/14*** (2006.01)
***C07D 487/00*** (2006.01) ***C07D 471/00*** (2006.01)
***A61K 31/435*** (2006.01) ***A61K 31/496*** (2006.01)
***A61K 31/498*** (2006.01) ***A61P 35/00*** (2006.01)

(52) Cooperative Patent Classification (CPC):
**A61K 31/435; A61K 31/496; A61K 31/498; A61P 35/00; C07D 401/14; C07D 471/00; C07D 487/00; C07D 491/052**

(86) International application number:
**PCT/CN2024/121721**

(87) International publication number:
**WO 2025/067417 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311273328**
**03.04.2024 CN 202410401501**
**07.08.2024 CN 202411076915**

(71) Applicant: **Chengdu Zenitar Biomedical Technology Co., Ltd.**
**Chengdu, Sichuan 610000 (CN)**

(72) Inventors:
- **JIA, Tao**
  **Chengdu, Sichuan 610000 (CN)**
- **WANG, Taijin**
  **Chengdu, Sichuan 610000 (CN)**
- **LI, Gang**
  **Chengdu, Sichuan 610000 (CN)**

(74) Representative: **karo IP**
**Patentanwälte PartG mbB**
**Steinstraße 16-18**
**40212 Düsseldorf (DE)**

# (54) FUSED LACTAM RING COMPOUND AND USE THEREOF

(57) The present invention discloses a fused lactam ring compound and the use thereof, and belongs to the technical field of chemical pharmaceuticals. The fused lactam ring compound as represented by formula I provided in the present invention can be used as a PARP1 inhibitor, has the advantages of high activity and high selectivity, and also has excellent pharmacokinetic properties and levels of safety.

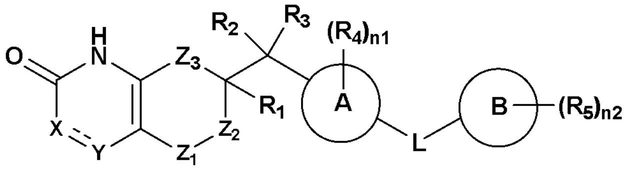

**formula I**

EP 4 768 487 A1

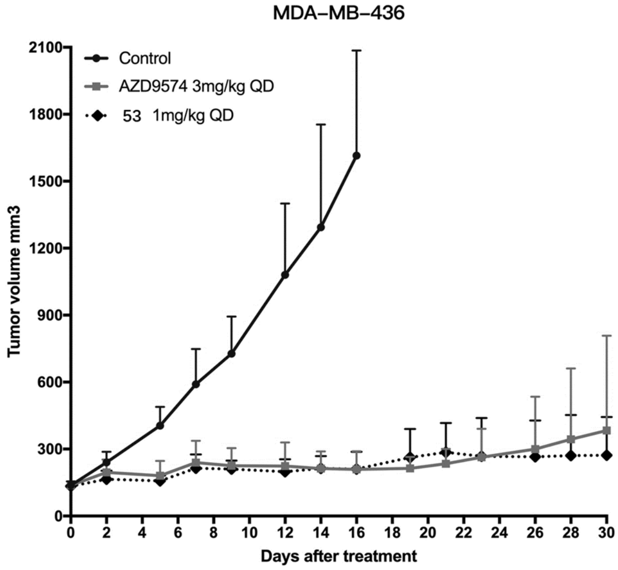
MDA–MB–436
Control
AZD9574 3mg/kg QD
53 1mg/kg QD
Tumor volume mm3
2100
1800
1500
1200
900
600
300
0
0
2
4
6
8
10
12
14
16
18
20
22
24
26
28
30
Days after treatment

FIG. 2

## Description

### TECHNICAL FIELD

The present disclosure pertains to the field of chemical medicine, and relates to a class of fused lactam ring compounds and use thereof.

### BACKGROUND

During the growth of a cell, its DNA is continuously damaged by various internal and surrounding adverse factors. In DNA damage, the most severe types of damage are single-strand breaks and double-strand breaks, with single-strand breaks being more common. If these breaks are not repaired in a timely and accurate manner, they can lead to genomic instability, which in turn may cause carcinogenesis or even directly result in cell death. For DNA single-strand breaks, their repair primarily relies on PARP enzymes. For double-strand breaks, there are two repair mechanisms for double-stranded DNA: one is non-homologous end joining repair, and the other is homologous recombination repair. Homologous recombination repair is a high-fidelity, error-free repair mechanism, and is also the primary approach for double-stranded DNA repair. Homologous recombination repair involves numerous proteins, among which BRCA proteins are the most well-known. Two studies in 2005 (Farmer H, Mccabe N, et al. Targeting the DNA repair defect in BRCA mutant cells as a therapeutic strategy[J]. Nature, 2005, 434(7035):917-921. Bryant, H., Schultz, N., Thomas, H. et al. Specific killing of BRCA2-deficient tumours with inhibitors of poly(ADP-ribose) polymerase. Nature 434, 913-917 (2005)) demonstrated that tumor cells lacking BRCA1 or BRCA2 can be selectively inhibited by PARP inhibitors. Based on this research finding, scholars proposed the concept of synthetic lethality: the loss of either the BRCA or PARP gene alone is not lethal, but the simultaneous inactivation of both genes can lead to cell death. Based on the synthetic lethality theory, PARP inhibitors (PARPi) have been developed to selectively target cancer cells with BRCA1/2 mutations.

PARP inhibitors have shown excellent clinical therapeutic efficacy in cancer patients with homologous recombination deficiency. However, hematological toxicities (anemia, neutropenia, and thrombocytopenia) and other toxicities limit the application of such drugs, whether used as monotherapy or in combination therapy. Relevant research has shown that (Harris P A, Boloor A, Cheung M, et al. Discovery of 5-[[4-[(2,3-dimethyl-2H-indazol-6-yl)methylamino]-2-pyrimidinyl] amino]-2-methyl-benzene sulfonamide (Pazopanib), a novel and potent vascular endothelial growth factor receptor inhibitor.[J]. Journal of Medicinal Chemistry, 2008, 51(15):4632.) these adverse reactions may originate from the inhibition of PARP2 by marketed PARP inhibitors, while PARP2 is not essential for therapeutic efficacy. Highly selective PARP1 inhibitors can reduce hematological toxicity, improve the therapeutic safety window, and increase the potential for use in combination with other chemotherapy or targeted drugs.

Therefore, there exists an unmet clinical need for effective and safe PARP inhibitors, particularly those with selectivity for PARP1. The novel PARP1 inhibitors described in the present disclosure exhibit unexpectedly high selectivity for PARP1 over other PARP family members (such as PARP2, PARP3, PARP5a, and PARP6), and can be used for treating diseases associated with PARP function.

### SUMMARY

An objective of the present disclosure is to provide a class of fused heterocyclic compounds and use thereof, so as to achieve highly selective and effective prevention or treatment of diseases associated with PARP function.

In a first aspect, the present disclosure provides a compound represented by formula I or a pharmaceutically acceptable form thereof, wherein the structure of formula I is as follows:

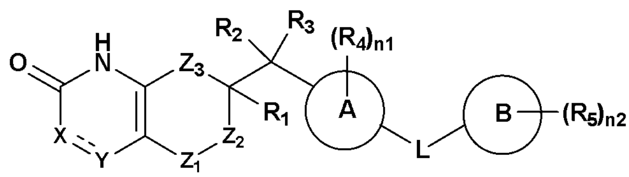

**formula I**

wherein

the structural fragment

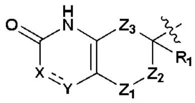

is selected from the following structures:

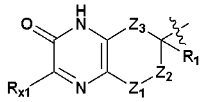 , 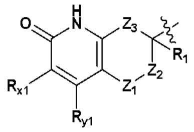 , 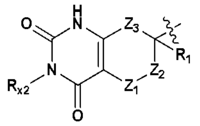 , 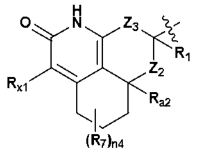 , 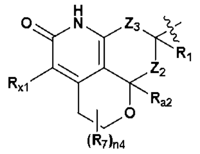 ,

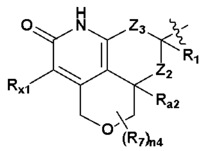 , 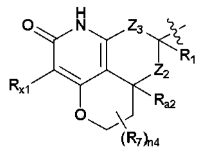 , 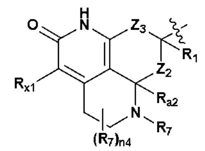 , 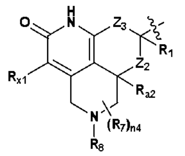 , 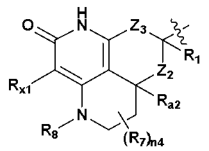 ,

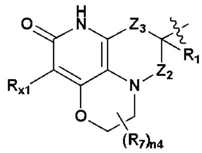 , 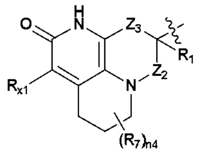 , 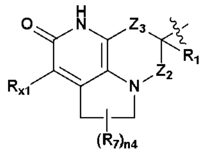 , 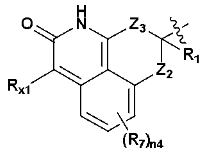 , 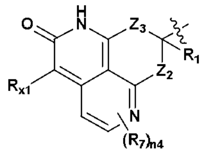 ,

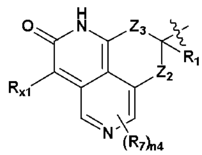 , 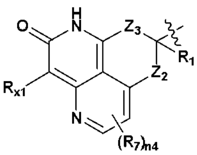 , 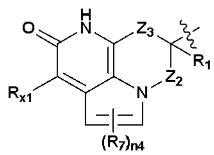 , 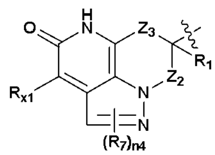 , and 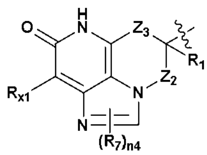 ;

$R_{x1}$ and $R_{y1}$ are independently selected from hydrogen, deuterium, halogen, cyano, amino, and the following group optionally substituted with 0-3 substituents: $C_{1-4}$ alkyl, -NH-$C_{1-4}$ alkyl, -N($C_{1-4}$ alkyl)$_2$, -O-$C_{1-4}$ alkyl, -S-$C_{1-4}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{x1}$ and $R_{y1}$, the substituent is selected from: deuterium, halogen, oxo, -OH, -$NH_2$, -CN, $C_{1-4}$ alkyl, $C_{1-4}$ fluoroalkyl, -NH-$C_{1-4}$ alkyl, -N($C_{1-4}$ alkyl)$_2$, -O-$C_{1-4}$ alkyl, and 3- to 6-membered cycloalkyl; in $R_{x1}$ and $R_{y1}$, the 4- to 6-membered heterocycloalkyl or 5- to 6-membered heteroaryl contains 1-3 heteroatoms selected from at least one of N, S, and O;

$R_{x2}$ is selected from hydrogen, deuterium, and the following group optionally substituted with 0-3 substituents: $C_{1-4}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{x2}$, the substituent is selected from: deuterium, halogen, -OH, -$NH_2$, -CN, oxo, $C_{1-4}$ alkyl, $C_{1-4}$ fluoroalkyl, -NH-$C_{1-4}$ alkyl, -N($C_{1-4}$ alkyl)$_2$, -O-$C_{1-4}$ alkyl, and 3- to 6-membered cycloalkyl; in $R_{x2}$, the 4-to 6-membered heterocycloalkyl or 5- to 6-membered heteroaryl contains 1-3 heteroatoms selected from at least one of N, S, and O;

$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -O-$C_{1-4}$ alkyl, -O-$C_{1-4}$ fluoroalkyl, -O-$C_{1-4}$ deuterated alkyl, -NH-$C_{1-4}$ alkyl, and -N($C_{1-4}$ alkyl)$_2$; n4 is selected from 1, 2, 3, 4, 5, and 6;

$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 6-membered cycloalkyl;

$Z_1$ is selected from a bond, O, $NR_{a1}$, $CR_{a2}R_{a3}$, and C=$Z_4$;

$Z_2$ is selected from O, $NR_{b1}$, and $CR_{b2}R_{b3}$;

$Z_3$ is selected from O and $CR_{c1}R_{c2}$;

$R_{a1}$ and $R_{b1}$ are independently selected from hydrogen, deuterium, and the following group optionally substituted with 0-6 substituents: $C_{1-6}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{a1}$ and $R_{b1}$, the substituent is selected from: deuterium, halogen,

-OH, $-NH_2$, -CN, and 3- to 6-membered cycloalkyl; in $R_{a1}$ and $R_{b1}$, the 4- to 6-membered heterocycloalkyl or 5- to 6-membered heteroaryl contains 1-3 heteroatoms selected from at least one of N, S, and O;

$R_{a2}$, $R_{a3}$, $R_{b2}$, $R_{b3}$, $R_{c1}$, and $R_{c2}$ are independently selected from hydrogen, deuterium, fluorine, hydroxyl, amino, $C_{1-4}$ alkyl, $C_{1-4}$ deuterated alkyl, $C_{1-4}$ fluoroalkyl, and hydroxyl-substituted $C_{1-4}$ alkyl;

$R_1$ is selected from hydrogen, deuterium, fluorine, hydroxyl, amino, cyano, $C_{1-4}$ alkyl, $C_{1-4}$ deuterated alkyl, $C_{1-4}$ fluoroalkyl, and hydroxyl-substituted $C_{1-4}$ alkyl;

$R_2$ and $R_3$ are independently selected from hydrogen, deuterium, fluorine, hydroxyl, amino, $C_{1-4}$ alkyl, $C_{1-4}$ deuterated alkyl, $C_{1-4}$ fluoroalkyl, and hydroxyl-substituted $C_{1-4}$ alkyl; or $R_2$ and $R_3$, together with the atom to which they are attached, form a 3- to 6-membered carbocycle;

ring A is selected from 4- to 8-membered heterocycloalkyl; in ring A, the 4- to 8-membered heterocycloalkyl contains 1-3 N heteroatoms, and one of the N heteroatoms is connected to the structural fragment

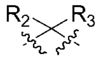
;

$R_4$ is selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; or any two $R_4$, together with the atom(s) to which they are attached, form a 3- to 6-membered carbocycle or a 5- to 7-membered heterocycle; when any two $R_4$, together with the atom(s) to which they are attached, form a ring, the 5- to 7-membered heterocycle contains 1-3 heteroatoms selected from at least one of N and O;

L is selected from a bond, O, $NR_{L1}$, and $CR_{L2}R_{L3}$;

$R_{L1}$ is selected from hydrogen, deuterium, $C_{1-4}$ alkyl, $C_{1-4}$ deuterated alkyl, $C_{1-4}$ fluoroalkyl, and hydroxyl-substituted $C_{1-4}$ alkyl;

$R_{L2}$ and $R_{L3}$ are independently selected from hydrogen, deuterium, fluorine, hydroxyl, amino, $C_{1-4}$ alkyl, $C_{1-4}$ deuterated alkyl, $C_{1-4}$ fluoroalkyl, and hydroxyl-substituted $C_{1-4}$ alkyl;

ring B is selected from 5- to 10-membered aryl and 5- to 10-membered heteroaryl; in ring B, the 5- to 10-membered heteroaryl contains 1-3 heteroatoms selected from at least one of N, S, and O;

$R_5$ is selected from hydrogen, deuterium, halogen, cyano, $-(CR_{5a}R_{5b})_{n3}OR_{5c}$, $-(CR_{5a}R_{5b})_{n3}N(R_{5c}R_{5d})$, $-COOR_{5c}$, $-CONH_2$, $-CONHR_{5c}$, $-CON(R_{5c}R_{5d})$, $-NHCOR_{5c}$, $-SO_2R_{5c}$, $-SO_2NHR_{5c}$, $-SO_2NR_{5c}R_{5d}$,

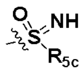
,
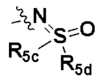
,

and the following group substituted with 1 or more $R_6$: $C_{1-6}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; or two adjacent $R_5$, together with the atoms to which they are attached, form a 5- to 6-membered carbocycle, a 5- to 6-membered heterocycle, a benzene ring, or a 5- to 6-membered heteroaromatic ring; in $R_5$, the 4- to 6-membered heterocycloalkyl or 5- to 6-membered heteroaryl contains 1-3 heteroatoms selected from at least one of N, S, and O; when two adjacent $R_5$, together with the atoms to which they are attached, form a ring, the 5- to 6-membered heterocycle or 5- to 6-membered heteroaromatic ring contains 1-3 heteroatoms selected from at least one of N, S, and O;

or $R_5$ and $R_4$, together with the atoms to which they are attached, form a 5- to 7-membered heterocycle; when $R_5$ and $R_4$, together with the atoms to which they are attached, form a ring, the 5- to 7-membered heterocycle contains 1-3 heteroatoms selected from at least one of N and O;

$R_{5a}$ and $R_{5b}$ are independently selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; or $R_{5a}$ and $R_{5b}$, together with the atom to which they are attached, form a 3- to 6-membered carbocycle or 4- to 6-membered heterocycle substituted with 0-6 substituents; when $R_{5a}$ and $R_{5b}$, together with the atom to which they are attached, form a ring, the substituent is selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; when $R_{5a}$ and $R_{5b}$, together with the atom to which they are attached, form a ring, the 5- to 6-membered heterocycle contains 1-3 heteroatoms selected from at least one of N, S, and O;

$R_{5c}$ and $R_{5d}$ are independently selected from hydrogen, deuterium, and the following group substituted with 0-6 substituents: $C_{1-4}$ alkyl, 6- to 10-membered aryl, 5- to 10-membered heteroaryl, 4- to 8-membered heterocycloalkyl, 3- to 8-membered cycloalkyl, 6- to 10-membered spirocycloalkyl, 6- to 10-membered heterospirocycloalkyl, 6- to 10-membered bridged cycloalkyl, and 6- to 10-membered bridged heterocycloalkyl; in $R_{5c}$ and $R_{5d}$, the substituent is selected from: deuterium, halogen, -OH, -CN, $C_{1-4}$ alkyl, $C_{1-4}$ deuterated alkyl, $C_{1-4}$ fluoroalkyl, $C_{1-4}$ alkoxy, 3- to 6-membered cycloalkyl, 4- to 6-membered heterocycloalkyl, 3- to 6-membered cycloalkylmethyl, 4- to 6-membered heterocycloalkylmethyl -NH-$C_{1-6}$ alkyl, -N($C_{1-6}$ alkyl)$_2$, -O$C_{1-6}$ alkyl, -$CONH_2$, and -CONH-$C_{1-6}$ alkyl;

in $R_{5c}$ and $R_{5d}$, the 5- to 10-membered heteroaryl, 4- to 8-membered heterocycloalkyl, 6- to 10-membered heterospirocycloalkyl, or 6- to 10-membered bridged heterocycloalkyl contain 1-3 heteroatoms selected from at least one of N, S, and O; in the substituents in $R_{5c}$ and $R_{5d}$, the 4- to 6-membered heterocycloalkyl or 4- to 6-membered heterocycloalkylmethyl contains 1-3 heteroatoms selected from at least one of N, S, and O;

or $R_{5a}$ and $R_{5c}$, $R_{5a}$ and $R_{5d}$, $R_{5b}$ and $R_{5c}$, $R_{5b}$ and $R_{5d}$, or $R_{5c}$ and $R_{5d}$, together with the atom(s) to which they are attached, form a 4- to 6-membered heterocycle substituted with 0-6 substituents selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy, wherein the 4- to 6-membered heterocycle contains 1-3 heteroatoms selected from at least one of N, S, and O;

$R_6$ is selected from H, deuterium, fluorine, $C_{1-4}$ alkyl, $C_{1-4}$ deuterated alkyl, and $C_{1-4}$ fluoroalkyl;

n1 is selected from 1, 2, 3, 4, 5, 6, 7, and 8;

n2 is selected from 1, 2, 3, 4, and 5;

n3 is selected from 0, 1, 2, 3, 4, and 5;

the pharmaceutically acceptable form is selected from a pharmaceutically acceptable salt, ester, stereoisomer, polymorph, solvate, nitrogen oxide, isotopically labeled compound, metabolite or prodrug.

In some embodiments of the present disclosure, $R_1$ is selected from hydrogen, deuterium, fluorine, hydroxyl, amino, and $C_{1-4}$ alkyl.

In some preferred embodiments of the present disclosure, $R_1$ is selected from hydrogen, deuterium, fluorine, and methyl.

In some embodiments of the present disclosure, $R_2$ and $R_3$ are independently selected from hydrogen, deuterium, fluorine, $C_{1-4}$ alkyl, and hydroxyl-substituted $C_{1-4}$ alkyl.

In some preferred embodiments of the present disclosure, $R_2$ and $R_3$ are independently selected from hydrogen, deuterium, fluorine, methyl, and hydroxymethyl.

In some embodiments of the present disclosure, L is selected from a bond, O, $NR_{L1}$, and $CR_{L2}R_{L3}$; $R_{L1}$ is selected from hydrogen, deuterium, methyl, deuterated methyl, fluoromethyl, and hydroxymethyl; $R_{L2}$ and $R_{L3}$ are independently selected from hydrogen, deuterium, fluorine, hydroxyl, amino, methyl, deuterated methyl, fluoromethyl, and hydroxymethyl.

In some preferred embodiments of the present disclosure, L is selected from a bond, O, and NH.

In some embodiments of the present disclosure, $R_4$ is selected from at least one of hydrogen, deuterium, fluorine, hydroxyl, amino, methyl, deuterated methyl, fluoromethyl, ethyl, methoxy, deuterated methoxy, fluoromethoxy, and hydroxymethyl; or two $R_4$ attached to adjacent C atoms, together with the atoms to which they are attached, form a 3- to 5-membered carbocycle or a 5- to 6-membered heterocycle; when two $R_4$ attached to adjacent C atoms, together with the atoms to which they are attached, form a ring, the 5- to 6-membered heterocycle contains 1-2 heteroatoms selected from at least one of N and O; n1 is selected from 1, 2, 3, and 4.

In some preferred embodiments of the present disclosure, $R_4$ is selected from at least one of hydrogen, deuterium, fluorine, hydroxyl, amino, methyl, ethyl, hydroxymethyl, and methoxy; or two $R_4$ attached to adjacent carbon atoms, together with the atoms to which they are attached, form a 3- to 5-membered carbocycle or a 5- to 6-membered heterocycle; when two $R_4$ attached to adjacent C atoms, together with the atoms to which they are attached, form a ring, the 5- to 6-membered heterocycle contains 1 O heteroatom; n1 is selected from 1 and 2.

In some embodiments of the present disclosure, the structural fragment

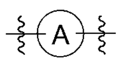

is selected from:

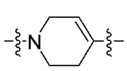, 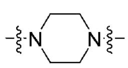, 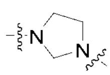, 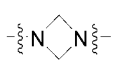, 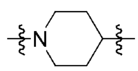, 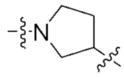, 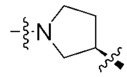,

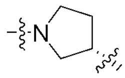, 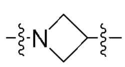, and 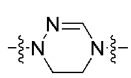

(in these structures, the left end is connected to the structural fragment

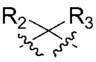,

and the right end is connected to L).

In some preferred embodiments of the present disclosure, the structural fragment

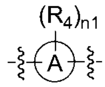

is selected from:

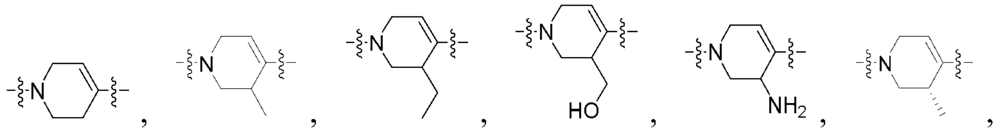,

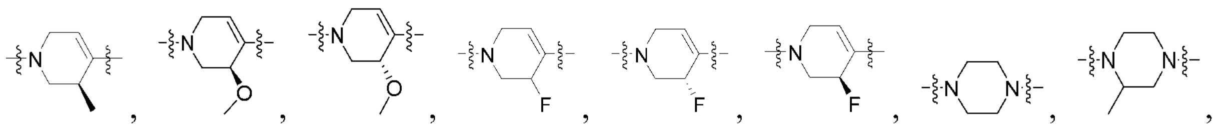,

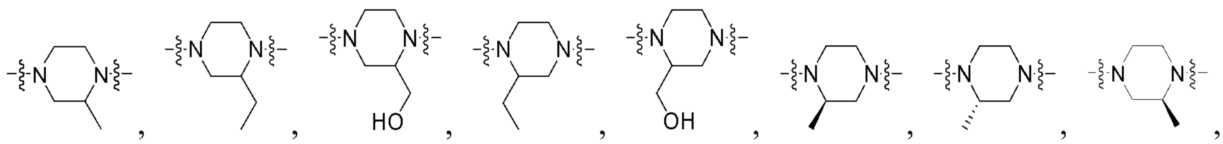,

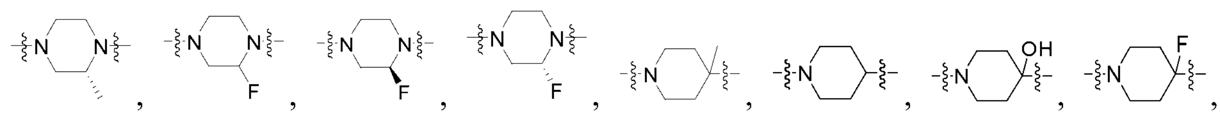,

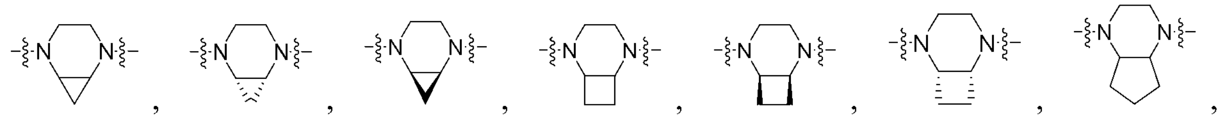,

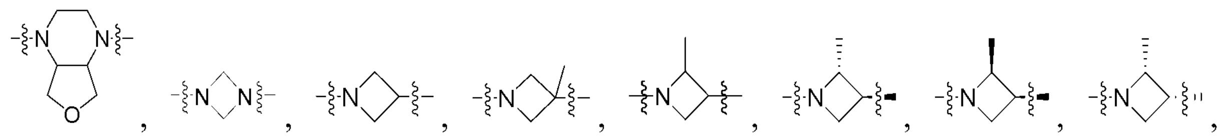,

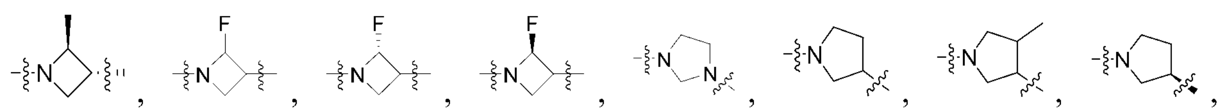,

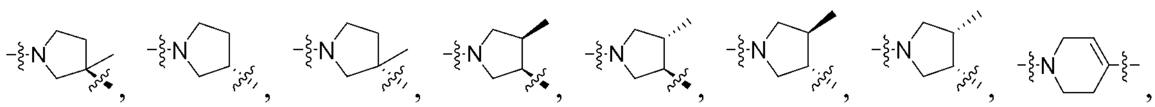,

and

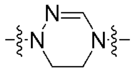

(in these structures, the left end is connected to the structural fragment

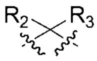,

and the right end is connected to L).

In some more preferred embodiments of the present disclosure, the structural fragment

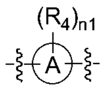

is selected from (the left end is connected to

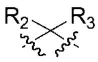,

and the right end is connected to L)

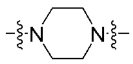, 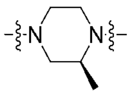, 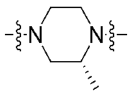, 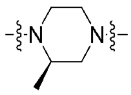, 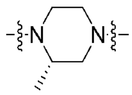, 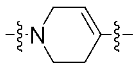, 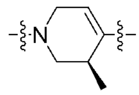,

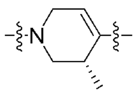, 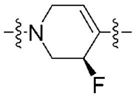, 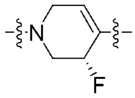, 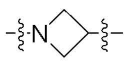, 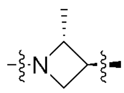, 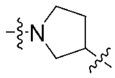, 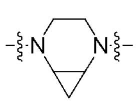, 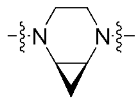,

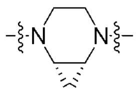, 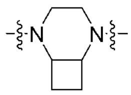, 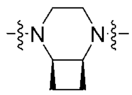, and 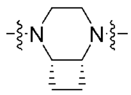

(in these structures, the left end is connected to the structural fragment

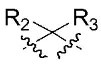,

and the right end is connected to L).

In some embodiments of the present disclosure, ring B is selected from phenyl, pyridyl, imidazolyl, imidazo[1,2-*a*]pyrazinyl, 1,7-naphthyridinyl, pyridazinyl, pyrazinyl, pyridothiazolyl, pyrazolo[1,5-*a*]pyrimidinyl, benzopyrazolyl, benzimidazolyl, thiazolyl, [1,2,4]triazolo[1,5-*a*]pyridinyl, thienyl, furyl, pyrazolyl, pyridazinopyrrolyl, pyridopyrazolyl, pyridooxazolyl, and pyrimidinyl.

In some preferred embodiments of the present disclosure, ring B is selected from

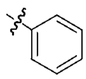,

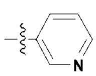, 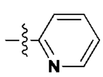, 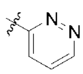, 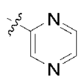, 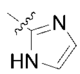, 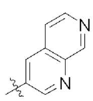, and 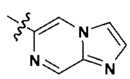

(in these structures, the wavy line bond is connected to L).

In some embodiments of the present disclosure, $R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, and 5- to 6-membered heteroarylaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5-to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3.

In some preferred embodiments of the present disclosure, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl, and

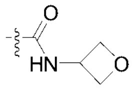;

n2 is selected from 1 and 2.

In some embodiments of the present disclosure, the structural fragment

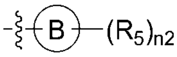

is selected from:

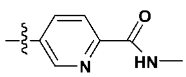, 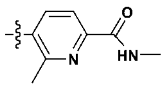, 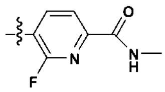, 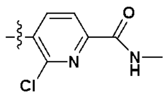, 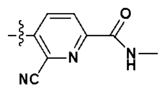,

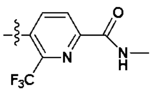, 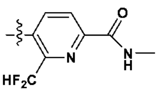, 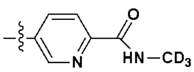, 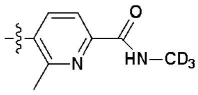, 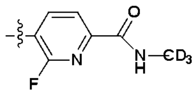, 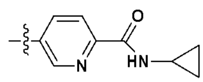,

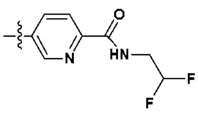, 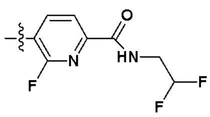, 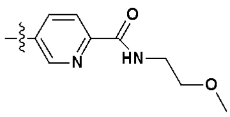, 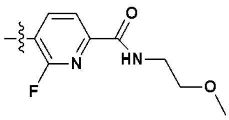, 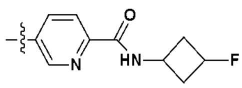,

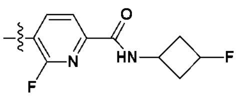, 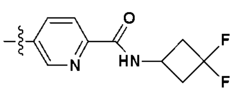, 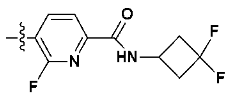, 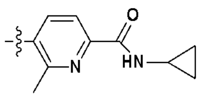, 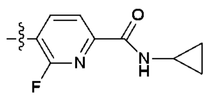,

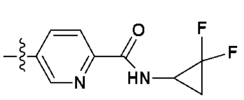, 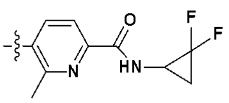, 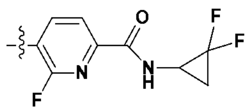, 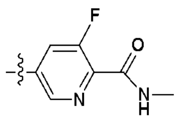, 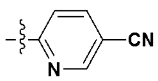,

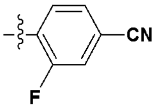, 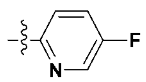, 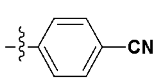, 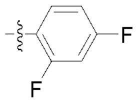, 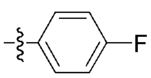, 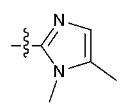, 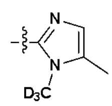,

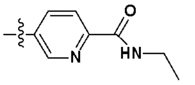, 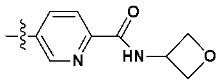, 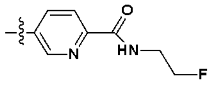, 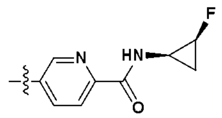, 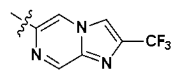,

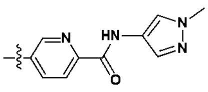, 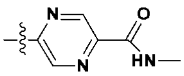, 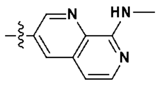, and 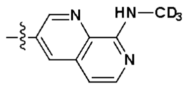.

In some embodiments of the present disclosure, $Z_3$ is selected from O, $CH_2$, CHF, and $CF_2$.

In some preferred embodiments of the present disclosure, $Z_3$ is selected from O and $CH_2$.

In some embodiments of the present disclosure, $Z_2$ is selected from O, $NR_{b1}$, and $CR_{b2}R_{b3}$; $R_{b1}$ is selected from hydrogen, deuterium, and the following group optionally substituted with 0-3 substituents: $C_{1-4}$ alkyl, 3- to 6-membered cycloalkyl, or 4- to 6-membered heterocycloalkyl; in $R_{b1}$, the substituent is selected from: deuterium, fluorine, -OH, $-NH_2$, -CN, and 3- to 4-membered cycloalkyl; in $R_{b1}$, the 4- to 6-membered heterocycloalkyl contains 1-2 heteroatoms selected from N and O; $R_{b2}$ and $R_{b3}$ are independently selected from hydrogen, deuterium, fluorine, and $C_{1-4}$ alkyl.

In some preferred embodiments of the present disclosure, $Z_2$ is selected from O, $NR_{b1}$, and $CR_{b2}R_{b3}$; $R_{b1}$ is selected from hydrogen, deuterium, and the following group optionally substituted with 0-3 substituents: $C_{1-4}$ alkyl or 3- to 4-membered cycloalkyl; in $R_{b1}$, the substituent is selected from: deuterium, fluorine, -OH, $-NH_2$, -CN, and 3- to 4-membered cycloalkyl; $R_{b2}$ and $R_{b3}$ are independently selected from hydrogen, deuterium, fluorine, and methyl.

In some more preferred embodiments of the present disclosure, $Z_2$ is selected from O, $CH_2$, NH, $NCH_3$, $NCD_3$, and *N*-cyclopropyl.

In some embodiments of the present disclosure, when $Z_1$ is selected from a bond, O, $NR_{a1}$, and $CR_{a2}R_{a3}$, $R_{a1}$ is selected from hydrogen, deuterium, and the following group optionally substituted with 0-3 substituents: $C_{1-4}$ alkyl, 3- to 6-membered cycloalkyl, or 4- to 6-membered heterocycloalkyl; in $R_{a1}$, the substituent is selected from: deuterium, fluorine, -OH, $-NH_2$, -CN, and 3- to 4-membered cycloalkyl; in $R_{a1}$, the 4- to 6-membered heterocycloalkyl contains 1-2 heteroatoms selected from N and O; $R_{a2}$ and $R_{a3}$ are independently selected from hydrogen, deuterium, fluorine, and $C_{1-4}$ alkyl.

In some preferred embodiments of the present disclosure, when $Z_1$ is selected from a bond, O, $NR_{a1}$, and $CR_{a2}R_{a3}$, $R_{a1}$ is selected from hydrogen, deuterium, and the following group optionally substituted with 0-3 substituents: $C_{1-4}$ alkyl or 3- to 4-membered cycloalkyl; in $R_{a1}$, the substituent is selected from: deuterium, fluorine, -OH, $-NH_2$, -CN, and 3- to 4-membered cycloalkyl; $R_{a2}$ and $R_{a3}$ are independently selected from hydrogen, deuterium, fluorine, and methyl.

In some more preferred embodiments of the present disclosure, when $Z_1$ is selected from a bond, O, $NR_{a1}$, and $CR_{a2}R_{a3}$, $Z_1$ is selected from O, $CH_2$, NH, $NCH_3$, $NCD_3$, and *N*-cyclopropyl.

In some preferred embodiments of the present disclosure, $R_{x1}$ and $R_{y1}$ are independently selected from hydrogen, deuterium, halogen, cyano, amino, $C_{1-4}$ alkyl, $C_{1-4}$ deuterated alkyl, $C_{1-4}$ fluoroalkyl, -NH-$C_{1-4}$ alkyl, -N($C_{1-4}$ alkyl)$_2$, -O-$C_{1-4}$ alkyl, -S-$C_{1-4}$ alkyl, 3- to 6-membered cycloalkyl, 3- to 6-membered fluorocycloalkyl, and phenyl;

$R_{x2}$ is selected from hydrogen, deuterium, $C_{1-4}$ alkyl, $C_{1-4}$ deuterated alkyl, $C_{1-4}$ fluoroalkyl, 3-to 6-membered cycloalkyl, 3- to 6-membered fluorocycloalkyl, and phenyl;

$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -NH-$C_{1-4}$ alkyl, and -N($C_{1-4}$ alkyl)$_2$; n4 is selected from 1, 2, 3, and 4;

$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 4-membered cycloalkyl.

In some more preferred embodiments of the present disclosure, $R_{x1}$ and $R_{y1}$ are independently selected from hydrogen, deuterium, fluorine, chlorine, methyl, deuterated methyl, fluoromethyl, ethyl, fluoroethyl, cyclopropyl, and fluorocyclopropyl;

$R_{x2}$ is selected from hydrogen, deuterium, methyl, deuterated methyl, fluoromethyl, ethyl, fluoroethyl, cyclopropyl, and fluorocyclopropyl;

$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;

$R_8$ is selected from hydrogen, methyl, deuterated methyl, and fluoromethyl.

In some most preferred embodiments of the present disclosure, the structural fragment

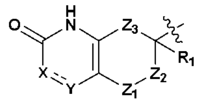

is selected from:

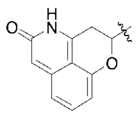, 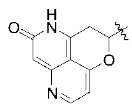, 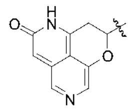, 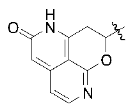, 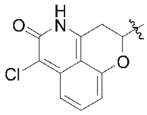,

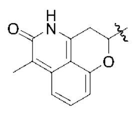, 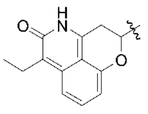, 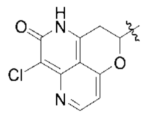, 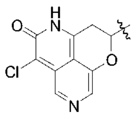, 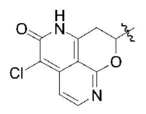, 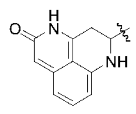, 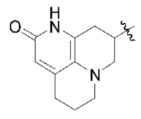,

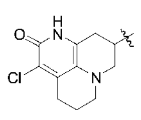, 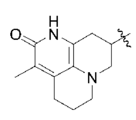, 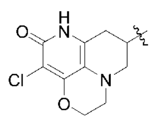, 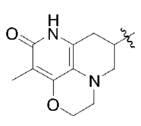, 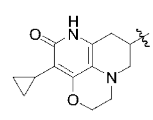, 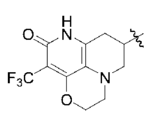, 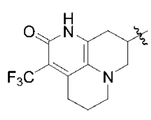,

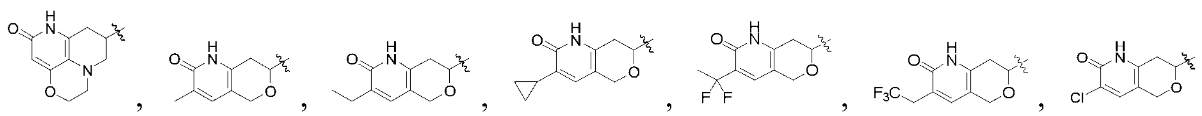
, , , , , , ,

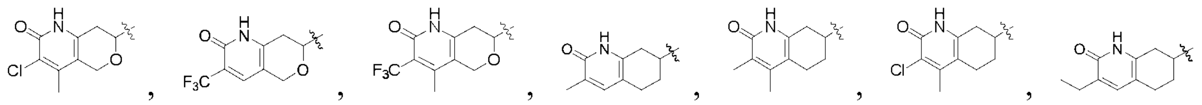
, , , , , , ,

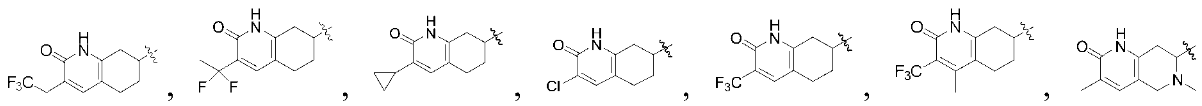
, , , , , , ,

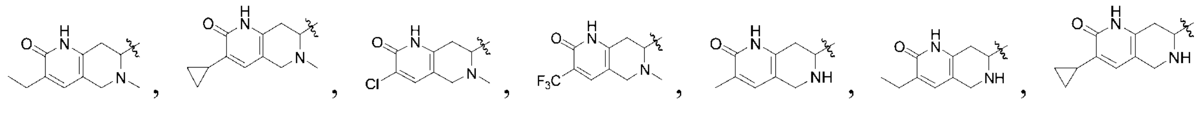
, , , , , , ,

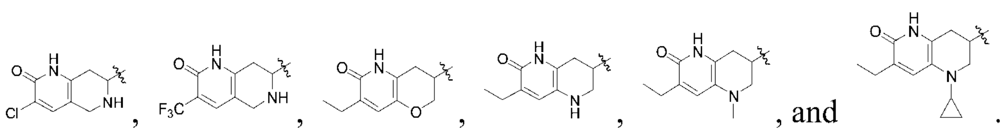
, , , , , and .

The present disclosure further provides some specific compounds represented by formula I, wherein the compounds are selected from:

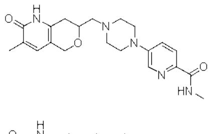 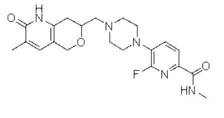 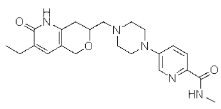 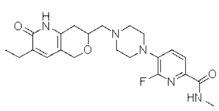 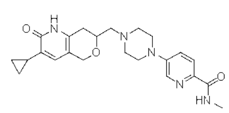

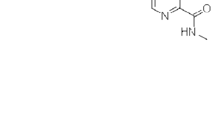 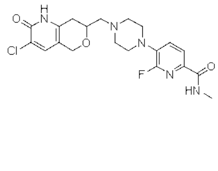 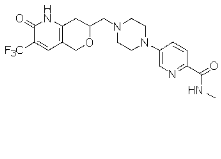 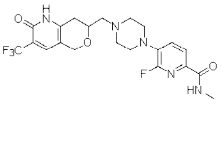 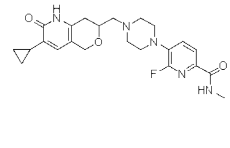

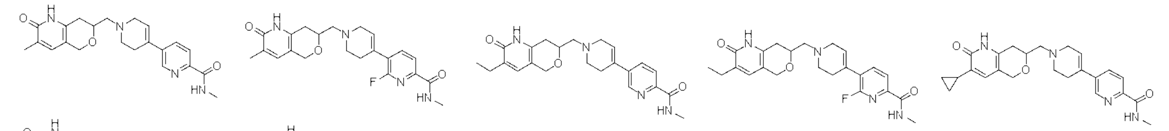
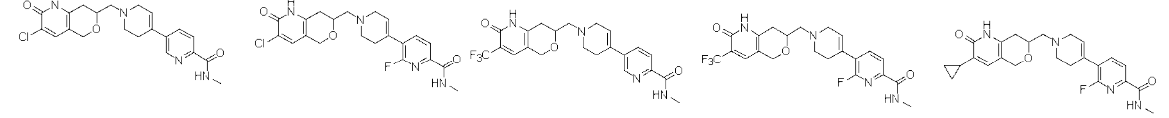
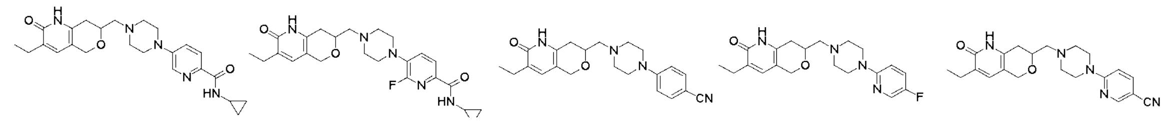
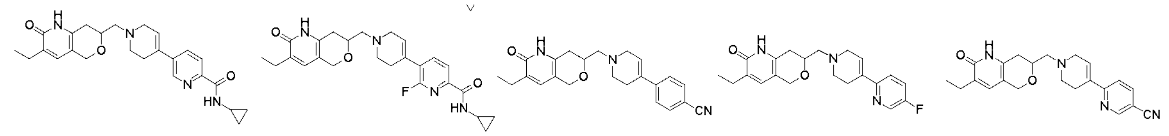
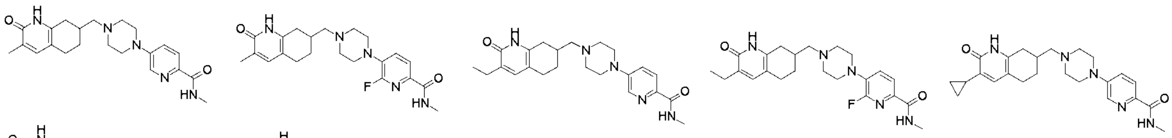
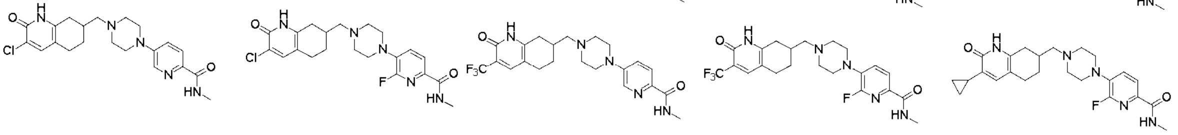
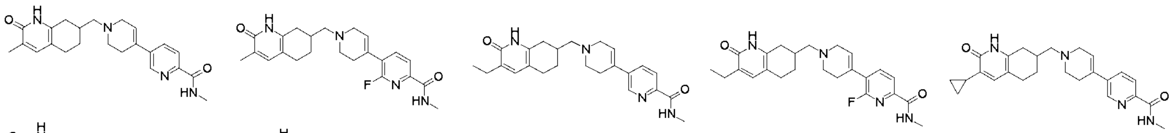
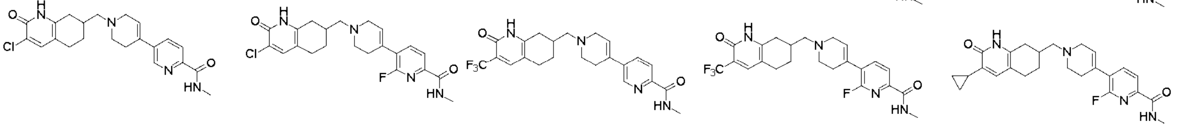
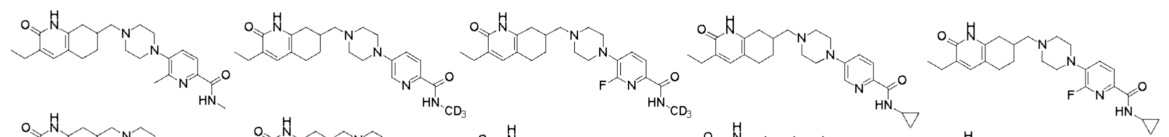
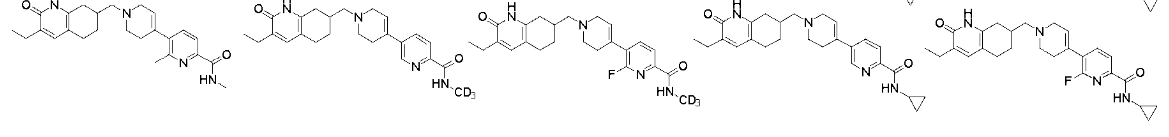
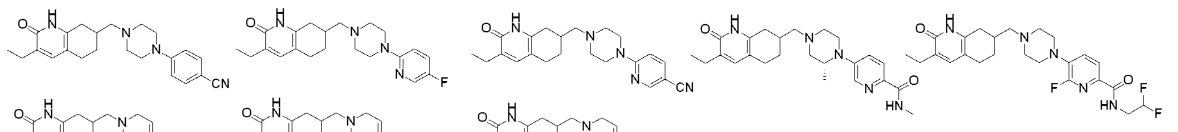
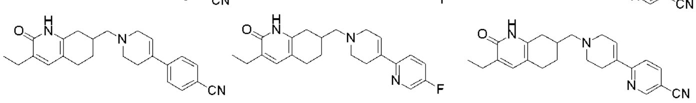

Based on the compounds of formula I described above, the present disclosure further provides a class of compounds represented by formula I , wherein in some embodiments of the present disclosure, the structural fragment

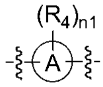

is selected from

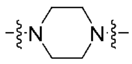, 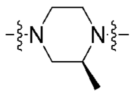,

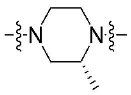, 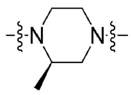, 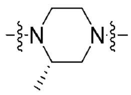, 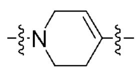, 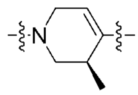, 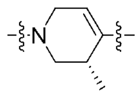, 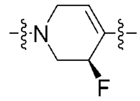,

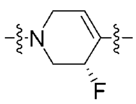, 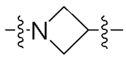, 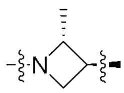, 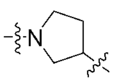, 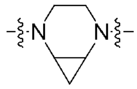, 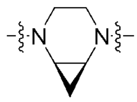, 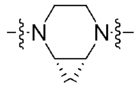, 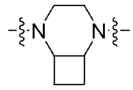,

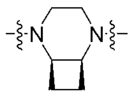, 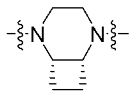, and 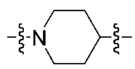

(in these structures, the left end is connected to the structural fragment

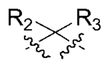,

and the right end is connected to L).

In some embodiments of the present disclosure, $R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, 5- to 6-membered heteroarylaminoacyl, $C_{1-4}$ alkoxyaminoacyl, 3-to 6-membered cycloalkyl-substituted $C_{1-4}$ alkoxyaminoacyl, and 3- to 6-membered cycloalkoxyaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5- to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3.

In some preferred embodiments of the present disclosure, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl,

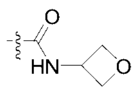, 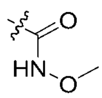, 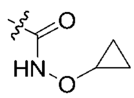, and 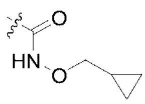;

n2 is selected from 1 and 2.

In some embodiments of the present disclosure, the structural fragment

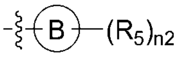

is selected from:

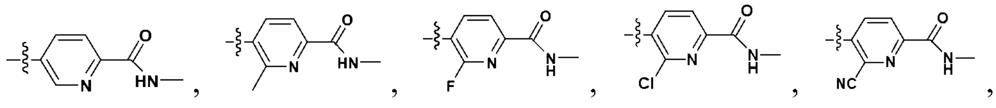

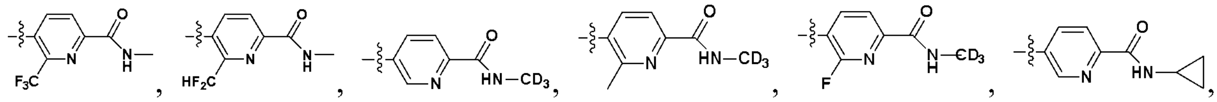

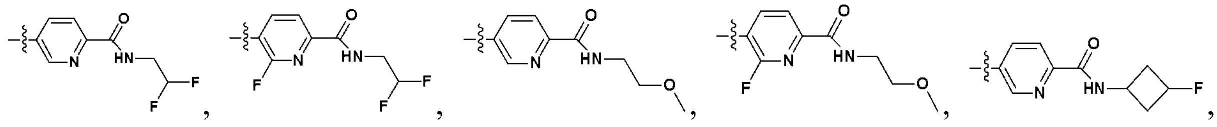

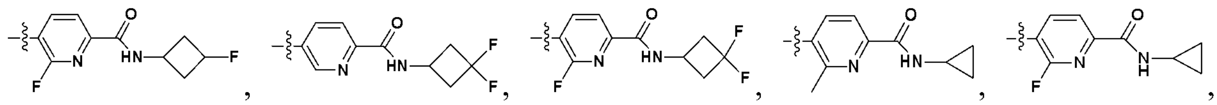

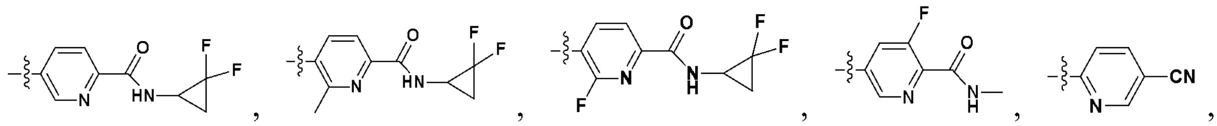

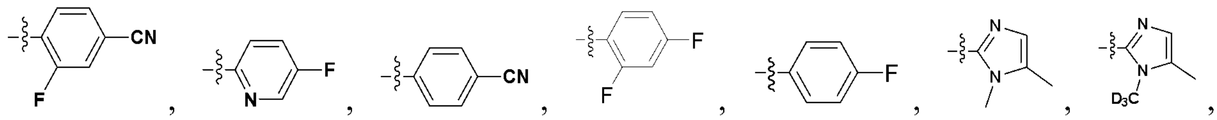

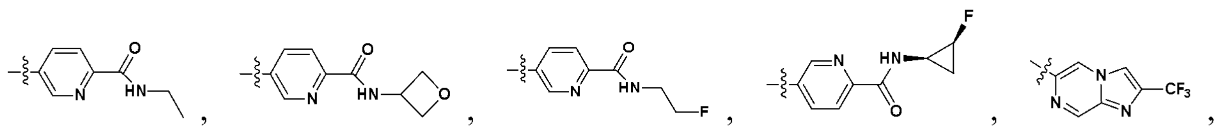

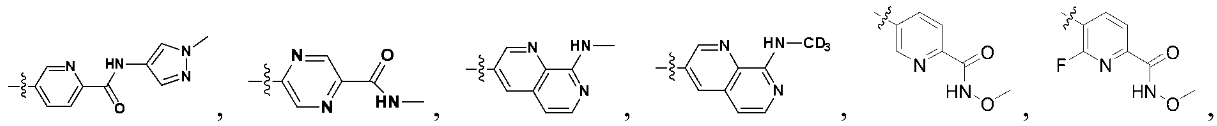

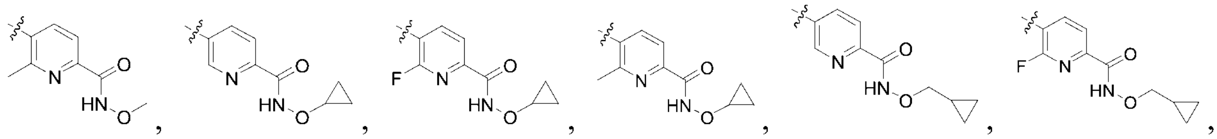

and

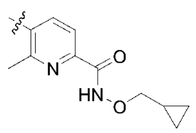

.

In some embodiments of the present disclosure, $R_{x1}$ and $R_{y1}$ are independently selected from hydrogen, deuterium, fluorine, chlorine, bromine, methyl, deuterated methyl, fluoromethyl, ethyl, fluoroethyl, cyclopropyl, and

fluorocyclopropyl;

$R_{x2}$ is selected from hydrogen, deuterium, methyl, deuterated methyl, fluoromethyl, ethyl, fluoroethyl, cyclopropyl, and fluorocyclopropyl;
$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;
$R_8$ is selected from hydrogen, methyl, deuterated methyl, and fluoromethyl.

In some preferred embodiments of the present disclosure, the structural fragment

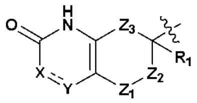

is selected from:

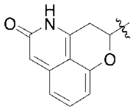, 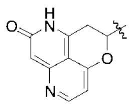, 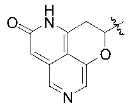, 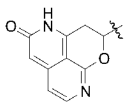, 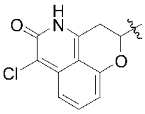,

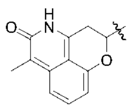, 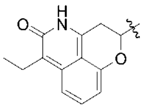, 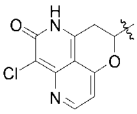, 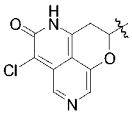, 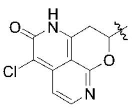, 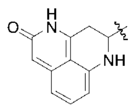, 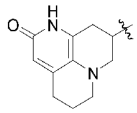,

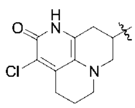, 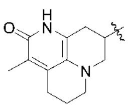, 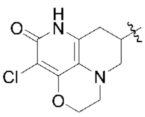, 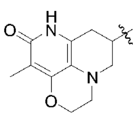, 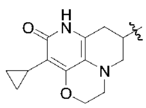, 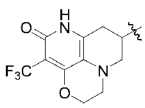, 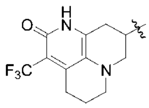,

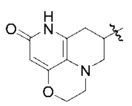, 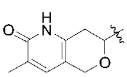, 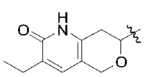, 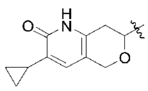, 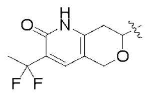, 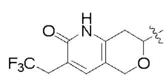, 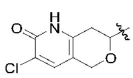,

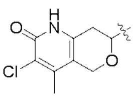, 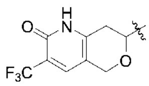, 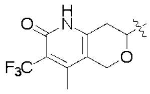, 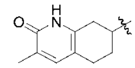, 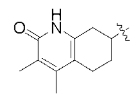, 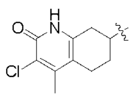, 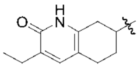,

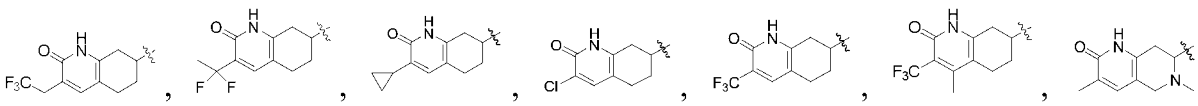, , , , , , ,

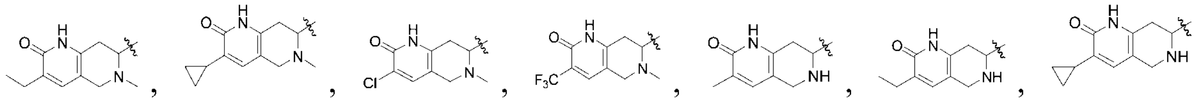, , , , , , ,

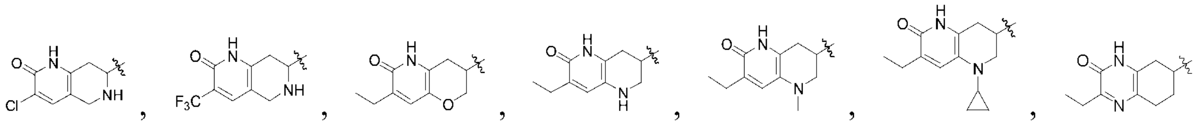, , , , , , ,

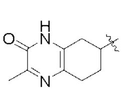, 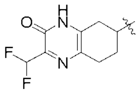, 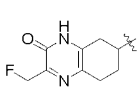, 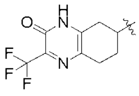, 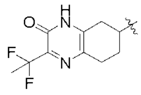, 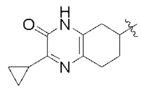, 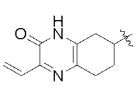,

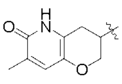, 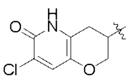, 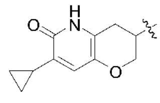, 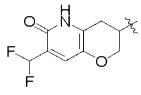, 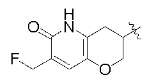, 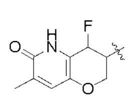, 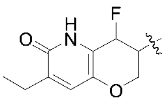,

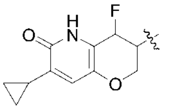, 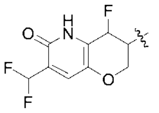, 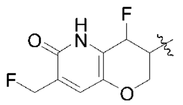, 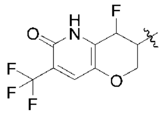, 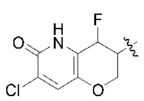, 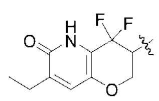,

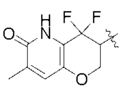, 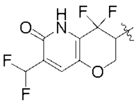, 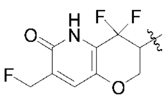, 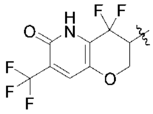, 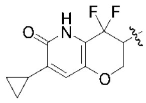, 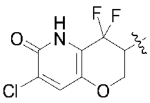, 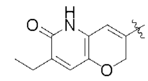,

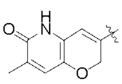, 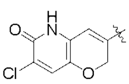, 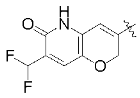, 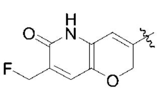, 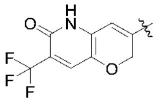, 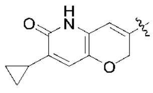, 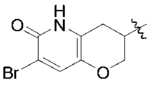,

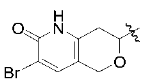, 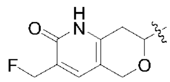, 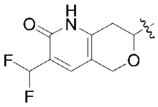, and 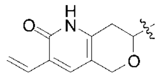.

In some embodiments of the present disclosure, the structural fragment

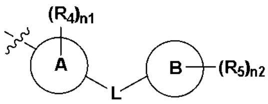

is replaced with:

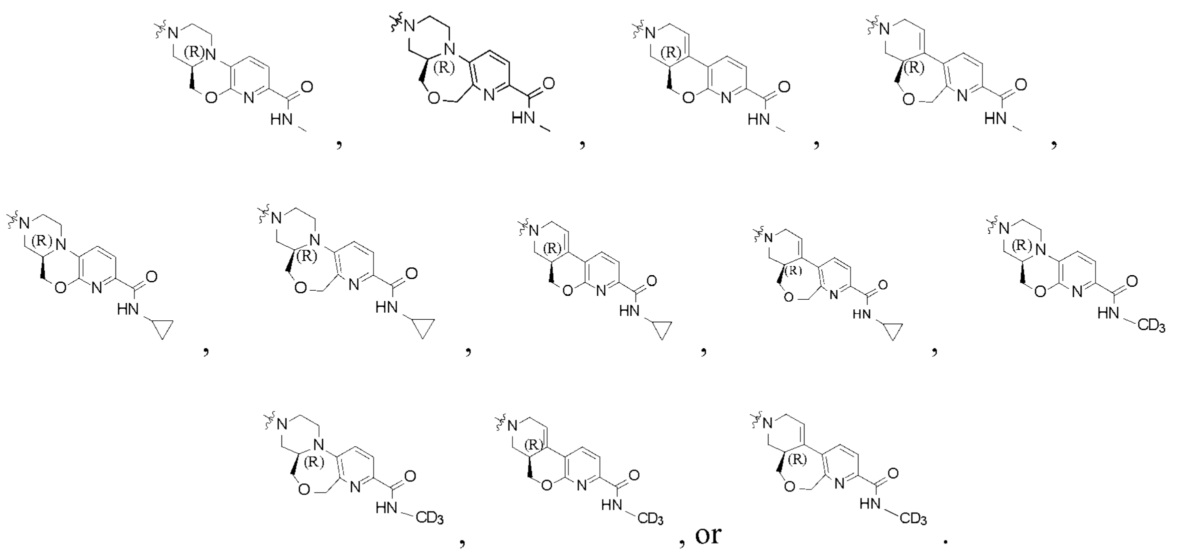

, , , , , , , , , , , or .

The present disclosure further provides some specific compounds represented by formula I, wherein the

compounds are selected from:

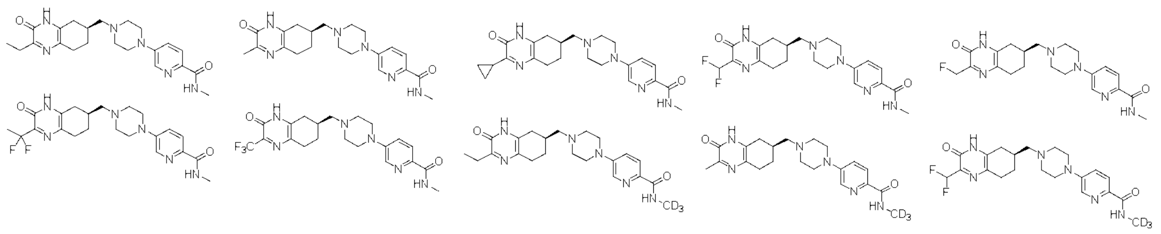

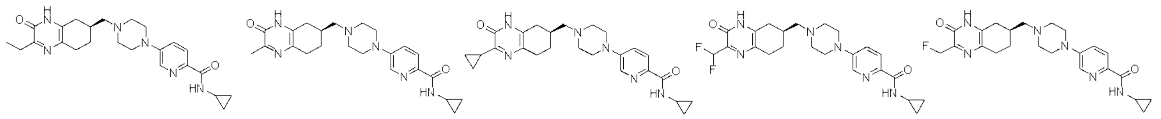

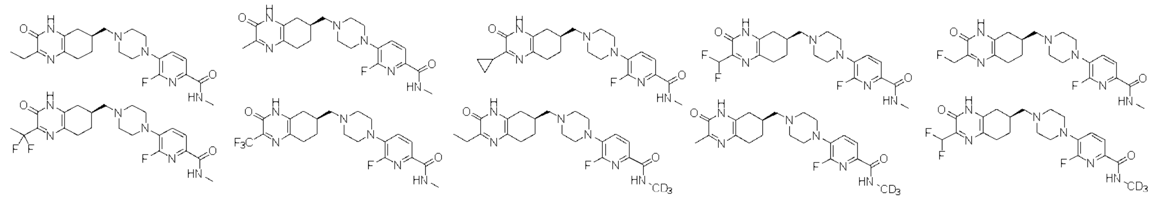

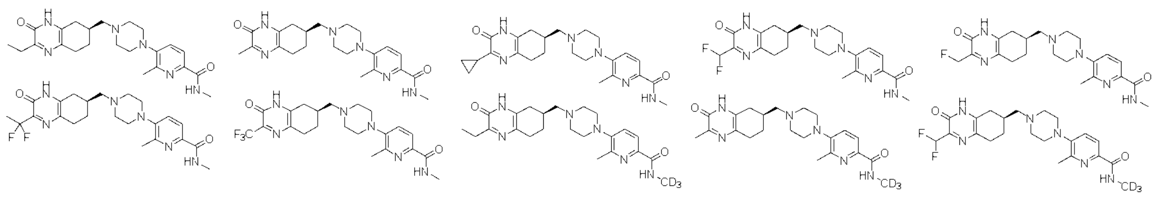

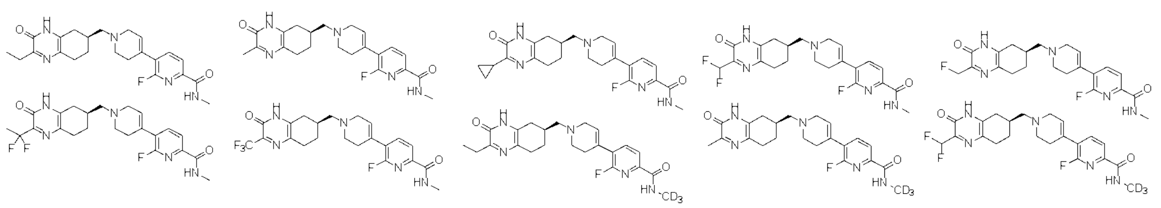

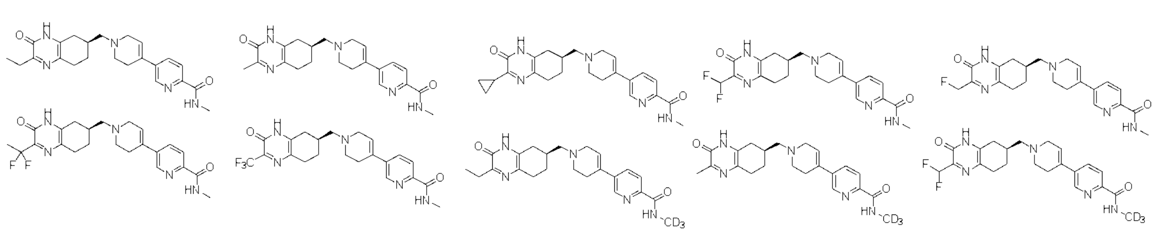

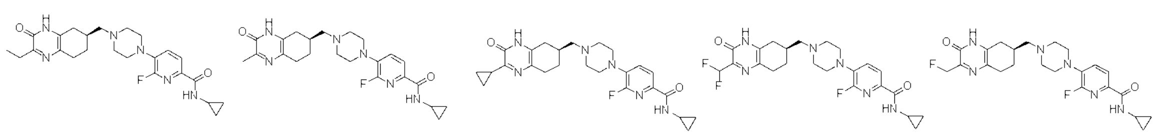

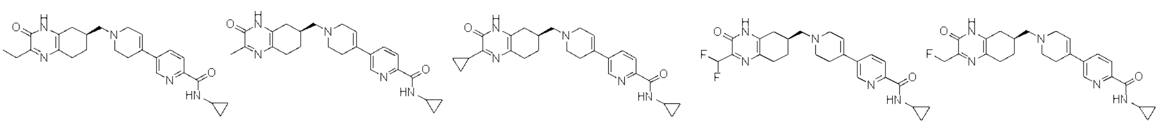

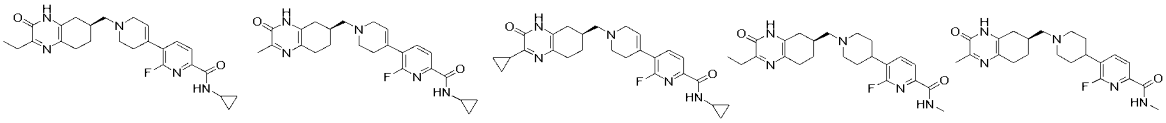

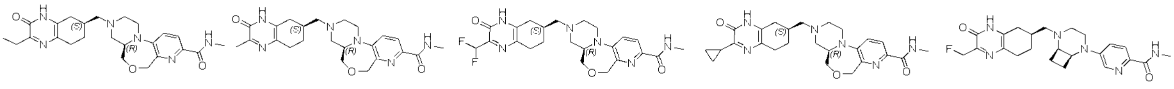

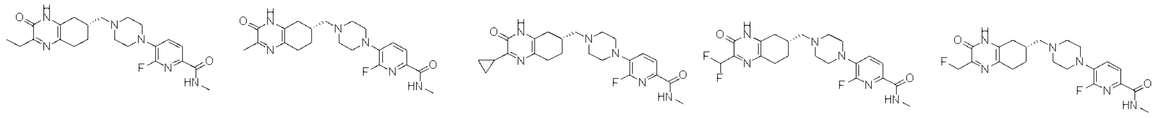

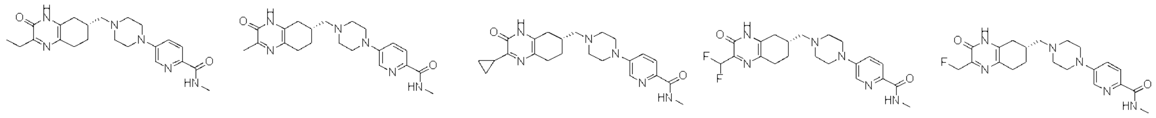

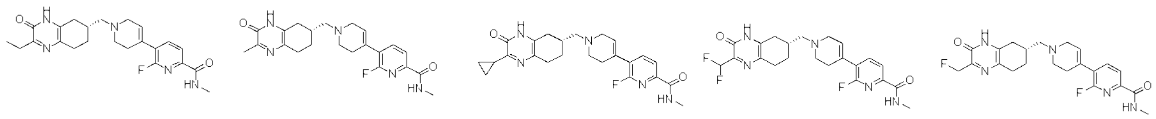

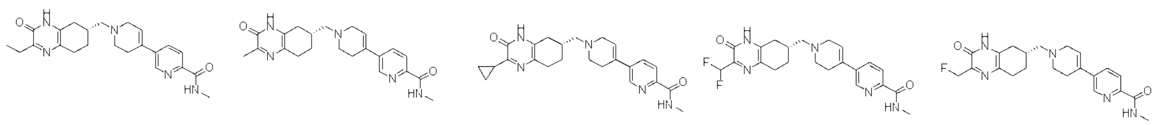

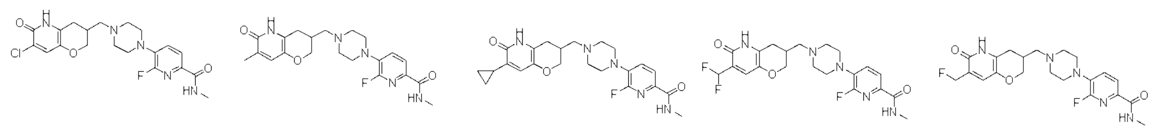

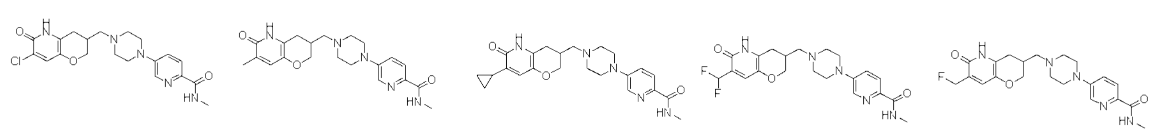

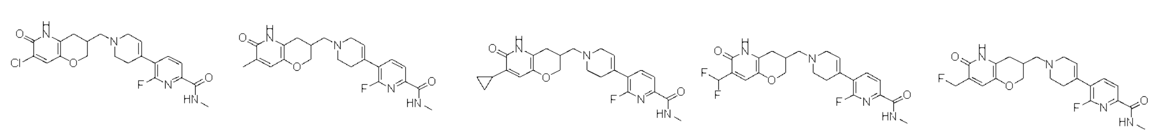

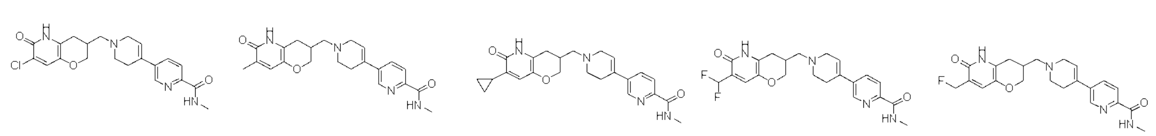

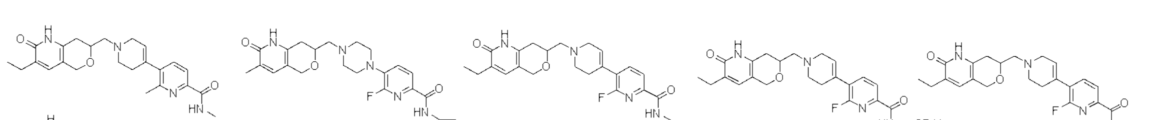

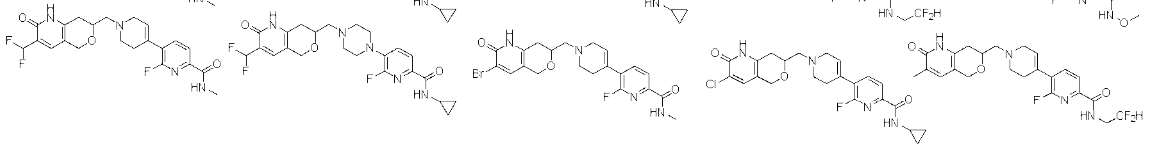

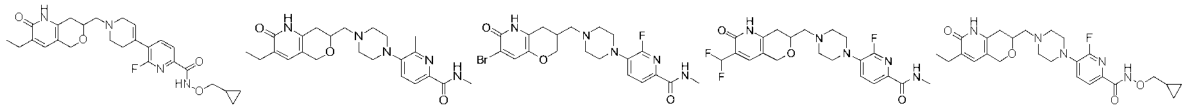

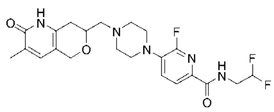 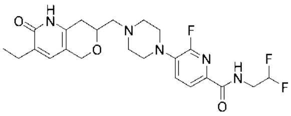 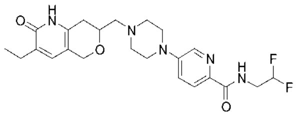 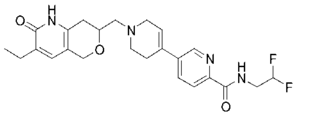

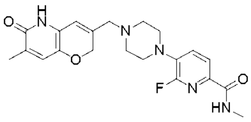.

Based on the compounds of formula I described above, the present disclosure further provides a class of compounds represented by formula I , wherein in some embodiments of the present disclosure, the structural fragment

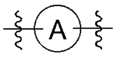

is selected from:

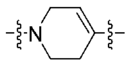, 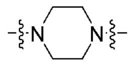,

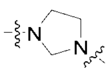, 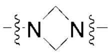, 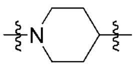, 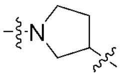, 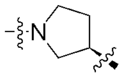, 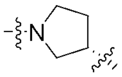, 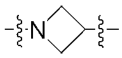, 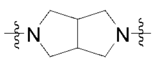,

and

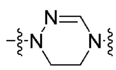

(in these structures, the left end is connected to the structural fragment

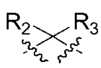,

and the right end is connected to L).

In some preferred embodiments of the present disclosure, the structural fragment

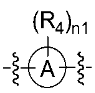

is selected from:

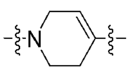, 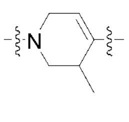, 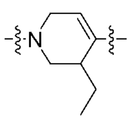, 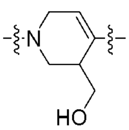, 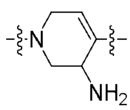, 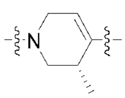,

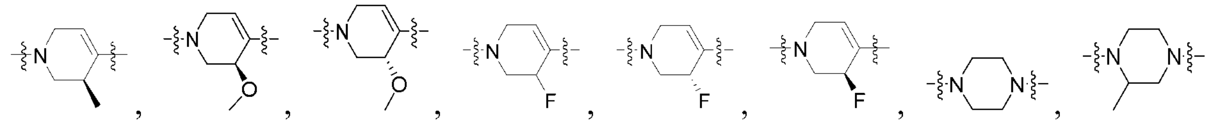
, , , , , , , ,

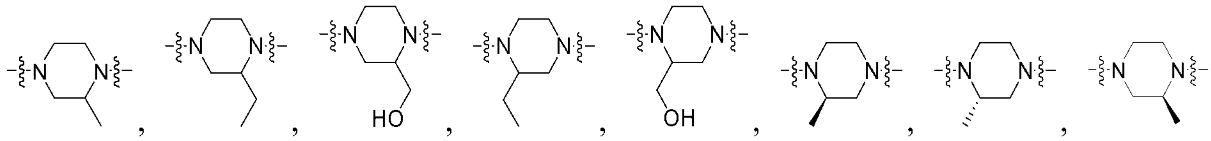
, , , , , , , ,

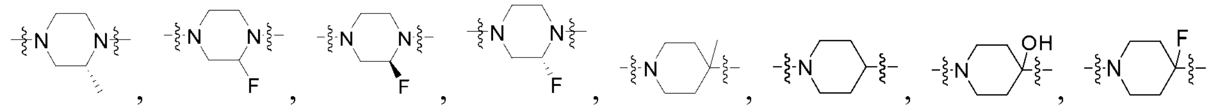
, , , , , , , ,

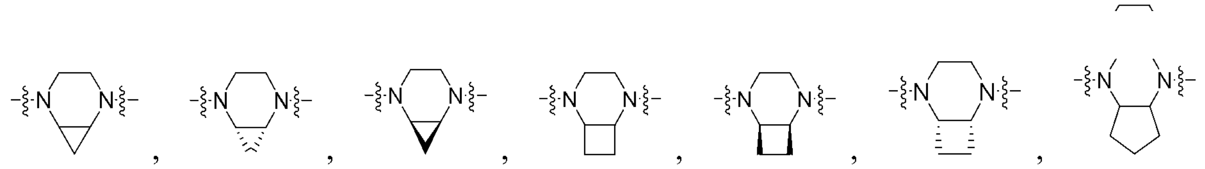
, , , , , , ,

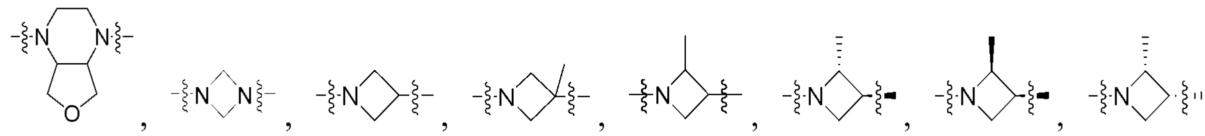
, , , , , , , ,

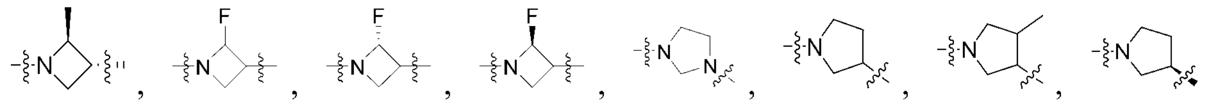
, , , , , , , ,

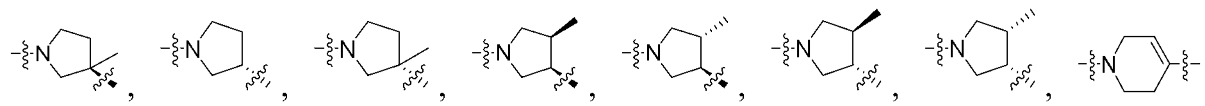
, , , , , , , ,

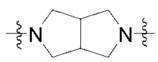
, and
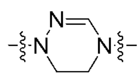

(in these structures, the left end is connected to the structural fragment

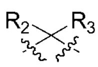
,

and the right end is connected to L).

In some more preferred embodiments of the present disclosure, the structural fragment

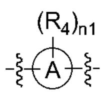

is selected from

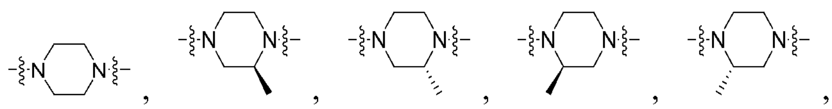
, , , , ,

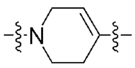, 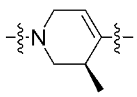, 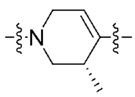, 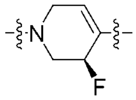, 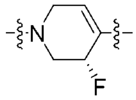, 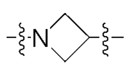, 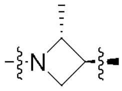, 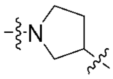,

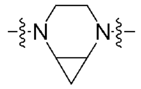, 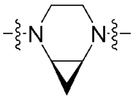, 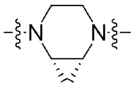, 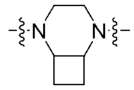, 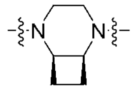, 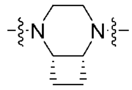, 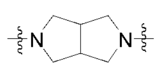,

and

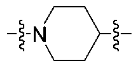

(in these structures, the left end is connected to the structural fragment

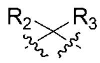,

and the right end is connected to L).

In some embodiments of the present disclosure, ring B is selected from phenyl, pyridyl, imidazolyl, imidazo[1,2-*a*]pyrazinyl, 1,7-naphthyridinyl, pyridazinyl, pyrazinyl, pyridothiazolyl, pyrazolo[1,5-*a*]pyrimidinyl, benzopyrazolyl, benzimidazolyl, thiazolyl, [1,2,4]triazolo[1,5-*a*]pyridinyl, thienyl, furyl, pyrrolyl, pyrazolyl, pyridazinopyrrolyl, pyridopyrazolyl, pyridooxazolyl, and pyrimidinyl.

In some preferred embodiments of the present disclosure, ring B is selected from

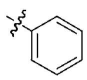,

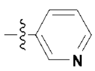, 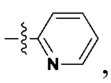, 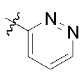, 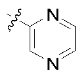, 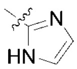, 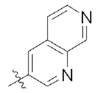, 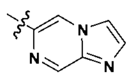, 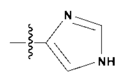, and 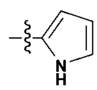.

In some embodiments of the present disclosure, $R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, 5- to 6-membered heteroarylaminoacyl, $C_{1-4}$ alkoxyaminoacyl, 3-to 6-membered cycloalkyl-substituted $C_{1-4}$ alkoxyaminoacyl, and 3- to 6-membered cycloalkoxyaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5- to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3.

In some preferred embodiments of the present disclosure, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl,

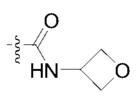, 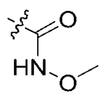, 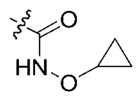, 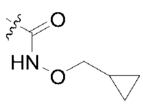, 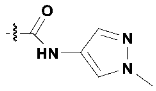,

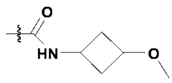 , 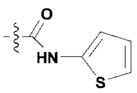 , 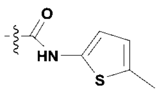 , 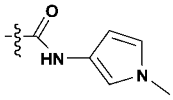 , 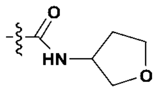 , 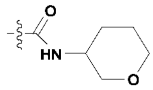 ,

and

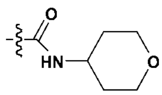 ;

n2 is selected from 1 and 2.

In some embodiments of the present disclosure, the structural fragment

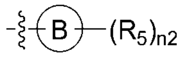

is selected from:

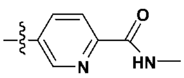 , 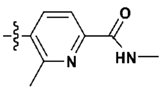 , 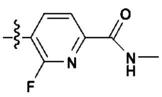 , 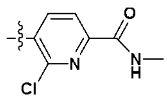 , 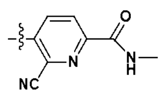 ,

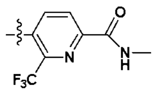 , 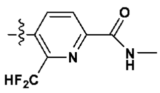 , 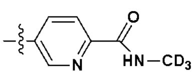 , 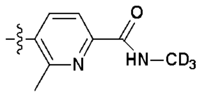 , 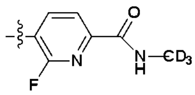 , 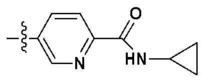 ,

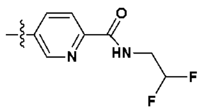 , 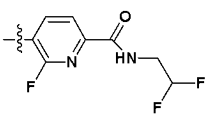 , 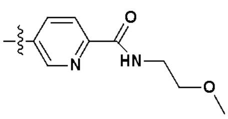 , 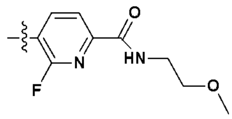 , 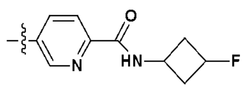 ,

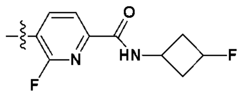 , 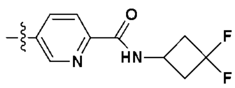 , 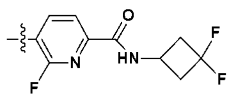 , 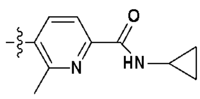 , 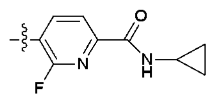 ,

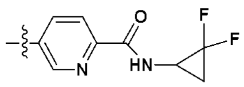 , 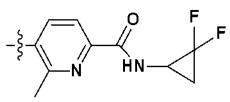 , 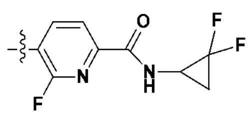 , 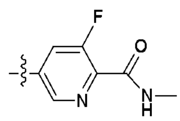 , 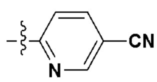 ,

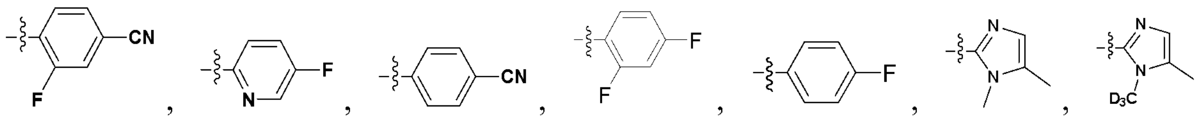 , , , , , , ,

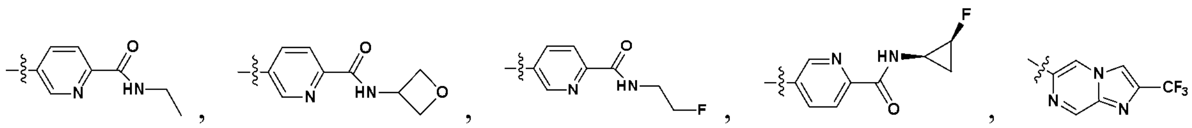 , , , , ,

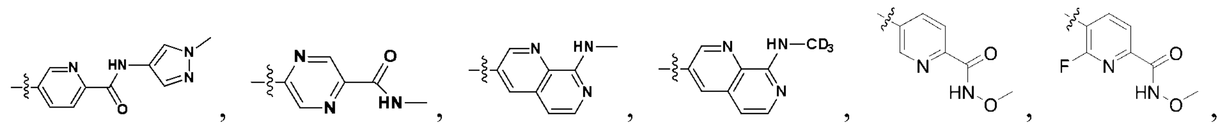
, , , , , ,

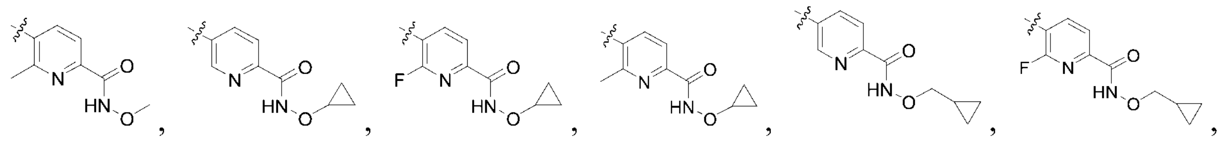
, , , , , ,

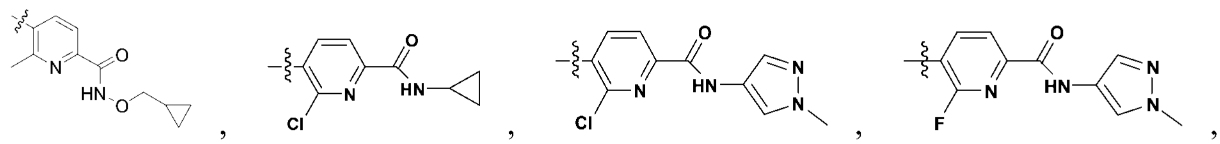
, , , ,

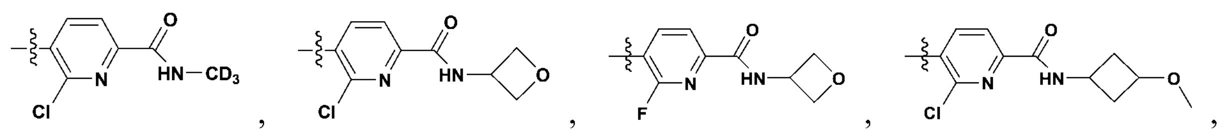
, , , ,

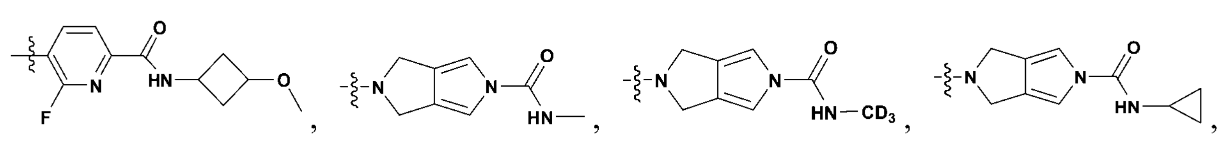
, , , ,

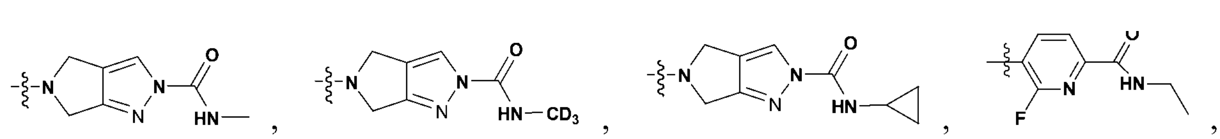
, , , ,

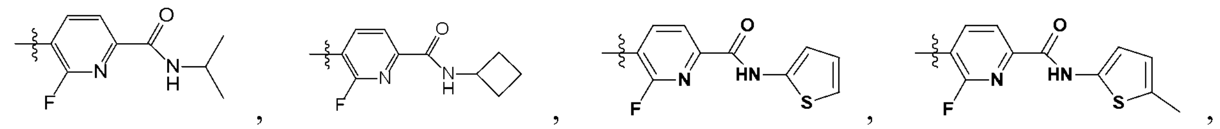
, , , ,

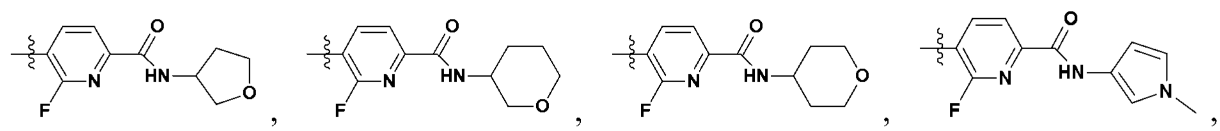
, , , ,

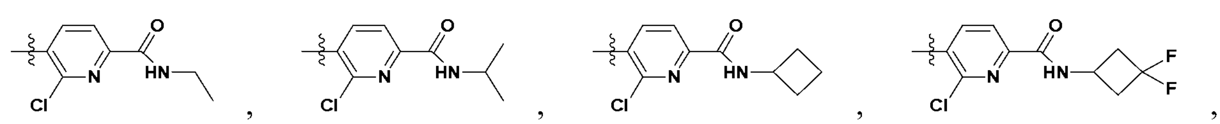
, , , ,

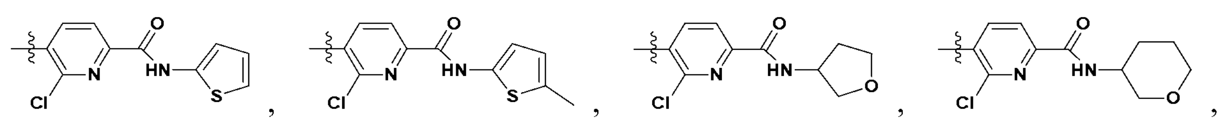
, , , ,

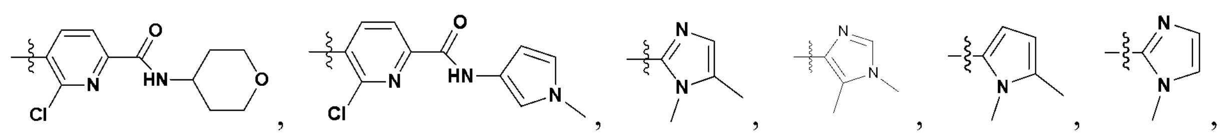
, , , , , ,

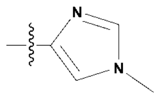, and 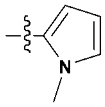.

In some embodiments of the present disclosure, $R_{x1}$ and $R_{y1}$ are independently selected from hydrogen, deuterium, halogen, cyano, amino, $C_{1-4}$ alkyl, $C_{1-4}$ deuterated alkyl, $C_{1-4}$ fluoroalkyl, -NH-$C_{1-4}$ alkyl, -N($C_{1-4}$ alkyl)$_2$, -O-$C_{1-4}$ alkyl, -S-$C_{1-4}$ alkyl, 3- to 6-membered cycloalkyl, 3-to 6-membered fluorocycloalkyl, vinyl, and phenyl;

$R_{x2}$ is selected from hydrogen, deuterium, $C_{1-4}$ alkyl, $C_{1-4}$ deuterated alkyl, $C_{1-4}$ fluoroalkyl, 3-to 6-membered cycloalkyl, 3- to 6-membered fluorocycloalkyl, and phenyl;
$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -NH-$C_{1-4}$ alkyl, and -N($C_{1-4}$ alkyl)$_2$; n4 is selected from 1, 2, 3, and 4;
$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 4-membered cycloalkyl.

In some preferred embodiments of the present disclosure, $R_{x1}$ and $R_{y1}$ are independently selected from hydrogen, deuterium, fluorine, chlorine, bromine, methyl, deuterated methyl, fluoromethyl, ethyl, fluoroethyl, cyclopropyl, methoxy, methylthio, vinyl, and fluorocyclopropyl;

$R_{x2}$ is selected from hydrogen, deuterium, methyl, deuterated methyl, fluoromethyl, ethyl, fluoroethyl, cyclopropyl, and fluorocyclopropyl;
$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;
$R_8$ is selected from hydrogen, methyl, deuterated methyl, and fluoromethyl.

In some more preferred embodiments of the present disclosure, the structural fragment

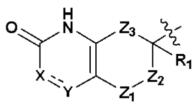

is selected from:

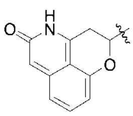, 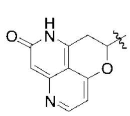, 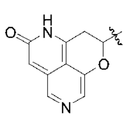, 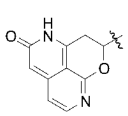, 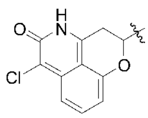,

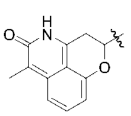, 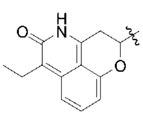, 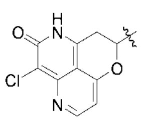, 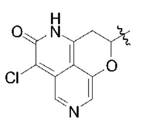, 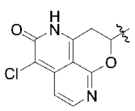, 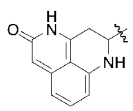, 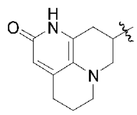,

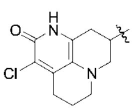, 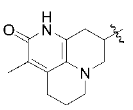, 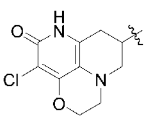, 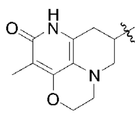, 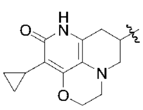, 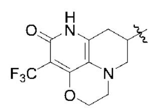, 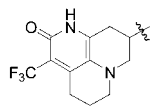,

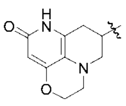, 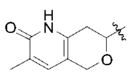, 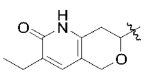, 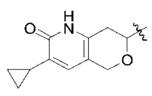, 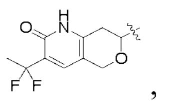, 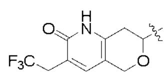, 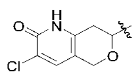,

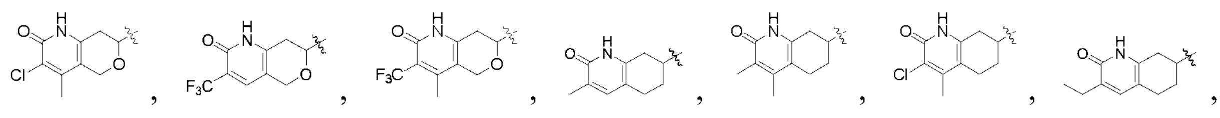
, , , , , , ,

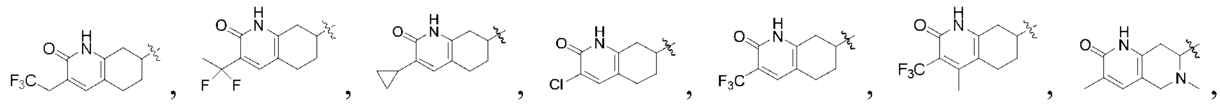
, , , , , , ,

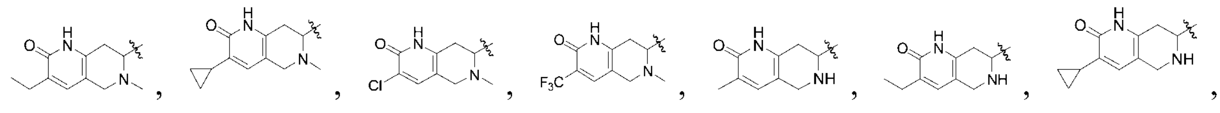
, , , , , , ,

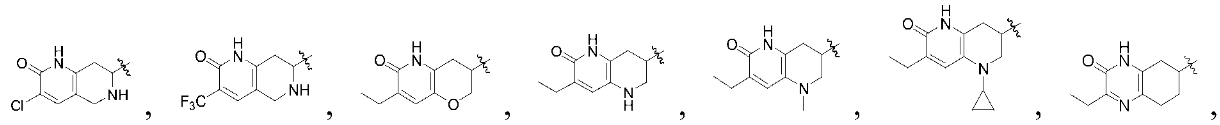
, , , , , , ,

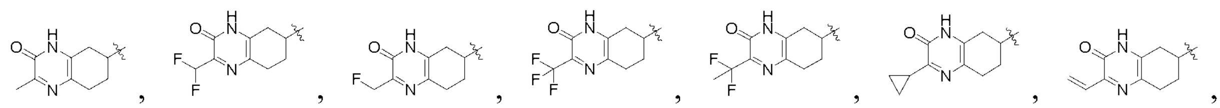
, , , , , , ,

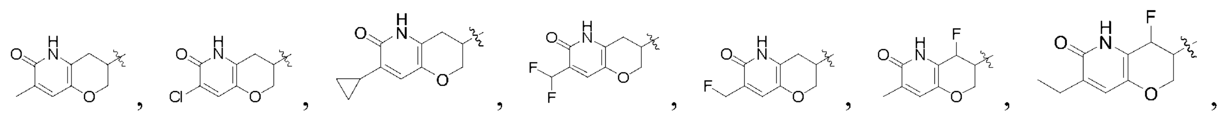
, , , , , , ,

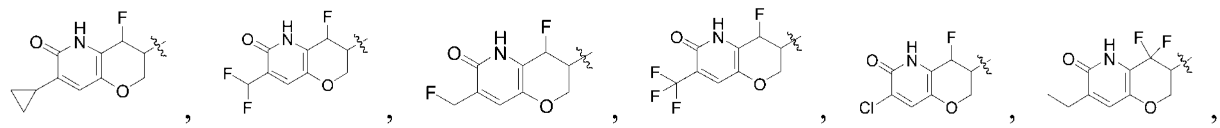
, , , , , , ,

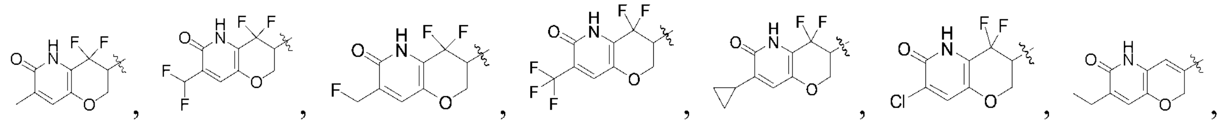
, , , , , , ,

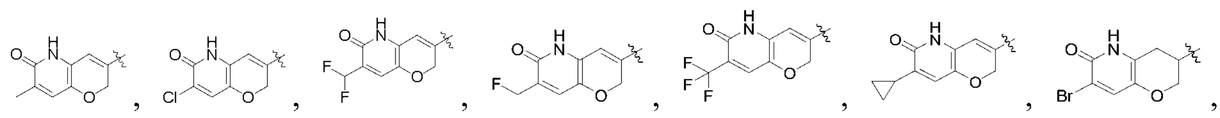
, , , , , , ,

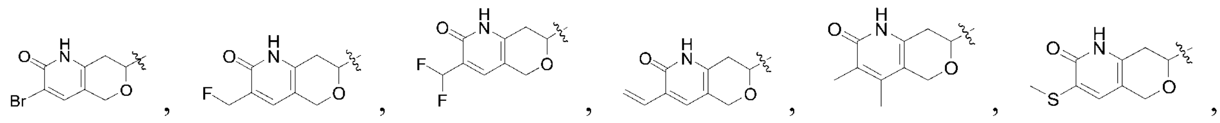
, , , , , , ,

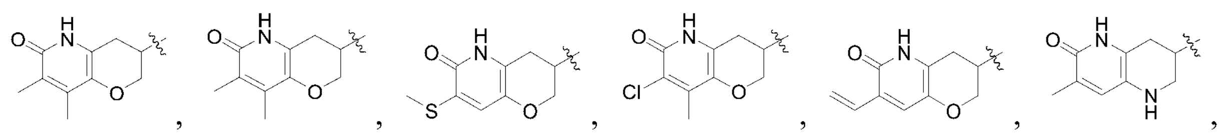
, , , , , , ,

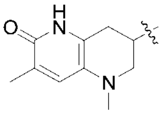, 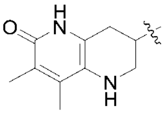, 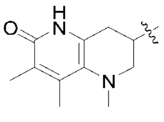, 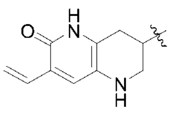, 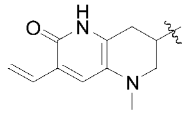, 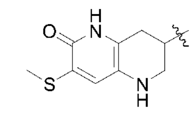,

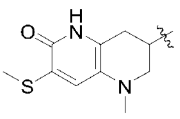, 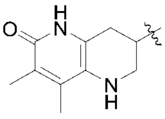, 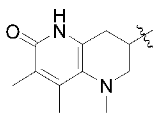, 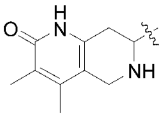, 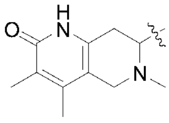, 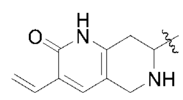,

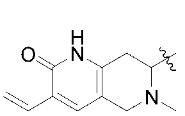, 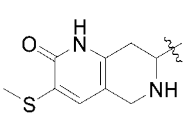, 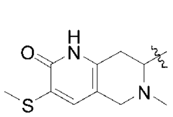, 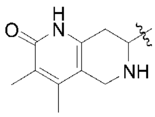, 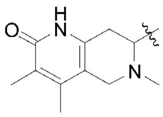, 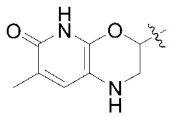,

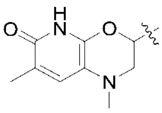, 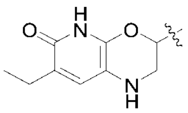, 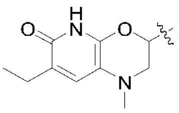, 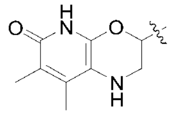, 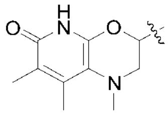, 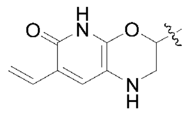,

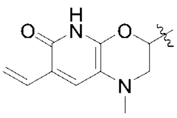, 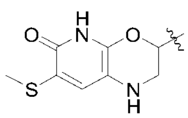, 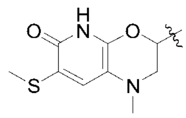, 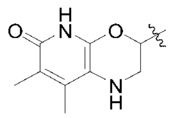, and 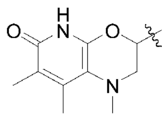.

In some embodiments of the present disclosure, the structural fragment

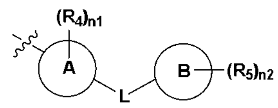

is replaced with:

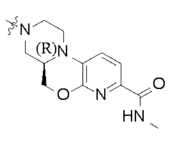, 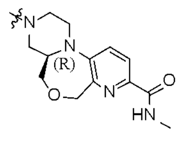, 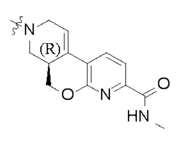, 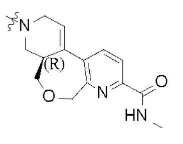,

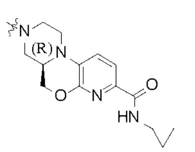, 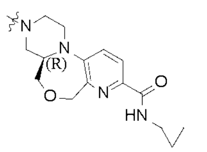, 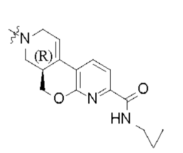, 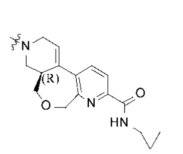, 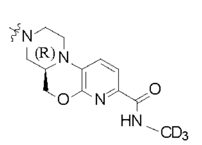,

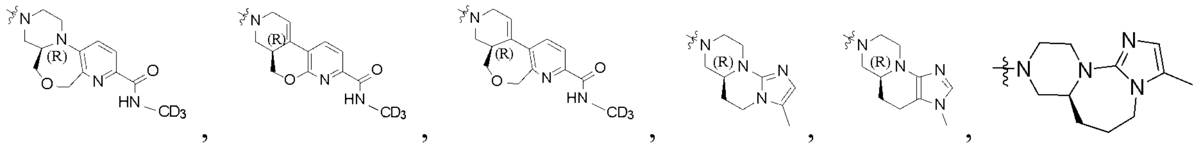,

or

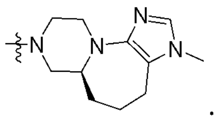

The present disclosure further provides some specific compounds represented by formula I, wherein the compounds are selected from:

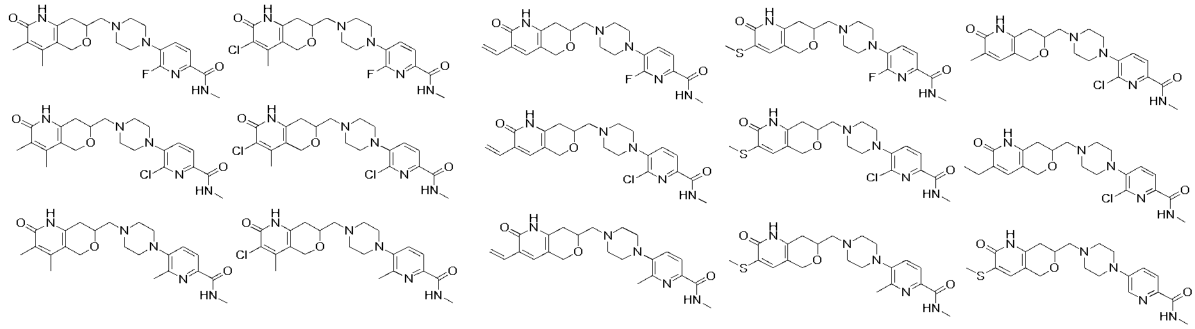

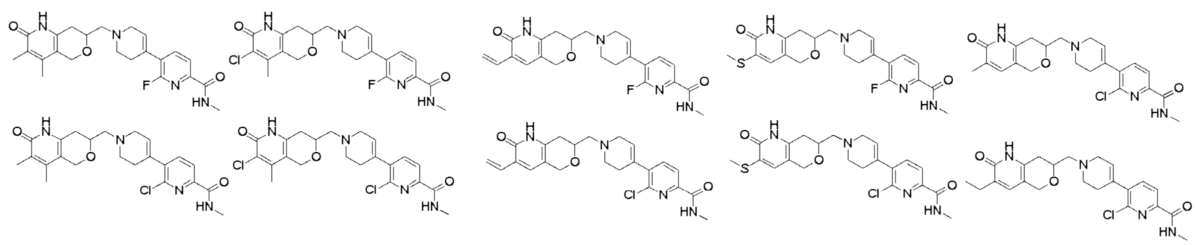

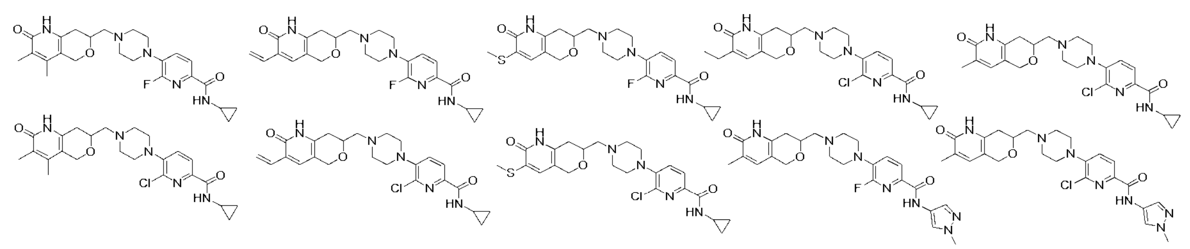

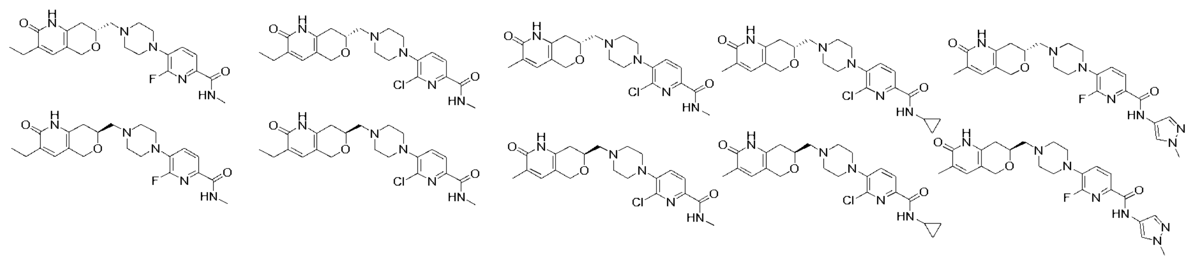

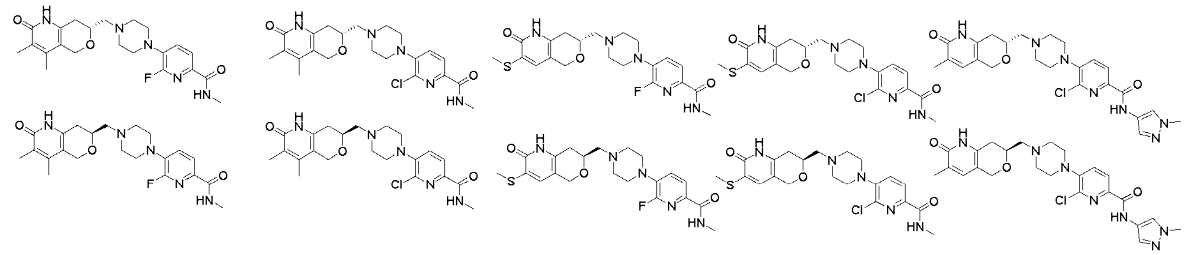

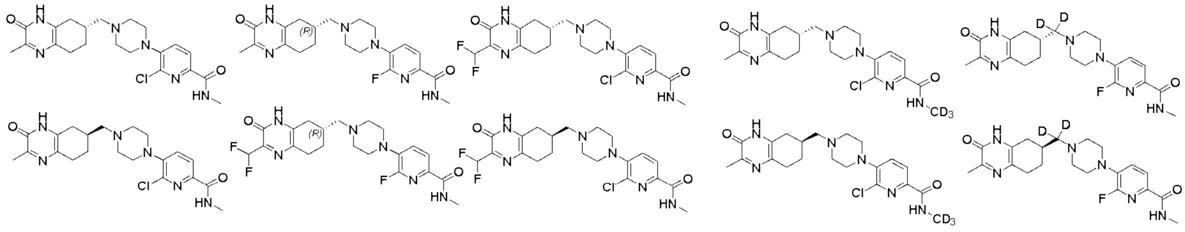

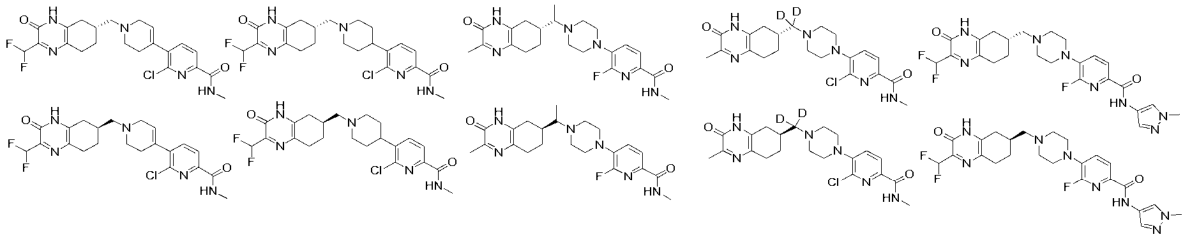

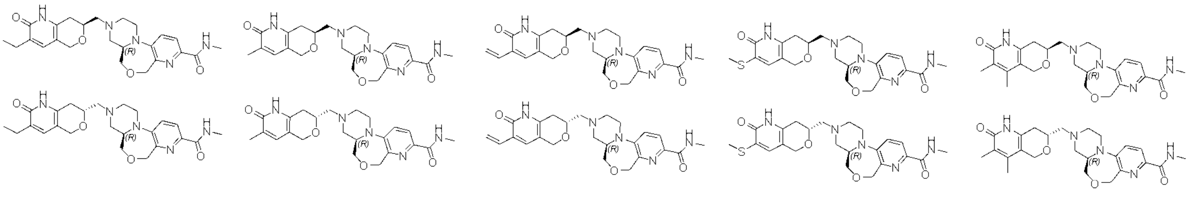

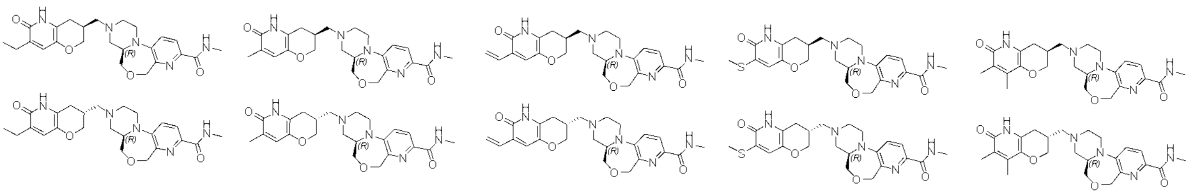

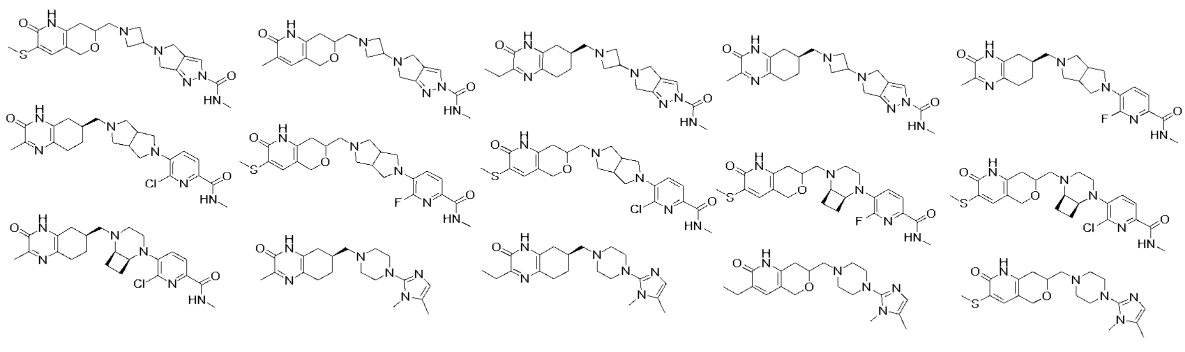

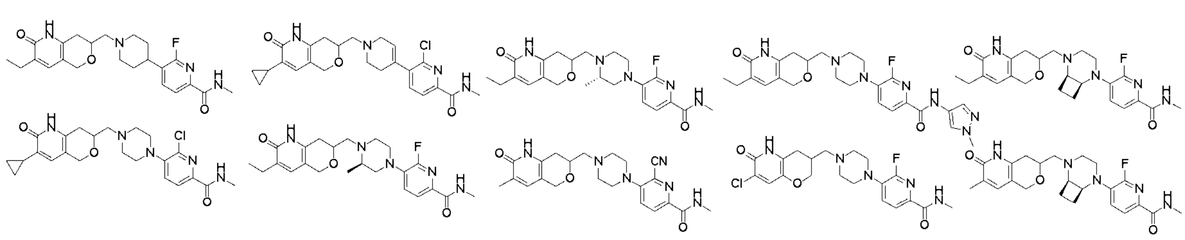

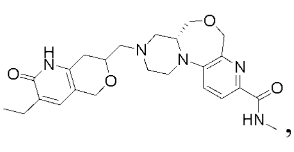,
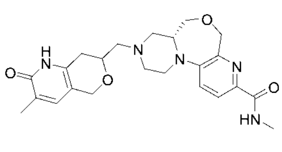,
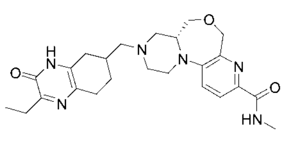,
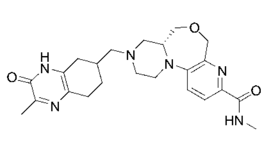

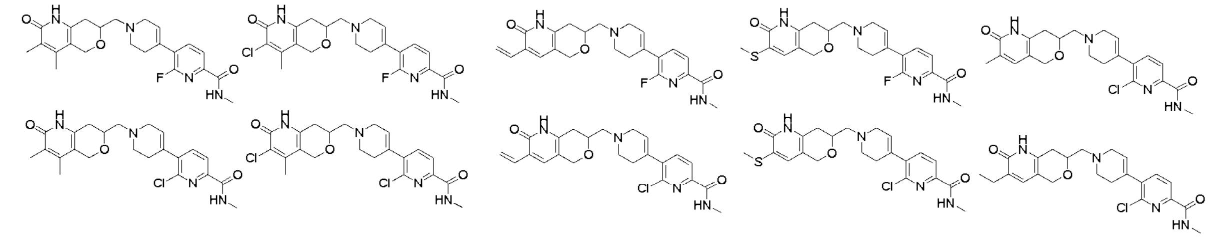

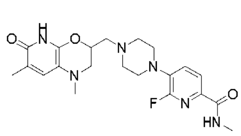, 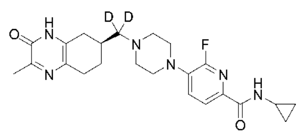, 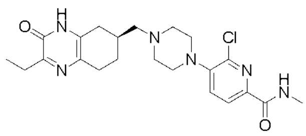, 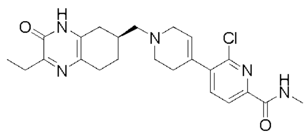,

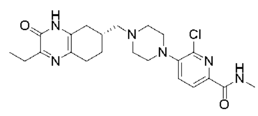, 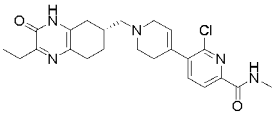, 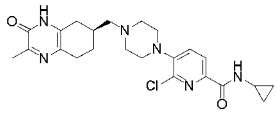, and 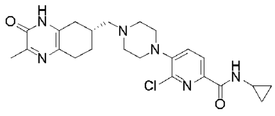.

The present disclosure further provides some specific compounds represented by formula I, wherein the compounds are selected from:

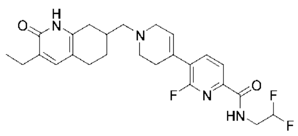, 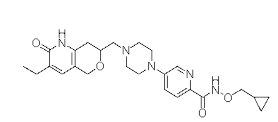, 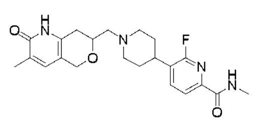, 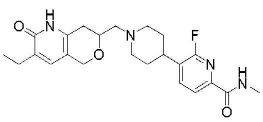,

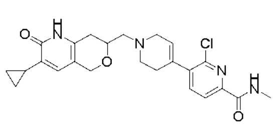, 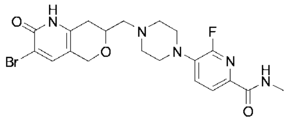, 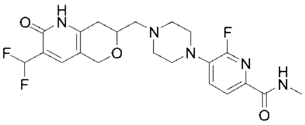, 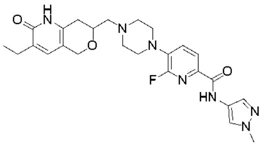,

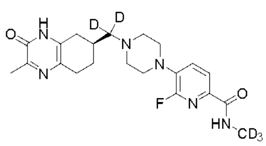, 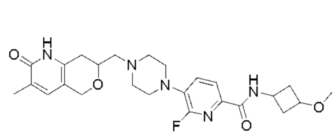, 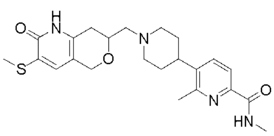, 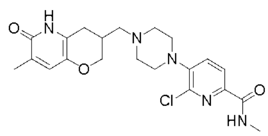,

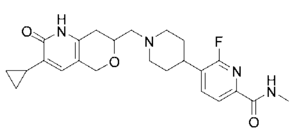, 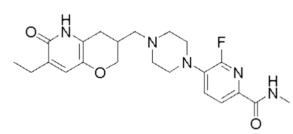, 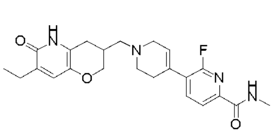, 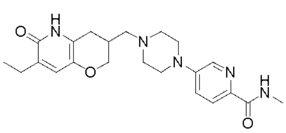,

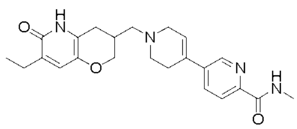, 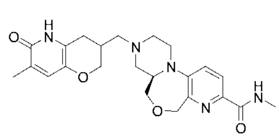, 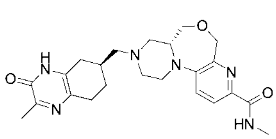, 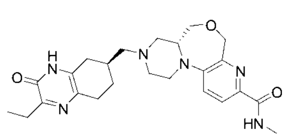,

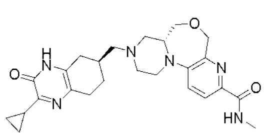, 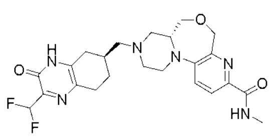, 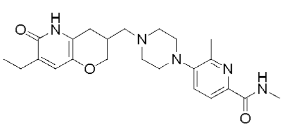, 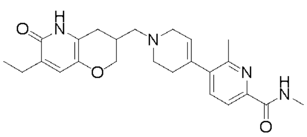,

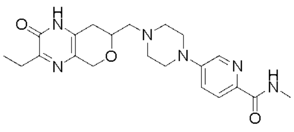, 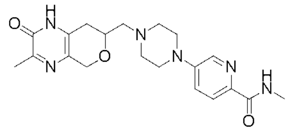, 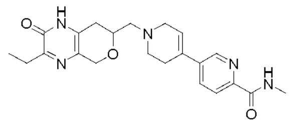, 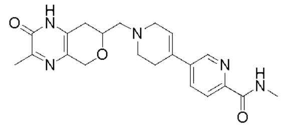,

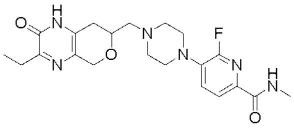, 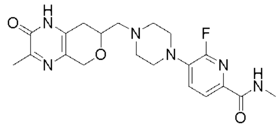, 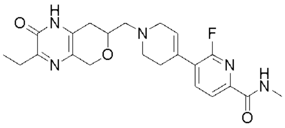, 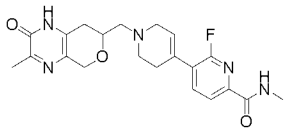,

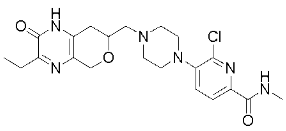, 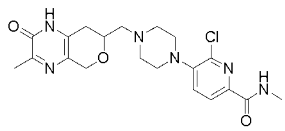, 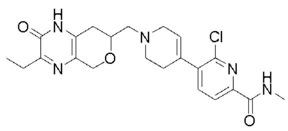, 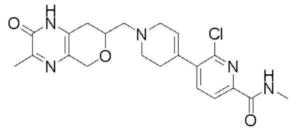,

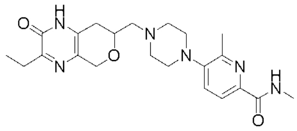, 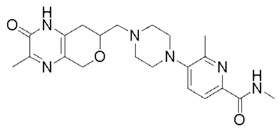, 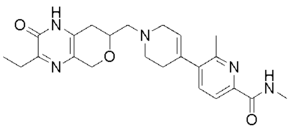, 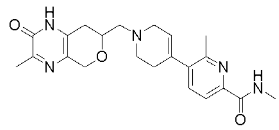,

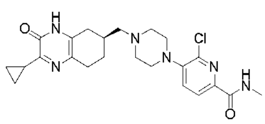, 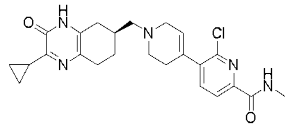, 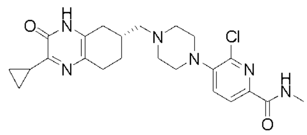, 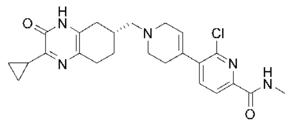,

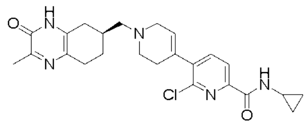, and 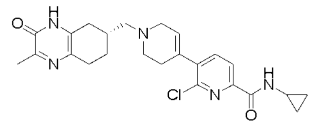.

In a second aspect, the present disclosure provides a pharmaceutical composition, comprising the aforementioned compound (formula I) or the pharmaceutically acceptable salt, ester, stereoisomer, tautomer, polymorph, solvate, nitrogen oxide, isotopically labeled compound, metabolite or prodrug thereof as an active ingredient, in combination with a pharmaceutically acceptable carrier.

A further objective of the present disclosure is to provide a method for preparing the pharmaceutical composition of the present disclosure, comprising: combining any compound of formula I or a pharmaceutically acceptable form thereof, or a mixture thereof, with one or more pharmaceutically acceptable carriers.

The pharmaceutically acceptable carrier that can be used in the pharmaceutical composition of the present disclosure is a pharmaceutically acceptable carrier, and examples of suitable pharmaceutically acceptable carriers are as described in Remington's Pharmaceutical Sciences (2005).

The pharmaceutical composition may be administered in any form as long as it achieves the prevention, alleviation, inhibition, or cure of a symptom in a human or animal patient. For example, it may be formulated into various suitable dosage forms according to the route of administration.

In some other embodiments, the administration of the compound or the pharmaceutical composition of the present disclosure may be combined with an additional treatment method. The additional treatment method may be selected from, but is not limited to: radiotherapy, chemotherapy, immunotherapy, or a combination thereof.

The present disclosure further relates to a pharmaceutical formulation, comprising any compound of formula I or a pharmaceutically acceptable form thereof, or a mixture thereof, as an active ingredient, or the pharmaceutical composition of the present disclosure. In some embodiments, the formulation is in the form of a solid formulation, a semi-solid formulation, a liquid formulation, or a gaseous formulation.

A further objective of the present disclosure is to provide an article of manufacture, for example, provided in the form of a kit. As used herein, the article of manufacture is intended to include, but is not limited to, a kit and a package. The article of manufacture of the present disclosure comprises: (a) a first container; (b) a pharmaceutical composition located

in the first container, wherein the composition comprises: a first therapeutic agent, the first therapeutic agent comprising: any compound of formula I or a pharmaceutically acceptable form thereof, or a mixture thereof; (c) optionally, a package insert indicating that the pharmaceutical composition can be used for treating a tumor-related disease (as defined below); and (d) a second container.

The first container is a container for accommodating a pharmaceutical composition. This container can be used for preparation, storage, transportation, and/or individual/bulk sale. The first container is intended to encompass a bottle, a jar, a vial, a flask, a syringe, a tube (e.g., for a cream product), or any other container for preparing, accommodating, storing, or dispensing a pharmaceutical product.

The second container is a container for accommodating the first container and optionally a package insert. Examples of the second container include, but are not limited to, a box (e.g., a paper box or a plastic box), a case, a carton, a bag (e.g., a paper bag or a plastic bag), a pouch, and a burlap sack. The package insert may be physically attached to the exterior of the first container via a cable tie, a glue, a staple, or other means of attachment, or it may be placed inside the second container without the need for any physical tools for attachment to the first container. Alternatively, the package insert is located on the exterior of the second container. When located on the exterior of the second container, the package insert is preferably physically attached via a cable tie, a glue, a staple, or other means of attachment. Alternatively, it may be placed adjacent to or in contact with the exterior of the second container without the need for physical attachment.

The package insert is a trademark, a label, a marking, or the like, which lists information related to the pharmaceutical composition located in the first container. The information listed is typically determined by the regulatory authority (e.g., the U.S. Food and Drug Administration) that governs the region where the article of manufacture is to be sold. Preferably, the package insert specifically lists the indications for which the pharmaceutical composition is approved. The package insert may be made of any material from which the information contained therein or thereon can be read. Preferably, the package insert is a printable material (e.g., paper, plastic, cardboard, foil, adhesive paper or plastic, etc.), on which the required information can be formed (e.g., printed or applied).

In a third aspect, the present disclosure provides use of the aforementioned compound of formula I and related specific compounds or pharmaceutically acceptable forms thereof, or the pharmaceutical composition of the present disclosure in the manufacture of a medicament for preventing or treating a PARP1 enzyme-related disease.

The present disclosure provides a method for preventing or treating a PARP1 enzyme-related disease, comprising administering to an individual in need thereof the compound of formula I or the pharmaceutically acceptable form thereof, or the pharmaceutical composition of the present disclosure.

The present disclosure provides the compound of formula I or the pharmaceutically acceptable form thereof, or the pharmaceutical composition of the present disclosure for use in preventing or treating a PARP1 enzyme-related disease.

The present disclosure provides a method for preventing or treating a PARP1 enzyme-related disease by combining the compound of formula I or the pharmaceutically acceptable form thereof, or the pharmaceutical composition of the present disclosure, with an additional treatment method, wherein the additional treatment method includes, but is not limited to: radiotherapy, chemotherapy, immunotherapy, or a combination thereof.

In some embodiments, the PARP1 enzyme-related disease is a disease that is sensitive to or responsive to PARP1 enzyme inhibition.

In some embodiments, the PARP1 enzyme-related disease is a tumor-related disease.

In some preferred embodiments, the tumor-related disease is deficient in an HR-dependent DNA DSB repair pathway.

In some preferred embodiments, the tumor-related disease comprises one or more cancer cells that have a reduced or absent ability to repair a DNA DSB through HR compared to normal cells.

In some preferred embodiments, the cancer cell has a BRCA1- or BRCA2-deficient phenotype.

In some embodiments, the PARP1 enzyme-related disease is a tumor-related disease, including but not limited to solid and hematological malignancies. In a further embodiment, the tumor-related disease includes, but is not limited to, breast cancer, colorectal cancer, colon cancer, lung cancer (including small cell lung cancer, non-small cell lung cancer, and bronchioloalveolar carcinoma), and prostate cancer, as well as bile duct cancer, bone cancer, bladder cancer, head and neck cancer, kidney cancer, liver cancer, gastrointestinal tissue cancer, esophageal cancer, ovarian cancer, pancreatic cancer, skin cancer, testicular cancer, thyroid cancer, uterine cancer, cervical cancer, and vulval cancer, along with leukemia (including chronic lymphocytic leukemia (CLL), acute lymphocytic leukemia (ALL), and chronic myeloid leukemia (CML)), multiple myeloma, or lymphoma.

In some preferred embodiments, the PARP1 enzyme-related disease is breast cancer, ovarian cancer, pancreatic cancer, prostate cancer, hematological cancer, gastrointestinal cancer, or lung cancer.

In a further preferred embodiment, the compound of the present disclosure may be used in combination with radiotherapy, chemotherapy, or immunotherapy to prevent or treat a cancer. The present disclosure provides use of the compound or the pharmaceutically acceptable salt, ester, stereoisomer, tautomer, polymorph, solvate, nitrogen oxide, isotopically labeled compound, metabolite or prodrug thereof described above, or the pharmaceutical composition

described above in the manufacture of a PARP1 inhibitor.

Beneficial Effects of Present Disclosure:

The present disclosure provides a novel class of highly active and highly selective PARP1 inhibitors, which can achieve at least one of the following technical effects: (1) high inhibitory activity against the PARP1 enzyme; (2) selective inhibition of the PARP1 enzyme, with high selectivity for PARP1 over other PARP family enzymes such as PARP2, PARP5a, and PARP5b; (3) potent inhibitory activity against homologous recombination-deficient tumor cells, with weak inhibitory effects on non-homologous recombination-deficient cells; (4) excellent pharmacokinetic properties (e.g., good bioavailability, appropriate half-life and duration of action); (5) excellent safety profile (lower toxicity and/or fewer side effects, wider therapeutic window), etc.

## BRIEF DESCRIPTION OF THE DRAWINGS

FIG. 1 shows the *in vivo* efficacy results of compound **2** in a subcutaneous xenograft tumor model of MDA-MB-436 breast cancer in nude mice.

FIG. 2 shows the *in vivo* efficacy results of compound **53** in a subcutaneous xenograft tumor model of MDA-MB-436 breast cancer in nude mice.

## DETAILED DESCRIPTION

The embodiments of the present disclosure will be explained with reference to the following examples. Those skilled in the art will appreciate that the following examples are only for illustrating the present disclosure, and should not be construed as limitations to the scope of the present disclosure. Procedures without specified techniques or conditions in the examples were performed according to the techniques or conditions described in the literature in the art or according to the product instructions.

All reagents and starting materials used in the examples of the present disclosure are commercially available.

Table 1. Letter abbreviations and meanings thereof in the present disclosure

| | | | |
|---|---|---|---|
| AcOH | Acetic acid | HPLC | High Performance Liquid Chromatography |
| Aq or aq | Aqueous solution | m/z | Mass/charge |
| BOC or Boc | *tert*-Butoxycarbonyl | Me | Methyl |
| CPME | Cyclopentyl methyl ether | MeCN | Acetonitrile |
| DCE | 1,2-Dichloroethane | MeOH | Methanol |
| DABCO | 1,4-Diazabicyclo[2.2.2]oct ane | NMR | Nuclear magnetic resonance |
| DCM | Dichloromethane | THF | Tetrahydrofuran |
| DMA | *N*,*N*-Dimethylacetamide | Sat or satd | Saturated |
| DMAP | 4-Dimethylaminopyridine | rt | Room temperature |
| EA | Ethyl acetate | UV | Ultraviolet |
| PE | Petroleum ether | t-BuOH | *tert*-Butanol |
| DMF | *N*,*N*-Dimethylformamide | Cbz | Benzyloxycarbonyl |
| TFA | Trifluoroacetic acid | mmol | Millimole |
| TEA | Triethylamine | mg | Milligram |
| DME | 1,2-Dimethoxyethane | min | Minute |
| DMSO | Dimethyl sulfoxide | mL | Milliliter |
| DPPF or dppf | 1,1'-Bis(diphenylphosphin o)ferrocene | BINAP | 2,2'-Bis(diphenylphosphino)-1,1 '-binaphthyl |
| eq or equiv | Equivalent | *m*-CPBA | *m*-Chloroperoxybenzoic acid |

(continued)

| | | | |
|---|---|---|---|
| AcOH | Acetic acid | HPLC | High Performance Liquid Chromatography |
| ESI | Electrospray ionization | TBS | *tert*-Butyldimethylsilyl |
| Et | Ethyl | $Pd_2(dba)_3$ | Tris(dibenzylideneacetone)dipal ladium |
| G | Gram | Xantphos | 4,5-Bis(diphenylphosphino)-9,9 -dimethylxanthene |
| H | Hour | NBS | *N*-bromosuccinimide |
| rt | Room temperature | TBAF | Tetra-*n*-butylammonium fluoride |

The structures of the compounds described in the present disclosure were determined by nuclear magnetic resonance (NMR) or mass spectrometry (MS). The NMR analysis was performed using a Bruker AVANCE-400 nuclear magnetic resonance instrument, with deuterated dimethyl sulfoxide (DMSO-$d_6$), deuterated chloroform ($CDCl_3$), and deuterated methanol ($CD_3OD$) as solvents and tetramethylsilane (TMS) as an internal standard. The chemical shifts were given in $10^{-6}$ (ppm). The MS analysis was performed using an Agilent SQD (ESI) mass spectrometer (manufacturer: Agilent, model: 6110).

The HPLC analysis was performed using an Agilent 1200DAD high-pressure liquid chromatograph (Sunfirc C18, 150 × 4.6 mm, 5wn, chromatography column) and a Waters 2695-2996 high-pressure liquid chromatograph (Gimini C18, 150 × 4.5 mm, 5ym chromatography column).

The thin-layer chromatography silica gel plates used were Qingdao Haiyang GF254 silica gel plates. The silica gel plates used in the thin-layer chromatography (TLC) analyses had a layer thickness of 0.15 mm-0.2 mm, and those used in the thin-layer chromatography separation and purification of products had a layer thickness of 0.4 mm-0.5 mm.

Qingdao Haiyang 100-200 and 200-300 mesh silica gels were generally used as carriers in column chromatography.

In the following examples, all reactions were carried out in an argon atmosphere or a nitrogen atmosphere unless otherwise specified. The argon atmosphere or nitrogen atmosphere means that the reaction flask was connected to a balloon containing about 1 L of argon or nitrogen gas. The hydrogen atmosphere means that the reaction flask was connected to a balloon containing about 1 L of hydrogen gas. The hydrogenation reactions generally involved 3 cycles of evacuation and hydrogen purging.

**Intermediate INT1:** *N*-Methyl-1',2',3',6'-tetrahydro-[3,4'-bipyridine]-6-carboxamide

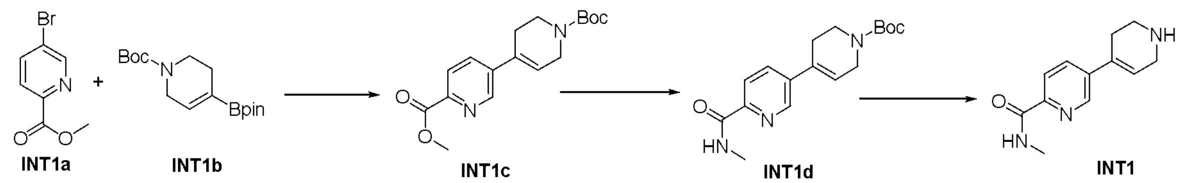

INT1a INT1b INT1c INT1d INT1

Step 1: Compound **INT1a** (1 g, 4.6 mmol), **INT1b** (1.7 g, 5.5 mmol), $Pd(dppf)Cl_2$ (0.3 g, 0.46 mmol), and potassium carbonate (1.6 g, 11.5 mmol) were added to a mixed solvent of 7 mL of dioxane, 3 mL of absolute ethanol, and 4 mL of water. Then, the mixture was purged three times with nitrogen and allowed to react at 90 °C for 2 h in a nitrogen atmosphere. After the reaction was completed as detected by TLC, the reaction mixture was cooled to room temperature, and 30 mL of dichloromethane and 20 mL of water were added. The mixture was then allowed to separate into layers in a separatory funnel. The aqueous phase was extracted twice with dichloromethane, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT1c** (1 g, white solid).

Step 2: **INT1c** (1 g, 3 mmol), an aqueous methylamine solution (5 g, 161.3 mmol), and absolute methanol (20 mL) were added to a 100 mL reaction flask, and the mixture was stirred at room temperature overnight. After the reaction was completed as monitored by TLC, the reaction mixture was concentrated under reduced pressure to dryness to obtain compound **INT1d** (0.8 g, white solid).

Step 3: Compound **INT1d** (0.5 g, 1.5 mmol) was added to 10 mL of absolute methanol, and then 10 mL of a 4 mol/L solution of hydrochloric acid in dioxane was added. The mixture was stirred at room temperature for 0.5-1 h. After the

reaction was completed as monitored by TLC, the reaction mixture was concentrated under reduced pressure to dryness to obtain compound **INT1** (0.5 g, white solid). MS/ESI $[M+H]^+$ = 218.1.

Intermediates **INT2** and **INT3** were prepared using a synthesis method similar to the preparation of intermediate **INT1**.

Table 2. Intermediates **INT2**-**INT3**

| Intermediate | Starting material | Structure | Name | Data |
|---|---|---|---|---|
| **INT2** | 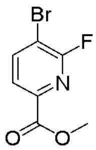 | 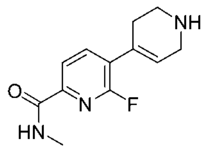 | 2-Fluoro-*N*-methyl-1',2',3',6'-tetrah ydro-[3,4'-bipyrid ine]-6-carboxamide | MS/ESI$[M+H]^+$=236.1; 1H NMR (400 MHz, DMSO) $\delta$9.56 (s, 2H), 8.70 (q, J = 4.4 Hz, 1H), 8.13 (dd, J = 9.8, 7.7 Hz, 1H), 7.98 (dd, J = 7.7, 1.7 Hz, 1H), 6.28 (s, 1H), 3.78 (d, J = 2.5 Hz, 2H), 3.67 (s, 2H), 3.29 (dd, J = 4.7, 2.9 Hz, 2H), 2.83-2.77 (m, 3H), 2.71 (s, 2H). |
| **INT3** | 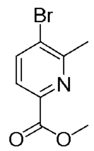 | 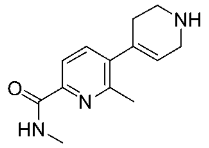 | *N*,2-Dimethyl-1',2',3',6'-tetrahydro -[3,4'-bipyridine] -6-carboxamide | MS/ESI$[M+H]^+$=232.1 |

**Intermediate INT4:** *N*-Cyclopropyl-1',2',3',6'-tetrahydro-[3,4'-bipyridine]-6-carboxamide

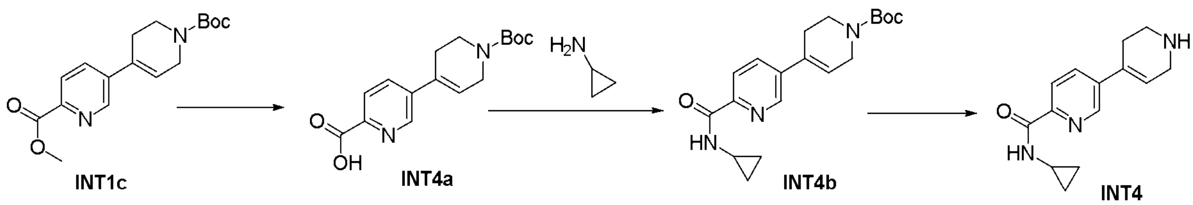

INT1c INT4a INT4b INT4

Step 1: **INT1c** (1.4 g, 4.5 mmol) was weighed out and dissolved in 20 mL of MeOH, and 5 mL of water was added, followed by the addition of lithium hydroxide (570 mg, 13.5 mmol). The mixture was allowed to react at room temperature for 12 h. After the reaction was completed as monitored by TLC, the mixture was adjusted to pH 6 with 2 M HCl and extracted with EA (3 $\times$ 25 mL). The organic phases were combined, dried over anhydrous sodium sulfate, and then concentrated to dryness by rotary evaporation under vacuum to obtain product **INT4a** (460 mg, pale yellow solid).

Step 2: **INT4a** (180 mg, 0.6 mmol), EDCI (144 mg, 0.75 mmol), and HOBT (100 mg, 0.75 mmol) were weighed out and added to 2 mL of DMF, and then *N*-methylmorpholine (290 mg, 3.2 mmol) and cyclopropylamine (34 mg, 0.6 mmol) were added. The mixture was allowed to react at room temperature for 12 h. After the starting materials were completely consumed as monitored by TLC, the mixture was diluted with water and extracted with EA. The organic phase was washed 5 times with water, dried over anhydrous sodium sulfate, and concentrated to dryness by rotary evaporation to obtain a crude product of **INT4b** (180 mg, yellow oily liquid).

Step 3: The crude product of **INT4b** (171 mg, 0.5 mmol) was dissolved in methanol (5 mL), and then a 4 M solution of HCl in 1,4-dioxane (0.9 mL) was added. The mixture was allowed to react at room temperature for 12 h. After the reaction was completed as monitored by TLC, potassium carbonate was added. The mixture was stirred for 30 min and then filtered to remove the potassium carbonate to obtain a crude product of compound **INT4** (180 mg, yellowish-brown solid). MS/ESI $[M+H]^+$ = 244.1.

Intermediates **INT5-INT8** were prepared using a synthesis method similar to the preparation of intermediate **INT4**.

Table 3. Intermediates **INT5-INT8**

| Intermediate | Starting material 1 | Starting material 2 | Structure | Name | Data |
|---|---|---|---|---|---|
| **INT5** | 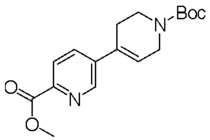 | $CD_3NH_2$ | 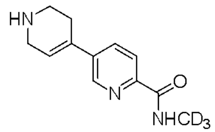 | *N*-(Methyl-*d*$_3$)-1',2',3',6'-tetrahydro-[3,4'-bipyridine ]-6-carboxamide | MS/ESI[M+H]$^+$=221.1 |
| **INT6** | 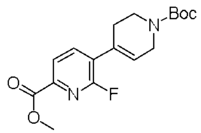 | 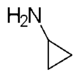 | 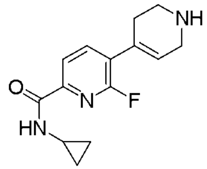 | *N*-Cyclopropyl-2-fluoro-1 ',2',3',6'-tetrahydro-[3,4'-bipyridine]-6-carboxamide | MS/ESI[M+H]$^+$=262.1 |
| **INT7** | 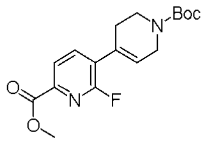 | $CD_3NH_2$ | 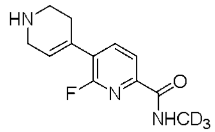 | 2-Fluoro-*N*-(methyl-*d*$_3$)-1' ,2',3',6'-tetrahydro-[3,4'-bi pyridine]-6-carboxamide | MS/ESI[M+H]$^+$=239.2 |
| **INT8** | 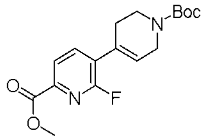 | 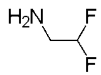 | 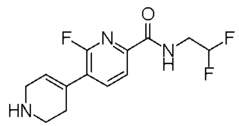 | *N*-(2,2-Difluoroethyl)-2-fluoro-1',2',3',6'-tetrahydro-[3,4'-bipyridine]-6-carbox amide | MS/ESI[M+H]$^+$=286.2 |

**Intermediate INT9:** *N*-Methyl-5-(piperazin-1-yl)picolinamide

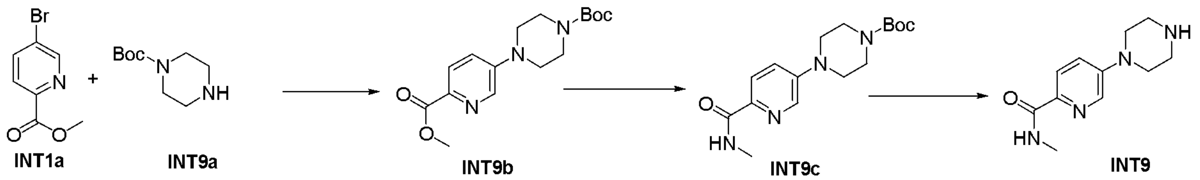

INT1a INT9a INT9b INT9c INT9

Step 1: Compound **INT1a** (5.66 g, 26.21 mmol, 1.05 eq), compound **INT9a** (4.65 g, 25.0 mmol, 1.00 eq), $Cs_2CO_3$ (16.27 g, 50.0 mmol, 2.00 eq), and RuPhos-Pd-G3 (1.04 g, 1.25 mmol, 0.05 eq) were added to 1,4-dioxane (50 mL), and the mixture was stirred at 120 °C overnight in a nitrogen atmosphere. After the reaction was completed as detected by LCMS, the mixture was allowed to cool to room temperature. The reaction mixture was diluted with water (100 mL) and then extracted with ethyl acetate (2 × 100 mL). The organic layers were combined, washed with brine (2 × 50 mL), dried over anhydrous $Na_2SO_4$, and filtered, and the filtrate was concentrated under reduced pressure. The residue was purified by silica gel column chromatography to obtain compound **INT9b** (white solid, 4.5 g). MS/ESI $[M+H]^+$ = 322.1.

Step 2: Compound **INT9b** (1.92 g, 6.0 mmol, 1.0 eq) was added to a solution of methylamine (8 mL, 25-30 wt% in water) in methanol (7 mL), and the mixture was stirred at room temperature for 3 h in a nitrogen atmosphere. The reaction was monitored by LCMS. The reaction was quenched with a saturated aqueous ammonium chloride solution (30 mL) at room temperature. The resulting mixture was extracted with dichloromethane (3 × 50 mL). The organic layers were combined, washed with brine (2 × 50 mL), dried over anhydrous $Na_2SO_4$, and filtered, and then the filtrate was concentrated under reduced pressure to obtain compound **INT9c** (1.65 g) as a light yellow oil. MS/ESI $[M+H]^+$ = 321.3.

Step 3: Compound **INT9c** (482 mg, 1.50 mmol, 1.00 eq) and a solution of HCl in 1,4-dioxane (3.7 mL, 15.0 mmol, 10.0 eq, 4.0 M) were stirred at room temperature for 2 h. After the reaction was completed as monitored by LCMS, the resulting mixture was concentrated under reduced pressure to obtain compound **INT9** (392 mg, crude product) as a yellow solid. The crude product was directly used in the next step without further purification. MS/ESI $[M+H]^+$ = 221.3.

Intermediates **INT10** and **INT11** were prepared using a synthesis method similar to the preparation of intermediate **INT9**.

Table 4. Intermediates **INT10** and **INT11**

| Intermediate | Starting material | Structure | Name | Data |
|---|---|---|---|---|
| **INT10** | 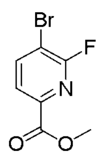 | 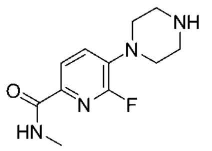 | 6-Fluoro-*N*-methyl-5-(piperazin -1-yl)picolinamide | MS/ESI$[M+H]^+$= 239.1 |
| **INT11** | 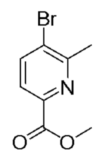 | 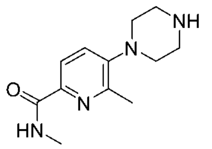 | *N*,6-Dimethyl-5-(piperazin-1-yl) picolinamide | MS/ESI$[M+H]^+$= 235.1 |

**Intermediate INT12:** *N*-Cyclopropyl-5-(piperazin-1-yl)picolinamide

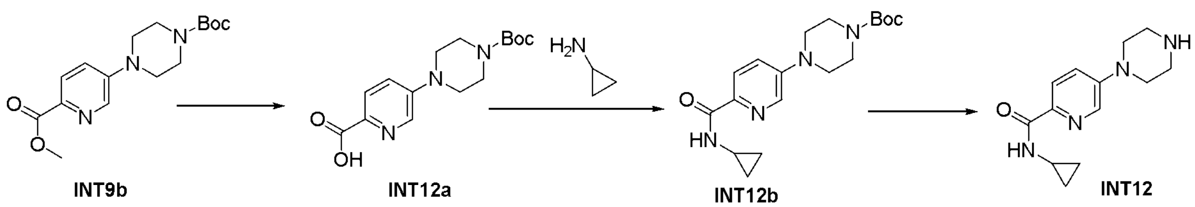

INT9b INT12a INT12b INT12

Step 1: **INT9b** (1.4 g, 4.5 mmol) was weighed out and dissolved in 20 mL of MeOH, and 5 mL of water was added, followed by the addition of lithium hydroxide (570 mg, 13.5 mmol). The mixture was allowed to react at room temperature for 12 h. After the reaction was completed as monitored by TLC, the mixture was adjusted to pH 6 with 2 M HCl and extracted with EA (3 × 25 mL). The organic phases were combined, dried over anhydrous sodium sulfate, and then concentrated to dryness by rotary evaporation under vacuum to obtain the product **INT12a** (845 mg).

Step 2: **INT12a** (180 mg, 0.6 mmol), EDCI (144 mg, 0.75 mmol), and HOBT (100 mg, 0.75 mmol) were weighed out and added to 2 mL of DMF, and then *N*-methylmorpholine (290 mg, 3.2 mmol) and cyclopropylamine (34 mg, 0.6 mmol) were added. The mixture was allowed to react at room temperature for 12 h. After the starting materials were completely consumed as monitored by TLC, the mixture was diluted with water and extracted with EA. The organic phase was washed 5 times with water, dried over anhydrous sodium sulfate, and concentrated under reduced pressure to remove the solvent to obtain a crude product of **INT12b** (165 mg). MS/ESI $[M+H]^+$ = 347.2.

Step 3: The crude product of **INT12b** (150 mg, 0.4 mmol) was dissolved in 5 mL of MeOH, and then 0.9 mL of a 4 M solution of HCl in dioxane was added. The mixture was allowed to react at room temperature for 12 h. After the reaction was completed as monitored by TLC, potassium carbonate was added. The mixture was stirred for 30 min and then filtered to remove the potassium carbonate to obtain a crude product of compound **INT12** (120 mg, yellowish-brown solid). MS/ESI $[M+H]^+$ = 247.1.

Intermediates **INT13-INT16** were prepared using a synthesis method similar to the preparation of intermediate **INT12.**

Table 5. Intermediates **INT13-INT16**

| Intermediate | Starting material 1 | Starting material 2 | Structure | Name | Data |
|---|---|---|---|---|---|
| **INT13** | 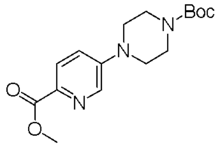 | $CD_3NH_2$ | 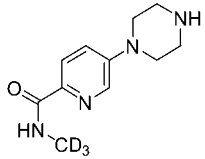 | *N*-(Methyl-$d_3$)-5-(pipe razin-1-yl)picolinamide | MS/ESI[M+ H]$^+$=234.1 |
| **INT14** | 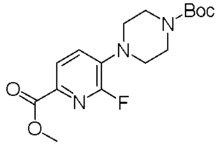 | 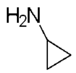 | 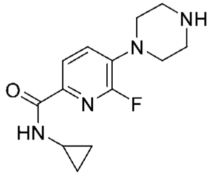 | *N*-Cyclopropyl-6-fluoro -5-(piperazin-1-yl)pic olinamide | MS/ESI[M+ H]$^+$=265.1 |
| **INT15** | 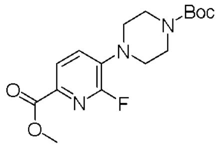 | $CD_3NH_2$ | 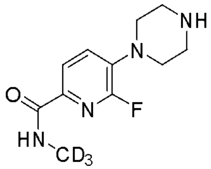 | 6-Fluoro-*N*-(methyl-$d_3$ )-5-(piperazin-1-yl)pic olinamide | MS/ESI[M+ H]$^+$=242.2 |
| **INT16** | 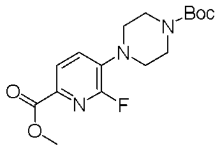 | 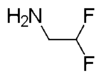 | 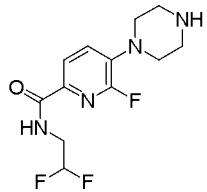 | *N*-(2,2-Difluoroethyl)-6-fluoro-5-(piperazin-1-yl)picolinamide | MS/ESI[M+ H]$^+$=289.2 |

**Intermediate INT17:** 5-Fluoro-1',2',3',6'-tetrahydro-2,4'-bipyridine

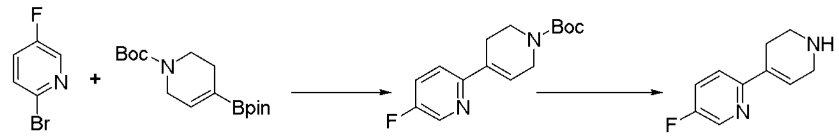

INT17a INT1b INT17b INT17

Step 1: Compound **INT17a** (0.5 g, 2.8 mmol), **INT1b** (0.92 g, 3.4 mmol), Pd(dppf)$Cl_2$ (0.21 g, 0.52 mmol), and potassium carbonate (0.99 g, 13 mmol) were added to a mixed solvent of 7 mL of dioxane, 3 mL of absolute ethanol, and 4 mL of water. Then, the mixture was purged three times with nitrogen and allowed to react at 90 °C for 2 h in a nitrogen atmosphere. After the reaction was completed as detected by TLC, the reaction mixture was cooled to room temperature, and 30 mL of dichloromethane and 20 mL of water were added. The mixture was then allowed to separate into layers in a separatory funnel. The aqueous phase was extracted twice with dichloromethane, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT17b** (0.7 g, white solid).

Step 2: Compound **INT17b** (0.5 g, 1.8 mmol) was added to 10 mL of absolute methanol, and then 10 mL of a 4 mol/L solution of hydrochloric acid in dioxane was added. The mixture was stirred at room temperature for 0.5-1 h. After the reaction was completed as monitored by TLC, the reaction mixture was concentrated under reduced pressure to dryness to obtain compound **INT17** (0.3 g, white solid). MS/ESI $[M+H]^+$ = 179.1.

Intermediates **INT18** and **INT19** were prepared using a synthesis method similar to the preparation of intermediate **INT17**.

Table 6. Intermediates INT18 and INT19

| Intermediate | Starting material | Structure | Name | Data |
|---|---|---|---|---|
| **INT18** | 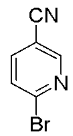 | 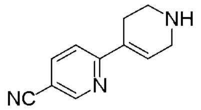 | 1',2',3',6'-Tetrahydro-[2,4'-bipyridine]-5-carbonitrile | MS/ESI$[M+H]^+$= 186.1 |
| **INT19** | 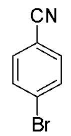 | 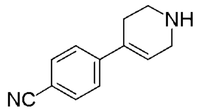 | *N*,6-Dimethyl-5-(piperazin-1-yl )picolinamide | MS/ESI$[M+H]^+$= 185.1 |

**Intermediate INT20**: 1-(5-Fluoropyridin-2-yl)piperazine

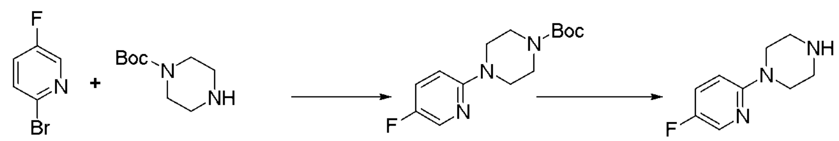

INT17a INT9a INT20a INT20

Step 1: Compound **INT17a** (1 g, 5.00 mmol) and compound **INT9a** (838 mg, 4.5 mmol) were dissolved in toluene (15 mL), and palladium acetate (112 mg, 0.5 mmol) and BINAP (311 mg, 0.5 mmol) were added. The reaction flask was purged with nitrogen and then placed in an oil bath at 120 °C for reaction. After 16 h of reaction, the reaction mixture was quenched with water (20 mL) and extracted with ethyl acetate (3 × 30 mL). The organic phases were combined and concentrated under reduced pressure. The crude product was purified by column chromatography (PE:EA = 5:1) to obtain

compound **INT20a** (yellow solid, 1.4 g), MS/ESI $[M+H]^+$ = 282.1.

Step 2: Compound **INT20a** (0.5 g, 1.8 mmol) was added to absolute methanol (10 mL), and then a 4 mol/L solution of hydrochloric acid in dioxane (10 mL) was added. The mixture was stirred at room temperature for 1 h. After the reaction was completed as monitored by TLC, the reaction mixture was concentrated under reduced pressure to obtain compound **INT20** (0.4 g, white solid). MS/ESI $[M+H]^+$ = 182.1.

Intermediates **INT21** and **INT22** were prepared using a synthesis method similar to the preparation of intermediate **INT20**.

Table 7. Intermediates **INT21** and **INT22**

| Intermediate | Starting material | Structure | Name | Data |
|---|---|---|---|---|
| **INT21** | 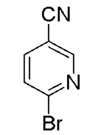 | 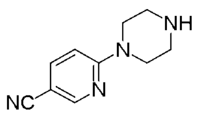 | 6-(Piperazin-1-yl) nicotinonitrile | MS/ESI$[M+H]^+$=189.1 |
| **INT22** | 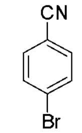 | 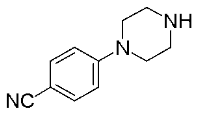 | 4-(Piperazin-1-yl) benzonitrile | MS/ESI$[M+H]^+$=188.1 |

**Intermediate INT23:** (*R*)-*N*-methyl-5-(2-methylpiperazin-1-yl)picolinamide

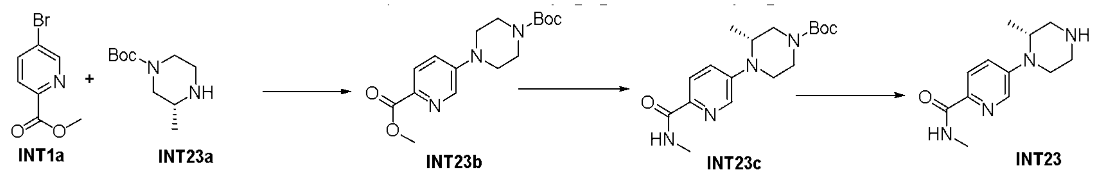

INT1a INT23a INT23b INT23c INT23

Step 1: Compound **INT1a** (5.66 g, 26.21 mmol, 1.05 eq), compound **INT23a** (5.00 g, 24.96 mmol, 1.00 eq), $Cs_2CO_3$ (16.27 g, 49.93 mmol, 2.00 eq), and RuPhos-Pd-G3 (1.04 g, 1.25 mmol, 0.05 eq) were added to 1,4-dioxane (50 mL), and the mixture was stirred at 120 °C overnight in a nitrogen atmosphere. After the reaction was completed as detected by LCMS, the mixture was allowed to cool to room temperature. The reaction mixture was diluted with water (100 mL) and then extracted with ethyl acetate (2 × 100 mL). The organic layers were combined, washed with brine (2 × 50 mL), dried over anhydrous $Na_2SO_4$, and filtered, and the filtrate was concentrated under reduced pressure. The residue was purified by silica gel column chromatography to obtain compound **INT23b** (white solid, 5.00 g, 59.71%). MS/ESI $[M+H]^+$ = 336.1.

Step 2: Compound **INT23b** (2.00 g, 5.96 mmol, 1.00 eq) was added to a solution of methylamine (8 mL, 25-30 wt% in water) in methanol (7 mL), and the mixture was stirred at room temperature for 3 h in a nitrogen atmosphere. The reaction was monitored by LCMS. The reaction was quenched with a saturated aqueous ammonium chloride solution (30 mL) at room temperature. The resulting mixture was extracted with dichloromethane (3 × 50 mL). The organic layers were combined, washed with brine (2 × 50 mL), dried over anhydrous $Na_2SO_4$, and filtered, and then the filtrate was concentrated under reduced pressure to obtain compound **INT23c** (1.70 g, 85.25%) as a light yellow oil. MS/ESI $[M+H]^+$ = 335.3.

Step 3: Compound **INT23c** (500 mg, 1.50 mmol, 1.00 eq) and a solution of HCl in 1,4-dioxane (3.7 mL, 14.95 mmol, 10.0 eq, 4.0 M) were stirred at room temperature for 2 h. After the reaction was completed as monitored by LCMS, the resulting mixture was concentrated under reduced pressure to obtain compound **INT23** (400 mg, crude product) as a yellow solid. The crude product was directly used in the next step without further purification. MS/ESI $[M+H]^+$ = 235.3.

**Intermediate INT24:** 7-(Bromomethyl)-3-ethyl-1,5,7,8-tetrahydro-2*H*-pyrano[4,3-*b*]pyridin-2-one

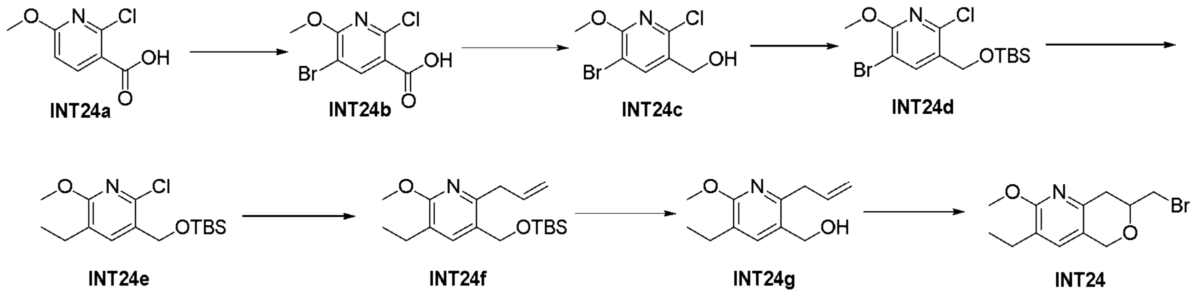


Step 1: Bromine (16.0 g, 100 mmol) was added to a suspension of 2-chloro-6-methoxypyridine-3-carboxylic acid **INT24a** (4.69 g, 25 mmol) and sodium acetate (4.10 g, 50 mmol) in glacial acetic acid (200 mL) at room temperature. The mixture was heated to 80 °C and allowed to react overnight. After the reaction was completed, the mixture was cooled to room temperature and poured into 500 mL of ice water with vigorous stirring. The solid was collected by filtration and washed with water to obtain compound **INT24b** (white solid, 5.2 g, yield: 78%). $^1$H NMR (400 MHz, DMSO-$d_6$): δ8.51 (s, 1H), 3.93 (s, 3H). MS/ESI $[M+H]^+$ = 266.1.

Step 2: Compound **INT24b** (2.0 g, 7.50 mmol) was added to a solution of $BH_3$·THF (30.0 mL, 1 mol/L), and the mixture was stirred at room temperature for 2 h. After the reaction was completed, the reaction mixture was quenched with MeOH and then concentrated under reduced pressure to remove the solvent. The residue was purified by column chromatography using petroleum ether/ethyl acetate (5/1, v/v) to obtain compound **INT24c** (white solid, 1.4 g, 73%). MS/ESI $[M+H]^+$ = 251.9.

Step 3: Compound **INT24c** (2.5 g, 10 mmol) and imidazole (1.0 g, 15 mmol) were added to DMF (50 mL), and *tert*-butyldimethylchlorosilane (1.8 g, 12 mmol) was added. The resulting solution was stirred at room temperature for 24 h, then diluted with diethyl ether, and washed with water and brine. The organic phases were combined, then dried over anhydrous sodium sulfate, and filtered, and the filtrate was concentrated under reduced pressure. Purification was performed by column chromatography to obtain the target compound **INT24d** (2.3 g). MS/ESI $[M+H]^+$ = 366.2.

Step 4: Compound **INT24d** (3.65 g, 10 mmol), ethylboronic acid (1.11 g, 15 mmol), cesium carbonate (9.78 g, 30 mmol), and Pd(dppf)$Cl_2$ (0.73 g, 1 mmol) were added to 1,4-dioxane/water (10:1, 50 mL), and the mixture was heated to 80 °C and allowed to react for 4 h in a nitrogen atmosphere. After the reaction was completed, the mixture was cooled to room temperature and diluted with water and ethyl acetate, and the aqueous phase was extracted with ethyl acetate. The organic phases were combined, then dried over anhydrous sodium sulfate, and filtered, and the filtrate was concentrated under reduced pressure. Purification was performed by column chromatography to obtain the target compound **INT24e** (1.5 g). MS/ESI $[M+H]^+$ = 316.2.

Step 5: Compound **INT24e** (3.65 g, 10 mmol), allylboronic acid pinacol ester (2.52 g, 15 mmol), cesium carbonate (9.78 g, 30 mmol), and Pd(dppf)$Cl_2$ (0.73 g, 1 mmol) were added to 1,4-dioxane/water (10:1, 50 mL), and the mixture was heated to 80 °C and allowed to react for 4 h in a nitrogen atmosphere. After the reaction was completed, the mixture was cooled to room temperature and diluted with water and ethyl acetate, and the aqueous phase was extracted with ethyl acetate. The organic phases were combined, then dried over anhydrous sodium sulfate, and filtered, and the filtrate was concentrated under reduced pressure. Purification was performed by column chromatography to obtain the target compound **INT24f** (2.2 g). MS/ESI $[M+H]^+$ = 322.2.

Step 6: Compound **INT24f** (1.6 g, 5 mmol) and a 48% aqueous HBr solution (1.1 mL) were added to acetic acid (30 mL), and the mixture was heated to 70 °C and stirred with heating for 6 h. After the reaction was completed, the mixture was cooled to room temperature and concentrated, and an aqueous $K_2CO_3$ solution was added. The mixture was stirred, then extracted twice with ethyl acetate, dried over anhydrous sodium sulfate, and filtered, and the filtrate was concentrated under reduced pressure to remove the solvent. The residue was purified by column chromatography to obtain compound **INT24g** (0.7 g). MS/ESI $[M+H]^+$ = 194.2.

Step 7: At 0 °C, compound **INT24g** (3.5 g, 18 mmol) was added to dry dichloromethane (50 mL), and *N*-bromosuccinimide (NBS, 3.56 g, 20.0 mmol) was added in portions. The reaction mixture was warmed to room temperature, stirred for 12 h, and then concentrated under reduced pressure to remove the solvent. The residue was purified by column chromatography to obtain compound **INT24** (2.7 g). MS/ESI $[M+H]^+$ = 272.2.

**Intermediate INT25:** 7-(Iodomethyl)-2-methoxy-3-methyl-7,8-dihydro-5*H*-pyrano[4,3-*b*]pyridine

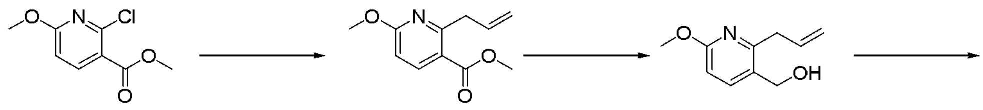

INT25a INT25b INT25c

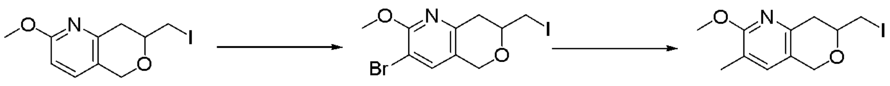

INT25d INT25e INT25

Step 1: Compound **INT25a** (25 g, 124.4 mmol), allylboronic acid pinacol ester (31.3 g, 186.6 mmol), Pd(dppf)$Cl_2$ (9.1 g, 12.4 mmol), and potassium phosphate (66.0 g, 310.9 mmol) were added to a mixed solvent of 250 mL of tetrahydrofuran and 25 mL of water. Then, the mixture was purged three times with nitrogen and allowed to react at 90 °C for 3 h in a nitrogen atmosphere. After the reaction was completed as detected by TLC, the reaction mixture was cooled to room temperature, and ethyl acetate and water were added. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT25b** (24.0 g, colorless oily liquid). LC-MS: ESI $[M+H]^+$ = 208.1.

Step 2: Compound **INT25b** (24.0 g, 115.9 mmol) was added to 240 mL of anhydrous tetrahydrofuran, and then 290 mL of a 1 mol/L lithium diisobutylaluminum hydride solution was added. The mixture was stirred at room temperature for 3 h. After the reaction was completed as monitored by TLC, saturated ammonium chloride was added to quench the reaction, followed by the addition of ethyl acetate and water. The mixture was then allowed to separate into layers in a separatory funnel. The aqueous phase was extracted 2-3 times with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT25c** (17.2 g, colorless oily liquid). LC-MS: ESI $[M+H]^+$ = 180.1.

Step 3: Compound **INT25c** (17.2 g, 96.1 mmol), $I_2$ (58.6 g, 230.6 mmol), and sodium bicarbonate (19.4 g, 230.6 mmol) were added to 200 mL of anhydrous acetonitrile, and the mixture was stirred at room temperature for 18 h. After the reaction was completed as monitored by thin-layer chromatography, a saturated sodium thiosulfate solution was added, followed by the addition of ethyl acetate and water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT25d** (6.2 g, white solid). LC-MS: ESI $[M+H]^+$ = 306.1.

Step 4: Compound **INT25d** (6.2 g, 20.3 mmol), NBS (5.8 g, 32.5 mmol), and dichloroacetic acid (0.26 g, 2.0 mmol) were added to 50 mL of glacial acetic acid, and the mixture was allowed to react at 90 °C for 3 h. After the reaction was completed as monitored by TLC, a saturated sodium thiosulfate solution was added, followed by the addition of ethyl acetate and water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT25e** (3.1 g, yellow solid). LC-MS: ESI $[M+H]^+$ = 384.1.

Step 5: Compound **INT25e** (1 g, 2.6 mmol), methylboronic acid (0.31 g, 5.2 mmol), Pd(dppf)$Cl_2$ (0.19 g, 0.26 mmol), and potassium carbonate (0.90 g, 6.5 mmol) were added to a mixed solvent of 10 mL of toluene and 1 mL of water. Then, the mixture was purged three times with nitrogen and allowed to react at 90 °C overnight in a nitrogen atmosphere. After the reaction was completed as detected by TLC, the reaction mixture was cooled to room temperature, and 30 mL of ethyl acetate and 50 mL of water were added. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT25** (0.6 g, white solid). LC-MS: ESI $[M+H]^+$ = 320.1.

**Intermediate INT26:** 3-Cyclopropyl-7-(iodomethyl)-2-methoxy-7,8-dihydro-5*H*-pyrano[4,3-*b*]pyridine

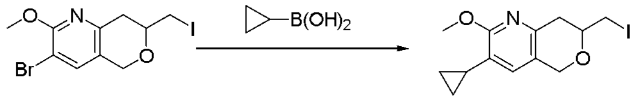


INT25e INT26

3-Cyclopropyl-7-(iodomethyl)-2-methoxy-7,8-dihydro-5*H*-pyrano[4,3-*b*]pyridine was prepared using a synthesis method similar to the preparation of intermediate **INT25**. LC-MS: ESI $[M+H]^+$ = 346.1.

**Intermediate INT27:** 3-(Difluoromethyl)-7-(iodomethyl)-1,5,7,8-tetrahydro-2*H*-pyrano[4,3-*b*]pyridin-2-one

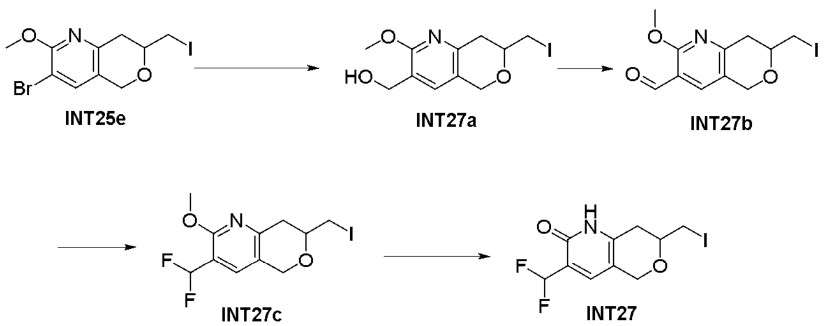

INT25e INT27a INT27b INT27c INT27

Step 1: **INT25e** (1.0 g, 2.6 mmol), (tributyltin)methanol (1.0 g, 3.1 mmol), and RuPhos Pd G3 (0.22 g, 0.26 mmol) were added to 10 mL of a dioxane solution. Then, the mixture was purged three times with nitrogen and allowed to react at 90 °C overnight in a nitrogen atmosphere. After the reaction was completed as detected by TLC, the reaction mixture was cooled to room temperature, and 30 mL of ethyl acetate and 50 mL of water were added. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT27a** (0.5 g, white solid).

Step 2: **INT27a** (0.5 g, 1.5 mmol) and DMP (0.76 g, 1.8 mmol) were added to 5 mL of a dichloromethane solution, and the mixture was stirred at room temperature for 2 h. After the reaction was completed as detected by LC-MS, a saturated sodium bicarbonate solution was added, followed by the addition of 30 mL of ethyl acetate and 50 mL of water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, concentrated to dryness by rotary evaporation, and purified by column chromatography to obtain compound **INT27b** (0.4 g, white solid).

Step 3: **INT27b** (0.4 g, 1.2 mmol) and DAST (0.23 g, 1.4 mmol) were added to 5 mL of a dichloromethane solution, and the mixture was stirred at room temperature for 8 h. After the reaction was completed as detected by LC-MS, saturated sodium bicarbonate was added to quench the reaction, followed by the addition of 30 mL of ethyl acetate and 50 mL of water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT27c** (0.3 g, white solid).

Step 4: **INT27c** (0.3 g, 0.84 mmol) and 0.5 mL of a 48% aqueous hydrobromic acid solution were added to 5 mL of dioxane, and the mixture was heated to 80 °C and stirred for 1 h. After the reaction was completed as detected by LC-MS, a saturated sodium bicarbonate solution was added, followed by the addition of 30 mL of ethyl acetate and 50 mL of water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, concentrated to dryness by rotary evaporation, and purified by column chromatography to obtain compound **INT27** (0.12 g, white solid). LC-MS: ESI $[M+H]^+$ = 342.1.

**Intermediate INT28:** *N*-(Cyclopropylmethoxy)-5-(piperazin-1-yl)picolinamide

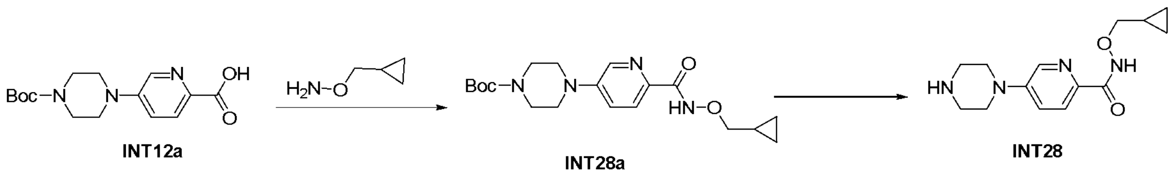

INT12a INT28a INT28

*N*-(Cyclopropylmethoxy)-5-(piperazin-1-yl)picolinamide was prepared using a synthesis method similar to the preparation of intermediate **INT12**. LC-MS: ESI $[M+H]^+$ = 277.2.

**Intermediate INT29**: 6-Fluoro-*N*-methyl-5-(piperidin-4-yl)picolinamide

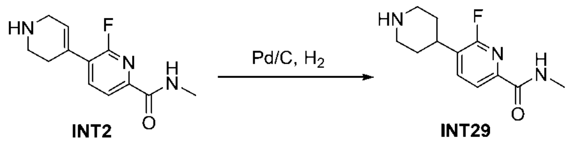


INT2 INT29

Compound **INT2** (0.2 g, 0.84 mmol) was dissolved in DCM (20 mL), and Pd/C (10%, 0.1 g) was added. The mixture was purged three times with hydrogen and then stirred at 25 °C for 12 h in a hydrogen atmosphere. The reaction mixture was filtered through celite, and the filtrate was concentrated under reduced pressure to remove the solvent, thus obtaining compound **INT29** (0.2 g, crude). LC-MS: ESI $[M+H]^+$ = 238.2.

**Intermediate INT30**: 7-(Bromomethyl)-3-chloro-2-methoxy-7,8-dihydro-5*H*-pyrano[4,3-*b*]pyridine

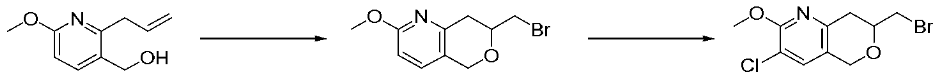

INT25c INT30a INT30

Step 1: Compound **INT25c** (17.2 g, 96.1 mmol), NBS (34.2 g, 192.2 mmol), and sodium bicarbonate (19.4 g, 230.6 mmol) were added to 200 mL of anhydrous acetonitrile, and the mixture was stirred at room temperature for 18 h. After the reaction was completed as monitored by thin-layer chromatography, a saturated sodium thiosulfate solution was added, followed by the addition of ethyl acetate and water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT30a** (3.2 g, white solid). LC-MS: ESI $[M+H]^+$ = 258.1.

Step 2: Compound **INT30a** (3.2 g, 12.5 mmol), NCS (3.3 g, 24.9 mmol), and dichloroacetic acid (0.26 g, 2.0 mmol) were added to 50 mL of glacial acetic acid, and the mixture was allowed to react at 90 °C for 3 h. After the reaction was completed as monitored by TLC, a saturated sodium thiosulfate solution was added, followed by the addition of ethyl acetate and water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, and concentrated to dryness by rotary evaporation. The resulting crude product was purified by column chromatography to obtain compound **INT30** (1.1 g, yellow solid). LC-MS: ESI $[M+H]^+$ = 292.1.

**Intermediate INT31**: 7-(Iodomethyl)-3-vinyl-1,5,7,8-tetrahydro-2*H*-pyrano[4,3-*b*]pyridin-2-one

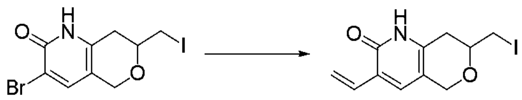

INT25e INT31

Compound **INT25e** (0.85 g, 2.3 mmol), tributyl(vinyl)tin (1.09 g, 3.5 mmol), and Ruphos Pd $G_3$ (0.19 g, 0.23 mmol) were added to 10 mL of a dioxane solution, and the mixture was heated to 100 °C and stirred for 2 h. After the reaction was completed as detected by LC-MS, 30 mL of ethyl acetate and 50 mL of water were added. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, concentrated to dryness by rotary evaporation, and purified by column chromatography to obtain compound **INT31** (0.35 g, white solid). LC-MS: ESI $[M+H]^+$ = 318.1.

**Intermediate INT32:** 2-Chloro-*N*-methyl-1',2',3',6'-tetrahydro-[3,4'-bipyridine]-6-carboxamide

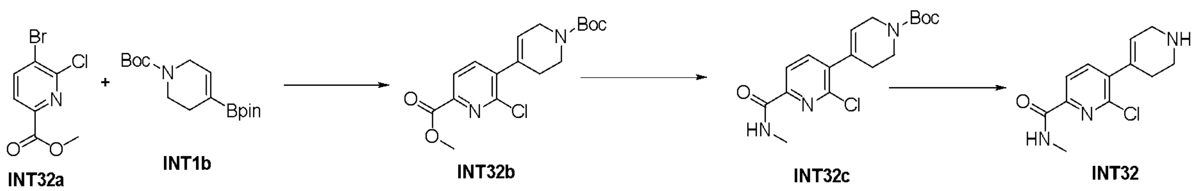

INT32a INT1b INT32b INT32c INT32

2-Chloro-*N*-methyl-1',2',3',6'-tetrahydro-[3,4'-bipyridine]-6-carboxamide was prepared using a synthesis method similar to the preparation of intermediate **INT1**. MS/ESI $[M+H]^+$ = 252.1.

**Intermediate INT33:** 6-Chloro-*N*-methyl-5-(piperazin-1-yl)picolinamide

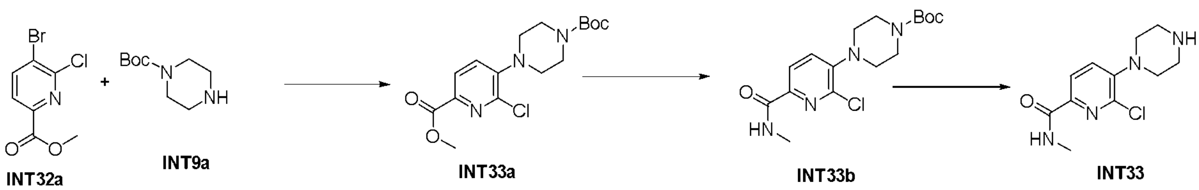

INT32a INT9a INT33a INT33b INT33

6-Chloro-*N*-methyl-5-(piperazin-1-yl)picolinamide was prepared using a synthesis method similar to the preparation of intermediate **INT9**. MS/ESI $[M+H]^+$ = 255.3.

**Intermediate INT34:** 6-Cyano-*N*-methyl-5-(piperazin-1-yl)picolinamide

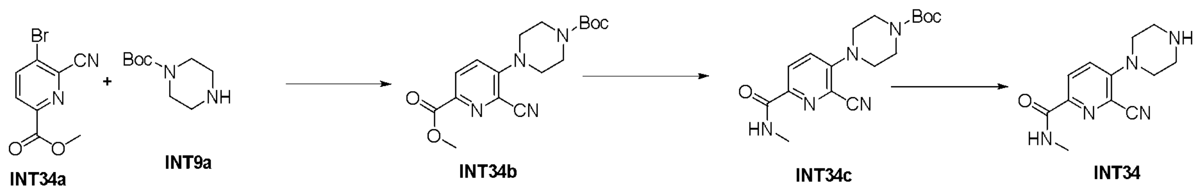

INT34a INT9a INT34b INT34c INT34

6-Cyano-*N*-methyl-5-(piperazin-1-yl)picolinamide was prepared using a synthesis method similar to the preparation of intermediate **INT9**. MS/ESI $[M+H]^+$ = 246.3.

**Intermediate INT35:** (*S*)-6-Fluoro-*N*-methyl-5-(3-methylpiperazin-1-yl)picolinamide

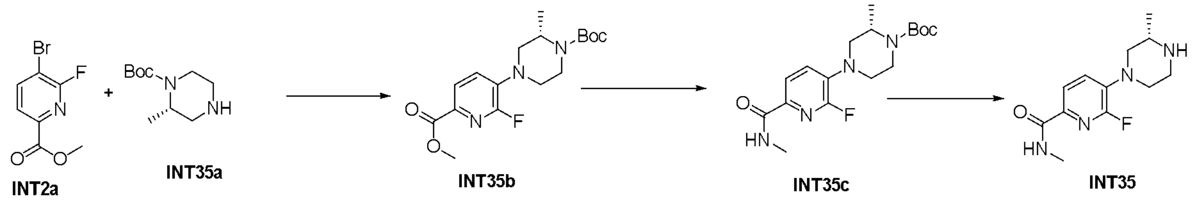

INT2a INT35a INT35b INT35c INT35

(*S*)-6-Fluoro-*N*-methyl-5-(3-methylpiperazin-1-yl)picolinamide was prepared using a synthesis method similar to the preparation of intermediate **INT23**. MS/ESI $[M+H]^+$ = 235.3.

**Intermediate INT36:** (*R*)-6-Fluoro-*N*-methyl-5-(3-methylpiperazin-1-yl)picolinamide

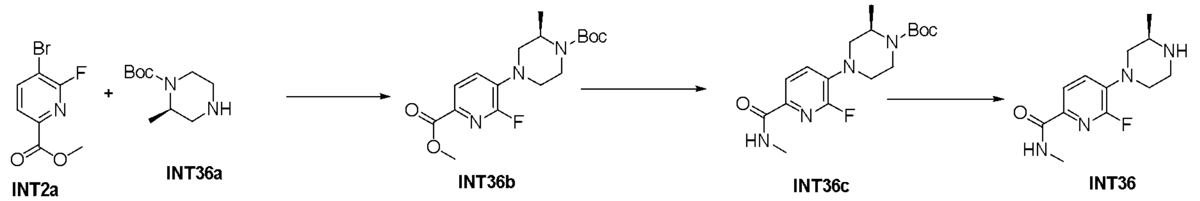

INT2a INT36a INT36b INT36c INT36

(*R*)-6-Fluoro-*N*-methyl-5-(3-methylpiperazin-1-yl)picolinamide was prepared using a synthesis method similar to the preparation of intermediate **INT23**. MS/ESI $[M+H]^+$ = 235.3.

**Intermediate INT37:** 6-Fluoro-*N*-(1-methyl-1*H*-pyrazol-4-yl)-5-(piperazin-1-yl)picolinamide

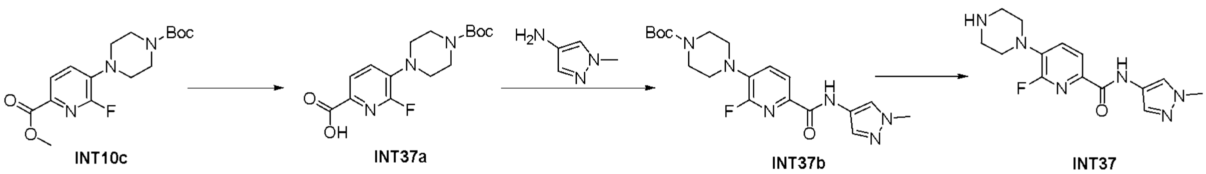

INT10c INT37a INT37b INT37

6-Fluoro-*N*-(1-methyl-1*H*-pyrazol-4-yl)-5-(piperazin-1-yl)picolinamide was prepared using a synthesis method similar to the preparation of intermediate **INT12**. MS/ESI $[M+H]^+$ = 305.3.

**Intermediate INT38:** 6-Fluoro-*N*-(3-methoxycyclobutyl)-5-(piperazin-1-yl)picolinamide

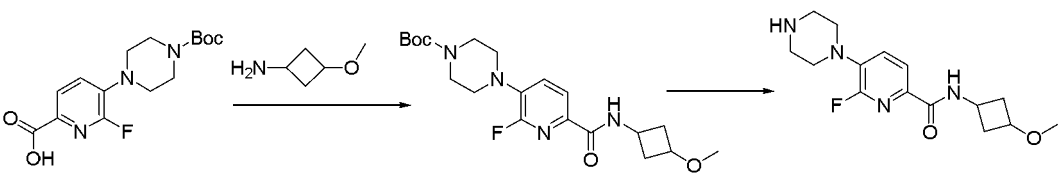

INT37a INT38a INT38

6-Fluoro-*N*-(3-methoxycyclobutyl)-5-(piperazin-1-yl)picolinamide was prepared using a synthesis method similar to the preparation of intermediate **INT12**. MS/ESI $[M+H]^+$ = 309.3.

**Intermediate INT39:** 5-((1*S*,6*R*)-2,5-Diazabicyclo[4.2.0]octan-2-yl)-6-fluoro-*N*-methylpicolinamide

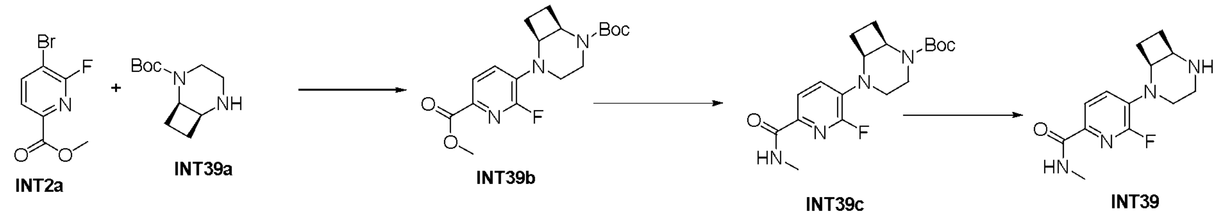
INT2a INT39a INT39b INT39c INT39

5-((1*S*,6*R*)-2,5-Diazabicyclo[4.2.0]octan-2-yl)-6-fluoro-*N*-methylpicolinamide was prepared using a synthesis method similar to the preparation of intermediate **INT23**. MS/ESI $[M+H]^+$ = 265.3.

**Intermediate INT40:** Preparation of (*R*)-*N*-methyl-1,2,3,4,4a,5-hexahydro-7*H*-pyrazino[2,1-*c*]pyrido[3,2-*e*][1,4]oxazolidine-9-car boxamide hydrochloride

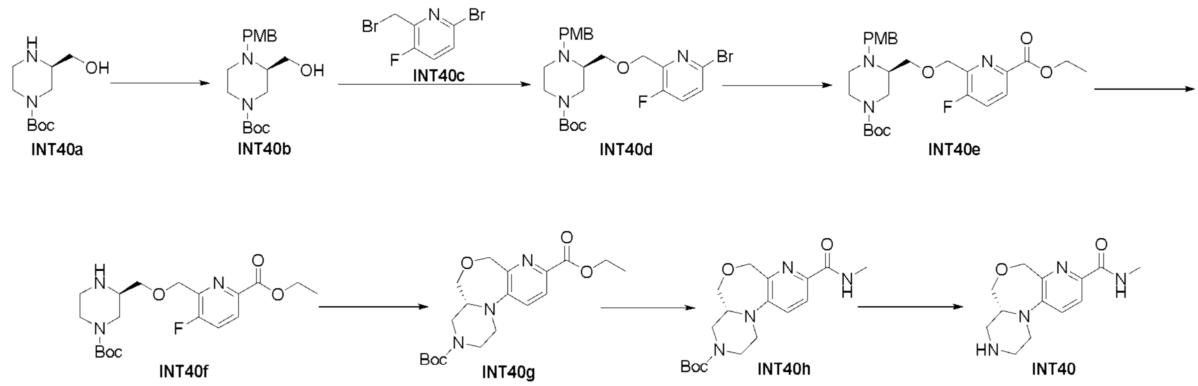
INT40a INT40b INT40c INT40d INT40e

INT40f INT40g INT40h INT40

Step 1: *tert*-Butyl (*R*)-3-(hydroxymethyl)piperazine-1-carboxylate (5 g, 23.118 mmol) was added to DMF (50 mL), and *p*-methoxybenzyl chloride (3.62 g, 23.118 mmol) and potassium carbonate (9.58 g, 69.355 mmol) were added. The mixture was heated to 60 °C and allowed to react for 24 h. After the reaction was completed, water was added to quench the reaction, and the mixture was extracted with ethyl acetate. The organic phase was washed 3 times with water and concentrated under reduced pressure. The resulting crude product was purified by column chromatography (petroleum ether:ethyl acetate = 10:1 to 5:1) to obtain compound **INT40b** (6 g, yield: 77.14%). LCMS = 337.4; $^1$H NMR (400 MHz, Chloroform-*d*) δ 7.14 (d, *J* = 8.3 Hz, 2H), 6.79 (d, *J* = 8.3 Hz, 2H), 3.88 (d, *J* = 13.2 Hz, 1H), 3.81 - 3.76 (m, 1H), 3.72 (s, 3H), 3.60 ( m, 1H), 3.51 (m, 2H), 3.28 (d, *J* = 13.1 Hz, 2H), 3.07 (s, 1H), 2.69 (d, *J* = 12.3 Hz, 1H), 2.56 - 2.42 (m, 1H), 2.18 (m, 1H), 1.38 (s, 9H).

Step 2: Compound **INT40b** (6 g, 17.834 mmol) and 6-bromo-2-(bromomethyl)-3-fluoropyridine (4.80 g, 17.834 mmol) were added to DMF (100 mL), and the mixture was cooled to 0 °C, followed by the addition of sodium hydride (1.07 g, 26.751 mmol). After the addition was completed, the mixture was allowed to react at room temperature for 12 h. After the reaction was completed, water was added to quench the reaction, and the mixture was extracted with ethyl acetate. The organic phase was washed 3 times with water and concentrated under reduced pressure. The resulting crude product was purified by column chromatography (petroleum ether:ethyl acetate = 5:1 to 1:1) to obtain compound **INT40d** (4.2 g). LCMS = 525.4. $^1$H NMR (400 MHz, Chloroform-*d*) δ 7.37-7.34 (m, 1H), 7.23-7.19 (m, 1H), 7.16 - 7.10 (m, 2H), 6.79 - 6.73 (m, 2H), 4.66 - 4.51 (m, 2H), 3.84 (s, 1H), 3.72 (s, 3H), 3.59-3.55 (m, 2H), 3.43 (d, *J* = 13.1 Hz, 1H), 3.29 (d, *J* = 13.3 Hz, 1H), 3.11 (s, 2H), 2.61-2.53 (m, 2H), 2.09 (s, 1H), 1.37 (s, 9H).

Step 3: Compound **INT40d** (4.2 g, 8.009 mmol), $PdCl_2(PPh_3)_2$ (1.12 g, 1.602 mmol), and DIEA (3.11 g, 24.026 mmol) were added to DMF (30 mL) and ethanol (30 mL). In a carbon monoxide atmosphere, the mixture was heated to 90 °C and allowed to react for 12 h. The reaction mixture was then added to water, followed by extraction with ethyl acetate. The organic phase was washed 3 times with water and concentrated under reduced pressure. The resulting crude product was purified by column chromatography (petroleum ether:ethyl acetate = 5:1 to 2:1) to obtain compound **INT40e** (2 g, yield: 48.25%). LCMS = 518.2.

Step 4: Compound **INT40e** (500 mg, 0.966 mmol) was added to acetonitrile (10 mL) and water (10 mL), and the

mixture was cooled to 0 °C. Ceric ammonium nitrate (2.647 g, 4.83 mmol) was then added, and the mixture was stirred at room temperature for 12 h. After the reaction was completed, the reaction mixture was added to a saturated aqueous sodium bicarbonate solution to adjust the pH to 8-9, followed by extraction with ethyl acetate. The organic phase was washed 3 times with water and concentrated under reduced pressure. The resulting crude product was purified by column chromatography (dichloromethane:methanol = 8:1) to obtain a crude product of compound **INT40f** (100 mg, yield: 26.05%). LCMS = 398.2.

Step 5: Compound **INT40f** (85 mg, 0.214 mmol) and DIEA (82.93 mg, 0.642 mmol) were added to NMP (3 mL), and the mixture was heated to 180 °C and stirred for 6 h. After the reaction was completed, the reaction mixture was added to water, followed by extraction with ethyl acetate. The organic phase was washed 3 times with water and concentrated under reduced pressure to obtain compound **INT40g** (80 mg, yield: 99.11%). LCMS = 378.2.

Step 6: Compound **INT40g** (80 mg, 0.212 mmol) and a methylamine alcohol solution (2 mL) were added to methanol (2 mL), and the mixture was stirred at room temperature for 1 h. After the reaction was completed, the reaction mixture was concentrated under reduced pressure to give a crude product of compound **INT40h** (80 mg, yield: 100%). LCMS = 363.4. Step 7: Compound **INT40h** (80 mg, 0.221 mmol) was added to dioxane (2 mL), and a solution of hydrochloric acid in dioxane (1 mL) was added. The mixture was stirred at room temperature for 1 h. After the reaction was completed, the reaction mixture was concentrated under reduced pressure to obtain compound **INT40** (hydrochloride, 80 mg). LCMS = 263.2.

**Intermediate INT41**: *N*,6-Dimethyl-5-(piperidin-4-yl)picolinamide

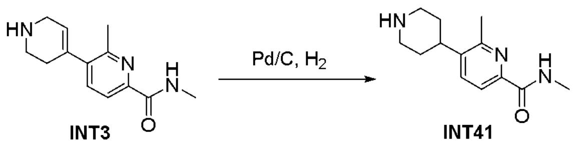

INT3 INT41

*N*,6-Dimethyl-5-(piperidin-4-yl)picolinamide was prepared using a synthesis method similar to the preparation of intermediate **INT29**. LC-MS: ESI $[M+H]^+$ = 234.2.

**Intermediate INT42**: 6-Chloro-*N*-cyclopropyl-5-(piperazin-1-yl)picolinamide

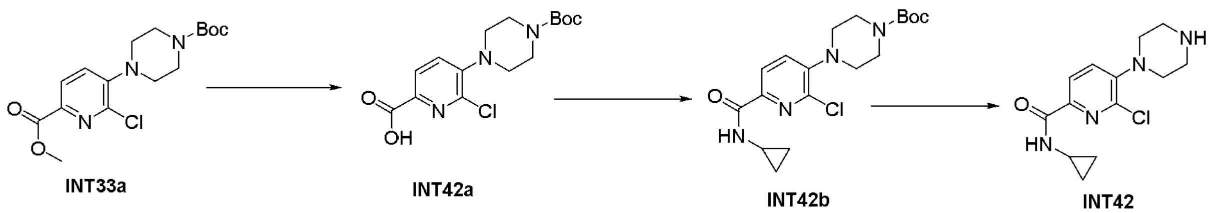
INT33a INT42a INT42b INT42

6-Chloro-*N*-cyclopropyl-5-(piperazin-1-yl)picolinamide was prepared using a synthesis method similar to the preparation of intermediate **INT12**. LC-MS: ESI $[M+H]^+$ = 281.2.

**Example 1**

5-(4-((3-Ethyl-2-oxo-1,5,7,8-tetrahydro-2*H*-pyrano[4,3-*b*]pyridin-7-yl)methyl)piperazin-1-yl) -*N*-methylpicolinamide

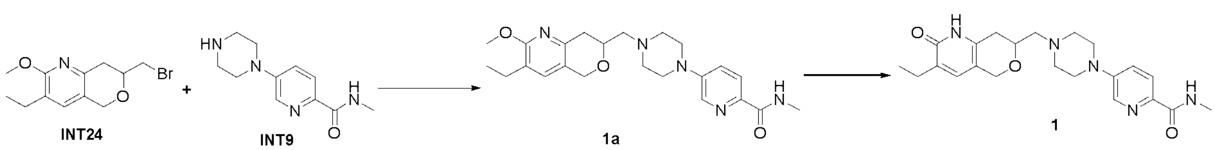
INT24 INT9 1a 1

Step 1: **INT9** (0.13 g, 0.6 mmol), DIEA (0.4 g, 3.2 mmol), **INT24** (0.17 g, 0.6 mmol), and KI (0.01 g, 0.06 mmol)

were added to 5 mL of acetonitrile, and the mixture was heated to 80 °C and stirred for 48 h. After the reaction was completed as detected by LC-MS, 5 mL of a saturated sodium bicarbonate solution was added. The mixture was extracted with ethyl acetate, concentrated to dryness by rotary evaporation, and purified by column chromatography to obtain compound **1a** (0.08 g, pale yellow solid).

Step 2: **1a** (0.08 g, 0.19 mmol) and 0.5 mL of a 48% aqueous hydrobromic acid solution were added to 4 mL of dioxane, and the mixture was heated to 80 °C and stirred for 2 h. After the reaction was completed as detected by LC-MS, a saturated sodium bicarbonate solution was added. The mixture was stirred, filtered, concentrated to dryness by rotary evaporation, and purified by column chromatography to obtain compound **1** (0.024 g, white solid). LC-MS: ESI $[M+H]^+$ = 412.2; $^1H$ NMR (400 MHz, DMSO-d6) δ 11.46 (s, 1H), 8.47 (q, *J* = 4.8 Hz, 1H), 8.33 (d, *J* = 2.9 Hz, 1H), 7.89 (d, *J* = 8.8 Hz, 1H), 7.46 (dd, *J* = 8.8, 2.9 Hz, 1H), 7.05 (s, 1H), 4.57 - 4.43 (m, 2H), 3.91 (dq, *J* = 10.2, 5.0 Hz, 1H),3.35 (m, *J* = 3.2 Hz, 4H), 2.84 (d, *J* = 4.8 Hz, 3H), 2.67 (m, *J* = 6.6 Hz, 4H), 2.62 - 2.58 (m, 2H), 2.50 (d, *J* = 11.5 Hz, 2H), 2.41 (q, *J* = 7.4 Hz, 2H), 1.12 (t, *J* = 7.4 Hz, 3H).

Compounds **2-38** of **Examples 2-38** were prepared using a synthesis method similar to the preparation of **Example 1**.

Table 8. Compounds **2-38**

| Compound | Structure | Name | Data |
|---|---|---|---|
| **2** | 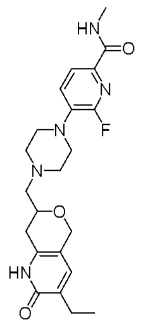 | 5-(4-((3-Ethyl-2-oxo-1,5,7,8 -tetrahydro-2*H*-pyrano[4,3-*b* ]pyridin-7-yl) methyl)pipera zin-1-yl)-6-fluoro-*N*-methyl picolinamide | MS/ESI$[M+H]^+$=430.2; $^1H$ NMR (400 MHz, DMSO-$d_6$) δ 11.40 (s, 1H), 8.40 (q, J = 4.7 Hz, 1H), 7.85 (dd, J = 8.1, 1.4 Hz, 1H), 7.57 (dd, J = 10.6, 8.1 Hz, 1H), 6.99 (s, 1H), 4.51 - 4.37 (m, 2H), 3.90 - 3.79 (m, 1H), 3.16 (t, J = 4.9 Hz, 4H), 2.77 (d, J = 4.8 Hz, 3H), 2.64 (q, J = 4.8 Hz, 4H), 2.61 - 2.40 (m, 4H), 2.35 (q, J = 7.5 Hz, 2H), 1.07 (t, J = 7.4 Hz, 3H). |
| **3** | 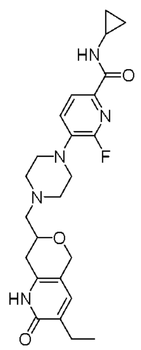 | *N*-Cyclopropyl-5-(4-((3-ethyl-2-oxo-1,5,7,8-tetrahydro-2*H*-pyrano [4,3-*b*]pyridin-7-yl)methyl)piperazin-1-yl)-6-fluoropicolinamide | MS/ESI$[M+H]^+$=456.2; $^1H$ NMR (400 MHz, DMSO-$d_6$) δ 11.42 - 11.38 (m, 1H), 8.36 (d, *J* = 4.9 Hz, 1H), 7.85 (dd, *J* = 8.1, 1.4 Hz, 1H), 7.57 (dd, *J* = 10.6, 8.1 Hz, 1H), 7.00 (s, 1H), 4.51 - 4.37 (m, 2H), 3.88 - 3.81 (m, 1H), 3.15 (t, *J* = 4.9 Hz, 4H), 2.85 (qd, *J* = 6.9, 4.0 Hz, 1H), 2.62 (tdd, *J* = 19.3, 12.6, 5.4 Hz, 4H), 2.51 - 2.39 (m, 2H), 2.35 (q, *J* = 7.5 Hz, 2H), 1.06 (t, *J* = 7.4 Hz, 3H), 0.72 - 0.60 (m, 4H). |
| **4** | 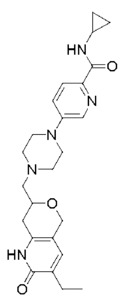 | *N*-Cyclopropyl-5-(4-((3-ethyl-2-oxo-1,5,7,8-tetrahydro-2*H*-pyrano [4,3-*b*]pyridin-7-yl)methyl)piperazin-1-yl) picolinamide | MS/ESI$[M+H]^+$=438.2 |

(continued)

| Compound | Structure | Name | Data |
|---|---|---|---|
| **5** | 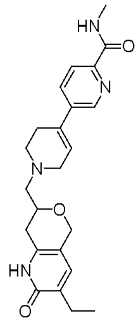 | 1'-((3-Ethyl-2-oxo-1,5,7,8-tetrahydro-2*H*-pyrano[4,3-*b*] pyridin-7-yl) methyl)-*N*-methyl -1',2',3',6'-tetrahydro-[3,4'-bipyridine]-6-carboxamide | MS/ESI[M+H]+=409.2;$^1$H NMR (400 MHz, DM SO-d6) δ 11.46 (s, 1H), 8.83 - 8.74 (m, 2H), 8.09 - 8.00 (m, 2H), 7.06 (s, 1H), 6.48 (t, *J* = 2.4 Hz, 1H), 4.58 - 4.44 (m, 2H), 3.93 (dd, *J* = 10.0, 4.9 Hz, 1H), 3.28 (p, *J* = 3.2 Hz, 2H), 2.88 (d, *J* = 4. 8 Hz, 3H), 2.80 (dt, *J* = 8.1, 5.7 Hz, 2H), 2.75 - 2 .60 (m, 4H), 2.55 - 2.47 (m, 2H), 2.41 (q, *J* = 7.4 Hz, 2H), 1.13 (t, *J* = 7.4 Hz, 3H). |
| **6** | 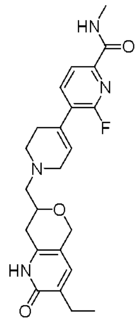 | 1'-((3-Ethyl-2-oxo-1,5,7,8-tetrahydro-2*H*-pyrano[4,3-*b*] pyridin-7-yl) methyl)-2-fluoro -*N*-methyl-1',2',3',6'-tetrahydro -[3,4'-bipyridine]-6-carbo xamide | MS/ESI[M+H]+=427.2; $^1$H NMR (400 MHz, DMSO-$d_6$) δ 11.38 (s, 1H), 8.64 (q, *J* = 4.7 Hz, 1H), 8.08 (dd, *J* = 9.9, 7.7 Hz, 1H), 7.92 (dd, *J* = 7.7, 1.8 Hz, 1H), 6.99 (s, 1H), 6.25 (t, *J* = 2.5 Hz, 1H), 4.54 - 4.38 (m, 2H), 3.86 (dq, *J* = 10.1, 5.3 Hz, 1H), 3.21 (q, *J* = 3.2 Hz, 2H), 2.79 (d, *J* = 4.8 Hz, 3H), 2.72 (h, *J* = 5.9 Hz, 2H), 2.71 - 2.53 (m, 2H), 2.48 (m, 3H), 2.44 (d, *J* = 10.0 Hz, 1H), 2.35 (q, *J* = 7.6 Hz, 2H), 1.06 (t, *J* = 7.4 Hz, 3H). |
| **7** | 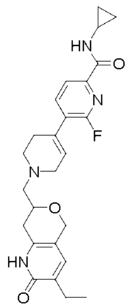 | *N*-Cyclopropyl-1'-((3-ethyl-2-oxo-1,5,7,8-tetrahydro-2*H* -pyrano [4,3-*b*]pyridin-7-yl) methyl)-2-fluoro-1',2',3',6'-tetrahydro-[3,4'-bipyridine]-6-carboxamide | MS/ESI[M+H]+=453.2 |
| **8** | 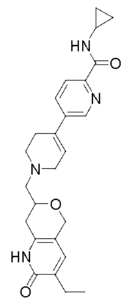 | *N*-Cyclopropyl-1'-((3-ethyl-2-oxo-1,5,7,8-tetrahydro-2*H* -pyrano [4,3-*b*]pyridin-7-yl) methyl)-1',2',3',6'-tetrahydro -[3,4'-bipyridine]-6-carboxamide | MS/ESI[M+H]+=435.2 |

(continued)

| Compound | Structure | Name | Data |
|---|---|---|---|
| **9** | 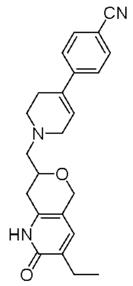 | 4-(1-((3-Ethyl-2-oxo-1,5,7,8 -tetrahydro-2*H*-pyrano[4,3-*b* ]pyridin-7-yl) methyl)-1,2,3, 6-tetrahydropyridin-4-yl)be nzonitrile | MS/ESI[M+H]$^+$=376.2 |
| **10** | 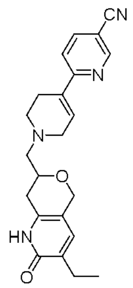 | 1'-((3-Ethyl-2-oxo-1,5,7,8-te trahydro-2*H*-pyrano[4,3-*b*]p yridin-7-yl) methyl)-1',2',3',6 '-tetrahydro-[2,4'-bipyridine] -5-carbonitrile | MS/ESI[M+H]$^+$=377.2; $^1$H NMR (400 MHz, DMSO-$d_6$) δ 11.51 (s, 1H), 9.03 (d, *J* = 2.2 Hz, 1H), 8.35 (dd, *J* = 8.4, 2.2 Hz, 1H), 7.86 (d, *J* = 8.5 Hz, 1H), 7.04 (s, 1H), 6.93 (s, 1H), 4.56 (s, 2H), 4.18 (s, 2H), 3.99 (s, 1H), 3.71 (s, 1H), 2.90 (s, 3H), 2.57 - 2.52 (m, 2H), 2.46 (s, 2H), 2.40 - 2.31 (m, 2H), 1.07 (t, *J* = 7.4 Hz, 3H). |
| **11** | 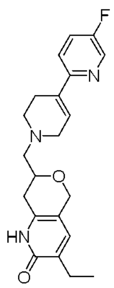 | 3-Ethyl-7-((5-fluoro-3',6'-di hydro-[2,4'-bipyridin]-1'(2'*H* )-yl) methyl)-1,5,7,8-tetrahy dro-2*H*-pyrano [4,3-*b*]pyridin -2-one | MS/ESI[M+H]$^+$=370.2; $^1$H NMR (400 MHz, DMSO-$d_6$) δ 11.39 (s, 1H), 8.50 (d, *J* = 2.9 Hz, 1H), 7.72 - 7.54 (m, 2H), 6.99 (s, 1H), 6.62 (d, *J* = 3.6 Hz, 1H), 4.46 (t, *J* = 10.5 Hz, 2H), 3.91 - 3.81 (m, 1H), 3.20 (d, *J* = 4.3 Hz, 2H), 2.74 - 2.66 (m, 2H), 2.65 - 2.52 (m, 4H), 2.49 - 2.42 (m, 2H), 2.35 (q, *J* = 7.5 Hz, 2H), 1.06 (t, *J* = 7.4 Hz, 3H). |
| **12** | 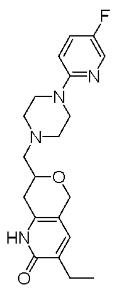 | 3-Ethyl-7-((4-(5-fluoropyrid in-2-yl)piperazin-1-yl)meth yl)-1,5,7,8-tetrahydro-2*H*-pyrano[4,3-*b*]pyridin-2-one | MS/ESI[M+H]$^+$=373.2; $^1$H NMR (400 MHz, DMSO-$d_6$) δ 10.13 (s, 1H), 8.17 (d, *J* = 3.1 Hz, 1H), 7.62 (ddd, *J* = 9.3, 8.3, 3.1 Hz, 1H), 7.10 - 6.90 (m, 2H), 4.57 (d, *J* = 2.3 Hz, 2H), 4.41 - 4.10 (m, 4H), 3.49 - 3.32 (m, 3H), 3.29 - 3.03 (m, 5H), 2.46 (t, *J* = 7.3 Hz, 2H), 2.37 (q, *J* = 7.5 Hz, 2H), 1.07 (t, *J* = 7.4 Hz, 3H). |
| **13** | 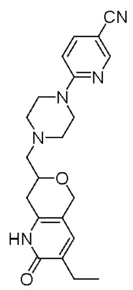 | 6-(4-((3-Ethyl-2-oxo-1,5,7,8 -tetrahydro-2*H*-pyrano[4,3-*b* ]pyridin-7-yl) methyl)pipera zin-1-yl)nicotinonitrile | MS/ESI[M+H]$^+$=380.2; $^1$H NMR (400 MHz, DMSO-$d_6$) δ 11.50 (s, 1H), 8.57 (d, *J* = 2.3 Hz, 1H), 7.98 (dd, *J* = 9.1, 2.3 Hz, 1H), 7.10 - 7.02 (m, 2H), 4.56 (s, 2H), 4.16 (s, 1H), 3.64 - 3.57 (m, 3H), 3.14 (s, 5H), 2.46 (t, *J* = 7.1 Hz, 3H), 2.36 (q, *J* = 7.6 Hz, 2H), 1.07 (t, *J* = 7.4 Hz, 3H). |

(continued)

| Compound | Structure | Name | Data |
|---|---|---|---|
| **14** | 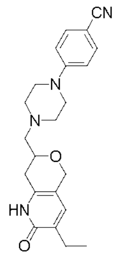 | 4-(4-((3-Ethyl-2-oxo-1,5,7,8 -tetrahydro-2*H*-pyrano[4,3-*b* ]pyridin-7-yl) methyl)pipera zin-1-yl)benzonitrile | MS/ESI[M+H]$^+$=379.2 |
| **15** | 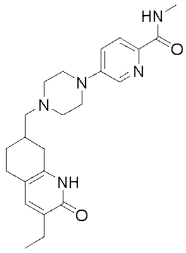 | 5-(4-((3-Ethyl-2-oxo-1,2,5,6 ,7,8-hexahydroquinoline-7-yl )methyl)piperazin-1-yl)-*N*-methylpicolinamide | MS/ESI[M+H]$^+$=410.2 |
| **16** | 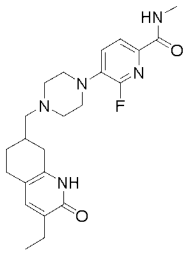 | 5-(4-((3-Ethyl-2-oxo-1,2,5,6 ,7,8-hexahydroquinoline-7-yl )methyl)piperazin-1-yl)-6-fl uoro-*N*-methylpicolinamide | MS/ESI[M+H]$^+$=428.2 |
| **17** | 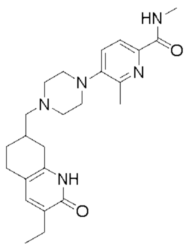 | 5-(4-((3-Ethyl-2-oxo-1,2,5,6 ,7,8-hexahydroquinoline-7-yl )methyl)piperazin-1-yl)-*N*,6 -dimethylpicolinamide | MS/ESI[M+H]$^+$=424.3 |
| **18** | 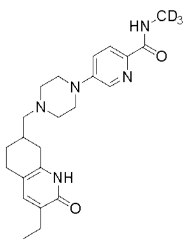 | 5-(4-((3-Ethyl-2-oxo-1,2,5,6 ,7,8-hexahydroquinoline-7-yl )methyl)piperazin-1-yl)-*N*-( methyl-$d_3$)picolinamide | MS/ESI[M+H]$^+$=413.3 |
| **19** | 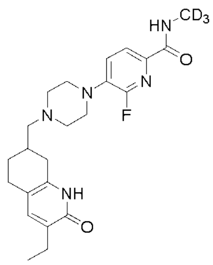 | 5-(4-((3-Ethyl-2-oxo-1,2,5,6 ,7,8-hexahydroquinoline-7-yl)methyl)piperazin-1-yl)-6-fl uoro-*N*-(methyl-$d_3$)picolina mide | MS/ESI[M+H]$^+$=431.3 |

(continued)

| Compound | Structure | Name | Data |
|---|---|---|---|
| **20** | 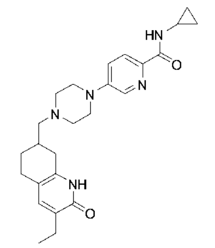 | *N*-Cyclopropyl-5-(4-((3-eth yl-2-oxo-1,2,5,6,7,8-hexahy droquinoline-7-yl)methyl)pi perazin-1-yl)picolinamide | MS/ESI[M+H]+=436.3 |
| **21** | 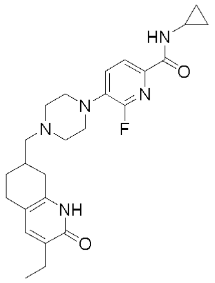 | *N*-Cyclopropyl-5-(4-((3-eth yl-2-oxo-1,2,5,6,7,8-hexahy droquinoline-7-yl)methyl)pi perazin-1-yl)-6-fluoropicoli namide | MS/ESI[M+H]+=454.3 |
| **22** | 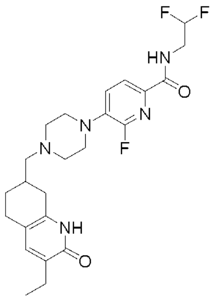 | *N*-(2,2-Difluoroethyl)-5-(4-( (3-ethyl-2-oxo-1,2,5,6,7,8-hexahydroquinoline-7-yl)met hyl)piperazin-1-yl)-6-fluoro picolinamide | MS/ESI[M+H]+=478.2 |
| **23** | 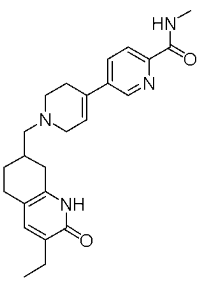 | 1'-((3-Ethyl-2-oxo-1,2,5,6,7, 8-hexahydroquinoline-7-yl) methyl)-*N*-methyl-1',2',3',6'-tetrahydro-[3,4'-bipyridine]-6-carboxamide | MS/ESI[M+H]+=407.2 |
| **24** | 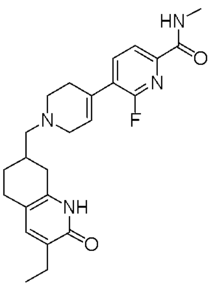 | 1'-((3-Ethyl-2-oxo-1,2,5,6,7, 8-hexahydroquinoline-7-yl) methyl)-2-fluoro-*N*-methyl-1',2',3',6'-tetrahydro-[3,4'-bi pyridine]-6-carboxamide | MS/ESI[M+H]+=425.2 |

(continued)

| Compound | Structure | Name | Data |
|---|---|---|---|
| **25** | 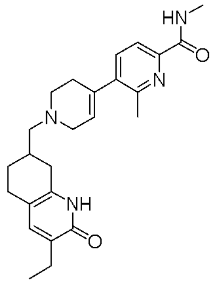 | 1'-((3-Ethyl-2-oxo-1,2,5,6,7, 8-hexahydroquinoline-7-yl) methyl)-*N*,2-dimethyl-1',2',3 ',6'-tetrahydro-[3,4'-bipyridi ne]-6-carboxamide | MS/ESI$[M+H]^+$=421.3 |
| **26** | 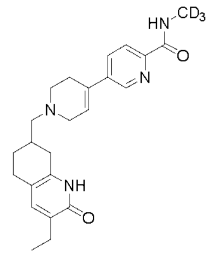 | 1'-((3-Ethyl-2-oxo-1,2,5,6,7, 8-hexahydroquinoline-7-yl) methyl)-*N*-(methyl-$d_3$)-1',2', 3',6'-tetrahydro-[3,4'-bipyrid ine]-6-carboxamide | MS/ESI$[M+H]^+$=410.3 |
| **27** | 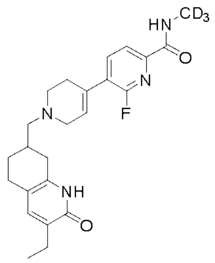 | 1'-((3-Ethyl-2-oxo-1,2,5,6,7, 8-hexahydroquinoline-7-yl) methyl)-2-fluoro-N-(methyl-$d_3$)-1',2',3',6'-tetrahydro-[3,4' -bipyridine]-6-carboxamide | MS/ESI$[M+H]^+$=428.3 |
| **28** | 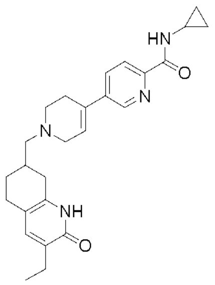 | *N*-Cyclopropyl-1'-((3-ethyl-2-oxo-1,2,5,6,7,8-hexahydro quinoline-7-yl)methyl)-1',2', 3',6'-tetrahydro-[3,4'-bipyridine]-6-carboxamide | MS/ESI$[M+H]^+$=433.3 |
| **29** | 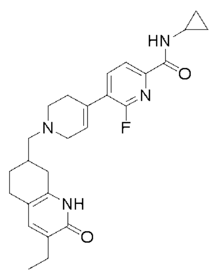 | *N*-Cyclopropyl-1'-((3-ethyl-2-oxo-1,2,5,6,7,8-hexahydro quinoline-7-yl)methyl)-2-flu oro-1',2',3',6'-tetrahydro-[3,4 '-bipyridine]-6-carboxamide | MS/ESI$[M+H]^+$=451.3 |

(continued)

| Compound | Structure | Name | Data |
|---|---|---|---|
| **30** | 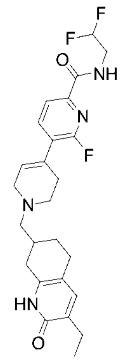 | *N*-(2,2-Difluoroethyl)-1'-((3 -ethyl-2-oxo-1,2,5,6,7,8-hex ahydroquinoline-7-yl)methyl )-2-fluoro-1',2',3',6'-tetrahy dro-[3,4'-bipyridine]-6-carb oxamide | MS/ESI[M+H]+=475.2 |
| **31** | 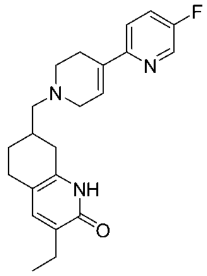 | 3-Ethyl-7-((5-fluoro-3',6'-di hydro-[2,4'-bipyridin]-1'(2'*H* )-yl) methyl)-5,6,7,8-tetrahy droquinoline-2(1*H*)-one | MS/ESI[M+H]+=368.2 |
| **32** | 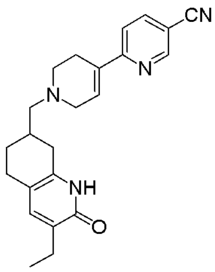 | 1'-((3-Ethyl-2-oxo-1,2,5,6,7, 8-hexahydroquinoline-7-yl) methyl)-1',2',3',6'-tetrahydro -[2,4'-bipyridine]-5-carbonit rile | MS/ESI[M+H]+=375.2 |
| **33** | 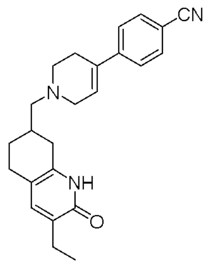 | 4-(1-((3-Ethyl-2-oxo-1,2,5,6 ,7,8-hexahydroquinoline-7-yl )methyl)-1,2,3,6-tetrahydro pyridin-4-yl)benzonitrile | MS/ESI[M+H]+=374.2 |
| **34** | 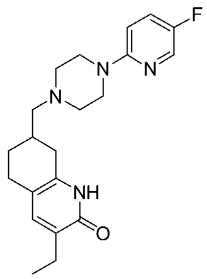 | 3-Ethyl-7-((4-(5-fluoropyrid in-2-yl)piperazin-1-yl)meth yl)-5,6,7,8-tetrahydroquinoli ne-2(1*H*)-one | MS/ESI[M+H]+=371.2 |

(continued)

| Compound | Structure | Name | Data |
|---|---|---|---|
| **35** | 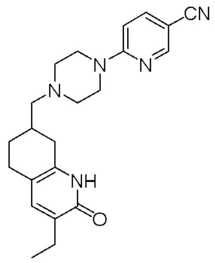 | 6-(4-((3-Ethyl-2-oxo-1,2,5,6 ,7,8-hexahydroquinoline-7-yl )methyl)piperazin-1-yl)nico tinonitrile | MS/ESI[M+H]+=378.2 |
| **36** | 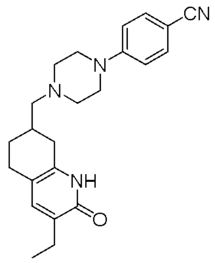 | 4-(4-((3-Ethyl-2-oxo-1,2,5,6 ,7,8-hexahydroquinoline-7-yl )methyl)piperazin-1-yl)ben zonitrile | MS/ESI[M+H]+=377.2 |
| **37** | 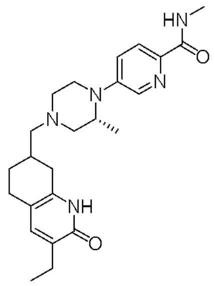 | 5-((2*R*)-4-((3-Ethyl-2-oxo-1, 2,5,6,7,8-hexahydroquinolin e-7-yl)methyl)-2-methylpipe razin-1-yl)-*N*-methylpicolin amide | MS/ESI[M+H]+=424.3 |
| **38** | 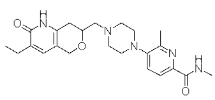 | 5-(4-((3-Ethyl-2-oxo-1,5,7,8 -tetrahydro-2*H*-pyrano[4,3-*b* ]pyridin-7-yl) methyl)pipera zin-1-yl)-*N*,6-dimethylpicoli namide | MS/ESI[M+H]+= 426.3; $^1$H NMR (400 MHz, DMSO-$d_6$) δ 11.40 (s, 1H), 8.44 (q, *J* = 4.9 Hz, 1H), 7.80 (d, *J* = 8.2 Hz, 1H), 7.48 (d, *J* = 8.3 Hz, 1H), 7.00 (s, 1H), 4.48 (d, *J* = 14.1 Hz, 1H), 4.42 (d, *J* = 14.1 Hz, 1H), 3.84 (s, 1H), 2.94 (t, *J* = 4.8 Hz, 4H), 2.81 (d, *J* = 4.8 Hz, 4H), 2.63 (dd, *J* = 20.9, 7.0 Hz, 5H), 2.59 - 2.38 (m, 4H), 2.35 (q, *J* = 7.4 Hz, 2H), 1.07 (t, *J* = 7.4 Hz, 3H). |

**Example 39**

6-Fluoro-*N*-methyl-5-(4-((3-methyl-2-oxo-1,5,7,8-tetrahydro-2*H*-pyrano[4,3-*b*]pyridin-7-yl)m ethyl)piperazin-1-yl)picolinamide

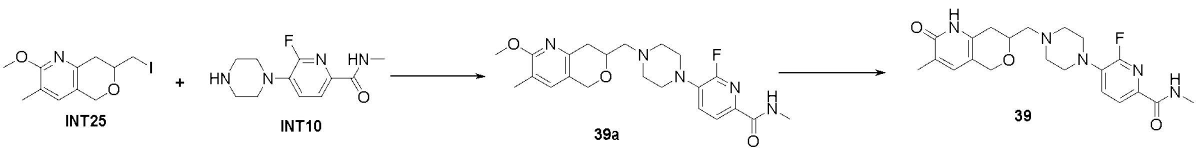


Step 1: **INT10** (0.14 g, 0.6 mmol), DIEA (0.4 g, 3.2 mmol), **INT25** (0.2 g, 0.6 mmol), and KI (0.01 g, 0.06 mmol) were added to 5 mL of acetonitrile, and the mixture was heated to 80 °C and stirred for 48 h. After the reaction was

completed as detected by LC-MS, 5 mL of a saturated sodium bicarbonate solution was added. The mixture was extracted with ethyl acetate, concentrated to dryness by rotary evaporation, and purified by column chromatography to obtain compound **39a** (0.07 g, pale yellow solid).

Step 2: **39a** (0.08 g, 0.19 mmol) and 0.5 mL of a 48% aqueous hydrobromic acid solution were added to 4 mL of dioxane, and the mixture was heated to 80 °C and stirred for 2 h. After the reaction was completed as detected by LC-MS, a saturated sodium bicarbonate solution was added. The mixture was stirred, filtered, concentrated to dryness by rotary evaporation, and purified by column chromatography to obtain compound **39** (0.014 g, white solid). LC-MS: ESI $[M+H]^+$ = 416.2; $^1$H NMR (400 MHz, DMSO-$d_6$) δ 11.42 (s, 1H), 8.41 (q, *J* = 4.8 Hz, 1H), 7.85 (dd, *J* = 8.1, 1.4 Hz, 1H), 7.57 (dd, *J* = 10.6, 8.1 Hz, 1H), 7.04 (d, *J* = 1.3 Hz, 1H), 4.49 - 4.36 (m, 2H), 3.88 - 3.79 (m, 1H), 3.15 (t, *J* = 4.9 Hz, 4H), 2.77 (d, *J* = 4.8 Hz, 3H), 2.64 (d, *J* = 4.3 Hz, 2H), 2.62 (d, *J* = 5.0 Hz, 2H), 2.57 (dd, *J* = 13.1, 6.3 Hz, 1H), 2.45 (t, *J* = 8.3 Hz, 2H), 1.93 (s, 3H).

**Example 40**

5-(4-((3-Bromo-2-oxo-1,5,7,8-tetrahydro-2*H*-pyrano[4,3-*b*]pyridin-7-yl)methyl)piperazin-1-y l)-6-fluoro-*N*-methylpicolinamide

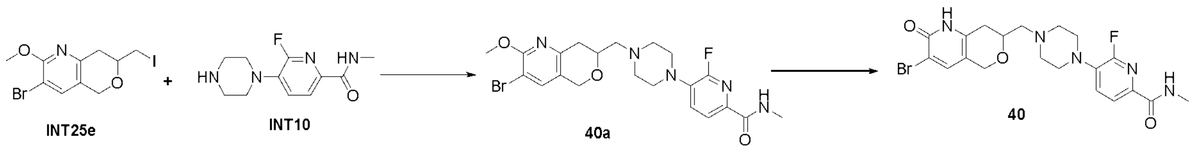

INT25e INT10 40a 40

5-(4-((3-Bromo-2-oxo-1,5,7,8-tetrahydro-2*H*-pyrano[4,3-*b*]pyridin-7-yl)methyl)piperazin-1-y l)-6-fluoro-*N*-methylpicolinamide was prepared using a synthesis method similar to the preparation of Example 39. LC-MS: ESI $[M+H]^+$ = 480.3; $^1$H NMR (400 MHz, DMSO-$d_6$) δ 12.03 (s, 1H), 8.41 (q, *J* = 4.7 Hz, 1H), 7.85 (dd, *J* = 8.0, 1.4 Hz, 1H), 7.71 (s, 1H), 7.57 (dd, *J* = 10.6, 8.1 Hz, 1H), 4.61 - 4.31 (m, 2H), 3.85 (q, *J* = 4.8 Hz, 1H), 3.16 (t, *J* = 4.8 Hz, 4H), 2.77 (d, *J* = 4.7 Hz, 3H), 2.63 (hept, *J* = 6.6, 6.1 Hz, 4H), 2.58 - 2.53 (m, 2H), 2.44 (dd, *J* = 17.5, 10.0 Hz, 2H).

**Example 41**

*N*-Cyclopropyl-6-fluoro-5-(4-((3-methyl-2-oxo-1,5,7,8-tetrahydro-2*H*-pyrano[4,3-*b*]pyridin-7 -yl)methyl)piperazin-1-yl) picolinamide

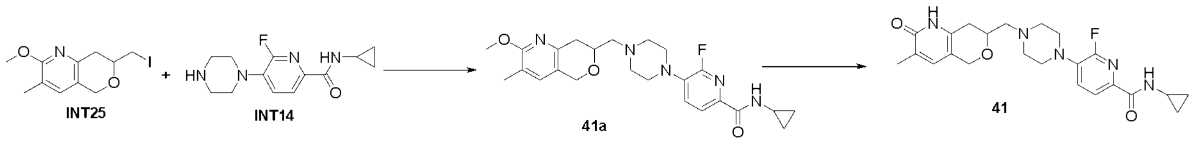

INT25 INT14 41a 41

*N*-Cyclopropyl-6-fluoro-5-(4-((3-methyl-2-oxo-1,5,7,8-tetrahydro-2*H*-pyrano[4,3-*b*]pyridin-7 -yl)methyl)piperazin-1-yl)picolinamide was prepared using a synthesis method similar to the preparation of Example **39.** LC-MS: ESI $[M+H]^+$ = 442.3; $^1$H NMR (400 MHz, DMSO-$d_6$) δ 11.42 (s, 1H), 8.36 (d, *J* = 4.9 Hz, 1H), 7.85 (dd, *J* = 8.0, 1.4 Hz, 1H), 7.57 (dd, *J* = 10.6, 8.1 Hz, 1H), 7.04 (d, *J* = 1.3 Hz, 1H), 4.50 - 4.37 (m, 2H), 3.89 - 3.77 (m, 1H), 3.16 (d, *J* = 4.2 Hz, 4H), 2.91 - 2.79 (m, 1H), 2.64 (s, 3H), 2.49 - 2.36 (m, 2H), 1.93 (s, 3H), 1.26 (q, *J*= 6.1 Hz, 3H), 0.66 (tt, *J* = 6.3, 2.1 Hz, 4H).

**Example 42**

5-(4-((3-Cyclopropyl-2-oxo-1,5,7,8-tetrahydro-2*H*-pyrano[4,3-*b*]pyridin-7-yl)methyl)piperazi n-1-yl)-6-fluoro-*N*-methylpicolinamide

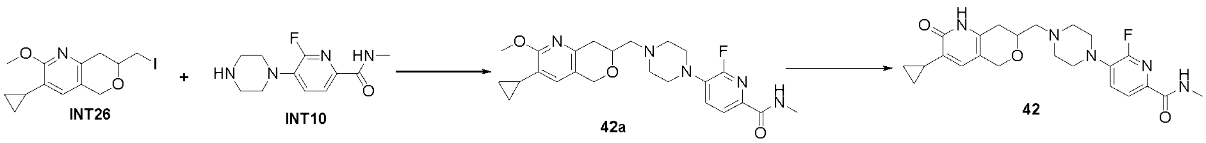

5-(4-((3-Cyclopropyl-2-oxo-1,5,7,8-tetrahydro-2*H*-pyrano[4,3-*b*]pyridin-7-yl)methyl)piperazi n-1-yl)-6-fluoro-N-methylpicolinamide was prepared using a synthesis method similar to the preparation of Example 39. LC-MS: ESI [M+H]$^+$ = 442.3; $^1$H NMR (400 MHz, DMSO-$d_6$) δ 11.41 (s, 1H), 8.41 (q, *J* = 4.7 Hz, 1H), 7.85 (dd, *J* = 8.0, 1.4 Hz, 1H), 7.57 (dd, *J* = 10.6, 8.1 Hz, 1H), 6.71 (s, 1H), 4.51 - 4.27 (m, 2H), 3.87 - 3.74 (m, 1H), 3.15 (t, *J* = 4.8 Hz, 4H), 2.77 (d, *J* = 4.7 Hz, 3H), 2.63 (q, *J* = 4.9, 4.5 Hz, 4H), 2.56 (t, *J* = 6.5 Hz, 3H), 2.48 - 2.35 (m, 2H), 0.80 (dd, *J* = 8.4, 2.3 Hz, 2H), 0.62 - 0.50 (m, 2H).

## Example 43

5-(4-((3-(Difluoromethyl)-2-oxo-1,5,7,8-tetrahydro-2*H*-pyrano[4,3-*b*]pyridin-7-yl)methyl)pip erazin-1-yl)-6-fluoro-*N*-methylpicolinamide

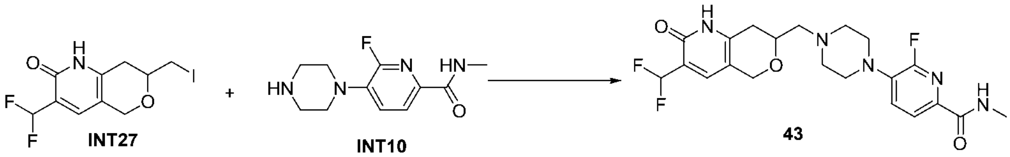

5-(4-((3-(Difluoromethyl)-2-oxo-1,5,7,8-tetrahydro-2*H*-pyrano[4,3-*b*]pyridin-7-yl)methyl)pip erazin-1-yl)-6-fluoro-*N*-methylpicolinamide was prepared using a synthesis method similar to the preparation of Example 1. LC-MS: ESI [M+H]$^+$ = 452.3; $^1$H NMR (400 MHz, DMSO-$d_6$) δ 12.23 (s, 1H), 8.53 (q, *J* = 4.7 Hz, 1H), 7.96 (dd, *J* = 8.1, 1.3 Hz, 1H), 7.75 (dd, *J* = 10.6, 8.0 Hz, 1H), 7.65 (s, 1H), 6.89 (s, 1H), 4.73 (d, *J* = 14.4 Hz, 1H), 4.65 (d, *J* = 14.4 Hz, 1H), 4.26 (s, 1H), 3.23 (t, 4H), 2.84 (d, *J* = 4.8 Hz, 4H), 2.68 - 2.59 (m, 3H), 2.58 (s, 4H).

## Example 44

*N*-(Cyclopropylmethoxy)-5-(4-((3-ethyl-2-oxo-1,5,7,8-tetrahydro-2*H*-pyrano[4,3-*b*]pyridin-7-yl)methyl)piperazin-1-yl)-6-fluoropicolinamide

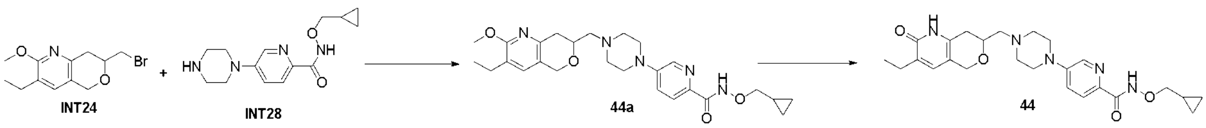

*N*-(Cyclopropylmethoxy)-5-(4-((3-ethyl-2-oxo-1,5,7,8-tetrahydro-2*H*-pyrano[4,3-*b*]pyridin-7-yl)methyl)piperazin-1-yl)-6-fluoropicolinamide was prepared using a synthesis method similar to the preparation of Example 1. LC-MS: ESI [M+H]$^+$ = 486.3; $^1$H NMR (400 MHz, DMSO-$d_6$) δ 11.42 (s, 1H), 8.24 (s, 1H), 7.60 (d, *J* = 8.0 Hz, 1H), 7.41 (s, 1H), 6.79 (s, 1H), 4.28 (s, 2H), 3.87 (s, 1H), 3.44 (d, *J* = 7.2 Hz, 4H), 2.89 (dd, *J* = 7.4, 4.1 Hz, 3H), 2.22 (d, *J* = 11.5 Hz, 3H), 2.12 (q, *J* = 7.3 Hz, 3H), 0.83 (t, *J* = 7.4 Hz, 4H), 0.32 - 0.23 (m, 2H).

## Example 45

(S)-6-Fluoro-N-methyl-5-(4-((2-methyl-3-oxo-3,4,5,6,7,8-hexahydroquinoxaline-6-yl)methyl) piperazin-1-yl)picolinamide

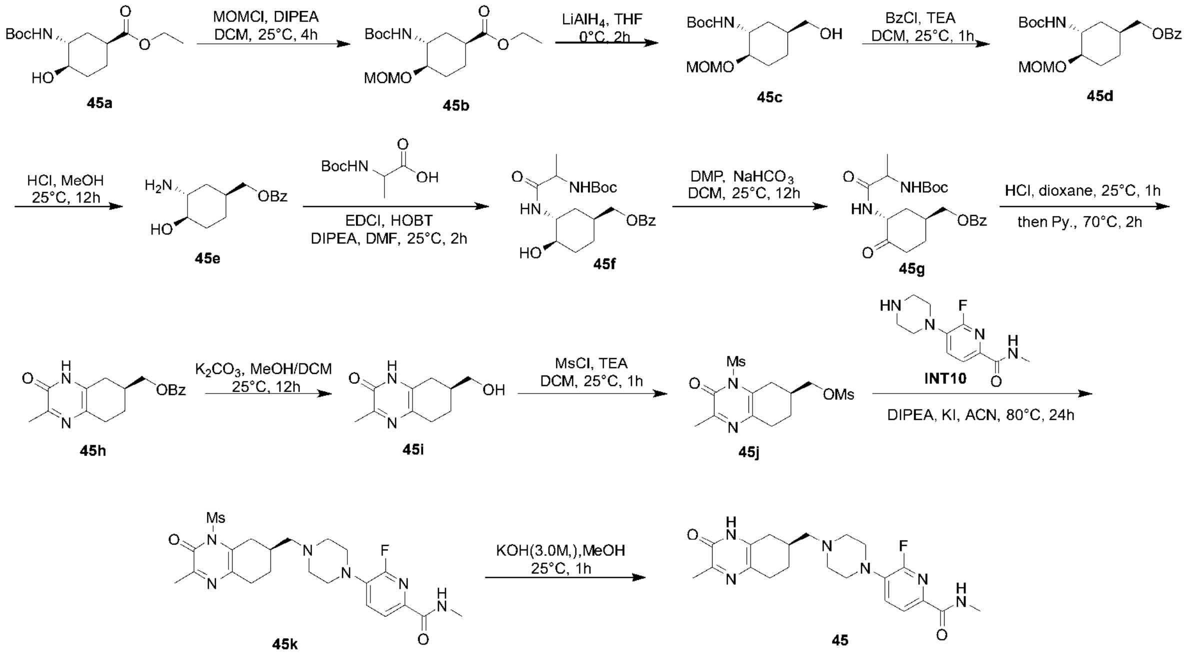


45a 45b 45c 45d 45e 45f 45g 45h 45i 45j INT10 45k 45

Step 1: Compound **45a** (15.0 g, 52.2 mmol) was dissolved in DCM (200 mL), and compound DIPEA (43.0 mL, 261 mmol) was added. MOMCl (15.0 g, 130.5 mmol) was then slowly added dropwise at 0 °C, and the mixture was slowly warmed to 25 °C and stirred for 4 h. The reaction mixture was slowly poured into 200 mL of a saturated sodium bicarbonate solution to quench the reaction, and the resulting mixture was extracted with DCM (4 × 50 mL) and dried over sodium sulfate to obtain compound **45b** (17.0 g, crude).

Step 2: Compound **45b** (17.0 g, 52.2 mmol) was dissolved in anhydrous THF (300 mL), and solid lithium aluminum hydride (3.0 g, 78.3 mmol) was slowly added at 0 °C. The mixture was stirred at 0 °C for 2 h. The reaction was quenched with $H_2O$/THF (1/10, 30 mL), and then NaOH (1.0 M, 20 mL) was added. The mixture was stirred until a solid was precipitated. The mixture was then filtered, and the filtrate was collected and concentrated under reduced pressure to obtain compound **45c**. (13.0 g, 45 mmol, yield: 87%).

Step 3: Compound **45c** (13.0 g, 45.0 mmol) was dissolved in DCM (100 mL), and compound TEA (18.0 mL, 135 mmol) was added. BzCl (7.8 mL, 67.47 mmol) was then slowly added dropwise at 0 °C, and the mixture was slowly warmed to 25 °C and stirred for 14 h. The reaction mixture was slowly poured into 100 mL of a saturated sodium bicarbonate solution to quench the reaction, and the resulting mixture was extracted with DCM (4 × 40 mL), dried over sodium sulfate, and subjected to column chromatography (EA/PE = 1/3) to obtain compound **45d** (17.0 g, 45 mmol, 100%). LC-MS: ESI $[M+H]^+$ = 394.2.

Step 4: Compound **45d** (17.0 g, 45.0 mmol) was dissolved in HCl/MeOH (4.0 M, 30 mL), and the solution was stirred at 25 °C for 12 h. The reaction solution was concentrated under reduced pressure to obtain compound **45e** (12.36 g, 42 mmol, 94%). LC-MS: ESI $[M+H]^+$ = 250.1.

Step 5: Compound **45e** (5.0 g, 17.54 mmol) was dissolved in DMF (50 mL), and 2-((*tert*-butoxycarbonyl)amino) propanoic acid (3.7 g, 19.29 mmol), DIPEA (13.0 mL, 70.16 mmol), and HOBT (2.8 g, 21.05 mmol) were added. The mixture was stirred at 25 °C for 10 min. EDCI (4.0 g, 21.05 mmol) was then added, and the mixture was stirred at 25 °C for another 2 h. The reaction mixture was slowly poured into 100 mL of a saturated ammonium chloride solution to quench the reaction, and the resulting mixture was extracted with EA (4 × 40 mL), dried over sodium sulfate, and purified by column chromatography (EA) to obtain compound **45f** (4.94 g, 11.76 mmol, 67%). LC-MS: ESI $[M+H]^+$ = 421.2.

Step 6: Compound **45f** (4.94 g, 11.76 mmol) was dissolved in DCM (50 mL), and solid sodium bicarbonate (5.0 g, 58.8 mmol) was added. DMP (15.03 g, 35.29 mmol) was then added at 0 °C, and the mixture was stirred at 25 °C for 12 h. The reaction mixture was slowly poured into 200 mL of a saturated sodium bicarbonate solution to quench the reaction, and the resulting mixture was extracted with DCM (4 × 50 mL) and dried over sodium sulfate to obtain compound **45g** (4.0 g, crude). LC-MS: ESI $[M-56+H]^+$ = 363.1.

Step 7: Compound **45g** (4.0 g, 9.57 mmol) was dissolved in HCl/MeOH (4.0 M, 40 mL), and the solution was stirred at 25 °C for 1 h. The reaction solution was concentrated under reduced pressure, and then pyridine (20 mL) was added. The mixture was stirred at 70 °C for 2 h, then concentrated under reduced pressure, and purified by column chromatography (EA) to obtain compound **45h** (1.0 g, 3.34 mmol, 35%). LC-MS: ESI $[M+H]^+$ = 299.1.

Step 8: Compound **45h** (1.0 g, 3.34 mmol) was dissolved in MeOH/DCM (30/5 mL), and potassium carbonate (0.9 g, 6.68 mmol) was added. The mixture was stirred at 25 °C for 12 h. The reaction mixture was concentrated under

reduced pressure, and then a saturated ammonium chloride solution (10 mL) was added to quench the reaction. The mixture was extracted with DCM (4 × 30 mL), dried over sodium sulfate, concentrated under reduced pressure, and purified by Prep-TLC (DCM/MeOH = 5/1) to obtain compound **45i** (0.37 g, crude). LC-MS: ESI $[M+H]^+$ = 195.2.

Step 9: Compound **45i** (0.1 g, 0.51 mmol) was dissolved in DCM (20 mL), and TEA (0.35 mL, 2.55 mmol) and MsCl (0.2 mL, 1.53 mmol) were added dropwise at 0 °C. The mixture was warmed to 25 °C and stirred for 1 h. 20 mL of a saturated sodium bicarbonate solution was slowly poured into the reaction mixture to quench the reaction, and the resulting mixture was extracted with DCM (3 × 30 mL), dried over sodium sulfate, subjected to suction filtration, and concentrated under reduced pressure to obtain compound **45j** (0.17 g, crude). LC-MS: ESI $[M+H]^+$ = 351.2.

Step 10: Compound **45j** (0.17 g, 0.51 mmol) was dissolved in ACN (10 mL), and DIPEA (0.5 mL, 2.80 mmol), KI (0.05 g), and compound **INT10** (0.12 g, 0.51 mmol) were added. The mixture was stirred at 80 °C for 24 h. 20 mL of a saturated ammonium chloride solution was slowly poured into the reaction mixture to quench the reaction, and the resulting mixture was extracted with DCM (4 × 30 mL), dried over sodium sulfate, subjected to suction filtration, concentrated under reduced pressure, and purified by prep-TLC (DCM/MeOH = 20/1) to obtain compound **45k** (0.05 g, 0.10 mmol, 20%). LC-MS: ESI $[M+H]^+$ = 493.2.

Step 11: Compound **45k** (0.05 g, 0.10 mmol) was dissolved in MeOH (5 mL), and KOH (5 mL, 3.0 M) was added. The mixture was stirred at 25 °C for 1 h. 30 mL of a saturated ammonium chloride solution was slowly poured into the reaction mixture to quench the reaction, and the resulting mixture was extracted with EA (4 × 30 mL), dried over sodium sulfate, subjected to suction filtration, concentrated under reduced pressure, and purified by prep-TLC (DCM/MeOH = 15/1) to obtain compound **45** (7.5 mg, 0.018 mmol, yield: 18%) as a white solid. LC-MS: ESI $[M+H]^+$ = 415.1. $^1H$ NMR (400 MHz, $CDCl_3$) δ 7.99 (d, *J* = 7.9 Hz, 1H), 7.49 (d, *J* = 4.8 Hz, 1H), 7.34 - 7.26 (m, 1H), 3.21 (d, *J* = 4.1 Hz, 4H), 2.98 (t, *J* = 9.2 Hz, 3H), 2.79 - 2.53 (m, 7H), 2.42 (s, 3H), 2.38 (d, *J* = 7.1 Hz, 3H), 2.03 (s, 2H), 1.48 (dd, *J* = 22.2, 18.4 Hz, 1H).

**Example 46**

(*S*)-2-Fluoro-*N*-methyl-1'-((2-methyl-3-oxo-3,4,5,6,7,8-hexahydroquinoxalin-6-yl)methyl)-1', 2',3',6'-tetrahydro-[3,4'-bipyridine]-6-carboxamide

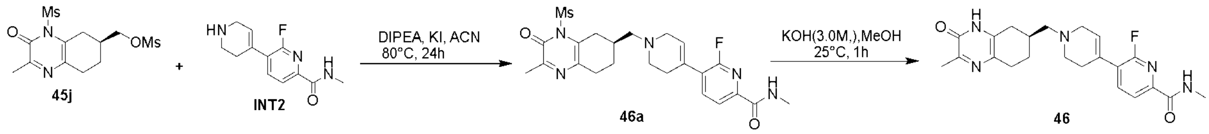


45j + INT2 → 46a → 46

(*S*)-2-Fluoro-*N*-methyl-1'-((2-methyl-3-oxo-3,4,5,6,7,8-hexahydroquinoxalin-6-yl)methyl)-1', 2',3',6'-tetrahydro-[3,4'-bipyridine]-6-carboxamide was prepared using a synthesis method similar to the preparation of Example 45. LC-MS: ESI $[M+H]^+$ = 412.1. $^1H$ NMR (400 MHz, $CDCl_3$) δ 8.05 (d, *J* = 6.7 Hz, 1H), 7.84 (t, *J* = 8.8 Hz, 1H), 7.65 (d, *J* = 5.0 Hz, 1H), 6.21 (s, 1H), 3.21 (s, 2H), 3.02 (d, *J* = 5.0 Hz, 3H), 2.75 - 2.69 (m, 4H), 2.57 (s, 2H), 2.46 (d, *J* = 7.2 Hz, 2H), 2.42 (s, 3H), 2.15 - 1.96 (m, 3H), 1.55 - 1.42(m, 2H).

**Example 47**

(*S*)-*N*,6-Dimethyl-5-(4-((2-methyl-3-oxo-3,4,5,6,7,8-hexahydroquinoxalin-6-yl)methyl)pipera zin-1-yl)picolinamide

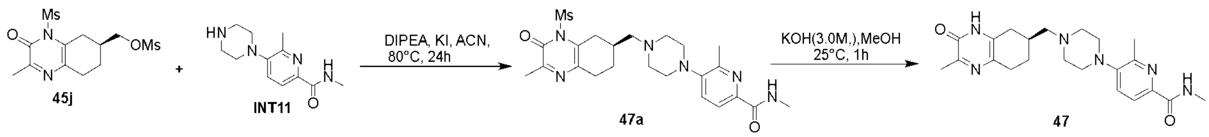


45j + INT11 → 47a → 47

(*S*)-*N*,6-Dimethyl-5-(4-((2-methyl-3-oxo-3,4,5,6,7,8-hexahydroquinoxalin-6-yl)methyl)pipera zin-1-yl)picolinamide was prepared using a synthesis method similar to the preparation of Example 45. LC-MS: ESI $[M+H]^+$ = 411.2. $^1H$ NMR (400 MHz, DMSO) δ 12.22 (s, 1H), 8.59 (q, *J* = 4.5 Hz, 1H), 8.12 - 7.97 (m, 1H), 7.94 - 7.79 (m, 1H), 4.67 (t, *J* = 8.8 Hz, 2H), 3.58 (s, 2H), 3.46 - 3.39 (m, 4H), 2.95 (d, *J* = 11.3 Hz, 2H), 2.77 (d, *J* = 4.8 Hz, 3H), 2.70 (dt, *J* = 11.8, 3.8 Hz, 1H), 2.50 (s, 3H), 2.42 (s, 3H), 2.14 (t, *J* = 10.5 Hz, 2H), 1.82 - 1.59 (m, 4H).

### Example 48

(*S*)-5-(4-((2-Ethyl-3-oxo-3,4,5,6,7,8-hexahydroquinoxalin-6-yl)methyl)piperazin-1-yl)-6-fluor o-*N*-methylpicolinamide

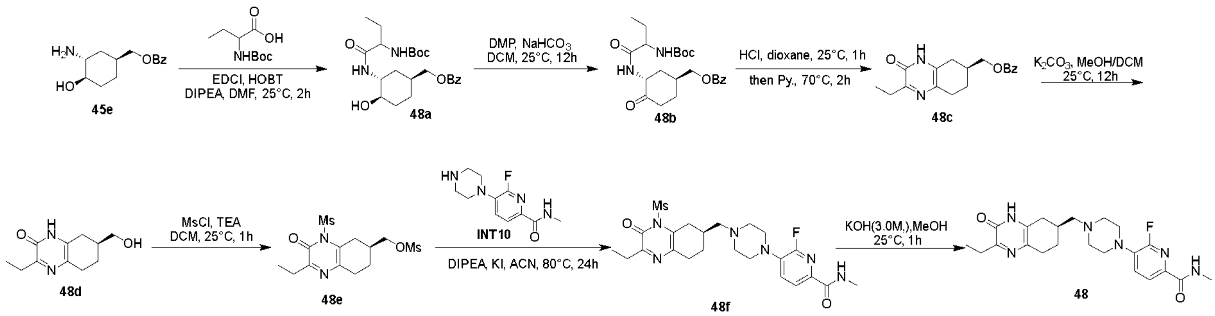


45e 48a 48b 48c 48d 48e INT10 48f 48

(*S*)-5-(4-((2-Ethyl-3-oxo-3,4,5,6,7,8-hexahydroquinoxalin-6-yl)methyl)piperazin-1-yl)-6-fluor o-*N*-methylpicolinamide was prepared using a synthesis method similar to the preparation of Example 45. LC-MS: ESI $[M+H]^+$ = 429.2. $^1$H NMR (400 MHz, DMSO-$d_6$) $\delta$ 11.76 (s, 1H), 8.41 (q, *J* = 4.7 Hz, 1H), 7.85 (dd, *J* = 8.0, 1.4 Hz, 1H), 7.57 (dd, *J* = 10.6, 8.1 Hz, 1H), 3.17 (t, *J* = 5.0 Hz, 4H), 2.77 (d, *J* = 4.8 Hz, 3H), 2.65 - 2.58 (m, 4H), 2.56 (s, 4H), 2.52 (s, 1H), 2.35 - 2.26 (m, 2H), 2.20 (dd, *J* = 17.5, 9.6 Hz, 1H), 2.01 (s, 1H), 1.90 (d, *J* = 12.9 Hz, 1H), 1.45 - 1.33 (m, 1H), 1.10 (t, *J* = 7.5 Hz, 3H).

### Example 49

*N*-(2,2-Difluoroethyl)-5-(4-((3-ethyl-2-oxo-1,5,7,8-tetrahydro-2*H*-pyrano[4,3-*b*]pyridin-7-yl) methyl)piperazin-1-yl)-6-fluoropicolinamide

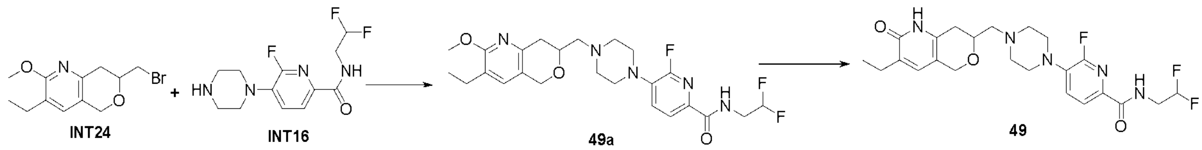

INT24 + INT16 49a 49

*N*-(2,2-Difluoroethyl)-5-(4-((3-ethyl-2-oxo-1,5,7,8-tetrahydro-2*H*-pyrano[4,3-*b*]pyridin-7-yl) methyl)piperazin-1-yl)-6-fluoropicolinamide was prepared using a synthesis method similar to the preparation of Example 1. LC-MS: ESI $[M+H]^+$ = 480.2. $^1$H NMR (400 MHz, DMSO-$d_6$) $\delta$ 11.41 (s, 1H), 8.73 (t, *J* = 6.2 Hz, 1H), 7.89 (dd, *J* = 8.1, 1.4 Hz, 1H), 7.59 (dd, *J* = 10.6, 8.1 Hz, 1H), 7.00 (s, 1H), 6.11 (tt, *J* = 56.2, 4.1 Hz, 1H), 4.51 - 4.37 (m, 2H), 3.89 - 3.82 (m, 1H), 3.72 - 3.63 (m, 2H), 3.17 (d, *J* = 5.2 Hz, 4H), 2.64 (q, *J* = 4.7 Hz, 3H), 2.59 - 2.51 (m, 4H), 2.35 (q, *J* = 7.5 Hz, 2H), 1.06 (t, *J* = 7.4 Hz, 3H).

### Example 50

2-Fluoro-*N*-methyl-1'-((3-methyl-2-oxo-1,5,7,8-tetrahydro-2*H*-pyrano[4,3-*b*]pyridin-7-yl)met hyl)-1',2',3',6'-tetrahydro-[3,4'-bipyridine]-6-carboxamide

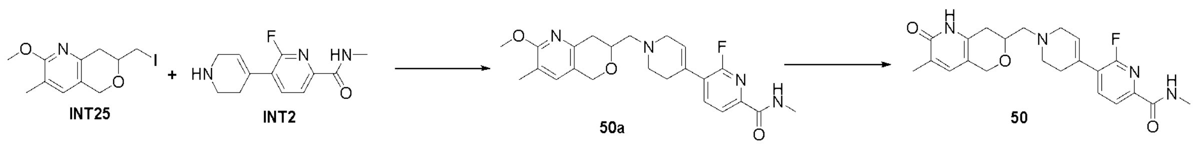

INT25 + INT2 50a 50

2-Fluoro-*N*-methyl-1'-((3-methyl-2-oxo-1,5,7,8-tetrahydro-2*H*-pyrano[4,3-*b*]pyridin-7-yl)met hyl)-1',2',3',6'-tetrahydro-[3,4'-bipyridine]-6-carboxamide was prepared using a synthesis method similar to the preparation of Example

39. LC-MS: ESI $[M+H]^+$ = 413.2. $^1H$ NMR (400 MHz, DMSO-$d_6$) δ 11.41 (s, 1H), 8.64 (d, *J* = 4.9 Hz, 1H), 8.09 (dd, *J* = 9.9, 7.7 Hz, 1H), 7.93 (dd, *J* = 7.7, 1.8 Hz, 1H), 7.05 (d, *J* = 1.2 Hz, 1H), 6.25 (s, 1H), 4.44 (d, *J* = 5.2 Hz, 2H), 3.87 (dd, *J* = 10.0, 5.2 Hz, 1H), 3.22 (q, *J* = 2.9 Hz, 2H), 2.80 (d, *J* = 4.8 Hz, 3H), 2.76 - 2.68 (m, 2H), 2.68 - 2.54 (m, 2H), 2.48 (s, 3H), 2.41 (d, *J* = 17.2 Hz, 1H), 1.94 (s, 3H).

**Example 51**

1'-((3-Cyclopropyl-2-oxo-1,5,7,8-tetrahydro-2*H*-pyrano[4,3-*b*]pyridin-7-yl)methyl)-2-fluoro-*N*-methyl-1',2',3',6'-tetrahydro-[3,4'-bipyridine]-6-carboxamide

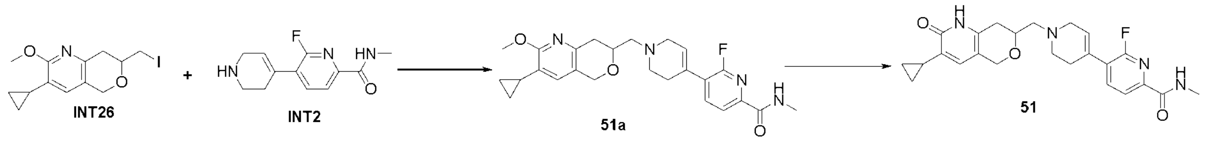

1'-((3-Cyclopropyl-2-oxo-1,5,7,8-tetrahydro-2*H*-pyrano[4,3-*b*]pyridin-7-yl)methyl)-2-fluoro-*N*-methyl-1',2',3',6'-tetrahydro-[3,4'-bipyridine]-6-carboxamide was prepared using a synthesis method similar to the preparation of Example 39. LC-MS: ESI $[M+H]^+$ = 439.2. $^1H$ NMR (400 MHz, DMSO-$d_6$) δ 11.41 (s, 1H), 8.64 (q, *J* = 4.8 Hz, 1H), 8.08 (dd, *J* = 9.9, 7.7 Hz, 1H), 7.92 (dd, *J* = 7.7, 1.8 Hz, 1H), 6.71 (s, 1H), 6.28 - 6.21 (m, 1H), 4.48 - 4.34 (m, 2H), 3.85 (q, *J* = 4.7 Hz, 1H), 3.21 (t, *J* = 3.1 Hz, 2H), 2.80 (d, *J* = 4.8 Hz, 3H), 2.72 (td, *J* = 5.7, 3.9 Hz, 2H), 2.60 (qd, *J* = 12.9, 5.7 Hz, 2H), 2.50 - 2.35 (m, 4H), 1.93 (tt, *J* = 8.4, 5.3 Hz, 1H), 0.86 - 0.72 (m, 2H), 0.64 - 0.50 (m, 2H).

**Example 52**

1'-((3-(Difluoromethyl)-2-oxo-1,5,7,8-tetrahydro-2*H*-pyrano[4,3-*b*]pyridin-7-yl)methyl)-2-flu oro-*N*-methyl-1',2',3',6'-tetrahydro-[3,4'-bipyridine]-6-carboxamide

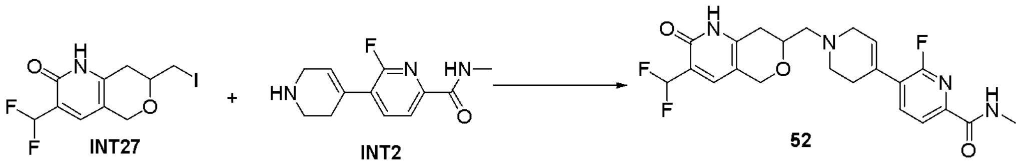

1'-((3-(Difluoromethyl)-2-oxo-1,5,7,8-tetrahydro-2*H*-pyrano[4,3-*b*]pyridin-7-yl)methyl)-2-flu oro-*N*-methyl-1',2',3',6'-tetrahydro-[3,4'-bipyridine]-6-carboxamide was prepared using a synthesis method similar to the preparation of Example 1. LC-MS: ESI $[M+H]^+$ = 449.2; $^1H$ NMR (400 MHz, DMSO-$d_6$) δ 12.02 (s, 1H), 8.64 (q, *J* = 4.8 Hz, 1H), 8.08 (dd, *J* = 9.9, 7.7 Hz, 1H), 7.92 (dd, *J* = 7.7, 1.8 Hz, 1H), 7.52 (s, 1H), 6.81 (t, *J* = 55.3 Hz, 1H), 6.24 (d, *J* = 3.7 Hz, 1H), 4.62 - 4.42 (m, 2H), 3.94 - 3.87 (m, 1H), 3.24 - 3.21 (m, 2H), 2.80 (d, *J* = 4.8 Hz, 3H), 2.77 - 2.71 (m, 2H), 2.70-2.55 (m, 4H), 2.48 (s, 1H).

**Example 53**

(*S*)-1'-((2-Ethyl-3-oxo-3,4,5,6,7,8-hexahydroquinoxalin-6-yl)methyl)-2-fluoro-*N*-methyl-1',2', 3',6'-tetrahydro-[3,4'-bipyridine]-6-carboxamide

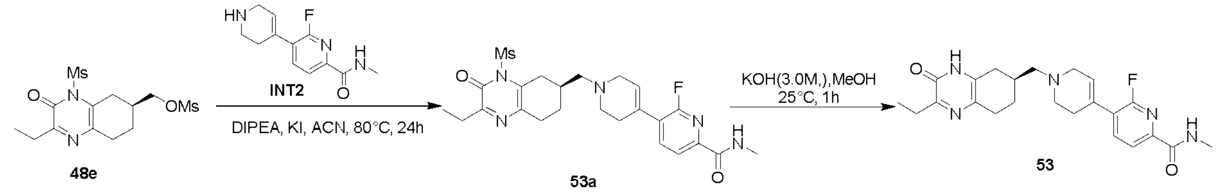


(*S*)-1'-((2-Ethyl-3-oxo-3,4,5,6,7,8-hexahydroquinoxalin-6-yl)methyl)-2-fluoro-*N*-methyl-1',2', 3',6'-tetrahydro-[3,4'-bipyridine]-6-carboxamide was prepared using a synthesis method similar to the preparation of Example 45. LC-MS: ESI $[M+H]^+$ = 426.3; $^1H$ NMR (400 MHz, DMSO-d6) δ 11.78 (s, 1H), 8.64 (q, *J* = 4.8 Hz, 1H), 8.09 (dd, *J* = 9.9, 7.7 Hz, 1H), 7.92 (dd, *J* = 7.7, 1.8 Hz, 1H), 6.26 (d, *J* = 3.7 Hz, 1H), 3.14 (qd, *J* = 17.7, 3.2 Hz, 2H), 2.80 (d, *J* = 4.8 Hz, 3H), 2.66 (d, *J* = 5.8 Hz, 2H), 2.61 (d, *J* = 4.5 Hz, 2H), 2.58 (d, *J* = 7.6 Hz, 4H), 2.51 (s, 1H), 2.40 - 2.30 (m, 2H), 2.21 (dd, *J* = 17.5, 9.7 Hz, 1H), 2.04 (s, 1H), 1.90 (d, *J* = 13.0 Hz, 1H), 1.45 - 1.31 (m, 1H), 1.10 (t, *J* = 7.4 Hz, 3H).

### Example 54

(*S*)-5-(1-((2-Ethyl-3-oxo-3,4,5,6,7,8-hexahydroquinoxalin-6-yl)methyl)piperidin-4-yl)-6-fluor o-*N*-methylpicolinamide

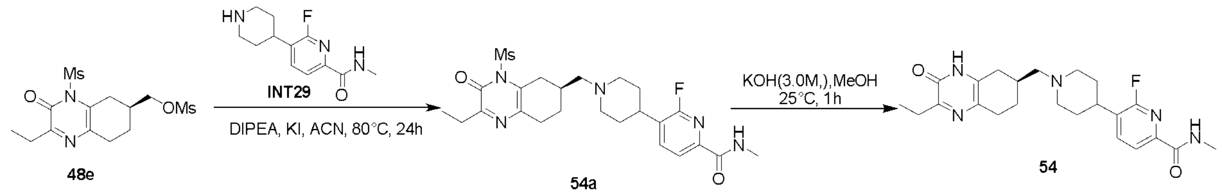


(*S*)-5-(1-((2-Ethyl-3-oxo-3,4,5,6,7,8-hexahydroquinoxaline-6-yl)methyl)piperidin-4-yl)-6-fluo ro-*N*-methylpicolinamide was prepared using a synthesis method similar to the preparation of Example 45. LC-MS: ESI $[M+H]^+$ = 427.2; $^1H$ NMR (400 MHz, DMSO-$d_6$) δ 11.65 (s, 1H), 8.61 (q, *J* = 4.7 Hz, 1H), 8.06 (dd, *J* = 9.7, 7.7 Hz, 1H), 7.91 (dd, *J* = 7.7, 1.6 Hz, 1H), 3.03 - 2.95 (m, 2H), 2.79 (d, *J* = 4.8 Hz, 3H), 2.64 - 2.55 (m, 4H), 2.54 (d, *J* = 4.9 Hz, 1H), 2.33 - 2.22 (m, 2H), 2.22 - 2.15 (m, 1H), 2.08 - 1.96 (m, 3H), 1.95 - 1.86 (m, 1H), 1.82 - 1.65 (m, 4H), 1.43 - 1.29 (m, 1H), 1.10 (t, *J* = 7.5 Hz, 3H).

### Example 55

(*S*)-1'-((2-Ethyl-3-oxo-3,4,5,6,7,8-hexahydroquinoxaline-6-yl)methyl)-*N*-methyl-1',2',3',6'-tetr ahydro-[3,4'-bipyridine]-6-carboxamide

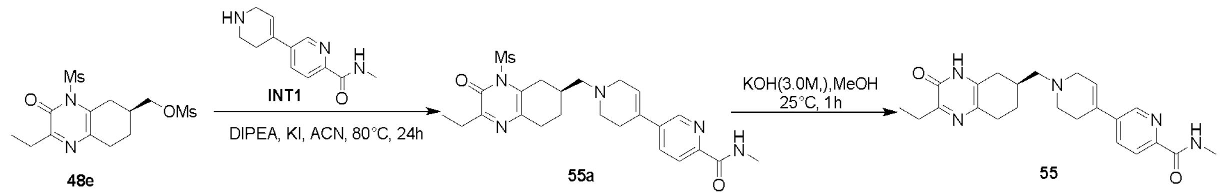


(*S*)-1'-((2-Ethyl-3-oxo-3,4,5,6,7,8-hexahydroquinoxalin-6-yl)methyl)-*N*-methyl-1',2',3',6'-tetra hydro-[3,4'-bipyridine]-6-carboxamide was prepared using a synthesis method similar to the preparation of Example 45. LC-MS: ESI $[M+H]^+$ = 426.1; $^1H$ NMR (400 MHz, DMSO-$d_6$) δ 11.52 (s, 1H), 8.40 (q, *J* = 4.8 Hz, 1H), 8.27 (d, *J* = 2.9 Hz, 1H), 7.83 (d, *J* = 8.8 Hz, 1H), 7.40 (dd, *J* = 8.9, 2.9 Hz, 1H), 3.31 - 3.23 (m, 4H), 2.78 (d, *J* = 4.8 Hz, 3H), 2.67 - 2.61 (m, 1H), 2.59 (d, *J* = 7.5 Hz, 2H), 2.57 - 2.52 (m, 4H), 2.47 (d, *J* = 4.8 Hz, 1H), 2.36 - 2.22 (m, 2H), 2.20 (dd, *J* = 17.4, 9.7 Hz, 1H), 2.01 (s, 1H), 1.96 - 1.86 (m, 1H), 1.37 (ddt, *J* = 12.8, 10.5, 8.1 Hz, 1H), 1.10 (t, *J* = 7.4 Hz, 3H).

### Example 56

(*S*)-1'-((2-Cyclopropyl-3-oxo-3,4,5,6,7,8-hexahydroquinoxaline-6-yl)methyl)-2-fluoro-*N*-met hyl-1',2',3',6'-tetrahydro-[3,4'-bipyridine]-6-carboxamide

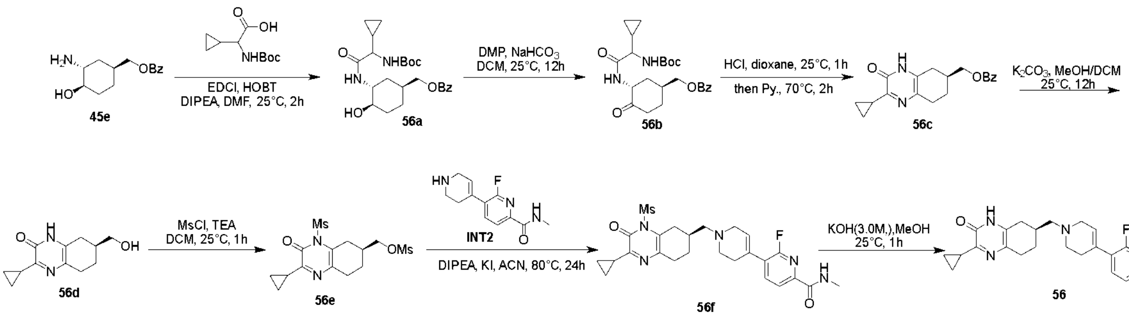


(*S*)-1'-((2-Cyclopropyl-3-oxo-3,4,5,6,7,8-hexahydroquinoxalin-6-yl)methyl)-2-fluoro-*N*-meth yl-1',2',3',6'-tetrahydro-[3,4'-bipyridine]-6-carboxamide was prepared using a synthesis method similar to the preparation of Example 45. LC-MS: ESI $[M+H]^+$ = 438.2; $^1$H NMR (400 MHz, DMSO-d6) δ 11.75 (s, 1H), 8.64 (q, *J* = 4.7 Hz, 1H), 8.09 (dd, *J* = 9.9, 7.7 Hz, 1H), 7.92 (dd, *J* = 7.8, 1.8 Hz, 1H), 6.26 (d, *J* = 3.6 Hz, 1H), 3.22 - 3.06 (m, 2H), 2.80 (d, *J* = 4.8 Hz, 3H), 2.68 (dd, *J* = 11.4, 5.5 Hz, 1H), 2.63 - 2.57 (m, 2H), 2.50 - 2.45 (m, 3H), 2.44 - 2.39 (m, 1H), 2.24 - 2.12 (m, 1H), 2.01 (s, 1H), 1.91 - 1.83 (m, 1H), 1.40 - 1.30 (m, 1H), 0.94 - 0.75 (m, 4H).

**Example 57**

(*S*)-5-(4-((2-Cyclopropyl-3-oxo-3,4,5,6,7,8-hexahydroquinoxaline-6-yl)methyl)piperazin-1-yl )-6-fluoro-*N*-methylpicolinamide

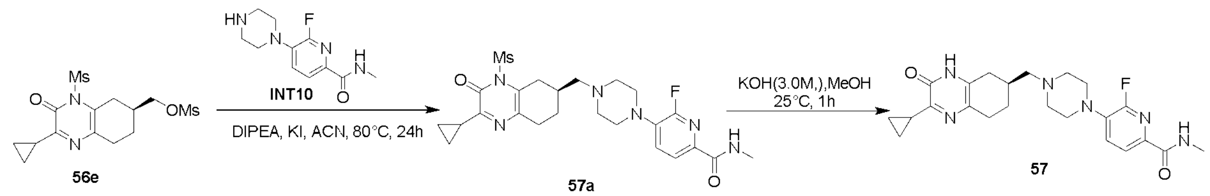


(*S*)-5-(4-((2-Cyclopropyl-3-oxo-3,4,5,6,7,8-hexahydroquinoxalin-6-yl)methyl)piperazin-1-yl) -6-fluoro-*N*-methylpicolinamide was prepared using a synthesis method similar to the preparation of Example 45. LC-MS: ESI $[M+H]^+$ = 441.3; $^1$H NMR (400 MHz, DMSO-$d_6$) δ 8.41 (q, *J* = 4.7 Hz, 1H), 7.85 (dd, *J* = 8.0, 1.4 Hz, 1H), 7.57 (dd, *J* = 10.7, 8.1 Hz, 1H), 3.16 (t, *J* = 5.1 Hz, 4H), 2.77 (d, *J* = 4.8 Hz, 3H), 2.62 (d, *J* = 5.1 Hz, 1H), 2.58 - 2.54 (m, 2H), 2.52 (d, *J* = 2.1 Hz, 2H), 2.49 - 2.45 (m, 2H), 2.42 (dd, *J* = 8.0, 4.9 Hz, 1H), 2.02 - 1.94 (m, 1H), 1.93 - 1.82 (m, 1H), 1.41 - 1.27 (m, 1H), 0.90 - 0.84 (m, 2H), 0.82 - 0.78 (m, 2H).

**Example 58**

(*S*)-5-(4-((2-Cyclopropyl-3-oxo-3,4,5,6,7,8-hexahydroquinoxalin-6-yl)methyl)piperazin-1-yl) -*N*-methylpicolinamide

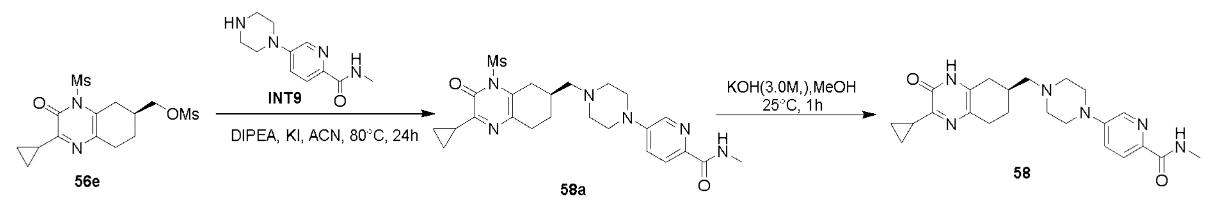

(*S*)-5-(4-((2-Cyclopropyl-3-oxo-3,4,5,6,7,8-hexahydroquinoxaline-6-yl)methyl)piperazin-1-yl )-*N*-methylpicolinamide was prepared using a synthesis method similar to the preparation of Example 45. LC-MS: ESI $[M+H]^+$ = 423.1; $^1H$ NMR (400 MHz, DMSO-$d_6$) δ 11.70 (s, 1H), 8.44 - 8.33 (m, 1H), 8.27 (d, *J* = 2.8 Hz, 1H), 7.83 (d, *J* = 8.7 Hz, 1H), 7.40 (dd, *J* = 8.9, 2.9 Hz, 1H), 3.32 (t, *J* = 5.2 Hz, 4H), 2.78 (d, *J* = 4.8 Hz, 3H), 2.66 - 2.59 (m, 1H), 2.58 - 2.53 (m, 1H), 2.51 (d, *J* = 2.4 Hz, 2H), 2.48 (d, *J* = 7.2 Hz, 5H), 2.42 (dd, *J* = 8.1, 4.9 Hz, 1H), 2.35 - 2.23 (m, 2H), 2.21 - 2.13 (m, 1H), 2.05 - 1.94 (m, 1H), 1.92 - 1.83 (m, 1H), 1.42 - 1.27 (m, 1H), 0.91 - 0.86 (m, 2H), 0.84 - 0.80 (m, 2H).

**Example 59**

6-Fluoro-*N*-methyl-5-(1-((3-methyl-2-oxo-1,5,7,8-tetrahydro-2*H*-pyrano[4,3-*b*]pyridin-7-yl)m ethyl)piperidin-4-yl)picolinamide

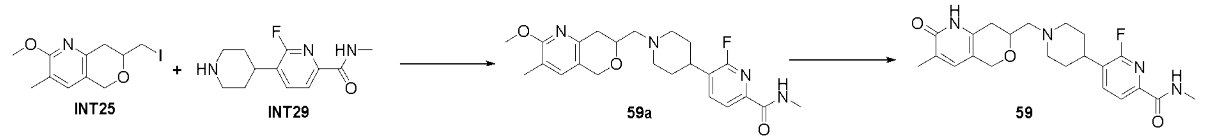

6-Fluoro-*N*-methyl-5-(1-((3-methyl-2-oxo-1,5,7,8-tetrahydro-2*H*-pyrano[4,3-*b*]pyridin-7-yl)m ethyl)piperidin-4-yl)picolinamide was prepared using a synthesis method similar to the preparation of Example 39. LC-MS: ESI $[M+H]^+$ = 415.2; $^1H$ NMR (400 MHz, DMSO-$d_6$) δ 11.40 (s, 1H), 8.61 (q, *J* = 4.8 Hz, 1H), 8.06 (dd, *J* = 9.7, 7.6 Hz, 1H), 7.90 (dd, *J* = 7.6, 1.6 Hz, 1H), 7.03 (d, *J* = 1.3 Hz, 1H), 4.48 - 4.35 (m, 2H), 3.81 (dq, *J* = 10.2, 5.3 Hz, 1H), 3.04 (td, *J* = 7.5, 3.7 Hz, 2H), 2.79 (d, *J* = 4.8 Hz, 3H), 2.59 - 2.53 (m, 1H), 2.47 - 2.36 (m, 1H), 2.14 (dtd, *J* = 14.4, 11.3, 3.4 Hz, 2H), 1.93 (s, 3H), 1.73 (qt, *J* = 8.7, 4.9 Hz, 4H).

**Example 60**

5-(1-((3-Ethyl-2-oxo-1,5,7,8-tetrahydro-2*H*-pyrano[4,3-*b*]pyridin-7-yl)methyl)piperidin-4-yl)-6-fluoro-*N*-methylpicolinamide

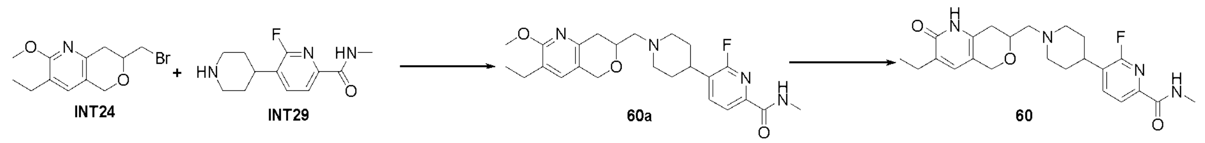

5-(1-((3-Ethyl-2-oxo-1,5,7,8-tetrahydro-2*H*-pyrano[4,3-*b*]pyridin-7-yl)methyl)piperidin-4-yl)-6-fluoro-*N*-methylpicolinamide was prepared using a synthesis method similar to the preparation of Example 39. LC-MS: ESI $[M+H]^+$ = 428.3; $^1H$ NMR (400 MHz, DMSO-$d_6$) δ 11.38 (s, 1H), 8.61 (q, *J* = 4.8 Hz, 1H), 8.06 (dd, *J* = 9.7, 7.6 Hz, 1H), 7.90 (dd, *J* = 7.6, 1.6 Hz, 1H), 6.99 (s, 1H), 4.50 - 4.37 (m, 2H), 3.85 - 3.78 (m, 1H), 3.08 - 3.00 (m, 2H), 2.79 (d, *J* = 4.7 Hz, 3H), 2.76 - 2.70 (m, 1H), 2.58 - 2.52 (m, 1H), 2.50 - 2.41 (m, 1H), 2.35 (q, *J* = 7.3 Hz, 2H), 2.19 - 2.09 (m, 2H), 1.77 - 1.64 (m, 4H), 1.06 (t, *J* = 7.4 Hz, 3H).

**Example 61**

5-(1-((3-Cyclopropyl-2-oxo-1,5,7,8-tetrahydro-2*H*-pyrano[4,3-*b*]pyridin-7-yl)methyl)piperidi n-4-yl)-6-fluoro-*N*-methylpicolinamide

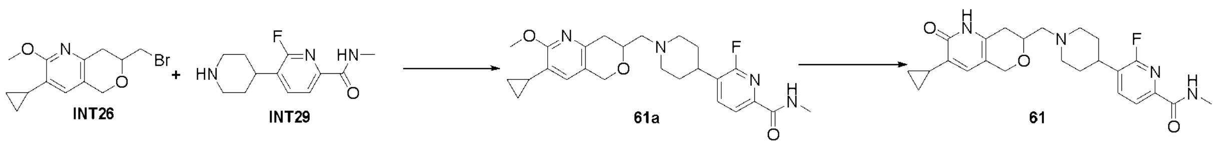

5-(1-((3-Cyclopropyl-2-oxo-1,5,7,8-tetrahydro-2*H*-pyrano[4,3-*b*]pyridin-7-yl)methyl)piperidi n-4-yl)-6-fluoro-*N*-methylpicolinamide was prepared using a synthesis method similar to the preparation of Example 39. LC-MS: ESI $[M+H]^+$ = 441.2; $^1$H NMR (400 MHz, DMSO-$d_6$) δ 11.39 (s, 1H), 8.61 (q, *J* = 4.7 Hz, 1H), 8.06 (dd, *J* = 9.7, 7.7 Hz, 1H), 7.90 (dd, *J* = 7.6, 1.6 Hz, 1H), 6.69 (s, 1H), 4.39 (d, *J* = 8.5 Hz, 2H), 3.80 (dd, *J* = 9.9, 5.0 Hz, 1H), 3.03 (s, 2H), 2.79 (d, *J* = 4.7 Hz, 3H), 2.78 - 2.70 (m, 1H), 2.57 - 2.52 (m, 1H), 2.48 - 2.31 (m, 3H), 2.13 (q, *J* = 11.4 Hz, 2H), 1.97 - 1.90 (m, 1H), 1.73 (d, *J* = 10.0 Hz, 4H), 0.79 (dd, *J* = 8.4, 2.2 Hz, 2H), 0.62 - 0.50 (m, 2H).

**Example 62**

6-Chloro-*N*-cyclopropyl-5-(4-((3-methyl-2-oxo-1,5,7,8-tetrahydro-2*H*-pyrano[4,3-*b*]pyridin-7 -yl)methyl)piperazin-1-yl) picolinamide

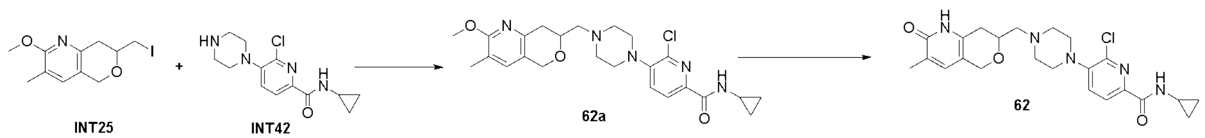

INT25 INT42 62a 62

6-Chloro-*N*-cyclopropyl-5-(4-((3-methyl-2-oxo-1,5,7,8-tetrahydro-2*H*-pyrano[4,3-*b*]pyridin-7 -yl)methyl)piperazin-1-yl)picolinamide was prepared using a synthesis method similar to the preparation of Example 39. LC-MS: ESI $[M+H]^+$ = 458.2; $^1$H NMR (400 MHz, DMSO-d6) δ 11.41 (s, 1H), 8.37 (d, *J* = 4.9 Hz, 1H), 7.93 (d, *J* = 8.1 Hz, 1H), 7.66 (d, *J* = 8.2 Hz, 1H), 7.03 (s, 1H), 4.50 - 4.36 (m, 2H), 3.89 - 3.78 (m, 1H), 3.09 (t, *J* = 4.7 Hz, 4H), 2.91 - 2.80 (m, 1H), 2.71 - 2.59 (m, 4H), 2.60 - 2.51 (m, 2H), 2.49 - 2.40 (m, 2H), 1.93 (s, 3H), 0.72 - 0.63 (m, 4H).

**Example 63**

1'-((3-Chloro-2-oxo-1,5,7,8-tetrahydro-2*H*-pyrano[4,3-*b*]pyridin-7-yl)methyl)-2-fluoro-*N*-met hyl-1',2',3',6'-tetrahydro-[3,4'-bipyridine]-6-carboxamide

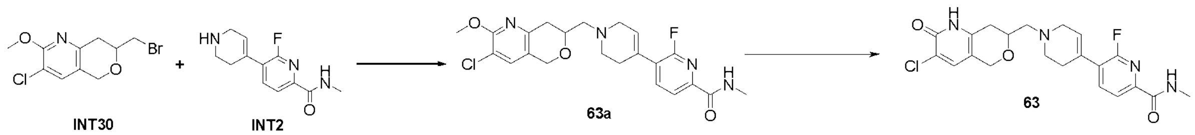

INT30 INT2 63a 63

1'-((3-Chloro-2-oxo-1,5,7,8-tetrahydro-2*H*-pyrano[4,3-*b*]pyridin-7-yl)methyl)-2-fluoro-*N*-met hyl-1',2',3',6'-tetrahydro-[3,4'-bipyridine]-6-carboxamide was prepared using a synthesis method similar to the preparation of Example 39. LC-MS: ESI $[M+H]^+$ = 433.1; $^1$H NMR (400 MHz, DMSO-$d_6$) δ 12.08 (s, 1H), 8.64 (q, *J* = 4.8 Hz, 1H), 8.12 - 8.04 (m, 1H), 7.92 (dd, *J* = 7.7, 1.8 Hz, 1H), 7.54 (s, 1H), 6.28 - 6.21 (m, 1H), 4.56 - 4.36 (m, 2H), 3.91 - 3.85 (m, 1H), 3.22 - 3.21 (m, 2H), 2.79 (d, *J* = 4.8 Hz, 3H), 2.75 - 2.67 (m, 4H), 2.66 - 2.50 (m, 4H).

**Example 64**

5-(4-((3-Chloro-2-oxo-1,5,7,8-tetrahydro-2*H*-pyrano[4,3-*b*]pyridin-7-yl)methyl)piperazin-1-y l)-6-fluoro-*N*-methylpicolinamide

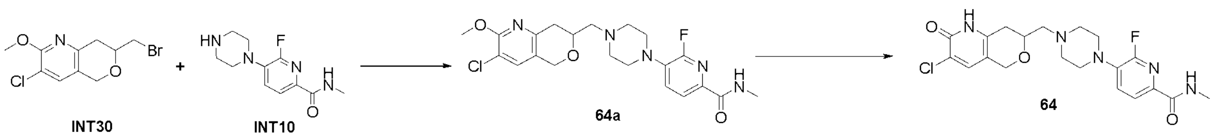

INT30 INT10 64a 64

5-(4-((3-Chloro-2-oxo-1,5,7,8-tetrahydro-2*H*-pyrano[4,3-*b*]pyridin-7-yl)methyl)piperazin-1-y l)-6-fluoro-*N*-methylpicolinamide was prepared using a synthesis method similar to the preparation of Example 39. LC-MS: ESI

[M+H]$^+$ = 436.1; $^1$H NMR (400 MHz, DMSO-$d_6$) δ 12.08 (s, 1H), 8.40 (q, *J* = 4.7 Hz, 1H), 7.84 (dd, *J* = 8.0, 1.4 Hz, 1H), 7.61 - 7.51 (m, 2H), 4.54 - 4.38 (m, 2H), 3.88 - 3.82 (m, 1H), 3.15 (t, *J* = 4.9 Hz, 4H), 2.76 (d, *J* = 4.8 Hz, 3H), 2.63 (q, *J* = 4.5 Hz, 4H), 2.56 (d, *J* = 6.3 Hz, 1H), 2.55 - 2.52 (m, 2H), 2.47 - 2.40 (m, 1H).

**Examples 65 and 66**

(*S*)-5-(4-((3-Ethyl-2-oxo-1,5,7,8-tetrahydro-2*H*-pyrano[4,3-*b*]pyridin-7-yl)methyl)piperazin-1 -yl)-6-fluoro-*N*-methylpicolinamide and (*R*)-5-(4-((3-ethyl-2-oxo-1,5,7,8-tetrahydro-2*H*-pyrano[4,3-*b*]pyridin-7-yl)methyl)piperazin-1 -yl)-6-fluoro-*N*-methylpicolinamide

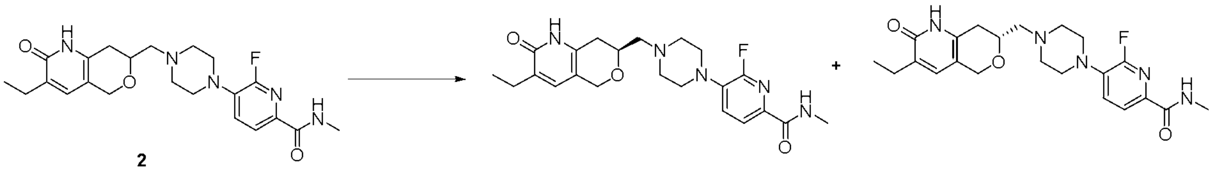

2

Compound **2** was separated and purified by preparative supercritical fluid chromatography (SFC) (column: CHIRALPAK IE 250 mm × 20 mm, 5 μm, 40% EtOH (NHOH 0.2%) to obtain two compounds **65** and **66**:

**Example 65** ($t_R$ = 3.489), MS/ESI [M+H]$^+$ = 430.2; $^1$H NMR (400 MHz, DMSO-$d_6$) δ 11.40 (s, 1H), 8.40 (q, *J* = 4.7 Hz, 1H), 7.85 (dd, *J* = 8.1, 1.4 Hz, 1H), 7.57 (dd, *J* = 10.6, 8.1 Hz, 1H), 6.99 (s, 1H), 4.51 - 4.37 (m, 2H), 3.90 - 3.79 (m, 1H), 3.16 (t, *J* = 4.9 Hz, 4H), 2.77 (d, *J* = 4.8 Hz, 3H), 2.64 (q, *J* = 4.8 Hz, 4H), 2.61 - 2.40 (m, 4H), 2.35 (q, *J* = 7.5 Hz, 2H), 1.07 (t, *J* = 7.4 Hz, 3H).

**Example 66** ($t_R$ = 4.696), MS/ESI [M+H]$^+$ = 430.2; $^1$H NMR (400 MHz, DMSO-$d_6$) δ 11.40 (s, 1H), 8.40 (q, *J* = 4.7 Hz, 1H), 7.85 (dd, *J* = 8.1, 1.4 Hz, 1H), 7.57 (dd, *J* = 10.6, 8.1 Hz, 1H), 6.99 (s, 1H), 4.51 - 4.37 (m, 2H), 3.90 - 3.79 (m, 1H), 3.16 (t, *J* = 4.9 Hz, 4H), 2.77 (d, *J* = 4.8 Hz, 3H), 2.64 (q, *J* = 4.8 Hz, 4H), 2.61 - 2.40 (m, 4H), 2.35 (q, *J* = 7.5 Hz, 2H), 1.07 (t, *J* = 7.4 Hz, 3H).

**Example 67**

6-Fluoro-*N*-methyl-5-(4-((2-oxo-3-vinyl-1,5,7,8-tetrahydro-2*H*-pyrano[4,3-*b*]pyridin-7-yl)met hyl)piperazin-1-yl)picolinamide

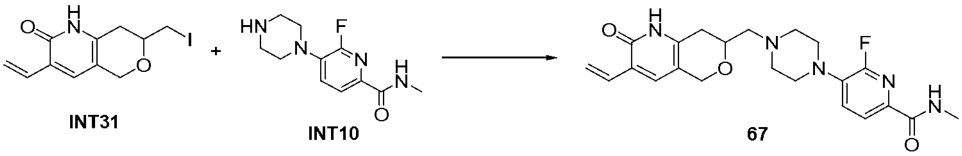

INT31 INT10 67

6-Fluoro-*N*-methyl-5-(4-((2-oxo-3-vinyl-1,5,7,8-tetrahydro-2*H*-pyrano[4,3-*b*]pyridin-7-yl)met hyl)piperazin-1-yl) picolinamide was prepared using a synthesis method similar to the preparation of Example 1. LC-MS: ESI [M+H]$^+$ = 427.2; $^1$H NMR (400 MHz, DMSO-$d_6$) δ 11.67 (s, 1H), 8.41 (d, *J* = 4.9 Hz, 1H), 7.85 (dd, *J* = 8.0, 1.5 Hz, 1H), 7.57 (dd, *J* = 10.6, 8.1 Hz, 1H), 7.34 (s, 1H), 6.65 (dd, *J* = 17.7, 11.4 Hz, 1H), 6.06 (dd, *J* = 17.7, 2.2 Hz, 1H), 5.20 (dd, *J* = 11.3, 2.2 Hz, 1H), 4.48 (d, *J* = 13.8 Hz, 2H), 3.86 (d, *J* = 5.1 Hz, 1H), 3.16 (t, *J* = 4.9 Hz, 4H), 2.77 (d, *J* = 4.8 Hz, 3H), 2.69 - 2.59 (m, 4H), 2.58 - 2.52 (m, 4H).

**Example 68**

2-Fluoro-*N*-methyl-1'-((2-oxo-3-vinyl-1,5,7,8-tetrahydro-2*H*-pyrano[4,3-*b*]pyridin-7-yl)methy l)-1',2',3',6'-tetrahydro-[3,4'-bipyridine]-6-carboxamide

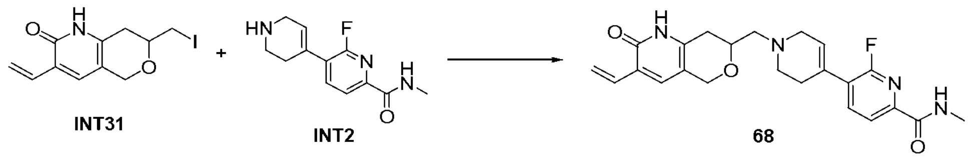

INT31 INT2 68

2-Fluoro-*N*-methyl-1'-((2-oxo-3-vinyl-1,5,7,8-tetrahydro-2*H*-pyrano[4,3-*b*]pyridin-7-yl)methy l)-1',2',3',6'-tetrahydro-[3,4'-bipyridine]-6-carboxamide was prepared using a synthesis method similar to the preparation of Example 1. LC-MS: ESI $[M+H]^+$ = 425.2.

**Example 69**

2-Chloro-1'-((3-cyclopropyl-2-oxo-1,5,7,8-tetrahydro-2*H*-pyrano[4,3-*b*]pyridin-7-yl)methyl)-*N*-methyl-1',2',3',6'-tetrahydro-[3,4'-bipyridine]-6-carboxamide

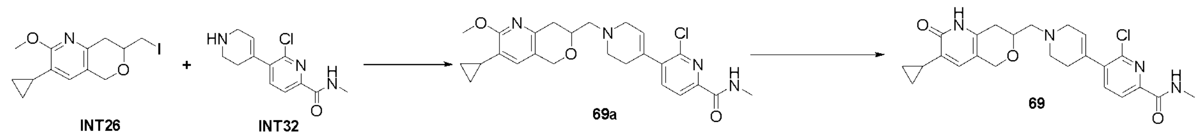

INT26 INT32 69a 69

2-Chloro-1'-((3-cyclopropyl-2-oxo-1,5,7,8-tetrahydro-2*H*-pyrano[4,3-*b*]pyridin-7-yl)methyl)-*N*-methyl-1',2',3',6'-tetrahydro-[3,4'-bipyridine]-6-carboxamide was prepared using a synthesis method similar to the preparation of Example 39. LC-MS: ESI $[M+H]^+$ = 455.1; $^1H$ NMR (400 MHz, DMSO-$d_6$) δ 11.41 (s, 1H), 8.61 (d, *J* = 4.9 Hz, 1H), 7.97 (d, *J* = 7.7 Hz, 1H), 7.89 (d, *J* = 7.7 Hz, 1H), 6.71 (s, 1H), 5.85 (s, 1H), 4.41 (d, *J* = 6.3 Hz, 2H), 3.91 - 3.80 (m, 1H), 3.20 - 3.17 (m, 2H), 2.81 (d, *J* = 4.8 Hz,3H), 2.77 - 2.53 (m, 4H), 2.49 - 2.36 (m, 4H), 1.96 - 1.91 (m, 1H), 0.82 - 0.78 (m, 2H), 0.59 - 0.55 (m, 2H).

**Example 70**

2-Chloro-*N*-methyl-1'-((3-methyl-2-oxo-1,5,7,8-tetrahydro-2*H*-pyrano[4,3-*b*]pyridin-7-yl)met hyl)-1',2',3',6'-tetrahydro-[3,4'-bipyridine]-6-carboxamide

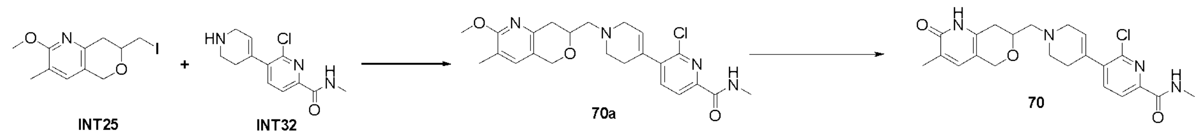

INT25 INT32 70a 70

2-Chloro-*N*-methyl-1'-((3-methyl-2-oxo-1,5,7,8-tetrahydro-2*H*-pyrano[4,3-*b*]pyridin-7-yl)met hyl)-1',2',3',6'-tetrahydro-[3,4'-bipyridine]-6-carboxamide was prepared using a synthesis method similar to the preparation of Example 39. LC-MS: ESI $[M+H]^+$ = 429.2; $^1H$ NMR (400 MHz, DMSO-$d_6$) δ 11.51 (s, 1H), 8.70 (d, *J* = 4.9 Hz, 1H), 8.12 - 7.91 (m, 2H), 7.13 (d, *J* = 1.3 Hz, 1H), 5.94 (d, *J* = 1.7 Hz, 1H), 4.63 - 4.43 (m, 2H), 4.02 - 3.89 (m, 1H), 3.29 - 3.27 (m, 2H), 2.95 (t, *J* = 5.1 Hz, 1H), 2.90 (d, *J* = 4.8 Hz, 3H), 2.85 - 2.64 (m, 4H), 2.58 - 2.47 (m, 4H), 2.03 (d, *J* = 3.1 Hz, 3H).

**Example 71**

(*S*)-6-Fluoro-*N*-methyl-5-(4-(1-(2-methyl-3-oxo-3,4,5,6,7,8-hexahydroquinoxalin-6-yl)ethyl)p iperazin-1-yl)picolinamide

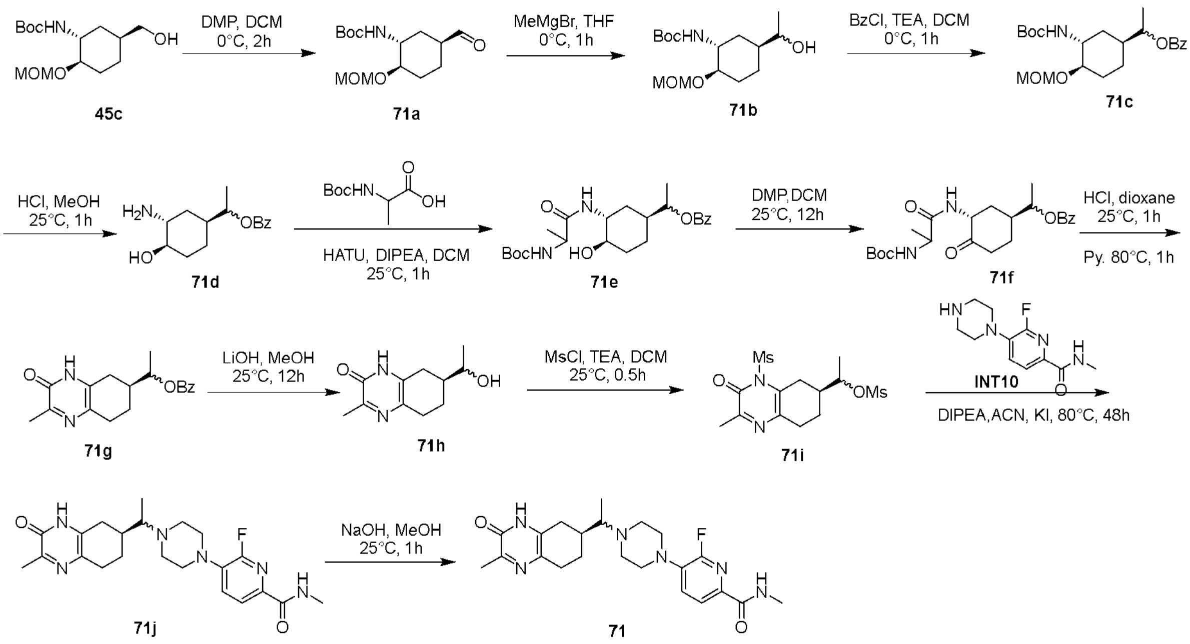


Step 1: Compound **45c** (25.0 g, 87.1 mmol) was dissolved in DCM (500 mL), and DMP (74.0 g, 35.29 mmol) was added at 0 °C. The mixture was stirred at 25 °C for 2 h. The reaction mixture was slowly poured into 500 mL of a saturated sodium bicarbonate solution to quench the reaction, and the resulting mixture was extracted with DCM (5 × 50 mL), dried over sodium sulfate, and purified by column chromatography (EA/PE = 1:1) to obtain compound **71a** (21.0 g, 73.6 mmol, 85%).

Step 2: Compound **71a** (21.0 g, 73.6 mmol) was dissolved in anhydrous THF (500 mL), and methylmagnesium bromide (61.0 mL, 184.0 mmol, 3.0 M) was slowly added at 0 °C. The mixture was stirred at 0 °C for 1 h. The reaction mixture was slowly added dropwise to a saturated ammonium chloride solution, and the resulting mixture was extracted with EA (5 × 50 mL), dried over sodium sulfate, and concentrated under reduced pressure to obtain compound **71b** (20.0 g, crude).

Step 3: Compound **71b** (20.0 g, 73.6 mmol) was dissolved in DCM (100 mL), and compound TEA (28.0 mL, 220.8 mmol) was added. BzCl (9 mL, 73.6 mmol) was then slowly added dropwise at 0 °C, and the mixture was slowly warmed to 25 °C and stirred for 1 h. The reaction mixture was slowly poured into 100 mL of a saturated sodium bicarbonate solution to quench the reaction, and the resulting mixture was extracted with DCM (4 × 40 mL), dried over sodium sulfate, and subjected to column chromatography (EA/PE = 1/3) to obtain compound **71c** (26.0 g, crude). LC-MS: ESI $[M+Na]^+$ = 430.2.

Step 4: Compound **71c** (26.0 g, 64.0 mmol) was dissolved in HCl/MeOH (4.0 M, 50 mL), and the solution was stirred at 25 °C for 1 h. The reaction solution was concentrated under reduced pressure to obtain compound **71d** (17 g, 64.0 mmol, 100%). LC-MS: ESI $[M+H]^+$ = 264.2.

Step 5: Compound **71d** (17.0 g, 64.0 mmol) was dissolved in DCM (300 mL), and 2-((*tert*-butoxycarbonyl)amino) propanoic acid (14.0 g, 71.59 mmol), DIPEA (53.0 mL, 325 mmol), and HATU (37.0 g, 97.5 mmol) were added. The mixture was stirred at 25 °C for 1 h. The reaction mixture was slowly poured into 200 mL of a saturated ammonium chloride solution to quench the reaction, and the resulting mixture was extracted with EA (4 × 50 mL), dried over sodium sulfate, and subjected to column chromatography (EA) to obtain compound **71e** (13.0 g, 29.88 mmol, 47%). LC-MS: ESI $[M+H]^+$ = 435.2.

Step 6: Compound **71e** (13.0 g, 29.88 mmol) was dissolved in DCM (500 mL), and DMP (38.0 g, 89.65 mmol) was added at 0 °C. The mixture was stirred at 25 °C for 12 h. The reaction mixture was slowly poured into 500 mL of a saturated sodium bicarbonate solution to quench the reaction, and the resulting mixture was extracted with DCM (4 × 50 mL), dried over sodium sulfate, and subjected to column chromatography (EA/PE = 1/1) to obtain compound **71f** (5.5 g, 12.73 mmol, 43%). LC-MS: ESI $[M+H]^+$ = 433.2.

Step 7: Compound **71f** (5.5 g, 12.7 mmol) was dissolved in HCl/MeOH (4.0 M, 40 mL), and the solution was stirred at 25 °C for 1 h. The reaction solution was concentrated under reduced pressure, and then pyridine (20 mL) was added. The mixture was stirred at 80 °C for 1 h, then concentrated under reduced pressure, and subjected to column chromatography (DCM/MeOH = 50/1) to obtain compound **71g** (1.8 g, 5.75 mmol, 45.3%). LC-MS: ESI $[M+H]^+$ = 313.2.

Step 8: Compound **71g** (1.8 g, 5.75 mmol) was dissolved in MeOH (30 mL), and lithium hydroxide (0.4 g, 17.25 mmol) was added. The mixture was stirred at 25 °C for 12 h. The reaction mixture was concentrated under reduced pressure, and then a saturated ammonium chloride solution (10 mL) was added to quench the reaction. The mixture was extracted with DCM (4 × 30 mL), dried over sodium sulfate, concentrated under reduced pressure, and purified by column

chromatography (DCM/MeOH = 20/1) to obtain compound **71h** (0.7 g). LC-MS: ESI $[M+H]^+$ = 209.2.

Step 9: Compound **71h** (0.4 g, 1.92 mmol) was dissolved in DCM (20 mL), and TEA (1.4 mL, 9.6 mmol) and MsCl (0.65 mL, 5.76 mmol) were added dropwise at 0 °C. The mixture was warmed to 25 °C and stirred for 0.5 h. 20 mL of a saturated sodium bicarbonate solution was slowly poured into the reaction mixture to quench the reaction, and the resulting mixture was extracted with DCM (3 × 30 mL), dried over sodium sulfate, subjected to suction filtration, and concentrated under reduced pressure to obtain compound **71i** (0.7 g, crude). LC-MS: ESI $[M+H]^+$ = 365.2.

Step 10: Compound **71i** (0.7 g, 1.92 mmol) was dissolved in ACN (10 mL), and DIPEA (1.3 mL, 7.80 mmol), KI (0.05 g), and compound **8** (0.5 g, 1.92 mmol) were added. The mixture was stirred at 80 °C for 24 h. 20 mL of a saturated ammonium chloride solution was slowly poured into the reaction mixture to quench the reaction, and the resulting mixture was extracted with DCM (4 × 30 mL), dried over sodium sulfate, subjected to suction filtration, concentrated under reduced pressure, and purified by column chromatography (DCM/MeOH = 20/1) to obtain compound **71j** (1.1 g, crude). LC-MS: ESI $[M+H]^+$ = 507.2.

Step 11: Compound **71j** (1.1 g, 2.17 mmol) was dissolved in MeOH (10 mL), and NaOH (10 mL, 2.0 M) was added. The mixture was stirred at 25 °C for 1 h. 30 mL of a saturated ammonium chloride solution was slowly poured into the reaction mixture to quench the reaction, and the resulting mixture was extracted with EA (4 × 30 mL), dried over sodium sulfate, subjected to suction filtration, concentrated under reduced pressure, and purified by prep-TLC (DCM/MeOH = 10/1) to obtain compound **71** (5 mg) as a white solid. LC-MS: ESI $[M+H]^+$ = 429.2; $^1$H NMR (400 MHz, CDCl3) δ 7.99 (d, *J* = 7.9 Hz, 1H), 7.50 (d, *J* = 4.8 Hz, 1H), 7.35 - 7.29 (m, 1H), 3.21 (s, 4H), 2.99 (d, *J* = 4.9 Hz, 3H), 2.78 (d, *J* = 4.2 Hz, 4H), 2.68 - 2.50 (m, 4H), 2.39 (s, 3H), 2.02 (s, 2H), 1.83 (s, 2H), 1.07 (d, J = 6.3 Hz, 3H).

**Example 72**

5-((3*S*)-4-((3-Ethyl-2-oxo-1,5,7,8-tetrahydro-2*H*-pyrano[4,3-*b*]pyridin-7-yl)methyl)-3-methyl piperazin-1-yl)-6-fluoro-*N*-methylpicolinamide

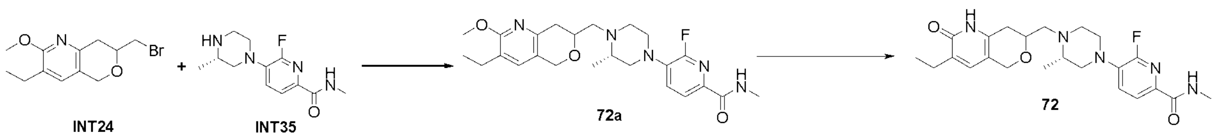

5-((3*S*)-4-((3-Ethyl-2-oxo-1,5,7,8-tetrahydro-2*H*-pyrano[4,3-*b*]pyridin-7-yl)methyl)-3-methyl piperazin-1-yl)-6-fluoro-*N*-methylpicolinamide was prepared using a synthesis method similar to the preparation of Example 39. LC-MS: ESI $[M+H]^+$ = 444.2; $^1$H NMR (400 MHz, DMSO-$d_6$) δ 11.37 (s, 1H), 8.40 (q, *J* = 4.8 Hz, 1H), 7.85 (d, *J* = 7.9 Hz, 1H), 7.57 (dd, *J* = 10.6, 8.1 Hz, 1H), 6.99 (s, 1H), 4.57 - 4.38 (m, 2H), 3.83 (d, *J* = 5.6 Hz, 1H), 3.32 (s, 3H), 3.05 - 2.93 (m, 2H), 2.92 - 2.82 (m, 1H), 2.77 (d, *J* = 4.7 Hz, 3H), 2.72 - 2.53 (m, 4H), 2.47 - 2.43 (m, 1H), 2.35 (q, *J* = 7.3 Hz, 2H), 1.13 - 1.01 (m, 6H).

**Example 73**

6-Chloro-5-(4-((3-cyclopropyl-2-oxo-1,5,7,8-tetrahydro-2*H*-pyrano[4,3-*b*]pyridin-7-yl)methyl )piperazin-1-yl)-*N*-methylpicolinamide

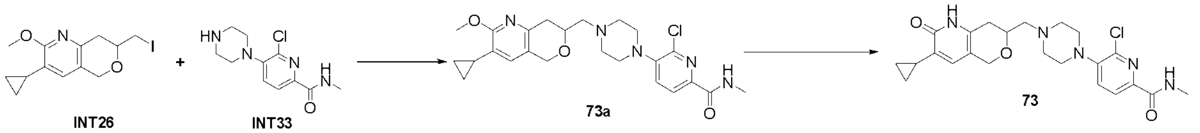

6-Chloro-5-(4-((3-cyclopropyl-2-oxo-1,5,7,8-tetrahydro-2*H*-pyrano[4,3-*b*]pyridin-7-yl)methyl )piperazin-1-yl)-*N*-methylpicolinamide was prepared using a synthesis method similar to the preparation of Example 39. LC-MS: ESI $[M+H]^+$ = 458.3; $^1$H NMR (400 MHz, DMSO-$d_6$) δ 11.43 (s, 1H), 8.44 (q, *J* = 4.8 Hz, 1H), 7.94 (d, *J* = 8.2 Hz, 1H), 7.67 (d, *J* = 8.2 Hz, 1H), 6.71 (s, 1H), 4.49 - 4.27 (m, 2H), 3.87 - 3.81 (m, 1H), 3.11 (t, *J* = 4.8 Hz, 4H), 2.80 (d, *J* = 4.8 Hz, 3H), 2.73 - 2.64 (m, 4H), 2.62 - 2.54 (m, 2H), 2.48 - 2.37 (m, 2H), 1.94 (tt, *J* = 8.5, 5.3 Hz, 1H), 0.89 - 0.70 (m, 2H), 0.62 - 0.47 (m, 2H).

**Example 74**

6-Chloro-*N*-methyl-5-(4-((3-methyl-2-oxo-1,5,7,8-tetrahydro-2*H*-pyrano[4,3-*b*]pyridin-7-yl) methyl)piperazin-1-yl)picolinamide

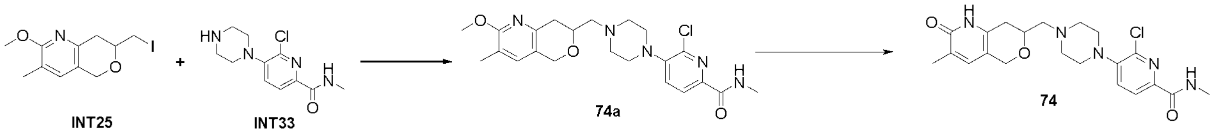

INT25 INT33 74a 74

6-Chloro-*N*-methyl-5-(4-((3-methyl-2-oxo-1,5,7,8-tetrahydro-2*H*-pyrano[4,3-*b*]pyridin-7-yl) methyl)piperazin-1-yl)picolinamide was prepared using a synthesis method similar to the preparation of Example 39. LC-MS: ESI $[M+H]^+$ = 432.2; $^1H$ NMR (400 MHz, DMSO-$d_6$) δ 11.42 (s, 1H), 8.44 (q, *J* = 4.7 Hz, 1H), 7.94 (d, *J* = 8.2 Hz, 1H), 7.67 (d, *J* = 8.2 Hz, 1H), 7.04 (s, 1H), 4.50 - 4.37 (m, 2H), 3.89 - 3.79 (m, 1H), 3.10 (t, *J* = 4.7 Hz, 4H), 2.80 (d, *J* = 4.8 Hz, 3H), 2.72 - 2.62 (m, 4H), 2.61 - 2.53 (m, 2H), 2.46 (t, *J* = 7.9 Hz, 2H), 1.94 (s, 3H).

**Example 75**

5-((3*R*)-4-((3-Ethyl-2-oxo-1,5,7,8-tetrahydro-2*H*-pyrano[4,3-*b*]pyridin-7-yl)methyl)-3-methyl piperazin-1-yl)-6-fluoro-*N*-methylpicolinamide

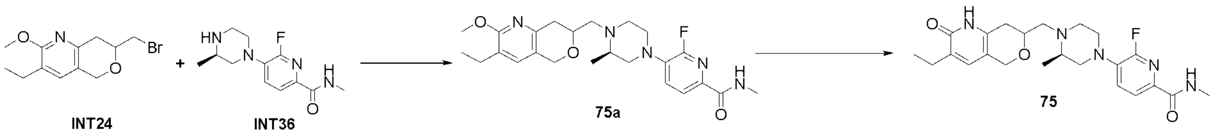

INT24 INT36 75a 75

5-((3*R*)-4-((3-Ethyl-2-oxo-1,5,7,8-tetrahydro-2*H*-pyrano[4,3-*b*]pyridin-7-yl)methyl)-3-methyl piperazin-1-yl)-6-fluoro-*N*-methylpicolinamide was prepared using a synthesis method similar to the preparation of Example 39. LC-MS: ESI $[M+H]^+$ = 444.2; $^1H$ NMR (400 MHz, DMSO-$d_6$) δ 11.38 (s, 1H), 8.40 (q, *J* = 4.8 Hz, 1H), 7.84 (d, *J* = 7.9 Hz, 1H), 7.56 (dd, *J* = 10.6, 8.5 Hz, 1H), 6.99 (s, 1H), 4.53 - 4.35 (m, 2H), 3.87 - 3.77 (m, 1H), 3.04 - 2.83 (m, 3H), 2.77 (d, *J* = 4.8 Hz, 3H), 2.74 - 2.54 (m, 3H), 2.49 - 2.40 (m, 2H), 2.35 (q, *J* = 7.3 Hz, 2H), 1.16 - 0.99 (m, 6H).

**Example 76**

6-Cyano-*N*-methyl-5-(4-((3-methyl-2-oxo-1,5,7,8-tetrahydro-2*H*-pyrano[4,3-*b*]pyridin-7-yl)m ethyl)piperazin-1-yl)picolinamide

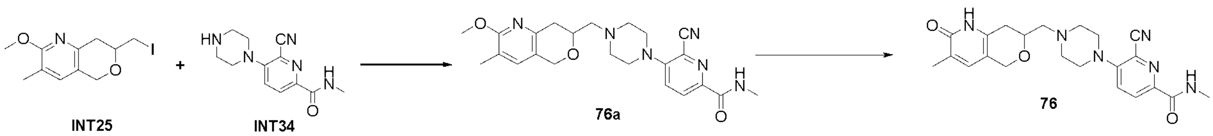

INT25 INT34 76a 76

6-Cyano-*N*-methyl-5-(4-((3-methyl-2-oxo-1,5,7,8-tetrahydro-2*H*-pyrano[4,3-*b*]pyridin-7-yl)m ethyl)piperazin-1-yl)picolinamide was prepared using a synthesis method similar to the preparation of Example 39. LC-MS: ESI $[M+H]^+$ = 423.3; $^1H$ NMR (400 MHz, DMSO-$d_6$) δ 11.42 (s, 1H), 8.57 (q, *J* = 4.7 Hz, 1H), 8.11 (d, *J* = 8.8 Hz, 1H), 7.74 (d, *J* = 8.9 Hz, 1H), 7.03 (d, *J* = 1.3 Hz, 1H), 4.49 - 4.36 (m, 2H), 3.90 - 3.79 (m, 1H), 3.36 (d, *J* = 2.7 Hz, 4H), 2.79 (d, *J* = 4.8 Hz, 3H), 2.73 - 2.63 (m, 4H), 2.62 - 2.53 (m, 2H), 2.49 - 2.36 (m, 2H), 1.93 (s, 3H).

**Example 77**

6-Fluoro-*N*-(1-methyl-1*H*-pyrazol-4-yl)-5-(4-((3-methyl-2-oxo-1,5,7,8-tetrahydro-2*H*-pyrano[ 4,3-*b*]pyridin-7-yl)methyl) piperazin-1-yl)picolinamide

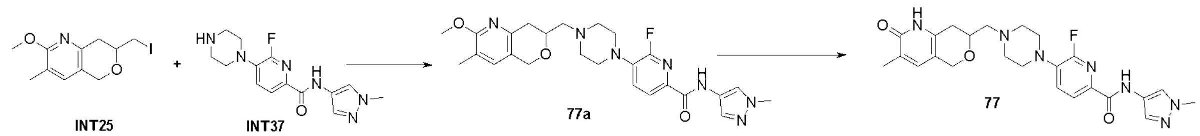

INT25 INT37 77a 77

6-Fluoro-*N*-(1-methyl-1*H*-pyrazol-4-yl)-5-(4-((3-methyl-2-oxo-1,5,7,8-tetrahydro-2*H*-pyrano[ 4,3-*b*]pyridin-7-yl) methyl)piperazin-1-yl)picolinamide was prepared using a synthesis method similar to the preparation of Example 39. LC-MS: ESI [M+H]$^+$ = 482.3; $^1$H NMR (400 MHz, DMSO-$d_6$) δ 11.41 (s, 1H), 10.51 (s, 1H), 8.04 (s, 1H), 7.93 (dd, *J* = 8.1, 1.5 Hz, 1H), 7.70 (s, 1H), 7.61 (dd, *J* = 10.6, 8.1 Hz, 1H), 7.03 (d, *J* = 1.3 Hz, 1H), 4.49 - 4.36 (m, 2H), 3.88 - 3.80 (m, 1H), 3.81 (s, 3H), 3.19 (t, *J* = 4.9 Hz, 4H), 2.65 (d, *J* = 5.2 Hz, 4H), 2.53 (d, *J* = 4.8 Hz, 2H), 2.49 - 2.36 (m, 2H), 1.93 (s, 3H).

**Example 78**

5-(4-((3-Ethyl-2-oxo-1,5,7,8-tetrahydro-2*H*-pyrano[4,3-*b*]pyridin-7-yl)methyl)piperazin-1-yl) -6-fluoro-*N*-(1-methyl-1*H*-pyrazol-4-yl)picolinamide

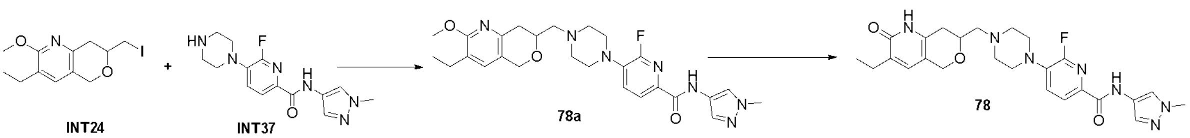

INT24 INT37 78a 78

5-(4-((3-Ethyl-2-oxo-1,5,7,8-tetrahydro-2*H*-pyrano[4,3-*b*]pyridin-7-yl)methyl)piperazin-1-yl) -6-fluoro-*N*-(1-methyl-1*H*-pyrazol-4-yl)picolinamide was prepared using a synthesis method similar to the preparation of Example 39. LC-MS: ESI [M+H]$^+$ = 496.1; $^1$H NMR (400 MHz, DMSO-$d_6$) δ 11.49 (s, 1H), 10.60 (s, 1H), 8.13 (s, 1H), 8.02 (dd, *J* = 8.1, 1.4 Hz, 1H), 7.79 (s, 1H), 7.70 (dd, *J* = 10.6, 8.2 Hz, 1H), 7.08 (s, 1H), 4.60 - 4.47 (m, 2H), 3.98 - 3.91 (m, 1H), 3.90 (s, 3H), 3.28 (t, *J* = 4.9 Hz, 4H), 2.78 - 2.69 (m, 4H), 2.68 - 2.61 (m, 2H), 2.58 - 2.50 (m, 2H), 2.44 (d, *J* = 7.4 Hz, 2H), 1.15 (t, *J* = 7.4 Hz, 3H).

**Example 79**

6-Fluoro-*N*-(3-methoxycyclobutyl)-5-(4-((3-methyl-2-oxo-1,5,7,8-tetrahydro-2*H*-pyrano[4,3-*b*]pyridin-7-yl)methyl)piperazin-1-yl)picolinamide

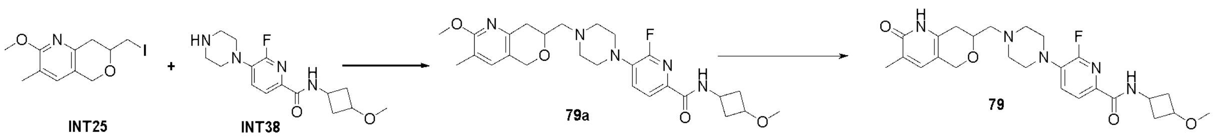

INT25 INT38 79a 79

6-Fluoro-*N*-(3-methoxycyclobutyl)-5-(4-((3-methyl-2-oxo-1,5,7,8-tetrahydro-2*H*-pyrano[4,3-*b*]pyridin-7-yl) methyl)piperazin-1-yl)picolinamide was prepared using a synthesis method similar to the preparation of Example 39. LC-MS: ESI [M+H]$^+$ = 486.2; $^1$H NMR (400 MHz, DMSO-$d_6$) δ 11.41 (s, 1H), 8.65 - 8.57 (m, 1H), 7.90 - 7.78 (m, 1H), 7.59 - 7.54 (m, 1H), 7.03 (d, *J* = 1.3 Hz, 1H), 4.50 - 4.36 (m, 2H), 4.07 - 3.92 (m, 1H), 3.85 - 3.81 (m, 1H), 3.64 - 3.53 (m, 1H), 3.19 - 3.12 (m, 4H), 3.13 (s, 2H), 2.65 - 2.56 (m, 4H), 2.59 - 2.51 (m, 2H), 2.44 (t, *J* = 7.7 Hz, 2H), 2.33 (d, *J* = 1.8 Hz, 2H), 2.38 - 2.16 (m, 2H), 2.07 - 2.00 (m, 2H), 1.93 (s, 3H).

**Example 80**

(*S*)-6-Fluoro-*N*-methyl-5-(1-((2-methyl-3-oxo-3,4,5,6,7,8-hexahydroquinoxalin-6-yl)methyl)p iperidin-4-yl)picolinamide

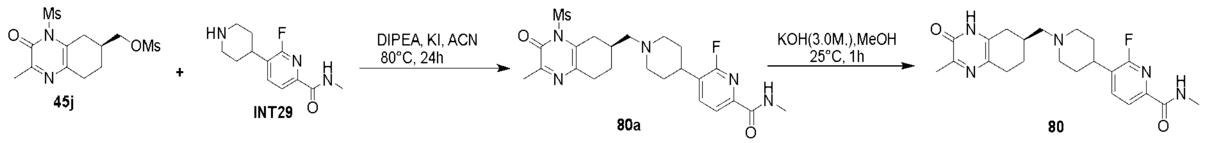


45j + INT29 → 80a → 80

(*S*)-6-Fluoro-*N*-methyl-5-(1-((2-methyl-3-oxo-3,4,5,6,7,8-hexahydroquinoxalin-6-yl)methyl)p iperidin-4-yl)picolinamide was prepared using a synthesis method similar to the preparation of Example 45. LC-MS: ESI $[M+H]^+$ = 414.1. $^1$H NMR (400 MHz, $CDCl_3$) δ 12.45 (s, 1H), 8.04 (d, *J* = 7.5 Hz, 1H), 7.85 - 7.76 (m, 1H), 7.65 (d, *J* = 4.9 Hz, 1H), 3.02 (t, *J* = 9.7 Hz, 5H), 2.88 - 2.61 (m, 4H), 2.43 (s, 3H), 2.39 - 2.30 (m, 3H), 2.18 - 1.96 (m, 4H), 1.88 - 1.74 (m, 4H), 1.55 - 1.40 (m, 1H).

**Example 81**

(*S*)-*N*-Cyclopropyl-6-fluoro-5-(4-((2-methyl-3-oxo-3,4,5,6,7,8-hexahydroquinoxalin-6-yl)met hyl)piperazin-1-yl)picolinamide

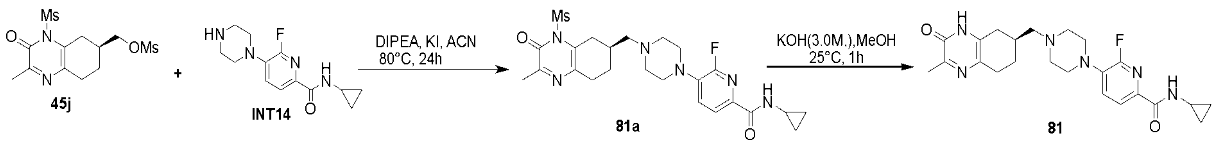


45j + INT14 → 81a → 81

(*S*)-*N*-Cyclopropyl-6-fluoro-5-(4-((2-methyl-3-oxo-3,4,5,6,7,8-hexahydroquinoxalin-6-yl)met hyl)piperazin-1-yl)picolinamide was prepared using a synthesis method similar to the preparation of Example 45. LC-MS: ESI $[M+H]^+$ = 441.1. $^1$H NMR (400 MHz, $CDCl_3$) δ 7.99 (d, *J* = 7.8 Hz, 1H), 7.53 (d, *J* = 3.3 Hz, 1H), 7.29 (dd, *J* = 10.0, 8.2 Hz, 1H), 3.23 (t, *J* = 4.6 Hz, 4H), 2.89 (tq, *J* = 7.4, 3.8 Hz, 1H), 2.82 - 2.57 (m, 7H), 2.42 (s, 3H), 2.37 (d, *J* = 17.5 Hz, 2H), 2.05 (d, *J* = 10.9 Hz, 2H), 1.55 - 1.45 (m, 1H), 1.42 (t, *J* = 5.1 Hz, 1H), 0.90 - 0.79 (m, 2H), 0.68 - 0.58 (m, 2H).

**Example 82**

(*S*)-6-Fluoro-*N*-(methyl-$d_3$)-5-(4-((2-methyl-3-oxo-3,4,5,6,7,8-hexahydroquinoxalin-6-yl)met hyl)piperazin-1-yl)picolinamide

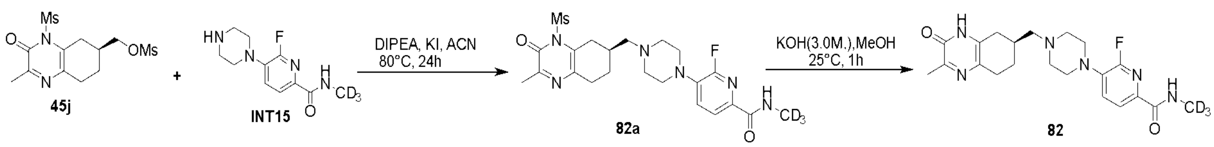


45j + INT15 → 82a → 82

(*S*)-6-Fluoro-*N*-(methyl-$d_3$)-5-(4-((2-methyl-3-oxo-3,4,5,6,7,8-hexahydroquinoxalin-6-yl)met hyl)piperazin-1-yl)picolinamide was prepared using a synthesis method similar to the preparation of Example 45. LC-MS: ESI $[M+H]^+$ = 418.1. $^1$H NMR (400 MHz, DMSO) δ 11.85 (s, 1H), 8.39 (s, 1H), 7.85 (d, *J* = 8.0 Hz, 1H), 7.59 (s, 1H), 4.14 (dd, *J* = 9.9, 4.8 Hz, 8H), 2.70 - 2.52 (m, 6H), 2.38 - 2.22 (m, 2H), 2.21 (d, *J* = 7.8 Hz, 4H).

**Example 83**

(*S*)-6-Fluoro-*N*-methyl-5-(4-((2-methyl-3-oxo-3,4,5,6,7,8-hexahydroquinoxalin-6-yl)methyl-*d* $_2$)piperazin-1-yl)picolinamide

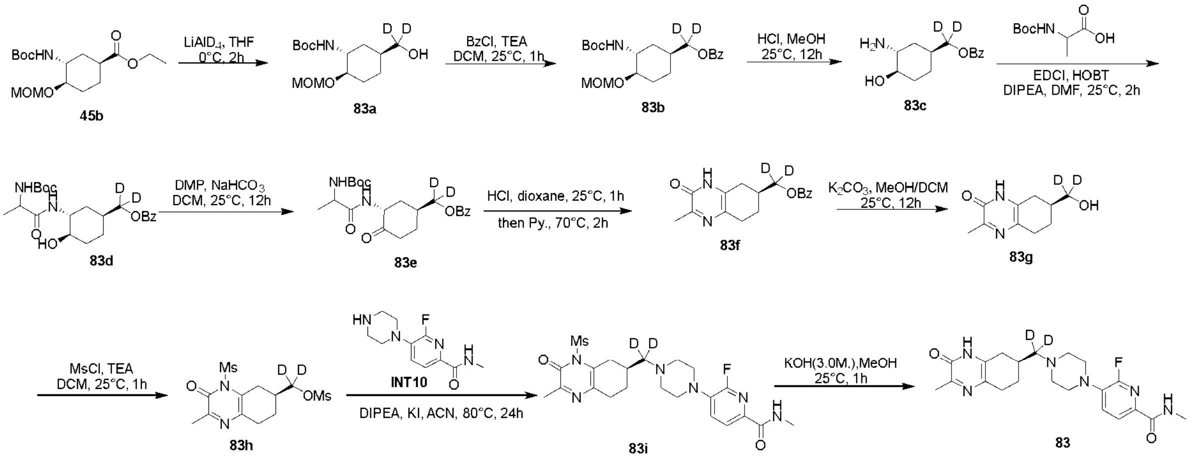


(*S*)-6-Fluoro-*N*-methyl-5-(4-((2-methyl-3-oxo-3,4,5,6,7,8-hexahydroquinoxalin-6-yl)methyl-*d* $_2$)piperazin-1-yl) picolinamide was prepared using a synthesis method similar to the preparation of Example 45. LC-MS: ESI $[M+H]^+$ = 443.1. $^1$H NMR (400 MHz, DMSO) δ 11.81 (s, 1H), 8.35 (d, *J* = 4.9 Hz, 1H), 7.84 (d, *J* = 7.9 Hz, 1H), 7.56 (dd, *J* = 10.5, 8.2 Hz, 1H), 3.15 (d, *J* = 4.4 Hz, 4H), 2.85 (dd, *J* = 11.4, 6.6 Hz, 1H), 2.65 - 2.52 (m, 7H), 2.25 - 2.14 (m, 4H), 1.99 (d, *J* = 9.9 Hz, 1H), 1.92 - 1.79 (m, 1H), 1.43 - 1.29 (m, 1H), 0.65 (d, *J* = 7.2 Hz, 4H).

**Example 84**

(*S*)-6-Fluoro-*N*-(methyl-*d*$_3$)-5-(4-((2-methyl-3-oxo-3,4,5,6,7,8-hexahydroquinoxalin-6-yl)met hyl-*d*$_2$)piperazin-1-yl)picolinamide

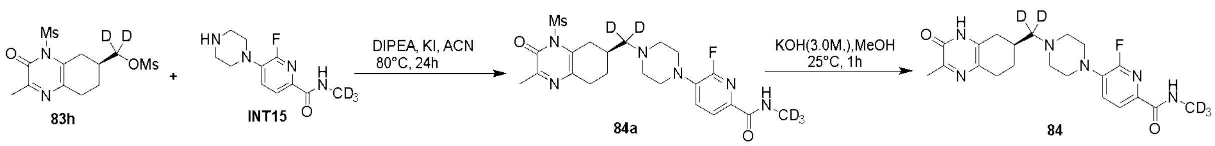


(*S*)-6-Fluoro-*N*-(methyl-*d*$_3$)-5-(4-((2-methyl-3-oxo-3,4,5,6,7,8-hexahydroquinoxalin-6-yl)met hyl-*d*$_2$)piperazin-1-yl)picolinamide was prepared using a synthesis method similar to the preparation of Example 45. LC-MS: ESI $[M+H]^+$ = 420.1. $^1$H NMR (400 MHz, DMSO) δ 11.81 (s, 1H), 8.37 (s, 1H), 7.84 (d, *J* = 8.0 Hz, 1H), 7.56 (dd, *J* = 10.5, 8.2 Hz, 1H), 3.15 (t, *J* = 4.5 Hz, 4H), 2.64 - 2.52 (m, 7H), 2.24 - 2.11 (m, 4H), 1.99 (d, *J* = 10.0 Hz, 1H), 1.92 - 1.80 (m, 1H), 1.36 (dq, *J* = 13.2, 8.1 Hz, 1H).

**Example 85**

(*S*)-6-Chloro-*N*-methyl-5-(4-((2-methyl-3-oxo-3,4,5,6,7,8-hexahydroquinoxalin-6-yl)methyl-*d*$_2$)piperazin-1-yl)picolinamide

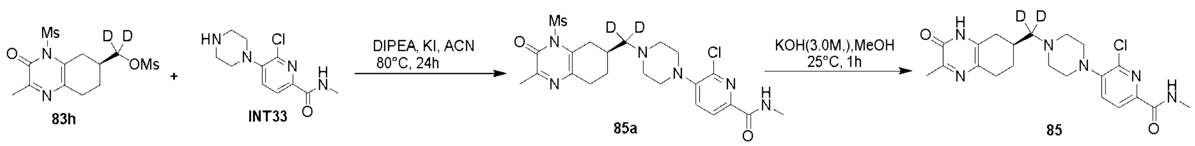


(*S*)-6-Chloro-*N*-methyl-5-(4-((2-methyl-3-oxo-3,4,5,6,7,8-hexahydroquinoxalin-6-yl)methyl-*d*$_2$)piperazin-1-yl) picolinamide was prepared using a synthesis method similar to the preparation of Example 45. LC-MS: ESI $[M+H]^+$ = 433.1. $^1$H NMR (400 MHz, DMSO) δ 11.82 (s, 1H), 8.43 (q, *J* = 4.6 Hz, 1H), 7.66 (dd, *J* = 8.2, 4.4 Hz, 1H), 3.10 (s, 4H), 2.79 (d, *J* = 4.8 Hz, 3H), 2.65 - 2.51 (m, 10H), 2.20 (s, 3H), 1.99 (t, *J* = 11.3 Hz, 1H), 1.93 - 1.83 (m, 1H), 1.43 - 1.31 (m, 1H).

**Example 86**

5-(4-((7-Chloro-6-oxo-3,4,5,6-tetrahydro-2*H*-pyrano[3,2-*b*]pyridin-3-yl)methyl)piperazin-1-y l)-6-fluoro-*N*-methylpicolinamide

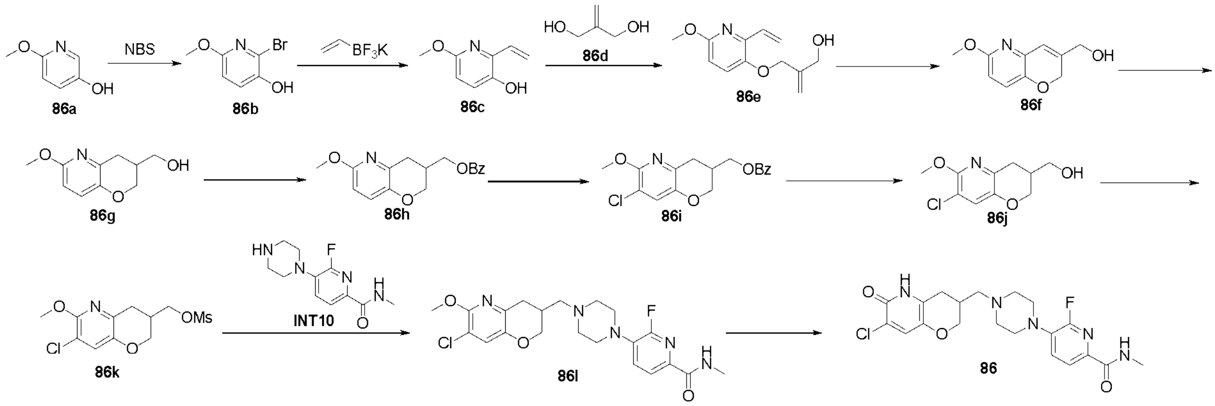


Step 1: Compound **86a** (14 g, 11.2 mmol) was dissolved in anhydrous acetonitrile (150 mL), and NBS (22 g, 12.3 mmol) was added. The mixture was allowed to react at room temperature overnight. A saturated aqueous sodium bicarbonate solution was added to quench the reaction, and the mixture was extracted three times with ethyl acetate. The organic phases were combined, washed with saturated brine, dried over anhydrous sodium sulfate, filtered, concentrated under reduced pressure, and subjected to column chromatography to obtain the target product **86b** (18 g). LC-MS: ESI $[M+H]^+$ = 203.9.

Step 2: Compound **86b** (5 g, 25 mmol), potassium vinyltrifluoroborate (5 g, 37 mmol), $Pd(dppf)Cl_2$ (1.8 g, 2.5 mmol), and potassium carbonate (10.4 g, 75 mmol) were dissolved in dioxane (50 mL) and water (5 mL). The system was purged with a nitrogen atmosphere and allowed to react at 100 °C for 5 h. The mixture was then filtered, mixed with silica gel, and subjected to column chromatography to obtain the target compound **86c** (2 g). LC-MS: ESI $[M+H]^+$ = 152.1.

Step 3: Compound **86c** (302 mg, 2 mmol), compound **86d** (88 mg, 3 mmol), and triphenylphosphine (786 mg, 3 mmol) were dissolved in anhydrous THF (10 mL). The system was purged with a nitrogen atmosphere and cooled to 0 °C, and then DIAD (606 mg, 3 mmol) was added dropwise. Subsequently, the mixture was transferred to room temperature, allowed to react overnight, then mixed with silica gel, and subjected to column chromatography to obtain the target compound **86e** (500 mg). LC-MS: ESI $[M+H]^+$ = 222.1.

Step 4: Compound **86e** (500 mg, 2.3 mmol) and Grubbs catalyst II (98 mg, 0.23 mmol) were dissolved in anhydrous DCM (20 mL). The system was purged with a nitrogen atmosphere and allowed to react at room temperature overnight. The mixture was then mixed with silica gel and subjected to column chromatography to obtain the target product **86f** (450 mg). LC-MS: ESI $[M+H]^+$ = 194.1.

Step 5: Compound **86f** (450 mg) was dissolved in methanol (10 mL), and 10% Pd/C (45 mg) was added. The system was purged with a hydrogen atmosphere and allowed to react at room temperature overnight. The reaction mixture was filtered through celite and concentrated under reduced pressure to obtain the target compound **86g** (430 mg). LC-MS: ESI $[M+H]^+$ = 196.1.

Step 6: Compound **86g** (200 mg, 1 mmol) and triethylamine (0.417 mL, 3 mmol) were dissolved in DCM (10 mL), and benzoyl chloride (0.17 g, 1.2 mmol) was added. The mixture was allowed to react at room temperature overnight. A saturated aqueous sodium bicarbonate solution was added to quench the reaction, and the mixture was extracted three times with DCM, washed with saturated brine, dried over anhydrous sodium sulfate, filtered, mixed with silica gel, and subjected to column chromatography to obtain the target product **86h** (193 mg). LC-MS: ESI $[M+H]^+$ = 300.1.

Step 7: Compound **86h** (140 mg, 0.47 mmol) was dissolved in anhydrous DMF (5 mL), and NCS (75 mg, 0.56 mmol) was added. The mixture was allowed to react at room temperature overnight. The reaction was quenched with a saturated aqueous sodium bicarbonate solution, and the mixture was extracted three times with EA, washed with saturated brine, dried over anhydrous sodium sulfate, filtered, mixed with silica gel, and subjected to column chromatography to obtain the target product **86i** (90 mg). LC-MS: ESI $[M+H]^+$ = 334.1.

Step 8: Compound **86i** (90 mg, 0.27 mmol) was dissolved in methanol (5 mL), and lithium hydroxide (19.37 mg, 0.81 mmol) was added. The mixture was allowed to react at room temperature overnight, then mixed with silica gel, and subjected to column chromatography to obtain the target product **86j** (65 mg). LC-MS: ESI $[M+H]^+$ = 230.1.

Step 9: Compound **86j** (65 mg, 0.28 mmol) and triethylamine (0.12 mL, 0.85 mmol) were dissolved in anhydrous

DCM (5 mL), and the solution was cooled to 0 °C. MsCl (65 mg, 0.57 mmol) was then added dropwise. Subsequently, the mixture was transferred to room temperature and allowed to react for 2 h. A saturated aqueous sodium bicarbonate solution was added to quench the reaction, and the mixture was extracted three times with DCM. The organic phases were combined, washed with saturated brine, dried over anhydrous sodium sulfate, filtered, and concentrated under reduced pressure to obtain a crude product of the target compound **86k** (77 mg). LC-MS: ESI $[M+H]^+$ = 308.0.

Step 10: Compound **86k** (77 mg, 0.25 mmol), compound **INT10** (66 mg, 0.28 mmol), potassium iodide (83 mg, 0.5 mmol), and DIEA (97 mg, 0.75 mmol) were dissolved in anhydrous ACN (5 mL). The solution was allowed to react at 80 °C overnight, then mixed with silica gel, and subjected to column chromatography to obtain the target product **86l** (45 mg). LC-MS: ESI $[M+H]^+$ = 450.2.

Step 11: Compound **86l** (40 mg, 0.09 mmol) was dissolved in ACN (5 mL), and a solution of hydrogen bromide in acetic acid (1 mL) was added. The mixture was allowed to react at 80 °C for 1 h. After the starting materials were completely consumed as monitored by LC-MS, the solution was cooled to room temperature, diluted with water, and extracted three times with EA to remove some organic impurities. The aqueous phase was adjusted to weakly alkaline with solid sodium bicarbonate and then extracted three times with 10% MeOH in DCM. The organic phases were combined, dried over anhydrous sodium sulfate, and filtered, and the filtrate was concentrated under reduced pressure. The residue was separated by preparative TLC to obtain the target product **86** (21 mg). LC-MS: ESI $[M+H]^+$ = 436.1; $^1$H NMR (400 MHz, DMSO) δ 11.83 (s, 1H), 8.41 (q, *J* = 4.6 Hz, 1H), 7.85 (dd, *J* = 8.0, 1.3 Hz, 1H), 7.57 (dd, *J* = 10.6, 8.2 Hz, 1H), 7.47 (s, 1H), 4.10 (d, *J* = 10.9 Hz, 1H), 3.75 (dd, *J* = 9.9, 4.9 Hz, 1H), 3.20 - 3.12 (m, 4H), 2.77 (d, *J* = 4.8 Hz, 2H), 2.71 - 2.63 (m, 1H), 2.59 - 2.53 (m, 4H), 2.42 - 2.25 (m, 2H).

**Example 87**

*N*,6-Dimethyl-5-(4-((3-(methylthio)-2-oxo-1,5,7,8-tetrahydro-2*H*-pyrano[4,3-*b*]pyridin-7-yl) methyl)piperazin-1-yl)picolinamide

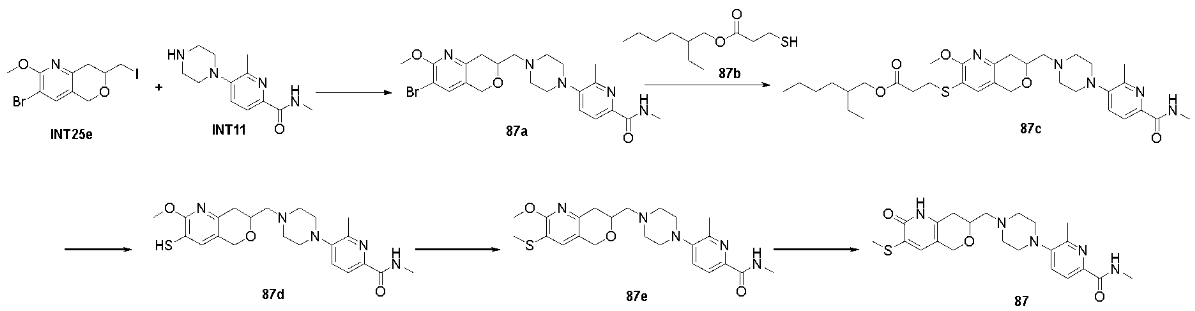

INT25e INT11 87a 87c 87d 87e 87

Step 1: Compound **INT25e** (500 mg, 1.31 mmol), compound **INT11** (367 mg, 1.57 mmol), and *N*,*N*-diisopropylethylamine (337 mg, 2.61 mmol) were added to 15 mL of acetonitrile, and the mixture was heated to 80 °C and stirred for 12 h. After the reaction was completed as monitored by TLC, 15 mL of a saturated aqueous sodium bicarbonate solution was added, and the mixture was extracted three times with 30 mL of dichloromethane. The organic phases were combined, then dried over anhydrous sodium sulfate, filtered, and dried in an oven. The resulting crude product was purified by column chromatography to obtain compound **87a** (400 mg, light yellow solid). LC-MS: ESI $[M+H]^+$ = 490.2.

Step 2: Compound **87a** (400 mg, 0.82 mmol), 2-ethylhexyl 3-mercaptopropionate (267.5 mg, 1.23 mmol), Xantphos (96.7 mg, 0.16 mmol), $Pd_2(dba)_3$ (75 mg, 0.082 mmol), and DIPEA (221 mg, 1.6 mmol) were added to anhydrous toluene, and the mixture was heated to 110 °C and allowed to react for 12 h in a $N_2$ atmosphere. After the reaction was completed as monitored by TLC, water and 30 mL of ethyl acetate were added, and the mixture was extracted three times. The organic phases were combined, then dried over anhydrous sodium sulfate, filtered, and dried in an oven. The resulting crude product was purified by column chromatography to obtain compound **87c** (300 mg, white solid). LC-MS: ESI $[M+H]^+$ = 628.4.

Step 3: Compound **87c** (300 mg, 0.92 mmol) was dissolved in ethanol, and sodium ethoxide (124.8 mg, 1.8 mmol) was added. The mixture was stirred at 25 °C for 2 h. After the reaction was completed as monitored by TLC, the reaction mixture was slowly poured into HCl (0.5 M, 10 mL) to quench the reaction. Water and ethyl acetate were then added, and the mixture was extracted three times. The organic phases were combined, then dried over anhydrous sodium sulfate, filtered, and dried again in an oven. The resulting crude product was purified by column chromatography to obtain compound **87d** (150 mg, white solid). LC-MS: ESI $[M+H]^+$ = 444.2.

Step 4: Compound **87d** (150 mg, 0.35 mmol), iodomethane (52 mg, 0.37 mmol), and cesium carbonate (165.5

mg, 0.51 mmol) were added to anhydrous acetonitrile, and the mixture was stirred at 25 °C for 1 h. After the reaction was completed as monitored by TLC, the reaction mixture was poured into saturated ammonium chloride to quench the reaction. Water and ethyl acetate were then added, and the mixture was extracted three times. The organic phases were combined, then dried over anhydrous sodium sulfate, filtered, and dried in an oven to obtain a crude product of compound **87e** (100 mg, yellow oil). LC-MS: ESI $[M+H]^+$ = 458.2. Step 5: The crude product of compound **87e** (100 mg, 0.22 mmol) and a 48% aqueous hydrobromic acid solution (0.5 mL) were added to a dioxane solution, and the mixture was heated to 80 °C and stirred for 2 h. After the reaction was completed as detected by LC-MS, a saturated sodium bicarbonate solution was added, followed by the addition of 30 mL of ethyl acetate and 50 mL of water. The mixture was then allowed to separate into layers in a separatory funnel. The aqueous phase was extracted twice with ethyl acetate, and the organic phases were combined, then washed sequentially with water and saturated brine, dried over anhydrous sodium sulfate, filtered, concentrated to dryness by rotary evaporation, and purified by column chromatography to obtain compound **87** (33 mg, white solid). LC-MS: ESI $[M+H]^+$ = 444.3; $^1$H NMR (400 MHz, DMSO-$d_6$) δ 11.65 (s, 1H), 8.42 (q, *J* = 4.8 Hz, 1H), 7.80 (d, *J* = 8.2 Hz, 1H), 7.48 (d, *J* = 8.3 Hz, 1H), 6.89 (s, 1H), 4.57 - 4.41 (m, 2H), 3.89 - 3.81 (m, 1H), 2.94 (t, *J* = 4.7 Hz, 4H), 2.80 (d, *J* = 4.9 Hz, 3H), 2.65 (s, 4H), 2.57 (dt, *J* = 11.8, 5.8 Hz, 2H), 2.49 (s, 3H), 2.27 (s, 3H).

**Example 88**

(*S*)-*N*-Cyclopropyl-6-fluoro-5-(4-((2-methyl-3-oxo-3,4,5,6,7,8-hexahydroquinoxalin-6-yl)met hyl-$d_2$)piperazin-1-yl)picolinamide

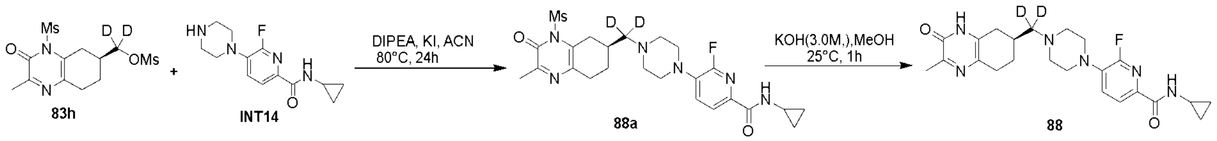


(*S*)-*N*-Cyclopropyl-6-fluoro-5-(4-((2-methyl-3-oxo-3,4,5,6,7,8-hexahydroquinoxalin-6-yl)met hyl-$d_2$)piperazin-1-yl)picolinamide was prepared using a synthesis method similar to the preparation of Example 45. LC-MS: ESI $[M+H]^+$ = 443.1. $^1$H NMR (400 MHz, DMSO) δ 11.81 (s, 1H), 8.35 (d, *J* = 4.9 Hz, 1H), 7.84 (d, *J* = 7.9 Hz, 1H), 7.56 (dd, *J* = 10.5, 8.2 Hz, 1H), 3.15 (d, *J* = 4.4 Hz, 4H), 2.85 (dd, *J* = 11.4, 6.6 Hz, 1H), 2.65 - 2.52 (m, 7H), 2.25 - 2.14 (m, 4H), 1.99 (d, *J* = 9.9 Hz, 1H), 1.92 - 1.79 (m, 1H), 1.43 - 1.29 (m, 1H), 0.65 (d, *J* = 7.2 Hz, 4H).

**Example 89**

6-Fluoro-*N*-methyl-5-((1*S*,6*R*)-5-((3-methyl-2-oxo-1,5,7,8-tetrahydro-2*H*-pyrano[4,3-*b*]pyridi n-7-yl)methyl)-2,5-diazabicyclo[4.2.0]octan-2-yl)picolinamide

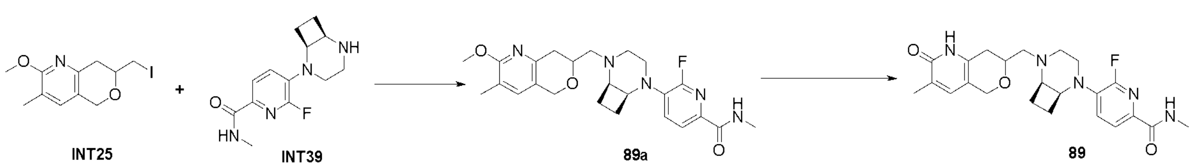

6-Fluoro-*N*-methyl-5-((1*S*,6*R*)-5-((3-methyl-2-oxo-1,5,7,8-tetrahydro-2*H*-pyrano[4,3-*b*]pyridi n-7-yl) methyl)-2,5-diazabicyclo[4.2.0]octan-2-yl)picolinamide was prepared using a synthesis method similar to the preparation of Example 39. LC-MS: ESI $[M+H]^+$ = 442.3; $^1$H NMR (400 MHz, DMSO-$d_6$) δ 11.49 (s, 1H), 8.44 (q, *J* = 4.7 Hz, 1H), 7.90 (dt, *J* = 8.1, 1.7 Hz, 1H), 7.53 - 7.43 (m, 1H), 7.12 (s, 1H), 4.59 - 4.44 (m, 2H), 4.30 - 4.18 (m, 1H), 3.97 - 3.86 (m, 1H), 3.60 - 3.51 (m, 1H), 3.34 - 3.19 (m, 1H), 3.07 (t, *J* = 8.2 Hz, 1H), 2.85 (d, *J* = 4.8 Hz, 3H), 2.57 - 2.39 (m, 2H), 2.02 (s, 3H), 1.93 - 1.76 (m, 2H), 1.46 - 1.26 (m, 4H), 0.99 - 0.90 (m, 2H).

**Example 90**

6-Fluoro-*N*-methyl-5-((1*S*,6*R*)-5-((3-methyl-2-oxo-1,5,7,8-tetrahydro-2*H*-pyrano[4,3-*b*]pyridi n-7-yl)methyl)-2,5-diazabicyclo[4.2.0]octan-2-yl)picolinamide

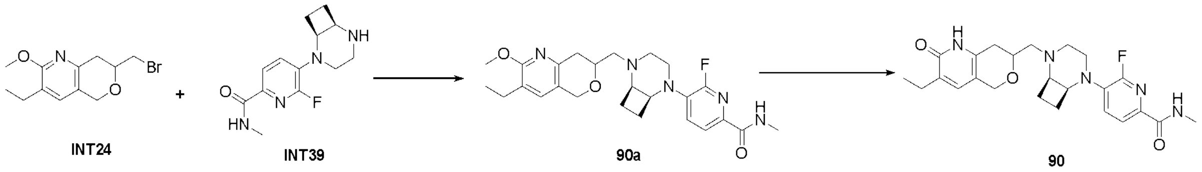

INT24 INT39 90a 90

6-Fluoro-*N*-methyl-5-((1*S*,6*R*)-5-((3-methyl-2-oxo-1,5,7,8-tetrahydro-2*H*-pyrano[4,3-*b*]pyridi n-7-yl) methyl)-2,5-diazabicyclo[4.2.0]octan-2-yl)picolinamide was prepared using a synthesis method similar to the preparation of Example 39. LC-MS: ESI $[M+H]^+$ = 442.3; $^1H$ NMR (400 MHz, DMSO-$d_6$) $\delta$ 11.49 (s, 1H), 8.44 (q, *J* = 4.7 Hz, 1H), 7.90 (dt, *J* = 8.1, 1.7 Hz, 1H), 7.53 - 7.43 (m, 1H), 7.12 (s, 1H), 4.59 - 4.44 (m, 2H), 4.30 - 4.18 (m, 1H), 3.97 - 3.86 (m, 1H), 3.60 - 3.51 (m, 1H), 3.34 - 3.19 (m, 1H), 3.07 (t, *J* = 8.2 Hz, 1H), 2.85 (d, *J* = 4.8 Hz, 3H), 2.57 - 2.39 (m, 2H), 2.02 (s, 3H), 1.93 - 1.76 (m, 2H), 1.46 - 1.26 (m, 4H), 0.99 - 0.90 (m, 2H).

## Example 91

(4a*R*)-3-((3-Ethyl-2-oxo-1,5,7,8-tetrahydro-2*H*-pyrano[4,3-*b*]pyridin-7-yl)methyl)-*N*-methyl-1,2,3,4,4a,5-hexahydro-7*H*-pyrazino[2,1-*c*]pyrido[3,2-*e*][1,4]oxazolidine-9-carboxamide

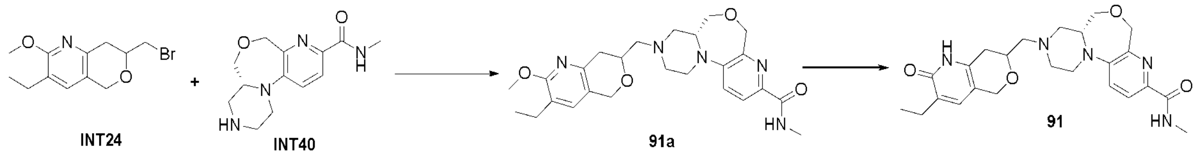

INT24 INT40 91a 91

(4a*R*)-3-((3-Ethyl-2-oxo-1,5,7,8-tetrahydro-2*H*-pyrano[4,3-*b*]pyridin-7-yl)methyl)-*N*-methyl-1,2,3,4,4a,5-hexahydro-7*H*-pyrazino[2,1-*c*]pyrido[3,2-*e*][1,4]oxazolidine-9-carboxamide was prepared using a synthesis method similar to the preparation of Example 39. LC-MS: ESI $[M+H]^+$ = 454.5. $^1H$ NMR (400 MHz, DMSO-$d_6$) $\delta$ 11.39 (s, 1H), 8.40 (d, *J* = 5.2 Hz, 1H), 7.82 (dd, *J* = 8.5, 1.9 Hz, 1H), 7.50 (d, *J* = 8.5 Hz, 1H), 6.99 (s, 1H), 4.95 (dd, *J* = 14.0, 4.4 Hz, 1H), 4.73 (dd, *J* = 14.0, 2.5 Hz, 1H), 4.46 - 4.39 (m, 2H), 4.01 - 3.94 (m, 1H), 3.87 - 3.76 (m, 2H), 3.53 - 3.43 (m, 1H), 3.30 (s, 2H), 2.79 (d, *J* = 4.8 Hz, 3H), 2.76 - 2.52 (m, 5H), 2.49 - 2.30 (m, 5H), 1.06 (t, *J* = 7.4 Hz, 3H).

## Example 92

(4a*R*)-*N*-Methyl-3-((3-methyl-2-oxo-1,5,7,8-tetrahydro-2*H*-pyrano[4,3-*b*]pyridin-7-yl)methyl) -1,2,3,4,4a,5-hexahydro-7*H*-pyrazino[2,1-*c*]pyrido[3,2-*e*][1,4]oxazolidine-9-carboxamide

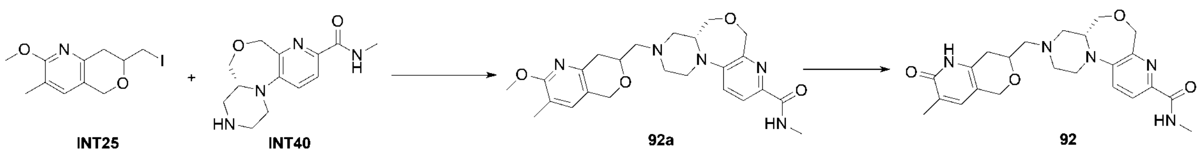

INT25 INT40 92a 92

(4a*R*)-*N*-Methyl-3-((3-methyl-2-oxo-1,5,7,8-tetrahydro-2*H*-pyrano[4,3-*b*]pyridin-7-yl)methyl) -1,2,3,4,4a,5-hexahydro-7*H*-pyrazino[2,1-*c*]pyrido[3,2-*e*][1,4]oxazolidine-9-carboxamide was prepared using a synthesis method similar to the preparation of Example 39. LC-MS: ESI $[M+H]^+$ = 440.5. $^1H$ NMR (400 MHz, DMSO-$d_6$) $\delta$ 11.16 (s, 1H), 8.40 (d, *J* = 5.1 Hz, 1H), 7.81 (d, *J* = 8.4 Hz, 1H), 7.50 (d, *J* = 8.5 Hz, 1H), 7.00 (s, 1H), 4.96 (dd, *J* = 14.1, 1.7 Hz, 1H), 4.73 (d, *J* = 13.9 Hz, 1H), 4.10 - 3.96 (m, 2H), 3.82 (dd, *J* = 12.2, 3.9 Hz, 1H), 3.75 - 3.64 (m, 1H), 3.50 (d, *J* = 7.5 Hz, 1H),3.31-3.27 (m, 2H), 2.79 (d, *J* = 4.8 Hz, 3H), 2.70 - 2.53 (m, 4H), 2.39 - 2.23 (m, 5H), 1.92 (s, 3H).

**Example 93**

(*R*)-*N*-Methyl-3-(((*S*)-2-methyl-3-oxo-3,4,5,6,7,8-hexahydroquinoxalin-6-yl)methyl)-1,2,3,4,4 a,5-hexahydro-7*H*-pyrazino[2,1-*c*]pyrido[3,2-*e*][1,4]oxazolidine-9-carboxamide

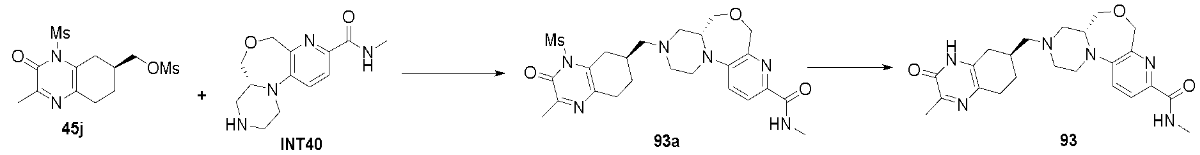

45j INT40 93a 93

(*R*)-*N*-Methyl-3-(((*S*)-2-methyl-3-oxo-3,4,5,6,7,8-hexahydroquinoxalin-6-yl)methyl)-1,2,3,4,4 a,5-hexahydro-7*H*-pyrazino[2,1-*c*]pyrido[3,2-*e*][1,4]oxazolidine-9-carboxamide was prepared using a synthesis method similar to the preparation of Example 45. LC-MS: ESI $[M+H]^+$ = 439.5.

**Examples 94 and 95**

(*S*)-6-Chloro-*N*-methyl-5-(4-((3-methyl-2-oxo-1,5,7,8-tetrahydro-2*H*-pyrano[4,3-*b*]pyridin-7-yl)methyl)piperazin-1-yl)picolinamide and (*R*)-6-chloro-*N*-methyl-5-(4-((3-methyl-2-oxo-1,5,7,8-tetrahydro-2*H*-pyrano[4,3-*b*]pyridin-7-yl)methyl) piperazin-1-yl)picolinamide

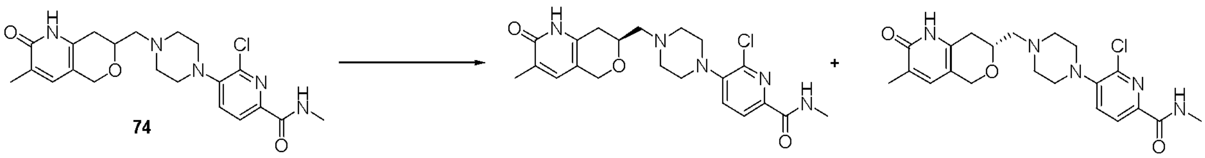

74

Compound **74** was separated and purified by preparative supercritical fluid chromatography (SFC) (column: CHIRALPAK IG 250 mm × 20 mm, 5 μm, 70% EtOH ($NH_3H_2O$ 0.1%) to obtain two compounds **94** and **95**:

**Example 94** ($t_R$ = 1.971), MS/ESI $[M+H]^+$ = 432.2; $^1$H NMR (400 MHz, DMSO-$d_6$) δ 11.42 (s, 1H), 8.44 (q, *J* = 4.7 Hz, 1H), 7.94 (d, *J* = 8.2 Hz, 1H), 7.67 (d, *J* = 8.2 Hz, 1H), 7.04 (s, 1H), 4.50 - 4.37 (m, 2H), 3.89 - 3.79 (m, 1H), 3.10 (t, *J* = 4.7 Hz, 4H), 2.80 (d, *J* = 4.8 Hz, 3H), 2.72 - 2.62 (m, 4H), 2.61 - 2.53 (m, 2H), 2.46 (t, *J* = 7.9 Hz, 2H), 1.94 (s, 3H).

**Example 95** ($t_R$ = 2.895), MS/ESI $[M+H]^+$ = 430.2; $^1$H NMR (400 MHz, DMSO-$d_6$) δ 11.42 (s, 1H), 8.44 (q, *J* = 4.7 Hz, 1H), 7.94 (d, *J* = 8.2 Hz, 1H), 7.67 (d, *J* = 8.2 Hz, 1H), 7.04 (s, 1H), 4.50 - 4.37 (m, 2H), 3.89 - 3.79 (m, 1H), 3.10 (t, *J* = 4.7 Hz, 4H), 2.80 (d, *J* = 4.8 Hz, 3H), 2.72 - 2.62 (m, 4H), 2.61 - 2.53 (m, 2H), 2.46 (t, *J* = 7.9 Hz, 2H), 1.94 (s, 3H).

**Example 96**

6-Fluoro-*N*-methyl-5-(4-((3-(methylthio)-2-oxo-1,5,7,8-tetrahydro-2H-pyrano[4,3-b]pyridin-7 -yl)methyl)piperazin-1-yl) picolinamide

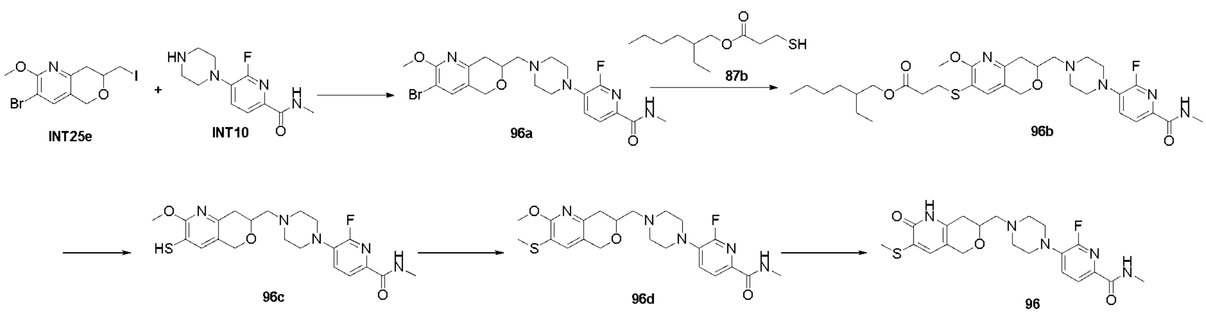

INT25e INT10 96a 87b 96b 96c 96d 96

6-Fluoro-N-methyl-5-(4-((3-(methylthio)-2-oxo-1,5,7,8-tetrahydro-2H-pyrano[4,3-b]pyridin-7 -yl)methyl)piperazin-1-yl)picolinamide was prepared using a synthesis method similar to the preparation of Example 87. LC-MS: ESI $[M+H]^+$ = 448.3; $^1$H NMR (400 MHz, DMSO-$d_6$) δ 11.64 (s, 1H), 8.40 (d, *J* = 4.9 Hz, 1H), 7.85 (dd, *J* = 8.0, 1.4 Hz, 1H), 7.57 (dd, *J* = 10.6, 8.1 Hz, 1H), 6.89 (s, 1H), 4.56 - 4.41 (m, 3H), 3.91 - 3.77 (m, 1H), 3.16 (t, *J* = 5.0 Hz, 4H), 2.86 - 2.77 (m, 1H), 2.76 (d, *J*= 4.8 Hz, 3H), 2.68 - 2.60 (m, 4H), 2.59 - 2.52 (m, 3H), 2.27 (s, 3H).

**Example 97**

N,6-Dimethyl-5-(1-((3-(methylthio)-2-oxo-1,5,7,8-tetrahydro-2*H*-pyrano[4,3-b]pyridin-7-yl) methyl)piperidin-4-yl)picolinamide

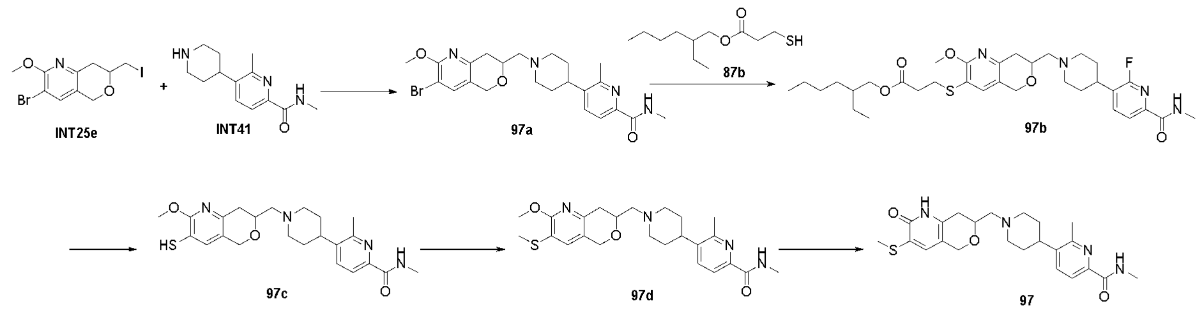


*N,6-Dimethyl-5-(1-((3-(methylthio)-2-oxo-1,5,7,8-tetrahydro-2H-pyrano[4,3-b]pyridin-7-yl)* methyl)piperidin-4-yl)picolinamide was prepared using a synthesis method similar to the preparation of Example 87. LC-MS: ESI $[M+H]^+$ = 443.2; $^1$H NMR (400 MHz, DMSO-$d_6$) δ 11.64 (s, 1H), 8.72 - 8.39 (m, 1H), 7.79 (d, *J* = 2.9 Hz, 2H), 6.89 (s, 1H), 4.59 - 4.37 (m, 2H), 3.88 - 3.78 (m, 1H), 3.10 - 3.00 (m, 2H), 2.81 (d, *J* = 4.9 Hz, 3H), 2.76 - 2.66 (m, 1H), 2.58 (s, 3H), 2.58 - 2.52 (m, 1H), 2.49 - 2.44 (m, 2H), 2.45 - 2.37 (m, 1H), 2.27 (s, 3H), 2.23 - 2.08 (m, 2H), 1.76 - 1.57 (m, 4H).

**Example 98**

(*R*)-5-(4-((1,7-Dimethyl-6-oxo-2,3,5,6-tetrahydro-1*H*-pyrido[2,3-*b*][1,4]oxazin-3-yl)methyl)p iperazin-1-yl)-6-fluoro-*N*-methylpicolinamide

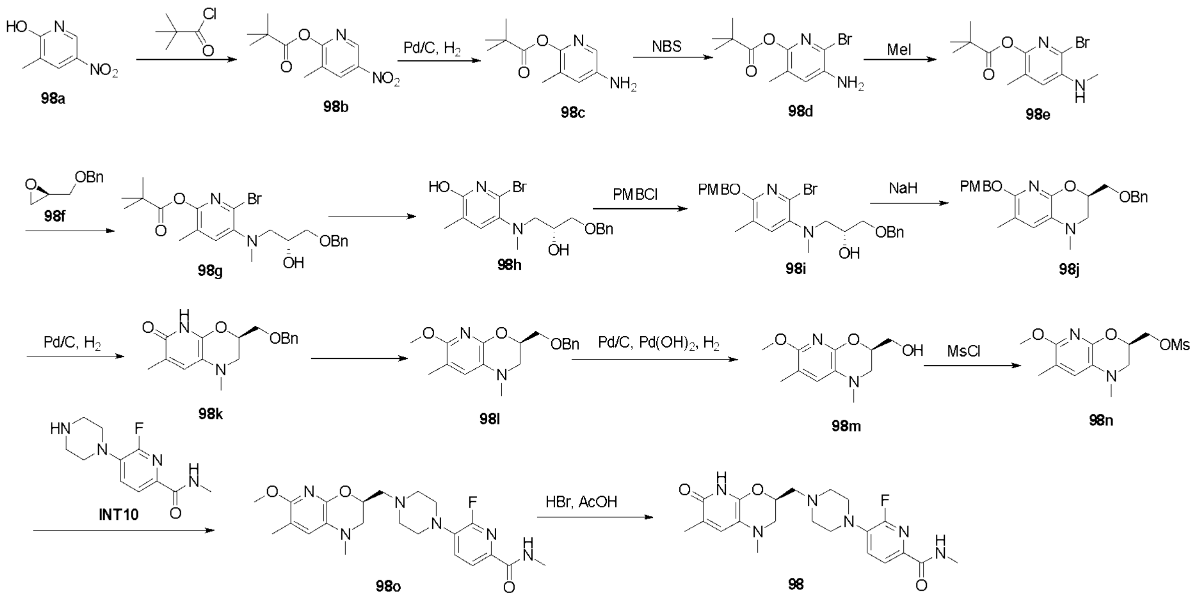


Step 1: **98a** (25 g, 162.2 mmol) was added to a round-bottom flask, followed by the addition of CAN (200 mL). The

mixture was stirred, but complete dissolution was not achieved. TEA (33.8 mL, 243.3 mmol) was then added. The mixture was stirred for 10 min to achieve complete dissolution, and then cooled to 0-10 °C. Pivaloyl chloride (21.6 mL, 175.2 mmol) was added dropwise, and the mixture was slowly warmed to room temperature and stirred for 4 h. After the reaction was completed, the reaction mixture was concentrated to dryness by rotary evaporation. The residue was purified by column chromatography (EA/PE = 0-20%) to obtain **98b** (36 g) as an off-white solid. LC-MS: $[M+H]^+$ = 239.2.

Step 2: **98b** (37.5 g, 157.4 mmol) was added to a round-bottom flask, followed by the addition of methanol (600 mL) and palladium on carbon (4.0 g, 37.6 mmol). The mixture was purged 3 times with hydrogen, stirred overnight in a hydrogen atmosphere, then filtered, and concentrated to dryness by rotary evaporation. The residue was purified by column chromatography (MeOH/DCM = 0-2%) to obtain compound **98c** (24 g) as an off-white solid. LC-MS: $[M+H]^+$ = 209.3.

Step 3: Compound **98c** (24.0 g, 115.2 mmol) was added to a round-bottom flask and completely dissolved in CAN (300 mL). The solution was cooled to 0 °C, and NBS (21.5 g, 121.0 mmol) was added. The mixture was stirred for 30 min, after which the reaction was completed. The reaction mixture was concentrated to dryness by rotary evaporation. The residue was purified by column chromatography (EA/PE = 0-20%) to obtain compound **98d** (20 g) as an off-white solid. LC-MS: $[M+H]^+$ = 287.2, 289.2.

Step 4: Compound **98d** (20.0 g, 69.6 mmol) was added to a round-bottom flask and dissolved in DMF (200 mL). The solution was cooled to -10 °C to 0 °C, and iodomethane (6.95 mL, 111.6 mmol) was added. The mixture was stirred for 10 min, and then sodium methoxide (5.31 g, 98.297 mmol) was added. The resulting mixture was stirred at -10 °C to 0 °C for 1.5 h, then warmed to room temperature, and stirred for another 3 h. TLC (PE/EA = 10:1) indicated that the reaction had reached equilibrium. Ice water (800 mL) was added to the system, and the mixture was extracted with EA. The organic phase was washed with water and brine, dried, and concentrated to dryness by rotary evaporation. The residue was purified by column chromatography (EA/PE = 0-10%) to obtain compound **98e** (3.43 g) as an off-white solid. LC-MS: $[M+H]^+$ = 301.2, 303.2.

Step 5: Compound **98e** (2.9 g, 9.6 mmol) was added to a round-bottom flask and dissolved in hexafluoroisopropanol (50 mL). (*R*)-(-)-Benzyl glycidyl ether (6.33 g, 38.5 mmol) was added, and the mixture was purged with $N_2$, heated to 75 °C, and stirred for 9 h. The system was concentrated to dryness by rotary evaporation. The residue was purified by column chromatography (EA/PE = 0-25%) to obtain compound **98g** (2.7 g) as a pale yellowish-green oil. LC-MS: $[M+H]^+$ =467.2, 469.2.

Step 6: Compound **98g** (2.7 g, 5.8 mmol) was added to a round-bottom flask and completely dissolved in methanol (50 mL). A solution of sodium hydroxide (0.7 g, 17.4 mmol) in water (20 mL) was added, and the mixture was stirred at room temperature for 30 min, then cooled to 0-10 °C, adjusted to pH 4-6 with HCl (2 N), extracted with EA, dried, and concentrated to dryness by rotary evaporation to obtain a crude product of compound **98h** (2.32 g) as a brown oil. LC-MS: $[M+H]^+$ = 383.1, 385.1.

Step 7: Compound **98h** (2.34 g, 6.1 mmol) was added to a round-bottom flask and dissolved in DMF (50 mL). Potassium carbonate (1.1 g, 7.98 mmol) and *p*-methoxybenzyl chloride (1 mL, 7.365 mmol) were added, and the mixture was heated to 60 °C and stirred for 3 h. The reaction mixture was then poured into ice water, and the resulting mixture was extracted with EA. The organic phase was washed with water, dried over $Na_2SO_4$, and concentrated to dryness by rotary evaporation. The residue was purified by column chromatography (EA/PE = 0-25%) to obtain compound **98i** (2.2 g) as a colorless oil. LC-MS: $[M+H]^+$ = 503.2, 505.2. Step 8: Compound **98i** (2.2 g, 4.4 mmol) was added to a round-bottom flask and dissolved in DME (50 mL). The solution was cooled to 0-10 °C, and sodium hydride (0.614 g, 15.4 mmol, 60% dispersion in mineral oil) was added in portions. In a $N_2$ atmosphere, the mixture was heated to 85 °C and stirred for 2 h. The reaction mixture was then poured into ice water, and the resulting mixture was extracted with EA. The organic phase was dried and concentrated to dryness by rotary evaporation. The residue was purified by column chromatography (EA/PE = 0-25%) to obtain compound **98j** (1.72 g) as a colorless oil. LC-MS: $[M+H]^+$ = 421.2.

Step 9: Compound **98j** (1.72 g, 4.09 mmol) was added to a round-bottom flask and dissolved in methanol (20 mL). Palladium on carbon (150 mg) was added, and the mixture was purged with $H_2$ and stirred for 1 h in a $H_2$ atmosphere. DCM (20 mL) was then added to the reaction mixture, and the resulting mixture was filtered and concentrated to dryness by rotary evaporation to obtain a crude product of compound **98k** (1.12 g) as a white solid. LC-MS: $[M+H]^+$ = 301.2.

Step 10: Compound **98k** (0.56 g, 1.864 mmol) was added to a round-bottom flask and dissolved in DMSO (12 mL). Potassium hydroxide (9.34 mg, 0.166 mmol) and water (3 mL) were added, and the mixture was stirred at room temperature for 30 min. Dimethyl sulfate (0.177 mL, 1.864 mmol) was added, and the mixture was stirred at room temperature overnight. The reaction mixture was then poured into cold water, and the resulting mixture was extracted with EA. The organic phase was dried and concentrated to dryness by rotary evaporation. The residue was purified by column chromatography (EA/PE = 0-25%) to obtain compound **98l** (130 mg) as a colorless oil. $^1H$ NMR (400 MHz, DMSO) δ 7.40 - 7.26 (m, 5H), 7.03 (s, 1H), 4.56 (s, 2H), 4.51 - 4.44 (m, 1H), 3.71 (s, 3H), 3.65 (dq, *J* = 10.4, 5.2 Hz, 2H), 3.19 (dd, *J* = 11.7, 2.5 Hz, 1H), 2.91 (dd, *J* = 11.7, 7.7 Hz, 1H), 2.73 (s, 3H), 2.04 (s, 3H); LC-MS: $[M+H]^+$ = 315.2.

Step 11: Compound **98l** (130 mg, 0.414 mmol) was added to a round-bottom flask and dissolved in methanol (6 mL). Palladium on carbon (30 mg, 0.282 mmol) and palladium hydroxide (30 mg, 0.414 mmol) were added, and the mixture

was purged 3 times with $H_2$ and hydrogenated overnight. Most of the starting materials remained. The mixture was then filtered to remove the catalyst, and a fresh portion of the catalyst was added. The resulting mixture was purged with $H_2$ and hydrogenated for 10 h, after which the reaction was completed. The reaction mixture was filtered and concentrated to dryness by rotary evaporation to obtain a crude product of compound **98m** (80 mg) as an oil. LC-MS: $[M+H]^+$ = 225.1. The crude product was directly used in the next step without further purification.

Step 12: Compound **98m** (80 mg, 0.357 mmol) was added to a round-bottom flask and dissolved in DCM (10 mL). TEA (0.1 mL, 0.713 mmol) was added, and the mixture was cooled to 0-10 °C. MsCl (0.03 mL, 0.39 mmol) was added, and the mixture was stirred for 30 min. Water was then added to the reaction mixture, and the resulting mixture was extracted with DCM. The organic phase was dried and concentrated to dryness by rotary evaporation to obtain a crude product of compound **98n** (130 mg) as an oil. LC-MS: $[M+H]^+$ = 303.1. The crude product was directly used in the next step without further purification.

Step 13: Compound **98n** (130 mg, 0.43 mmol), compound **INT10** (133.80 mg, 0.43 mmol), TEA (0.3 mL, 2.15 mmol), and potassium iodide (71.37 mg, 0.43 mmol) were added to a round-bottom flask and dissolved in DMF (8 mL). The mixture was purged with nitrogen, heated to 100 °C, stirred overnight, then filtered, and concentrated to dryness by rotary evaporation. The residue was purified by column chromatography (MeOH/DCM = 0-6%) to obtain compound **98o** (17 mg) as a white solid. LC-MS: $[M+H]^+$ = 445.3.

Step 14: Compound **98o** (17 mg, 0.038 mmol) was added to a round-bottom flask and dissolved in ACN (5 mL). A 33% w/w (45% w/v) solution of hydrobromic acid in acetic acid (1 mL) was added, and the mixture was heated to 80 °C and stirred for 2 h. The reaction mixture was concentrated to dryness by rotary evaporation, and the residue was adjusted to pH 7-9 with $NaHCO_3$ (aq) and extracted with DCM. The organic phase was dried and concentrated to dryness by rotary evaporation. The residue was purified by Prep-TLC to obtain compound **98** (10 mg) as an off-white solid. $^1$H NMR (400 MHz, DMSO) δ 9.81 (s, 1H), 8.41 (d, *J* = 4.8 Hz, 1H), 7.85 (d, *J* = 7.8 Hz, 1H), 7.57 (dd, *J* = 10.5, 8.2 Hz, 1H), 6.95 (s, 1H), 4.48 (d, *J* = 5.4 Hz, 1H), 3.30 (s, 1H), 3.22 - 3.10 (m, 5H), 2.83 (dd, *J* = 11.7, 7.6 Hz, 1H), 2.76 (d, *J* = 4.8 Hz, 3H), 2.72 (s, 4H), 2.62 (d, *J* = 6.1 Hz, 4H), 2.00 (s, 3H); LC-MS: $[M+H]^+$ = 431.2.

## Example 99

5-(4-((3,4-Dimethyl-2-oxo-1,5,7,8-tetrahydro-2*H*-pyrano[4,3-*b*]pyridin-7-yl)methyl)piperazin -1-yl)-6-fluoro-*N*-methylpicolinamide

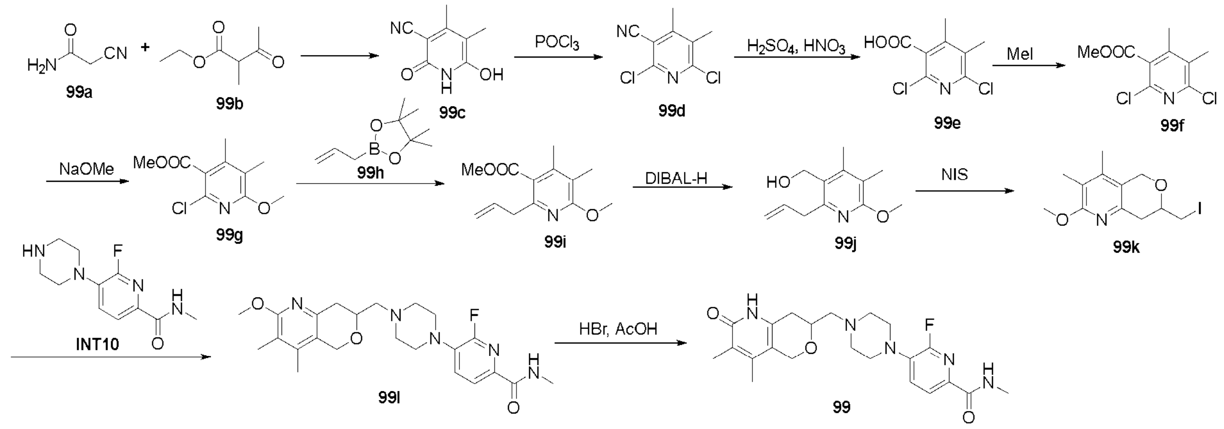


Step 1: Compound **99a** (25.0 g, 297.336 mmol) and compound **99b** (42.87 g, 297.336 mmol) were added to a round-bottom flask and stirred in methanol (200 mL) to achieve complete dissolution. A solution of potassium hydroxide (25 g, 446.0 mmol) in methanol (200 mL) was added. The system was heated to 65-70 °C, allowed to react for 4 h, then warmed to room temperature, and stirred overnight. The reaction mixture was filtered, and the filter cake was washed with a small amount of methanol. The resulting solid was added to 700 mL of water, and the mixture was stirred for 30 min, then adjusted to pH 1-2 with HCl (6 N), stirred again for 30 min, cooled to 10-20 °C, stirred for another 30 min, and filtered. The solid was dried to obtain compound **99c** (35 g) as a white powder. $^1$H NMR (400 MHz, DMSO) δ 2.18 (s, 3H), 1.87 (s, 3H); LC-MS: $[M+H]^+$ = 165.1.

Step 2: Compound **99c** (5.0 g, 30.458 mmol) and phosphorus oxychloride (10 mL) were added to a sealed tube. The tube was sealed, and the mixture was heated to 180 °C, allowed to react for 5 h, and then cooled to room temperature. TLC (PE/EA = 5:1) indicated that the reaction was completed. The reaction mixture was added to stirred ice water, followed

by extraction with EA. The organic phase was dried and concentrated to dryness by rotary evaporation. The residue was purified by column chromatography (EA/EA = 0-10) to obtain compound **99d** (5.46 g) as a white solid. LC-MS: $[M+H]^+$ = 201.2, 203.2.

Step 3: Compound **99d** (3.0 g, 14.922 mmol) was added to a round-bottom flask and dissolved in sulfuric acid (15 mL). Nitric acid (2.4 mL, mmol) was added, and the mixture was heated to 110 °C and stirred for 2 h. The reaction mixture was then poured into ice water, and the resulting mixture was extracted with EA. The organic phase was dried and concentrated to dryness by rotary evaporation to obtain a crude product of compound **99e** (3.28 g) as a white solid. LC-MS: $[M+H]^+$ = 220.2, 222.2.

Step 4: Compound **99e** (3.28 g, 14.91 mmol) was added to a round-bottom flask and dissolved in DMF (40 mL). Potassium carbonate (3.09 g, 22.359 mmol) and iodomethane (1.86 mL, 29.8 mmol) were added, and the mixture was stirred at room temperature for 2 h. Water (200 mL) was then added to the reaction mixture, and the resulting mixture was extracted with EA. The organic phase was dried and concentrated to dryness by rotary evaporation. The residue was purified by column chromatography (EA/PE = 0-20%) to obtain compound **99f** (3.4 g) as a colorless oil. LC-MS: $[M+H]^+$ = 234.2, 236.2.

Step 5: Compound **99f** (7.36 g, 31.44 mmol), $Pd_2(dba)_3$ (1.44 g, 1.57 mmol), Xantphos (1.82 g, 3.144 mmol), and TEA (21.85 mL, 157.21 mmol) were added to a round-bottom flask and dissolved in dioxane (75 mL). Sodium methoxide (3.4 g, 62.88 mmol) was added, and the mixture was purged 3 times with $N_2$, heated to 60 °C, and stirred for 3 days. The reaction mixture was filtered and concentrated to dryness by rotary evaporation. The residue was purified by column chromatography (EA/PE = 0-5%) to obtain compound **99g** (1.05 g) as an off-white solid. $^1H$ NMR (400 MHz, DMSO) δ 3.88 (d, *J* = 5.0 Hz, 6H), 2.18 (s, 3H), 2.09 (s, 3H); LC-MS: $[M+H]^+$=230.1.

Step 6: Compound **99g** (0.92 g, 4.0 mmol), compound **99h** (4.04 g, 24.04 mmol), $Pd(dppf)_2Cl_2$ (0.29 g, 0.40 mmol), potassium carbonate (0.83 g, 6.0 mmol), water (1.6 mL), and dioxane (80 mL) were added to a round-bottom flask. The mixture was purged 3 times with $N_2$, heated to 100 °C, and stirred overnight. The reaction mixture was cooled to room temperature, filtered, and concentrated to dryness by rotary evaporation. The residue was purified by column chromatography (EA/PE = 0-5%) to obtain compound **99i** (0.84 g) as a colorless oil. $^1H$ NMR (400 MHz, DMSO) δ 5.94 (dd, J = 17.0, 10.2 Hz, 1H), 5.08 - 4.96 (m, 2H), 3.87 (s, 3H), 3.83 (s, 3H), 3.38 (d, J = 6.7 Hz, 2H), 2.14 (s, 3H), 2.07 (s, 3H); LC-MS: $[M+H]^+$ = 236.1.

Step 7: Compound **99i** (0.84 g, 3.57 mmol) was added to a round-bottom flask and completely dissolved in THF (10 mL). DIBAL-H (21.42 mL, 21.42 mmol) was added dropwise, and the mixture was stirred at room temperature for 2 h. The reaction mixture was cooled to 0-10 °C. 10% KOH (aq) (60 mL) was then added dropwise, and the resulting mixture was extracted with EA. The organic phase was dried and concentrated to dryness by rotary evaporation. The residue was purified by column chromatography (EA/PE = 0-25%) to obtain compound **99j** (0.72 g) as a colorless oil. LC-MS: $[M+H]^+$ = 208.2.

Step 8: Compound **99j** (0.72 g, 3.47 mmol) was added to a round-bottom flask and dissolved in ACN (80 mL). *N*-Iodosuccinimide (1.95 g, 8.68 mmol) was added, and the mixture was stirred for 2 h. The reaction mixture was then added to water, followed by extraction with EA. The organic phase was washed with $NaHCO_3$ (aq), dried, and concentrated to dryness by rotary evaporation. The residue was purified by column chromatography (EA/PE = 0-10%) to obtain compound **99k** (0.55 g) as a white solid. $^1H$ NMR (400 MHz, DMSO) δ 4.80 (d, *J* = 14.8 Hz, 1H), 4.59 (d, *J* = 14.8 Hz, 1H), 3.81 (s, 3H), 3.63 (ddd, *J* = 10.2, 6.9, 3.6 Hz, 1H), 3.53 (dd, *J* = 10.4, 4.1 Hz, 1H), 3.39 (dd, *J* = 10.4, 6.6 Hz, 1H), 2.78 (dd, *J* = 16.6, 2.8 Hz, 1H), 2.63 (dd, *J* = 16.6, 10.4 Hz, 1H), 2.05 (s, 3H), 2.02 (s, 3H); LC-MS: $[M+H]^+$=334.2. Step 9: Compound **99k** (195 mg, 0.585 mmol) and compound **INT10** (182.13 mg, 0.585 mmol) were added to a round-bottom flask and dissolved in DMF (25 mL). DIEA (0.41 mL, 2.34 mmol) was added, and the mixture was heated to 80-100 °C, stirred for 2 days, and then concentrated under reduced pressure to remove the solvent. The residue was purified by column chromatography (MeOH/DCM = 0-8%) to give a crude product (120 mg), which was then purified by Prep-TLC (DCM/MeOH = 10:1) to obtain compound **99l** (35 mg) as a white solid. LC-MS: $[M+H]^+$ = 444.3.

Step 10: Compound **99l** (35 mg, 0.079 mmol) was added to a round-bottom flask and dissolved in ACN (6 mL). A 33% w/w (45% w/v) solution of hydrobromic acid in acetic acid (1 mL) was added, and the mixture was heated to 80 °C, stirred for 3 h, and concentrated under reduced pressure to remove the solvent. $NaHCO_3$ (aq) was then added to the residue to adjust the pH to 7-9, and the mixture was extracted with DCM/MeOH (10:1). The organic phase was dried and concentrated to dryness by rotary evaporation. The residue was purified by Prep-TLC (DCM/MeOH = 10:1) to obtain compound **99** (28 mg) as a white solid. $^1H$ NMR (400 MHz, DMSO) δ 11.28 (s, 1H), 8.40 (q, *J* = 4.6 Hz, 1H), 7.89 - 7.79 (m, 1H), 7.57 (dd, *J* = 10.6, 8.2 Hz, 1H), 4.55 (d, *J* = 14.2 Hz, 1H), 4.40 (d, *J* = 14.3 Hz, 1H), 3.87 - 3.74 (m, 1H), 3.16 (t, *J* = 4.6 Hz, 4H), 2.76 (d, *J* = 4.8 Hz, 3H), 2.70 - 2.59 (m, 4H), 2.59 - 2.51 (m, 2H), 2.43 (d, *J* = 9.2 Hz, 2H), 1.92 (s, 6H); LC-MS: $[M+H]^+$ = 430.2.

**Example 100**

6-Chloro-5-(4-((3,4-dimethyl-2-oxo-1,5,7,8-tetrahydro-2*H*-pyrano[4,3-*b*]pyridin-7-yl)methyl) piperazin-1-yl)-*N*-methylpicolinamide

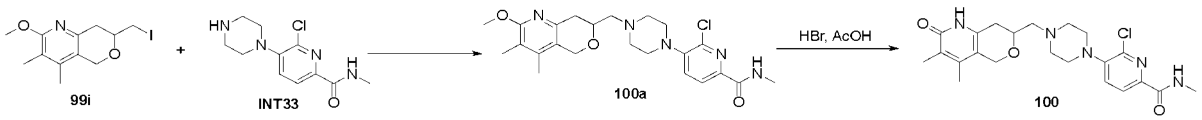


6-Chloro-5-(4-((3,4-dimethyl-2-oxo-1,5,7,8-tetrahydro-2*H*-pyrano[4,3-*b*]pyridin-7-yl)methyl) piperazin-1-yl)-*N*-methylpicolinamide was prepared using a synthesis method similar to the preparation of Example 99. $^1$H NMR (400 MHz, DMSO) δ 11.28 (s, 1H), 8.42 (dd, *J* = 8.9, 4.8 Hz, 1H), 7.95 (dd, *J* = 8.1, 5.2 Hz, 1H), 7.65 (dd, *J* = 12.4, 8.2 Hz, 1H), 4.56 (d, *J* = 14.2 Hz, 1H), 4.41 (d, *J* = 14.3 Hz, 1H), 3.81 (d, *J* = 3.6 Hz, 1H), 3.10 (s, 4H), 2.84 - 2.75 (m, 3H), 2.64 (d, *J* = 19.6 Hz, 4H), 2.56 (dd, *J* = 11.4, 5.4 Hz, 2H), 2.45 (s, 2H), 1.93 (s, 6H); LC-MS: $[M+H]^+$ = 446.2.

**Example 101**

6-Fluoro-*N*-methyl-5-(4-((7-methyl-6-oxo-3,4,5,6-tetrahydro-2*H*-pyrano[3,2-*b*]pyridin-3-yl)m ethyl)piperazin-1-yl)picolinamide

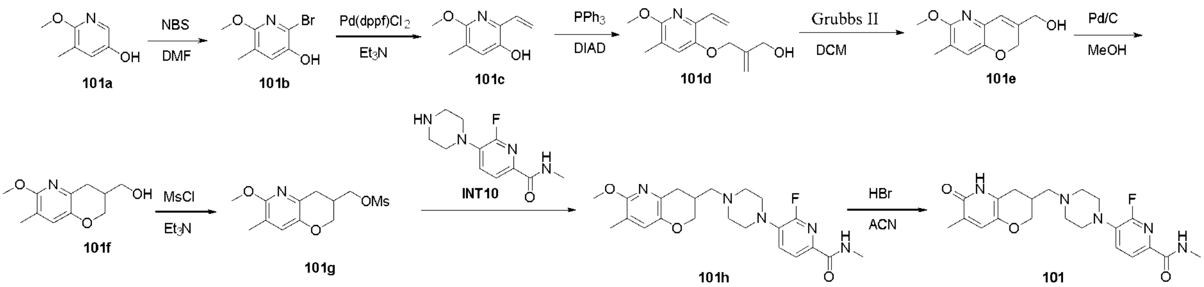


Step 1: Compound **101a** (1.7 g, 12.2 mmol) was dissolved in anhydrous ACN (20 mL), and NBS (2.4 g, 13.4 mmol) was added. The mixture was allowed to react at room temperature overnight. A saturated aqueous sodium bicarbonate solution was added to quench the reaction, and the mixture was extracted three times with ethyl acetate. The organic phases were combined, washed with saturated brine, dried over anhydrous sodium sulfate, filtered, concentrated under reduced pressure, and subjected to column chromatography to obtain the target product **101b** (2.2 g). LC-MS: $[M+H]^+$ = 217.9.

Step 2: Compound **101b** (1.2 g, 5.5 mmol), potassium vinyltrifluoroborate (1.47 g, 11 mmol), Pd(dppf)$Cl_2$ (0.4 g, 0.55 mmol), and triethylamine (1.67 g, 16.5 mmol) were dissolved in dioxane (30 mL). The system was purged with a nitrogen atmosphere and allowed to react at 100 °C for 3 h. The reaction mixture was filtered, mixed with silica gel, and subjected to column chromatography to obtain the target compound **101c** (650 mg). LC-MS: $[M+H]^+$ = 166.1.

Step 3: Compound **101c** (650 mg, 3.9 mmol), 2-methylene-1,3-propanediol (520 mg, 5.9 mmol), and triphenylphosphine (1.55 g, 5.9 mmol) were dissolved in anhydrous THF (10 mL). The system was purged with a nitrogen atmosphere and cooled to 0 °C, and then DIAD (1.2 g, 5.9 mmol) was added dropwise. Subsequently, the mixture was transferred to room temperature and allowed to react overnight. After the reaction was completed, the reaction mixture was mixed with silica gel and purified by column chromatography to obtain the target compound **101d** (893 mg). LC-MS: $[M+H]^+$ = 236.1.

Step 4: Compound **101d** (893 mg, 3.8 mmol) and Grubbs catalyst II (322 mg, 0.38 mmol) were dissolved in anhydrous DCM (25 mL). The system was purged with a nitrogen atmosphere and allowed to react at room temperature overnight. The mixture was then mixed with silica gel and purified by column chromatography to obtain the target product **101e** (400 mg). LC-MS: $[M+H]^+$ = 208.1.

Step 5: Compound **101e** (400 mg) was dissolved in methanol (10 mL), and 10% Pd/C (40 mg) was added. The system was purged with a hydrogen atmosphere and allowed to react at room temperature overnight. The reaction mixture was filtered through celite and concentrated under reduced pressure to obtain the target compound **101f** (169 mg). LC-MS: $[M+H]^+$ = 210.1.

Step 6: Compound **101f** (169 mg, 0.81 mmol) and triethylamine (0.34 mL, 2.4 mmol) were dissolved in anhydrous

DCM (10 mL), and the solution was cooled to 0 °C. MsCl (185 mg, 1.6 mmol) was then added dropwise. Subsequently, the mixture was transferred to room temperature and allowed to react for 2 h. A saturated aqueous sodium bicarbonate solution was added to quench the reaction, and the mixture was extracted three times with DCM. The organic phases were combined, washed with saturated brine, dried over anhydrous sodium sulfate, filtered, and concentrated under reduced pressure to obtain a crude product of the target compound **101g** (255 mg). LC-MS: $[M+H]^+$ = 288.1. The crude product was directly used in the next step.

Step 7: Compound **101g** (162 mg, 0.56 mmol), compound **INT10** (170 mg, 0.62 mmol), potassium iodide (187 mg, 1.13 mmol), and DIEA (219 mg, 1.69 mmol) were dissolved in anhydrous ACN (10 mL). The solution was allowed to react at 80 °C overnight, then mixed with silica gel, and subjected to column chromatography to obtain the target product **101h** (90 mg). LC-MS: $[M+H]^+$ = 430.2.

Step 8: Compound **101h** (90 mg, 0.2 mmol) was dissolved in ACN (15 mL), and a solution of hydrogen bromide in acetic acid (2 mL) was added. The mixture was allowed to react at 80 °C for 3 h. After the starting materials were completely consumed as monitored by LC-MS, the solution was cooled to room temperature, diluted with water, and extracted once with EA to remove some organic impurities. The aqueous phase was adjusted to weakly alkaline with solid sodium bicarbonate and then extracted three times with 10% MeOH in DCM. The organic phases were combined, dried over anhydrous sodium sulfate, and filtered, and the filtrate was concentrated under reduced pressure. The residue was separated by preparative TLC to obtain the target compound **101** (48 mg). LC-MS: $[M+H]^+$ = 416.2; $^1$H NMR (400 MHz, DMSO-d6) δ 11.14 (s, 1H), 8.41 (q, *J* = 4.7 Hz, 1H), 7.85 (d, *J* = 8.0 Hz, 1H), 7.57 (dd, *J* = 10.6, 8.1 Hz, 1H), 7.01 (s, 1H), 4.24 - 3.96 (m, 1H), 3.70 (dd, J = 10.7, 6.4 Hz, 1H), 3.24 - 3.10 (m, 4H), 2.77 (d, *J* = 4.7 Hz, 3H), 2.62 - 2.51 (m, 5H), 2.38 - 2.16 (m, 4H), 1.93 (s, 3H).

**Example 102**

6-Chloro-*N*-methyl-5-(4-((7-methyl-6-oxo-3,4,5,6-tetrahydro-2*H*-pyrano[3,2-*b*]pyridin-3-yl) methyl)piperazin-1-yl)picolinamide

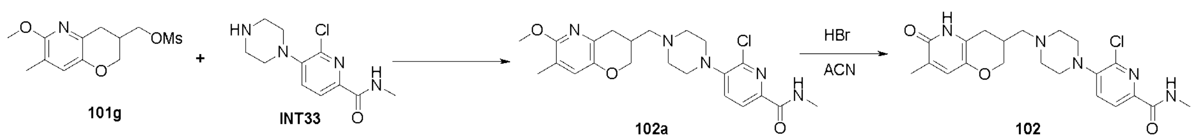

101g INT33 102a 102

6-Chloro-*N*-methyl-5-(4-((7-methyl-6-oxo-3,4,5,6-tetrahydro-2*H*-pyrano[3,2-*b*]pyridin-3-yl) methyl)piperazin-1-yl)picolinamide was prepared using a synthesis method similar to the preparation of Example 101. LC-MS: $[M+H]^+$ = 432.2; $^1$H NMR (400 MHz, DMSO-d6) δ 11.13 (s, 1H), 8.50 - 8.38 (m, 1H), 7.95 (dd, *J* = 8.2, 5.2 Hz, 1H), 7.65 (dd, *J* = 12.3, 8.2 Hz, 1H), 7.01 (s, 1H), 4.07 (d, *J* = 11.1 Hz, 1H), 3.70 (dd, *J* = 10.7, 6.7 Hz, 1H), 3.15 - 3.04 (m, 4H), 2.79 (dd, *J* = 4.8, 1.8 Hz, 3H), 2.68 - 2.53 (m, 5H), 2.38 - 2.23 (m, 4H), 1.93 (s, 3H).

**Example 103**

6-Fluoro-*N*-methyl-5-(4-((7-methyl-6-oxo-5,6-dihydro-2*H*-pyrano[3,2-*b*]pyridin-3-yl)methyl) piperazin-1-yl)picolinamide

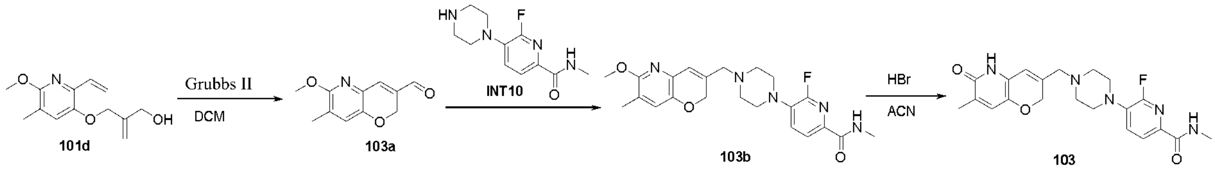

101d 103a 103b 103

Step 1: Compound **101d** (893 mg, 3.8 mmol) and Grubbs catalyst II (322 mg, 0.38 mmol) were dissolved in anhydrous DCM (25 mL). The system was purged with a nitrogen atmosphere and allowed to react at room temperature overnight. The mixture was then mixed with silica gel and purified by column chromatography to obtain the target product **103a** (108 mg). LC-MS: $[M+H]^+$ = 206.1.

Step 2: Compound **103a** (138 mg, 0.58) was dissolved in methanol (10 mL), and triethylamine (80 mg, 0.8 mmol), acetic acid (63 mg, 1.1 mmol), and compound INT10 (108 mg, 0.53 mmol) were added. The mixture was stirred at room

temperature for 1 h. Sodium cyanoborohydride (99 mg, 1.58 mmol) was added, and the mixture was allowed to react at room temperature overnight, then mixed with silica gel, and purified by column chromatography to obtain the target product **103b** (230 mg). LC-MS: $[M+H]^+$ = 428.2.

Step 3: Compound **103b** (60 mg, 0.14 mmol) was dissolved in ACN (10 mL), and a solution of hydrogen bromide in acetic acid (2 mL) was added. The mixture was allowed to react at 80 °C for 3 h. After the starting materials were completely consumed as monitored by LC-MS, the solution was cooled to room temperature, diluted with water, and extracted once with EA to remove some organic impurities. The aqueous phase was adjusted to weakly alkaline with solid sodium bicarbonate and then extracted three times with 10% MeOH in DCM. The organic phases were combined, dried over anhydrous sodium sulfate, and filtered, and the filtrate was concentrated under reduced pressure. The residue was separated and purified by preparative TLC to obtain the target compound **103** (9.5 mg). LC-MS: $[M+H]^+$ = 414.2; $^1$H NMR (400 MHz, DMSO-d6) δ 11.22 (s, 1H), 8.40 (q, *J* = 4.6 Hz, 1H), 7.85 (d, *J* = 8.0 Hz, 1H), 7.57 (dd, *J* = 10.6, 8.1 Hz, 1H), 7.06 (s, 1H), 6.24 (s, 1H), 4.63 (s, 2H), 3.21 - 3.14 (m, 4H), 3.11 (s, 2H), 2.76 (d, *J* = 4.7 Hz, 3H), 2.58 - 2.52 (m, 4H), 1.97 (s, 3H).

### Example 104

(4a*R*)-*N*-Methyl-3-((7-methyl-6-oxo-3,4,5,6-tetrahydro-2*H*-pyrano[3,2-*b*]pyridin-3-yl)methyl) -1,2,3,4,4a,5-hexahydro-7*H*-pyrazino[2,1-*c*]pyrido[3,2-*e*][1,4]oxazolidine-9-carboxamide

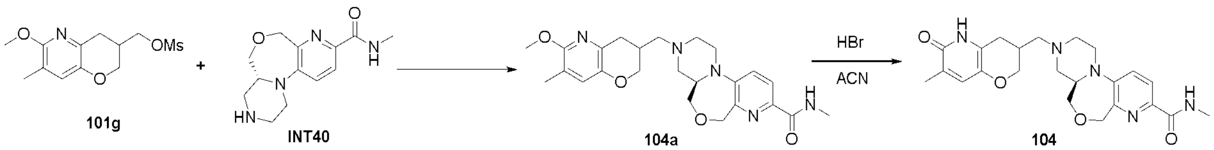


101g INT40 104a 104

(4a*R*)-*N*-Methyl-3-((7-methyl-6-oxo-3,4,5,6-tetrahydro-2*H*-pyrano[3,2-*b*]pyridin-3-yl)methyl) -1,2,3,4,4a,5-hexahydro-7*H*-pyrazino[2,1-*c*]pyrido[3,2-*e*][1,4]oxazolidine-9-carboxamide was prepared using a synthesis method similar to the preparation of Example 101. LC-MS: $[M+H]^+$ = 440.5; $^1$H NMR (400 MHz, DMSO-$d_6$) δ 11.41 (s, 1H), 8.44 - 8.35 (m, 1H), 7.82 (dd, *J* = 8.4, 1.6 Hz, 1H), 7.50 (d, *J* = 8.5 Hz, 1H), 7.03 (s, 1H), 4.95 (dd, *J* = 13.8, 3.9 Hz, 1H), 4.73 (d, *J* = 13.9 Hz, 1H), 4.47 - 4.36 (m, 2H),4.01 - 3.94 (m, 1H), 3.83 - 3.79 (m, 2H), 3.53 - 3.41 (m, 1H), 3.31 - 3.26 (m, 2H), 2.79 (d, *J* = 4.9 Hz, 3H), 2.75 - 2.59 (m, 2H), 2.58 - 2.52 (m, 2H), 2.49 - 2.30 (m, 4H), 1.93 (s, 3H).

### Example 105

(*R*)-3-(((*S*)-2-Ethyl-3-oxo-3,4,5,6,7,8-hexahydroquinoxalin-6-yl)methyl)-*N*-methyl-1,2,3,4,4a, 5-hexahydro-7*H*-pyrazino [2,1-*c*]pyrido[3,2-*e*][1,4]oxazolidine-9-carboxamide

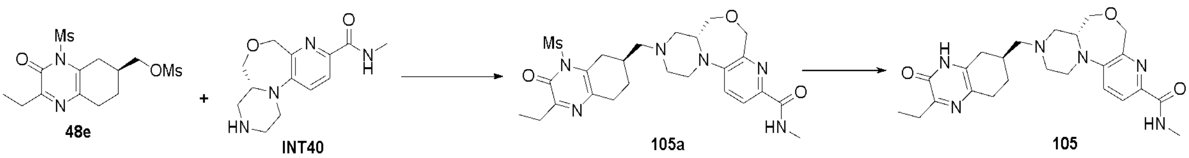

48e INT40 105a 105

(*R*)-3-(((*S*)-2-Ethyl-3-oxo-3,4,5,6,7,8-hexahydroquinoxalin-6-yl)methyl)-*N*-methyl-1,2,3,4,4a, 5-hexahydro-7*H*-pyrazino[2,1-*c*]pyrido[3,2-*e*][1,4]oxazolidine-9-carboxamide was prepared using a synthesis method similar to the preparation of Example 45. LC-MS: ESI $[M+H]^+$ = 453.4. $^1$H NMR (400 MHz, DMSO-$d_6$) δ 11.79 (s, 1H), 8.40 (q, *J* = 4.8 Hz, 1H), 7.82 (d, *J* = 8.4 Hz, 1H), 7.51 (d, *J* = 8.5 Hz, 1H), 4.96 (d, *J* = 13.9 Hz, 1H), 4.77 - 4.66 (m, 1H), 4.07 - 3.93 (m, 1H), 3.87 - 3.74 (m, 1H), 3.54 - 3.43 (m, 1H), 3.24 (s, 1H), 2.79 (d, *J* = 4.8 Hz, 3H), 2.70 - 2.58 (m, 4H), 2.61 - 2.52 (m, 4H), 2.28 (d, *J* = 7.5 Hz, 3H), 2.25 - 2.14 (m, 1H), 2.06 - 1.95 (m, 1H), 1.92 - 1.82 (m, 1H), 1.43 - 1.32 (m, 1H), 1.29 - 1.20 (m, 1H), 1.10 (t, *J* = 7.4 Hz, 3H).

### Example 106

(*S*)-6-Chloro-*N*-methyl-5-(4-((2-methyl-3-oxo-3,4,5,6,7,8-hexahydroquinoxalin-6-yl)methyl) piperazin-1-yl)picolinamide

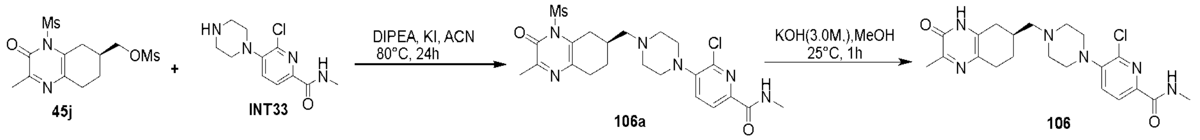


45j + INT33 → 106a → 106

(*S*)-6-Chloro-*N*-methyl-5-(4-((2-methyl-3-oxo-3,4,5,6,7,8-hexahydroquinoxalin-6-yl)methyl) piperazin-1-yl)picolinamide was prepared using a synthesis method similar to the preparation of Example 45. LC-MS: ESI $[M+H]^+$ = 431.1. $^1H$ NMR (400 MHz, DMSO) δ 11.82 (s, 1H), 8.43 (q, *J* = 4.5 Hz, 1H), 7.94 (d, *J* = 8.2 Hz, 1H), 7.67 (d, *J* = 8.2 Hz, 1H), 3.11 (s, 4H), 2.79 (d, *J* = 4.8 Hz, 3H), 2.68 - 2.52 (m, 7H), 2.35 - 2.27 (m, 2H), 2.25 - 2.14 (m, 4H), 2.21 - 1.95 (m, 1H), 1.93 - 1.87 (m, 1H), 1.48 - 1.28 (m, 1H).

**Bioactivity Assays:**

**1. PARP enzyme assay**

Experimental materials: PARP1 protein (BPS, Cat. No. 80501), PARP2 protein (BPS, Cat. No. 80502), PARP5A protein (BPS, Cat. No. 80504), Biotin-NAD+ (R&D, Cat. No. 6573), Strep-HRP (Thermo Pierce, Cat. No. 21127), NAD+ (TCI, Cat. No. D0919-5G), quantitative enhanced chemiluminescent HRP substrate kit (Thermo Pierce, Cat. No. 15159), histone (Active Motif, Cat. No. 81167), Activated DNA (Genscript, Cat. No. L05182-01&02&03), anti-rabbit IgG, HRP-linked antibody (CST, Cat. No. 7074P2), anti-Poly/Mono-ADP Ribose (E6F6A) Rabbit mAb (CST, Cat. No. 83732S), SuperSignal ELISA Femto Substrate (Thermo Pierce, Cat. No. 37074).

**1.1. PARP1 enzyme assay**

1.1.1. Buffer preparation: PBST: 1× PBS, 0.05% Tween-20; blocking buffer: 1× PBS, 0.05% Tween-20, 5% BSA; reaction buffer: 50 mM Tris-HCl (pH 7.5), 0.005% Tween-20, 0.01% BSA.

1.1.2. Coating: A 50 ng/mL Histone coating solution was prepared with 1× PBS and then transferred to a 384-well reaction plate at 25 μL/well. The plate was coated at 4 °C overnight.

1.1.3. Washing: After the coating was completed, the coating solution was discarded, and the plate was washed with the PBST solution. The method was as follows: PBST was transferred to the 384-well reaction plate at 50 μL/well, and the plate was left to stand for 5 min. The washing solution was then discarded, and the plate was refilled. The washing step was repeated 3 times. Finally, the reaction plate was patted dry and left for the next step of blocking.

1.1.4. Blocking: The blocking buffer was transferred to the 384-well reaction plate at 50 μL/well, and the plate was left to stand for 1 h.

Washing: After the blocking was completed, the blocking buffer was discarded, and the plate was washed 3 times with the PBST solution according to the method in step 2. Finally, the reaction plate was patted dry.

1.1.5. A 1000-fold solution of a compound was prepared and then transferred at 1 μL/well to a 96-well plate containing 199 μL of the reaction buffer. The mixture was well mixed, and then the well mixed compound solution was transferred to the 384-well reaction plate at 5 μL/well. 1.1.6. A 25/10-fold PARP1-DNA solution was prepared with the reaction buffer and then transferred to the 384-well reaction plate at 10 μL/well. For the negative control wells, 10 μL

of the DNA solution was transferred. The final concentrations of PARP1 and DNA were 0.02 nM and 0.8 nM, respectively.

1.1.7. A 25/10-fold NAD+ solution was prepared with the reaction buffer and then transferred to the 384-well reaction plate at 10 μL/well, with the final concentration of NAD+ being 3.5 μM. The plate was incubated at room temperature for 60 min.

1.1.8. A 25/10-fold NAD+ solution was prepared with the reaction buffer and then transferred to the 384-well reaction plate at 10 μL/well, with the final concentration of NAD+ being 3.5 μM. The plate was incubated at room temperature for 60 min.

1.1.9. Washing: After the reaction was completed, the reaction solution was discarded, and the plate was washed 3 times with the PBST solution according to the method in step 2. Finally, the reaction plate was patted dry.

1.1.10. A primary antibody (anti-Poly/Mono-ADP Ribose Rabbit mAb) was diluted 2000-fold with the blocking buffer and then added at 20 μL/well. The plate was incubated at room temperature for 1.5 h.

1.1.11. Washing: The primary antibody was discarded, and the plate was washed 3 times with the PBST solution

according to the method in step 2. Finally, the reaction plate was patted dry.

1.1.12. A secondary antibody (anti-rabbit IgG, HRP-linked Antibody) was diluted 2000-fold with the blocking buffer and then added at 20 μL/well. The plate was incubated at room temperature for 1 h.

1.1.13. Washing: The secondary antibody was discarded, and the plate was washed 3 times with the PBST solution according to the method in step 2. Finally, the reaction plate was patted dry.

1.1.14. Color development: Femto-ECL Substrate A and Femto-ECL Substrate B were mixed at a ratio of 1:1, and the mixture was transferred to the 384-well reaction plate at 25 μL/well. 1.1.15. Reading: The chemiluminescence values (RLU) were measured using Envision.

The assay results are shown in Table 9 below:

Table 9. PARP-1 enzyme assay results

| Compound | PARP-1 $IC_{50}$(nM) | Compound | PARP-1 $IC_{50}$(nM) | Compound | PARP-1 $IC_{50}$(nM) | Compound | PARP-1 $IC_{50}$(nM) |
|---|---|---|---|---|---|---|---|
| **1** | +++ | **2** | +++ | **3** | +++ | **4** | +++ |
| **5** | +++ | **6** | +++ | **7** | +++ | **8** | +++ |
| **15** | +++ | **16** | +++ | **17** | +++ | **18** | +++ |
| **19** | +++ | **20** | +++ | **21** | +++ | **22** | +++ |
| **23** | +++ | **24** | +++ | **25** | +++ | **26** | +++ |
| **27** | +++ | **28** | +++ | **29** | +++ | **30** | +++ |
| **38** | +++ | **39** | ++ | **40** | +++ | **41** | ++ |
| **42** | ++ | **43** | ++ | **44** | ++ | **45** | +++ |
| **46** | +++ | **47** | +++ | **48** | +++ | **49** | +++ |
| **50** | ++ | **51** | ++ | **52** | ++ | **53** | +++ |
| **54** | ++ | **55** | +++ | **56** | +++ | **57** | +++ |
| **58** | +++ | **59** | ++ | **60** | ++ | **61** | ++ |
| **62** | +++ | **63** | ++ | **64** | ++ | **65** | +++ |
| **66** | +++ | **67** | +++ | **68** | +++ | **69** | ++ |
| **70** | ++ | **71** | +++ | **72** | ++ | **73** | ++ |
| **74** | +++ | **75** | ++ | **76** | +++ | **77** | +++ |
| **78** | +++ | **81** | +++ | **82** | +++ | **83** | +++ |
| **84** | +++ | **85** | +++ | **86** | ++ | **87** | +++ |
| **88** | +++ | **92** | +++ | **94** | +++ | **95** | +++ |
| **96** | +++ | **100** | +++ | **101** | +++ | **105** | +++ |
| **106** | +++ | - | - | - | - | - | - |
| +++ means that $IC_{50}$ is less than or equal to 50 nM;<br>++ means that $IC_{50}$ is between 50 nM and 500 nM;<br>+ means that $IC_{50}$ is greater than 500 nM.<br>Conclusion: The compounds of the present disclosure exhibited good inhibitory activity against the PARP1 enzyme. | | | | | | | |

### 1.2. Assay for enzymatic selectivity for PARP family

Experimental materials: compounds Olaparib (Cat. No.: HY-10162, Batch No.: 136925) and XAV-939 (Cat. No.: HY-15147, Batch No.: 112781) were purchased from Shanghai MCE.

Table 10. Reagents and materials

| Name | Brand | Cat. No. |
|---|---|---|
| PARP1 Chemiluminescent assay kit | BPS | 80551 |

(continued)

| Name | Brand | Cat. No. |
|---|---|---|
| PARP2 Chemiluminescent assay kit | BPS | 80552 |
| TNKS1 Chemiluminescent assay kit | BPS | 78405 |
| PARP7 Chemiluminescent assay kit | BPS | 79729 |
| PARP11 Chemiluminescent assay kit | BPS | 80561 |
| PARP12 Chemiluminescent assay kit | BPS | 78504 |
| PARP1-His-tag | ICE | S2202T-H27H |
| PARP2-Gst-tag | ICE | S2203-H31G |
| PARP1-Biotin labeled DNA | Genscript | N20998-06&08 |
| PARP2- Biotin labeled DNA | Genscript | N20998-01&02&05 |
| $NAD^+$ | SIGMA | N6522 |
| SA-Tb | Revvity | 610SATLB |
| GST-XL665 | Revvity | 61GSTXLB |
| His-d2 | Revvity | 61HISDLB |
| DTT | Sigma-Aldrich | 646563 |
| 384 reaction plate | Greiner | 781074 |
| 384 reaction plate | Greiner | 784075 |
| 96-well polypropylene plate | Beijing Chuanglianyi Technology Co., Ltd. | CLY 96001 |

Experimental method: The formulation of the PARP enzyme reaction buffer is shown in Table 11, the formulations of the PARP enzymes are shown in Table 12, the formulations of the substrates are shown in Table 13, and the formulations of the assay solutions are shown in Table 14.

Table 11. Formulation of PARP enzyme reaction buffer

| Name | Storage concentration | Working concentration |
|---|---|---|
| 10 x PARP assay buffer | 10 x | 1 x |
| DTT | 1 M | 1 mM |
| $H_2O$ | \ | \ |

Table 12. Formulations of PARP enzymes

| Reagent | Storage concentration | Final concentration |
|---|---|---|
| PARP1 | 8.63 μM | 0.5 nM |
| PARP2 | 19.05 μM | 2 nM |
| PARP5A | 0.13 mg/mL | 0.3 ng/μL |
| PARP7 | 0.35 mg/mL | 3.9 ng/μL |
| PARP12 | 2.35 μM | 100 nM |

Table 13. Formulations of substrates

| Target | Substrate | Storage concentration | Final concentration |
|---|---|---|---|
| PARP1 | PARP Substrate Mixture 2 | 10 x | 0.1 x |
| PARP2 | PARP Substrate Mixture 1 | 10 x | 0.5 x |
| PARP5A | PARP Substrate Mixture 1 | 10 x | 0.5 x |

(continued)

| Target | Substrate | Storage concentration | Final concentration |
|---|---|---|---|
| PARP7 | PARP Substrate Mixture 2 | 10 x | 1 x |
| PARP11 | PARP Substrate Mixture 2 | 10 x | 1 x |
| PARP12 | PARP Substrate Mixture 2 | 10 x | 1 x |
| PARP1 trapping | PARP1-Biotin labeled DNA | 10 μM | 2 nM |
| PARP2 trapping | PARP2-Biotin labeled DNA | 10 μM | 4 nM |
| PARP1 trapping | $NAD^+$ | 200 mM | 12 μM |
| PARP2 trapping | $NAD^+$ | 200 mM | 3000 μM |

Table 14. Formulations of assay solutions

| Name | Stock solution concentration | Final concentration |
|---|---|---|
| SA-Tb | 200 x | 1 x |
| His-d2 | 50 x | 1 x |
| GST-XL665 | 50 x | 1 x |

Experimental procedures:

1) A 5× histone mixture was diluted 5-fold with PBS, and the resulting 1× histone mixture was added to an assay plate at 25 μL/well, followed by incubation at 4 °C overnight.
2) The assay plate was washed three times with 100 μL of a PBST buffer (5 min/wash). A blocking buffer was added at 25 μL/well, and then the plate was incubated at room temperature for 90 min.
3) The assay plate was washed three times with 100 μL of the PBST buffer (5 min/wash).
4) The diluted compound was transferred to a 384-well plate at 100 nL/well using acoustic liquid transfer technology (Echo 655), and the plate was centrifuged at 1000 rpm for 1 min.
5) The PARP enzyme solution was added at 5 μL/well, and the plate was centrifuged at 1000 rpm for 1 min.
6) The substrate mixture was added at 5 μL/well, and the plate was centrifuged at 1000 rpm for 1 min and then incubated at room temperature for 1 h.
7) The assay plate was washed three times with 100 μL of the PBST buffer (5 min/wash).
8) St-HRP diluted with the blocking buffer (dilution ratio: 1:2000) was added at 25 μL/well, followed by incubation at room temperature for 30 min.
9) The assay plate was washed three times with 100 μL of the PBST buffer (5 min/wash).
10) A mixture of ELISA ECL Sub A and ELISA ECL Sub B (mixed in equal volumes) was added at 25 μL/well.
11) The Luminescence signal values were measured using a BMG microplate reader.

The assay results are shown in Table 15 below:

Table 15. Assay results of enzymatic selectivity for PARP family

| Compound | PARP-1 $IC_{50}$ (nM) | PARP-2 $IC_{50}$ (nM) | PARP-5A $IC_{50}$ (nM) | PARP-7 $IC_{50}$ (nM) | PARP-12 $IC_{50}$ (nM) |
|---|---|---|---|---|---|
| **2** | 0.19 | 519 | 975 | >10000 | 1992 |
| **45** | 0.56 | 2018 | 1932 | 5986 | 862 |
| **Olaparib** | 0.97 | 0.41 | - | 1919 | 2514 |
| **XAV-939** | - | - | 3.92 | - | - |
| Conclusion: The compounds of the present disclosure selectively inhibited the PARP1 enzyme, exhibiting high selectivity for PARP1 over other enzymes in the PARP family. | | | | | |

**2. Assay for anti-proliferative activity against cells:**

BRCA-mutated MDA-MB-436 cells were cultured in a DMEM medium containing 10% fetal bovine serum, 100 U/mL penicillin, and 100 μg/mL streptomycin, and incubated in an incubator with 5% saturated $CO_2$ at 37 °C. When the cells grew to 80% confluence, they were collected, centrifuged at 300 g for 10 min, and then plated onto a 96-well plate at 1200 cells/well. After 24 h, different final concentrations (0 nM, 0.01 nM, 0.1 nM, 1 nM, 10 nM, 100 nM, and 1000 nM) of PARPi were added, and the cells were cultured for another 72 h.

The cells were subjected to medium exchange treatment (re-supplemented with PARPi at the same final concentrations) and then cultured for another 96 h. The 96-well plate was taken out.

The OD values at a wavelength of 450 nm were measured using the CCK8 method, and the cell inhibition rates were calculated:

Inhibition rate % = 1 - (average OD value of treatment group - average OD value of blank group)/(average OD value of control group - average OD value of blank group) × 100%.

BRCA wild-type DLD-1 cells were cultured in an RPMI-1640 medium containing 10% fetal bovine serum, 100 U/mL penicillin, and 100 μg/mL streptomycin, and incubated in an incubator with 5% saturated $CO_2$ at 37 °C. When the cells grew to 80% confluence, they were collected, centrifuged at 300 g for 10 min, and then plated onto a 96-well plate at 1000 cells/well. After 24 h, different final concentrations (0 μM, 1 μM, and 10 μM) of PARPi were added, and the cells were cultured for another 72 h. The cells were subjected to medium exchange treatment (re-supplemented with PARPi at the same final concentrations) and then cultured for another 96 h. The 96-well plate was taken out. The OD values at a wavelength of 450 nm were measured using the CCK8 method, and the cell inhibition rates were calculated:

Inhibition rate % = 1 - (average OD value of treatment group - average OD value of blank group)/(average OD value of control group - average OD value of blank group) × 100%.

The assay results are shown in Table 16 below:

Table 16. Assay results of anti-proliferative activity against cells

| Compound | MDA-MB-436 $IC_{50}$(nM) | DLD-1 $IC_{50}$(nM) | Compound | MDA-MB-436 $IC_{50}$(nM) | DLD-1 $IC_{50}$(nM) |
|---|---|---|---|---|---|
| **1** | 300 | >1000 | **2** | 0.25 | >1000 |
| **3** | 1.5 | >1000 | **4** | 4.0 | >1000 |
| **5** | 7.5 | >1000 | **6** | 1.0 | >1000 |
| **38** | 3.5 | >1000 | **39** | 40.0 | >1000 |
| **40** | 4.5 | >1000 | **41** | 16.0 | >1000 |
| **42** | 9.0 | >1000 | **44** | 15.0 | >1000 |
| **45** | 2.5 | >1000 | **46** | 6.7 | >1000 |
| **47** | 4.0 | >1000 | **48** | 1.2 | >1000 |
| **49** | 300 | >1000 | **50** | 350 | >1000 |
| **51** | 160 | >1000 | **52** | 200 | >1000 |
| **53** | 1.5 | >1000 | **54** | 250 | >1000 |
| **55** | 21 | >1000 | **56** | 45 | >1000 |
| **57** | 25 | >1000 | **58** | 140 | >1000 |
| **59** | 300 | >1000 | **60** | 120 | >1000 |
| **61** | 360 | >1000 | **62** | 3.5 | >1000 |
| **63** | 430 | >1000 | **64** | 65 | >1000 |
| **65** | 0.7 | >1000 | **66** | 4.5 | >1000 |
| **67** | 4.5 | >1000 | **68** | 6.0 | >1000 |
| **69** | 180 | >1000 | **70** | 300 | >1000 |

(continued)

| Compound | MDA-MB-436 $IC_{50}$(nM) | DLD-1 $IC_{50}$(nM) | Compound | MDA-MB-436 $IC_{50}$(nM) | DLD-1 $IC_{50}$(nM) |
|---|---|---|---|---|---|
| **71** | 2.5 | >1000 | **73** | 15 | >1000 |
| **74** | 7.0 | >1000 | **76** | 9.6 | >1000 |
| **77** | 3.9 | >1000 | **78** | 2.8 | >1000 |
| **79** | 130 | >1000 | **80** | 300 | >1000 |
| **81** | 4.1 | >1000 | **82** | 3.6 | >1000 |
| **83** | 6.5 | >1000 | **84** | 6.0 | >1000 |
| **85** | 7.0 | >1000 | **86** | 16 | >1000 |
| **87** | 8.5 | >1000 | **88** | 6.5 | >1000 |
| **92** | 25.5 | >1000 | **95** | 10.5 | >1000 |
| **96** | 1.0 | >1000 | **100** | 15.5 | >1000 |
| **101** | 6.0 | >1000 | **106** | 6.5 | >1000 |
| Conclusion: The compounds of the present disclosure exhibited significant inhibitory effects on BRCA-mutated MDA-MB-436 cells, but had no significant inhibitory effects on BRCA wild-type DLD-1 cells, indicating that the compounds of the present disclosure specifically inhibit homologous recombination-deficient tumor cells. | | | | | |

**3. Assay for inhibition of hERG potassium ion channel by compounds**

Cell culture and treatment: CHO cells stably expressing hERG were cultured in a cell culture flask and incubated in an incubator with 5% $CO_2$ at 37 °C. When the cell density reached 60%-80%, the cell culture medium was removed by pipetting. The cells were washed once with PBS, and then Detachin was added for detachment. After complete detachment, a culture medium was added for neutralization. The cells were then centrifuged, and the supernatant was removed by pipetting. A culture medium was further added for resuspension, and the cell density was adjusted to (2-5) $\times 10^6$ cells/mL for later use.

Compound preparation: The compound stock solution was diluted with 100% DMSO. Specifically, 10 μL of the compound stock solution was added to 20 μL of DMSO, followed by a 3-fold serial dilution to obtain 6 concentrations. 4 μL of each of the 6 compound solutions at different concentrations was taken and added to 396 μL of an extracellular fluid, resulting in a 100-fold dilution to obtain 6 intermediate concentrations. Then, 80 μL of each of the 6 intermediate-concentration compound solutions was taken and added to 320 μL of an extracellular fluid, resulting in a 5-fold dilution to achieve the final test concentrations. The highest test concentration was 40 μM. There were 6 concentrations in total, which were 40 μM, 13.33 μM, 4.44 μM, 1.48 μM, 0.49 μM, and 0.16 μM in sequence. The DMSO content in the final test concentration did not exceed 0.2%. DMSO at this concentration has no effect on the hERG potassium channel. During the compound preparation, the entire dilution process was completed by a Bravo instrument.

Electrophysiological recording process: The single-cell high-impedance sealing and whole-cell mode formation processes were all completed automatically by a Qpatch instrument. After achieving the whole-cell recording mode, the cells were clamped at -80 mV. Before a 5-second +40 mV depolarizing stimulus was applied, a 50-millisecond -50 mV prepulse voltage was first applied. The potential was then repolarized to -50 mV and held for 5 s, and finally returned to -80 mV. This voltage stimulation was applied every 15 s. After 2 min of recording, the extracellular fluid was administered, followed by recording for 5 min. Then, the compound administration process began, starting from the lowest test compound concentration. Each test concentration was applied for 2.5 min. After all the concentrations were applied consecutively, the positive control compound, 3 μM Cisapride, was administered. At least 3 cells ($n \geq 3$) were tested for each concentration.

Data processing: Data analysis and processing were performed using GraphPad Prism 5.0 and Excel software. The $IC_{50}$ of the compound was calculated using GraphPad Prism 5 software by equation fitting as follows:

$$Y=Bottom + (Top-Bottom)/(1+10^{\wedge}((LogIC50-X)*HillSlope))$$

where X is the Log value of the test concentration of the test sample, Y is the inhibition percentage at the corresponding concentration, and Bottom and Top are the minimum and maximum inhibition percentages, respectively.

Table 17. Results of inhibition of hERG potassium ion channel by compounds

| Compound | Inhibition rate at 20 μM | Compound | Inhibition rate at 20 μM |
|---|---|---|---|
| **2** | 6.70% | **6** | 7.97% |
| **45** | 13.04% | **62** | 49.82% |
| **67** | 4.18% | **74** | 17.69% |
| **83** | 0.92% | **87** | 7.34% |
| Conclusion: Compounds **2**, **6**, **45**, **62**, **67**, **74**, **83**, **87**, etc., of the present disclosure exhibited relatively weak inhibitory effects on the hERG potassium ion channel. | | | |

**4. Pharmacokinetic evaluation of compounds in Balb/c mice**

Experimental objective: To understand the pharmacokinetic profiles of the compounds. Experimental basis: Technical guidelines for non-clinical pharmacokinetic studies of chemical drugs, 2014.

Experimental scheme: The pharmacokinetic profiles of the compounds were investigated by intravenous administration (1 mg·kg$^{-1}$) and intragastric administration (1 mg·kg$^{-1}$) in Balb/c mice.

Sample preparation: About 0.2 mg of the compound was weighed out and dissolved in 10 μL of DMSO, and then a sodium chloride solution for injection was added to prepare a 0.1 mg·mL$^{-1}$ compound solution for later administration.

Sample collection: Six male Balb/c mice (Chengdu Dossy Experimental Animals Co., Ltd., License No.: SCXK (Sichuan) 2020-030) were used. Three of the mice were subjected to intravenous administration (IV) at 1 mg·kg$^{-1}$, while the other three were subjected to intragastric administration (PO) at 1 mg·kg$^{-1}$. About 0.05 mL of blood was collected at 5 min, 15 min, 30 min, 1 h, 2 h, 4 h, 6 h, 8 h, 10 h, 24 h, and 48 h after administration. The collected blood was centrifuged at 3500 rpm for 15 min. The supernatant plasma was then collected and cryopreserved at -40 °C for later analysis. The plasma concentration was quantitatively analyzed using the LC-MS/MS method, and pharmacokinetic parameters such as time to peak (Cmax), area under plasma concentration-time curve (AUC(0-t)), half-life ($T_{1/2}$), clearance rate (CL), tissue distribution (Vdss), and bioavailability (F) were calculated.

Table 18. Results of pharmacokinetic test of compounds in Balb/c mice

| Compound | Dose<br>Route of administration | $C_{max}$ ug/L | $T_{1/2}$ h | $AUC_{(0-t)}$ ug/L*h | CL L/h/kg | Vdss L/kg | F % |
|---|---|---|---|---|---|---|---|
| **2** | 1mg/kg, PO | 2353 | 1.36 | 5397 | 0.19 | 0.38 | - |
| **45** | 1mg/kg, PO | 359 | 7.98 | 1543 | 0.59 | 6.85 | - |
| **51** | 1mg/kg, PO | 6285 | 15.88 | 96792 | 0.01 | 0.04 | - |
| **53** | 1mg/kg, PO | 3650 | 7.10 | 22318 | 0.04 | 0.42 | - |
| **74** | 1mg/kg, PO | 2351 | 2.52 | 17096 | 0.29 | 1.06 | - |
| **Conclusion:** The compounds of the present disclosure exhibited good pharmacokinetic properties in Balb/c mice. | | | | | | | |

**5. Evaluation of cerebrospinal fluid and plasma distribution of compounds *in vivo***

Experimental procedures: The compound was weighed out, a small amount of DMSO was added, and then a sodium chloride solution for injection was added to prepare a 5 mg·mL$^{-1}$ compound solution for later administration. Male rats were subjected to intravenous administration at 5 mg·kg$^{-1}$, and cerebrospinal fluid and whole blood (n = 1) were collected at 0.25 h and 2 h after administration. The whole blood was centrifuged at 3500 rpm for 15 min, and the supernatant plasma was collected. 10 μL of the plasma and 10 μL of the cerebrospinal fluid were each added to a centrifuge tube, and 40 μL of acetonitrile containing 20 ng·mL$^{-1}$ internal standard SAHA was added for precipitation. The mixture was vortexed for 30 s and centrifuged at 13000 rpm for 15 min. The supernatant was collected and transferred to a sample vial for later analysis. Range of standard curve: 1-1000 ng·mL$^{-1}$.

Table 19. Assay results of cerebrospinal fluid and plasma distribution of compounds in rats after administration

| Compound | 0.25h | | 2h | |
|---|---|---|---|---|
| | Cerebrospinal fluid (ng/g) | Plasma (ng/mL) | Cerebrospinal fluid (ng/g) | Plasma (ng/mL) |
| **39** | 103 | 8199 | 26 | 4893 |
| **43** | 16 | 8027 | 3 | 7689 |
| **47** | 64 | 1008 | 13 | 43 |
| **51** | 53 | 41927 | 25 | 21710 |
| **64** | 102 | 1623 | 0 | 503 |
| **74** | 60 | 8788 | 19 | 5556 |
| **82** | 119 | 2741 | 0 | 77 |
| **83** | 86 | 1501 | 0 | 28 |
| **101** | 52 | 2825 | 8 | 592 |
| Conclusion: Some of the compounds of the present disclosure exhibit relatively good brain penetration potential; in particular, compounds **39**, **43**, **47**, **51**, **64**, **74**, **82**, **83**, **101**, etc., showed relatively high distribution concentrations in cerebrospinal fluid. | | | | |

**6. Assay for selectivity of compounds for PARP6**

The main instruments and reagents are shown in Table 20:

Table 20. Main instruments and reagents for PARP6 assay

| Reagent | Brand | Cat. No. |
|---|---|---|
| PARP6 Chemiluminescent assay kit | BPS | 80556 |
| 384-well plate | Greiner | 781074 |
| 96-well microplate | Nunc | 249944 |
| Instrument | Manufacturer | Model |
| Benchtop centrifuge | Cence | TDZ5-WS |
| High-throughput drug-screening multi-mode microplate reader | BMG | PHERAstar FSX |
| Echo | BECKMAN | 655 system |

Experimental procedures: The enzymatic activity of PARP6 was assayed by the ELISA method. First, substrate coating and blocking were performed: A 5× histone mixture was diluted 5-fold with PBS, and the diluted histone mixture was added to an assay plate at 25 μL/well, followed by incubation at 4 °C overnight. The assay plate was washed three times with 100 μL of a PBST buffer (5 min/wash), and 25 μL of a blocking buffer was added, followed by incubation for 90 min. The assay plate was washed three times with 100 μL of the PBST buffer (5 min/wash). Subsequently, the enzymatic activity assay of PARP6 was performed: The compound was added to the reaction plate at 100 nL/well, and the plate was centrifuged at 1000 rpm for 1 min. Then, the PARP enzyme was added at 5 μL/well, and the plate was centrifuged at 1000 rpm for 1 min. After that, a substrate mixture was added at 5 μL/well, and the plate was centrifuged at 1000 rpm for 1 min and then incubated at 25 °C for 1 h. Finally, experimental signal detection was performed: The assay plate was washed three times with 100 μL of the PBST buffer (5 min/wash), and St-HRP diluted with the blocking buffer (dilution ratio: 1:2000) was added at 25 μL/well, followed by incubation at 25 °C for 30 min. The assay plate was washed three times with 100 μL of the PBST buffer (5 min/wash). A mixture of ELISA ECL Sub A and ELISA ECL Sub B (mixed in equal volumes) was added at 25 μL/well. The Luminescence signal values were measured using a BMG microplate reader. The $IC_{50}$ values of the compounds were calculated using the nonlinear fitting formula in GraphPad software. The inhibition rates were calculated using the following formula: Inhibition rate (% Inh) = (signal of compound - signal of negative control)/(signal of positive control - signal of negative control) × 100%. Negative control: DMSO (maximum signal value); positive control: the highest concentration test value of the positive control compound (minimum signal value); reference compounds: AZD5305 and Olaparib purchased from Shanghai MedChemExpress.

The experimental results are shown in Table 21.

Table 21. Assay results of effects of compounds on enzymatic activity of PARP6

| Compound | PARP6 $IC_{50}$ nM | Compound | PARP6 $IC_{50}$ nM | Compound | PARP6 $IC_{50}$ nM |
|---|---|---|---|---|---|
| **AZD5305** | 8188 | **2** | >10000 | **43** | >10000 |
| **Olaparib** | 392 | - | - | - | - |
| Conclusion: Compounds **2**, **43**, etc., of the present disclosure exhibited relatively weak inhibitory effects on PARP6, demonstrating high selectivity. | | | | | |

**7. Hematological toxicity assay**

CD34+ hematopoietic stem and progenitor cells (hmPB34-P-SC) were cultured in an RPMI 1640 complete medium (supplemented with 25 ng/mL IL-3, 25 ng/mL IL-6, 25 ng/mL SCF, and 10% fetal bovine serum) at 37 °C with 5% $CO_2$ overnight. The next day, the cells were resuspended and seeded in a 96-well plate at a concentration of 1000 cells/well, and meanwhile, different concentrations of the drug were added for treatment, with 3 replicate wells set for each concentration. After 5 days of culture, the number of viable cells in each well was determined using a CellTiter-Glo 2.0 kit, followed by detection by a microplate reader.

Table 22. Assay results of inhibitory activity of compounds against CD34+ hematopoietic stem and progenitor cells (hmPB34-P-SC)

| Compound | hmPB34-P-SC $IC_{50}$ nM | Compound | hmPB34-P-SC $IC_{50}$ nM | Compound | hmPB34-P-SC $IC_{50}$ nM |
|---|---|---|---|---|---|
| **Olaparib** | 200 | **2** | 738 | **45** | 331 |
| **51** | >1000 | **67** | >1000 | **74** | >1000 |
| **77** | 307 | **81** | >1000 | **85** | >1000 |
| **106** | >1000 | - | - | - | - |
| Conclusion: The compounds of the present disclosure exhibited relatively weak inhibitory effects on CD34+ hematopoietic stem and progenitor cells (hmPB34-P-SC); in particular, compounds **51**, **67**, **74**, **81**, **85**, **106**, etc., showed $IC_{50}$ values greater than 1000 nM for their inhibitory activity against hmPB34-P-SC, which was significantly superior to that of the positive control drug Olaparib. | | | | | |

**8. In vivo pharmacodynamic study of compound 2 in MDA-MB-436 breast cancer subcutaneous xenograft tumor model in nude mice**

Experimental process: Twenty-four female NOD/SCID mice were used in the experiment. They were randomly divided into 4 groups of 6: a blank control group, compound **2** 1 mg/kg and 3 mg/kg dose groups, and a reference compound Olaparib 100 mg/kg group. All groups were subjected to oral administration once daily. Meanwhile, the mice were weighed every 2 days, and the length and width of the tumors were measured with a vernier caliper. After 30 days of administration, the tumor-bearing mice were anesthetized and sacrificed. The tumor tissues were dissected, weighed, and photographed, and the tumor growth inhibition rates were calculated.

Experimental conclusion: The experimental results are shown in FIG. 1. After cell inoculation, each treatment group was subjected to drug administration for 30 consecutive days. Compared to the blank control group, all treatment groups (the Olaparib 100 mg/kg group, and the compound **2** 1 mg/kg and 3 mg/kg groups) showed significant inhibitory effects on tumor growth in the NOD/SCID mouse subcutaneous tumor model of the human breast cancer cell line MM436. The tumor growth inhibition rates of the treatment groups were 65.81%, 92.61%, and 95.74%, respectively; the relative tumor proliferation rates (T/C%) thereof were 29.23%, 9.03%, and 6.02%, respectively. Among all treatment groups, the compound **2** 3 mg/kg group showed the best tumor inhibitory effect; the tumor inhibitory effect of the compound **2** 3 mg/kg group was significantly superior to that of the reference drug Olaparib 100 mg/kg group.

**9. In vivo pharmacodynamic study of compound 53 in MDA-MB-436 breast cancer subcutaneous xenograft tumor model in nude mice**

Experimental process: Eighteen female NOD/SCID mice were used in the experiment. They were randomly

divided into 3 groups of 6: a blank control group, a compound AZD9574 (3 mg/kg) group, and a compound **53** (1 mg/kg) group. All groups were subjected to oral administration once daily. Meanwhile, the mice were weighed every 2 days, and the length and width of the tumors were measured with a vernier caliper. After 30 days of administration, the tumor-bearing mice were anesthetized and sacrificed. The tumor tissues were dissected, weighed, and photographed, and the tumor growth inhibition rates were calculated. Compound AZD9574 was synthesized with reference to the synthesis method in Example 20 of patent WO2021260092.

Conclusion: The experimental results are shown in FIG. 2. After cell inoculation, each treatment group was subjected to drug administration for 30 consecutive days. Compared to the blank control group, the compound AZD9574 (3 mg/kg) group and the compound **53** (1 mg/kg) group showed significant inhibitory effects on tumor growth in the NOD/SCID mouse subcutaneous tumor model of the human breast cancer cell line MM436. The anti-tumor effect of the compound **53** (1 mg/kg) group was superior to that of the AZD9574 (3 mg/kg) group.

## Claims

1. A compound represented by formula I or a pharmaceutically acceptable form thereof, wherein the structure of formula I is as follows:

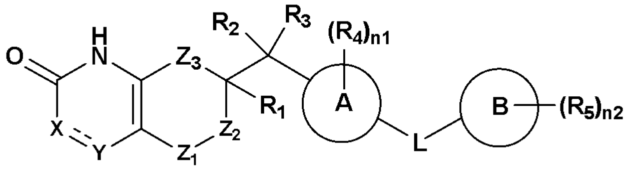

**formula I**

wherein
the structural fragment

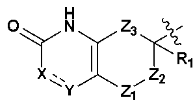

is selected from the following structures:

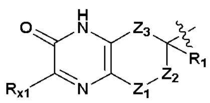 , 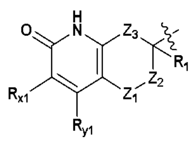 , 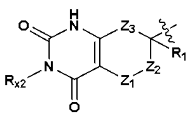 , 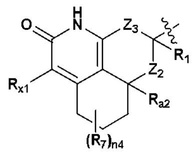 , 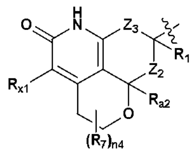 ,

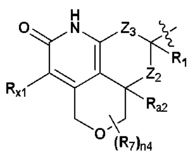 , 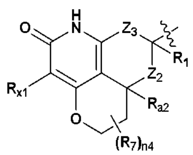 , 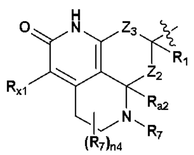 , 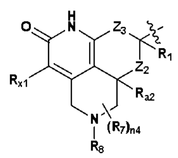 , 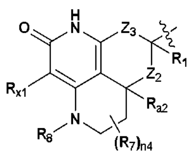 ,

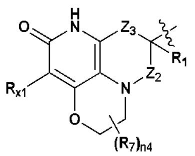 , 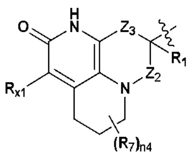 , 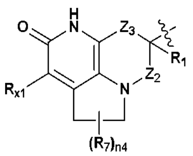 , 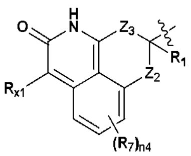 , 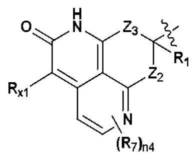 ,

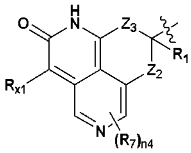, 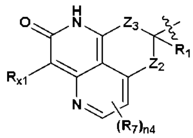, 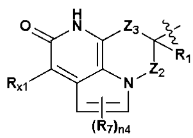, 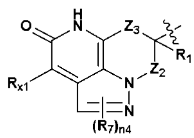, and 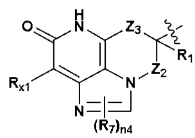;

$R_{x1}$ and $R_{y1}$ are independently selected from hydrogen, deuterium, halogen, cyano, amino, and the following group optionally substituted with 0-3 substituents: $C_{1-4}$ alkyl, -NH-$C_{1-4}$ alkyl, -N($C_{1-4}$ alkyl)$_2$, -O-$C_{1-4}$ alkyl, -S-$C_{1-4}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{x1}$ and $R_{y1}$, the substituent is selected from: deuterium, halogen, oxo, -OH, -$NH_2$, -CN, $C_{1-4}$ alkyl, $C_{1-4}$ fluoroalkyl, -NH-$C_{1-4}$ alkyl, -N($C_{1-4}$ alkyl)$_2$, -O-$C_{1-4}$ alkyl, and 3- to 6-membered cycloalkyl; in $R_{x1}$ and $R_{y1}$, the 4- to 6-membered heterocycloalkyl or 5- to 6-membered heteroaryl contains 1-3 heteroatoms selected from at least one of N, S, and O;

$R_{x2}$ is selected from hydrogen, deuterium, and the following group optionally substituted with 0-3 substituents: $C_{1-4}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{x2}$, the substituent is selected from: deuterium, halogen, -OH, -$NH_2$, -CN, oxo, $C_{1-4}$ alkyl, $C_{1-4}$ fluoroalkyl, -NH-$C_{1-4}$ alkyl, -N($C_{1-4}$ alkyl)$_2$, -O-$C_{1-4}$ alkyl, and 3- to 6-membered cycloalkyl; in $R_{x2}$, the 4-to 6-membered heterocycloalkyl or 5- to 6-membered heteroaryl contains 1-3 heteroatoms selected from at least one of N, S, and O;

$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -O-$C_{1-4}$ alkyl, -O-$C_{1-4}$ fluoroalkyl, -O-$C_{1-4}$ deuterated alkyl, -NH-$C_{1-4}$ alkyl, and -N($C_{1-4}$ alkyl)$_2$; n4 is selected from 1, 2, 3, 4, 5, and 6;

$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 6-membered cycloalkyl;

$Z_1$ is selected from a bond, O, $NR_{a1}$, $CR_{a2}R_{a3}$, and C=$Z_\mathbf{4}$;

$Z_2$ is selected from O, $NR_{b1}$, and $CR_{b2}R_{b3}$;

$Z_3$ is selected from O and $CR_{c1}R_{c2}$;

$R_{a1}$ and $R_{b1}$ are independently selected from hydrogen, deuterium, and the following group optionally substituted with 0-6 substituents: $C_{1-6}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; in $R_{a1}$ and $R_{b1}$, the substituent is selected from: deuterium, halogen, -OH, -$NH_2$, -CN, and 3- to 6-membered cycloalkyl; in $R_{a1}$ and $R_{b1}$, the 4- to 6-membered heterocycloalkyl or 5- to 6-membered heteroaryl contains 1-3 heteroatoms selected from at least one of N, S, and O;

$R_{a2}$, $R_{a3}$, $R_{b2}$, $R_{b3}$, $R_{c1}$, and $R_{c2}$ are independently selected from hydrogen, deuterium, fluorine, hydroxyl, amino, $C_{1-4}$ alkyl, $C_{1-4}$ deuterated alkyl, $C_{1-4}$ fluoroalkyl, and hydroxyl-substituted $C_{1-4}$ alkyl;

$R_1$ is selected from hydrogen, deuterium, fluorine, hydroxyl, amino, cyano, $C_{1-4}$ alkyl, $C_{1-4}$ deuterated alkyl, $C_{1-4}$ fluoroalkyl, and hydroxyl-substituted $C_{1-4}$ alkyl;

$R_2$ and $R_3$ are independently selected from hydrogen, deuterium, fluorine, hydroxyl, amino, $C_{1-4}$ alkyl, $C_{1-4}$ deuterated alkyl, $C_{1-4}$ fluoroalkyl, and hydroxyl-substituted $C_{1-4}$ alkyl; or $R_2$ and $R_3$, together with the atom to which they are attached, form a 3- to 6-membered cycloalkyl;

ring A is selected from 4- to 8-membered heterocycloalkyl; in ring A, the 4- to 8-membered heterocycloalkyl contains 1-3 N heteroatoms, and one of the N heteroatoms is connected to the structural fragment

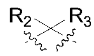;

$R_4$ is selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; or any two $R_4$, together with the atom(s) to which they are attached, form a 3- to 6-membered carbocycle or a 5- to 7-membered heterocycle; when any two $R_4$, together with the atom(s) to which they are attached, form a ring, the 5- to 7-membered heterocycle contains 1-3 heteroatoms selected from at least one of N and O;

L is selected from a bond, O, $NR_{L1}$, and $CR_{L2}R_{L3}$;

$R_{L1}$ is selected from hydrogen, deuterium, $C_{1-4}$ alkyl, $C_{1-4}$ deuterated alkyl, $C_{1-4}$ fluoroalkyl, and hydroxyl-substituted $C_{1-4}$ alkyl;

$R_{L2}$ and $R_{L3}$ are independently selected from hydrogen, deuterium, fluorine, hydroxyl, amino, $C_{1-4}$ alkyl, $C_{1-4}$

deuterated alkyl, $C_{1-4}$ fluoroalkyl, and hydroxyl-substituted $C_{1-4}$ alkyl;

ring B is selected from 5- to 10-membered aryl and 5- to 10-membered heteroaryl; in ring B, the 5- to 10-membered heteroaryl contains 1-3 heteroatoms selected from at least one of N, S, and O;

$R_5$ is selected from hydrogen, deuterium, halogen, cyano, $-(CR_{5a}R_{5b})_{n3}OR_{5c}$, $-(CR_{5a}R_{5b})_{n3}N(R_{5c}R_{5d})$, $-CO-OR_{5c}$, $-CONH_2$, $-CONHR_{5c}$, $-CON(R_{5c}R_{5d})$, $-NHCOR_{5c}$, $-SO_2R_{5c}$, $-SO_2NHR_{5c}$, $-SO_2NR_{5c}R_{5d}$,

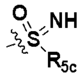,
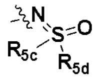,

and the following group substituted with 1 or more $R_6$: $C_{1-6}$ alkyl, $C_{2-6}$ alkenyl, $C_{2-6}$ alkynyl, 3- to 6-membered cycloalkyl, 4- to 6-membered heterocycloalkyl, phenyl, or 5- to 6-membered heteroaryl; or two adjacent $R_5$, together with the atoms to which they are attached, form a 5- to 6-membered carbocycle, a 5-to 6-membered heterocycle, a benzene ring, or a 5- to 6-membered heteroaromatic ring; in $R_5$, the 4- to 6-membered heterocycloalkyl or 5- to 6-membered heteroaryl contains 1-3 heteroatoms selected from at least one of N, S, and O; when two adjacent $R_5$, together with the atoms to which they are attached, form a ring, the 5- to 6-membered heterocycle or 5- to 6-membered heteroaromatic ring contains 1-3 heteroatoms selected from at least one of N, S, and O;

or $R_5$ and $R_4$, together with the atoms to which they are attached, form a 5- to 7-membered heterocycle; when $R_5$ and $R_4$, together with the atoms to which they are attached, form a ring, the 5- to 7-membered heterocycle contains 1-3 heteroatoms selected from at least one of N and O;

$R_{5a}$ and $R_{5b}$ are independently selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; or $R_{5a}$ and $R_{5b}$, together with the atom to which they are attached, form a 3- to 6-membered carbocycle or 4- to 6-membered heterocycle substituted with 0-6 substituents; when $R_{5a}$ and $R_{5b}$, together with the atom to which they are attached, form a ring, the substituent is selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy; when $R_{5a}$ and $R_{5b}$, together with the atom to which they are attached, form a ring, the 5- to 6-membered heterocycle contains 1-3 heteroatoms selected from at least one of N, S, and O;

$R_{5c}$ and $R_{5d}$ are independently selected from hydrogen, deuterium, and the following group substituted with 0-6 substituents: $C_{1-4}$ alkyl, 6- to 10-membered aryl, 5- to 10-membered heteroaryl, 4- to 8-membered heterocycloalkyl, 3- to 8-membered cycloalkyl, 6- to 10-membered spirocycloalkyl, 6- to 10-membered heterospirocycloalkyl, 6- to 10-membered bridged cycloalkyl, or 6- to 10-membered bridged heterocycloalkyl; in $R_{5c}$ and $R_{5d}$, the substituent is selected from: deuterium, halogen, -OH, -CN, $C_{1-4}$ alkyl, $C_{1-4}$ deuterated alkyl, $C_{1-4}$ fluoroalkyl, $C_{1-4}$ alkoxy, 3- to 6-membered cycloalkyl, 4- to 6-membered heterocycloalkyl, 3- to 6-membered cycloalkylmethyl, 4- to 6-membered heterocycloalkylmethyl, $-NH-C_{1-6}$ alkyl, $-N(C_{1-6}$ alkyl$)_2$, $-OC_{1-6}$ alkyl, $-CONH_2$, and $-CONH-C_{1-6}$ alkyl;

in $R_{5c}$ and $R_{5d}$, the 5- to 10-membered heteroaryl, 4- to 8-membered heterocycloalkyl, 6- to 10-membered heterospirocycloalkyl, or 6- to 10-membered bridged heterocycloalkyl contain 1-3 heteroatoms selected from at least one of N, S, and O; in the substituents in $R_{5c}$ and $R_{5d}$, the 4- to 6-membered heterocycloalkyl or 4- to 6-membered heterocycloalkylmethyl contains 1-3 heteroatoms selected from at least one of N, S, and O;

or $R_{5a}$ and $R_{5c}$, $R_{5a}$ and $R_{5d}$, $R_{5b}$ and $R_{5c}$, $R_{5b}$ and $R_{5d}$, or $R_{5c}$ and $R_{5d}$, together with the atom(s) to which they are attached, form a 4- to 6-membered heterocycle substituted with 0-6 substituents selected from at least one of hydrogen, deuterium, fluorine, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, and $C_{1-4}$ fluoroalkoxy, wherein the 4- to 6-membered heterocycle contains 1-3 heteroatoms selected from at least one of N, S, and O;

$R_6$ is selected from H, deuterium, fluorine, $C_{1-4}$ alkyl, $C_{1-4}$ deuterated alkyl, and $C_{1-4}$ fluoroalkyl;

n1 is selected from 1, 2, 3, 4, 5, 6, 7, and 8;

n2 is selected from 1, 2, 3, 4, and 5;

n3 is selected from 0, 1, 2, 3, 4, and 5;

the pharmaceutically acceptable form is selected from a pharmaceutically acceptable salt, ester, stereoisomer, polymorph, solvate, nitrogen oxide, isotopically labeled compound, metabolite or prodrug.

**2.** The compound according to claim 1, wherein

$R_1$ is selected from hydrogen, deuterium, fluorine, hydroxyl, amino, and $C_{1-4}$ alkyl; preferably,

$R_1$ is selected from hydrogen, deuterium, fluorine, and methyl.

3. The compound according to claim 1 or 2, wherein $R_2$ and $R_3$ are independently selected from hydrogen, deuterium, fluorine, $C_{1-4}$ alkyl, and hydroxyl-substituted $C_{1-4}$ alkyl; preferably, $R_2$ and $R_3$ are independently selected from hydrogen, deuterium, fluorine, methyl, and hydroxymethyl.

4. The compound according to any one of claims 1-3, wherein

L is selected from a bond, O, $NR_{L1}$, and $CR_{L2}R_{L3}$; $R_{L1}$ is selected from hydrogen, deuterium, methyl, deuterated methyl, fluoromethyl, and hydroxymethyl; $R_{L2}$ and $R_{L3}$ are independently selected from hydrogen, deuterium, fluorine, hydroxyl, amino, methyl, deuterated methyl, fluoromethyl, and hydroxymethyl;
preferably, L is selected from a bond, O, and NH.

5. The compound according to any one of claims 1-4, wherein

$R_4$ is selected from at least one of hydrogen, deuterium, fluorine, hydroxyl, amino, methyl, deuterated methyl, fluoromethyl, ethyl, methoxy, deuterated methoxy, fluoromethoxy, and hydroxymethyl; or two $R_4$ attached to adjacent C atoms, together with the atoms to which they are attached, form a 3- to 5-membered carbocycle or a 5- to 6-membered heterocycle;
when two $R_4$ attached to adjacent C atoms, together with the atoms to which they are attached, form a ring, the 5- to 6-membered heterocycle contains 1-2 heteroatoms selected from at least one of N and O; n1 is selected from 1, 2, 3, and 4;
preferably, $R_4$ is selected from at least one of hydrogen, deuterium, fluorine, hydroxyl, amino, methyl, ethyl, hydroxymethyl, and methoxy; or two $R_4$ attached to adjacent carbon atoms, together with the atoms to which they are attached, form a 3- to 5-membered carbocycle or a 5- to 6-membered heterocycle; when two $R_4$ attached to adjacent C atoms, together with the atoms to which they are attached, form a ring, the 5- to 6-membered heterocycle contains 1 heteroatom selected from O; n1 is selected from 1 and 2.

6. The compound according to any one of claims 1-5, wherein the structural fragment

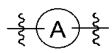

is selected from:

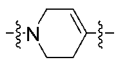, 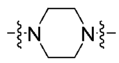, 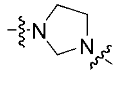, 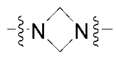,

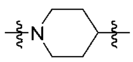, 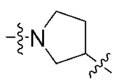, 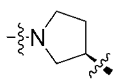, 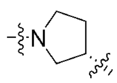, 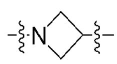, and 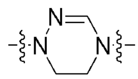;

preferably, the structural fragment

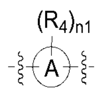

is selected from:

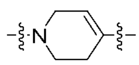, 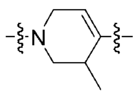,

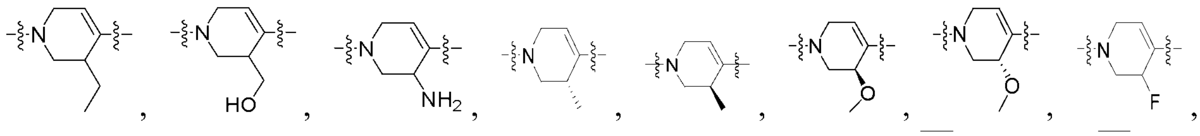

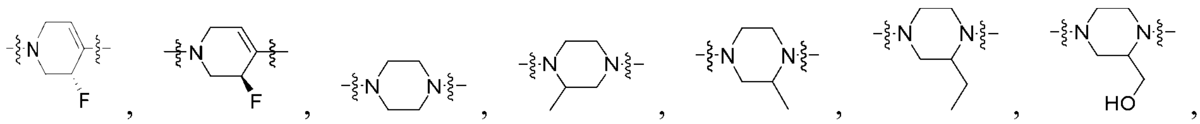

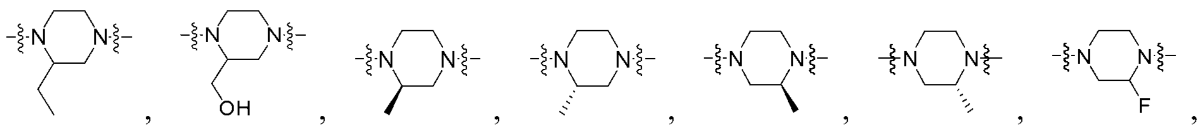

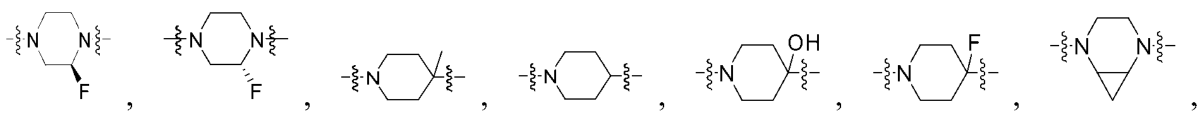

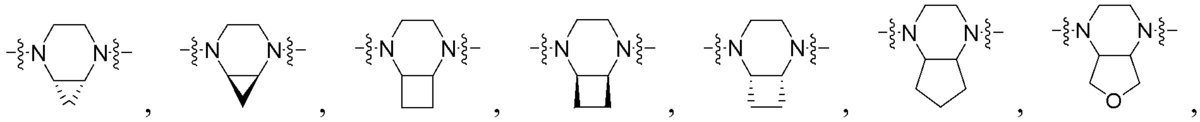

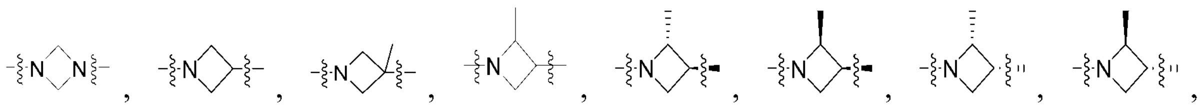

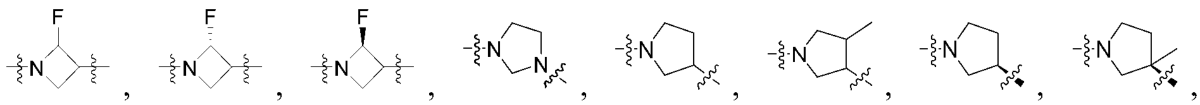

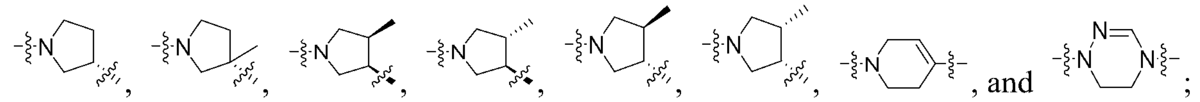
, and ;

more preferably, the structural fragment

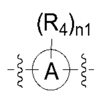

is selected from

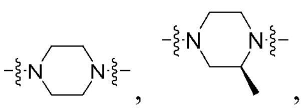

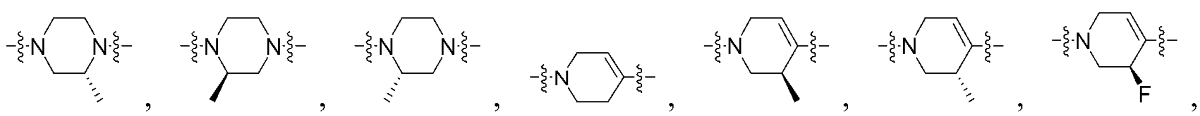

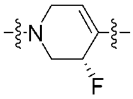, 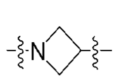, 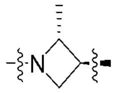, 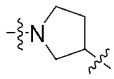, 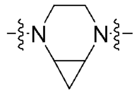, 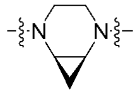, 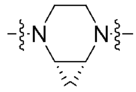, 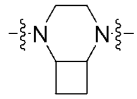,

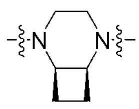, and 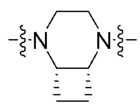.

7. The compound according to any one of claims 1-6, wherein

ring B is selected from phenyl, pyridyl, imidazolyl, imidazo[1,2-*a*]pyrazinyl, 1,7-naphthyridinyl, pyridazinyl, pyrazinyl, pyridothiazolyl, pyrazolo[1,5-*a*]pyrimidinyl, benzopyrazolyl, benzimidazolyl, thiazolyl, [1,2,4]triazolo [1,5-*a*]pyridinyl, thienyl, furyl, pyrazolyl, pyridazinopyrrolyl, pyridopyrazolyl, pyridooxazolyl, and pyrimidinyl; preferably, ring B is selected from

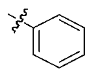, 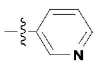, 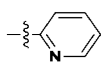, 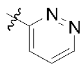, 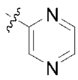, 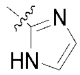, 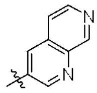,

and

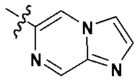.

8. The compound according to any one of claims 1-7, wherein

$R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, and 5- to 6-membered heteroarylaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5- to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3;
preferably, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl, and

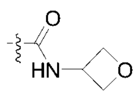;

n2 is selected from 1 and 2.

9. The compound according to any one of claims 1-8, wherein the structural fragment

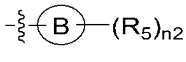

is selected from:

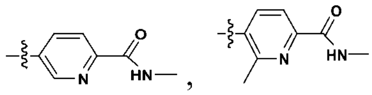,

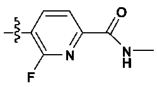, 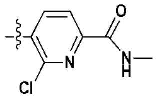, 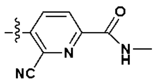, 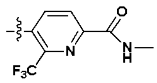, 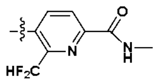, 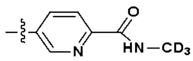,

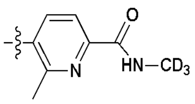, 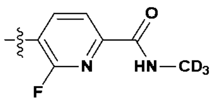, 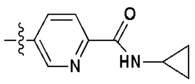, 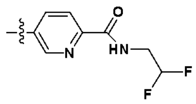, 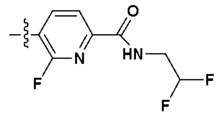,

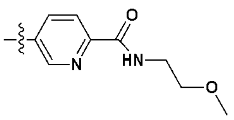, 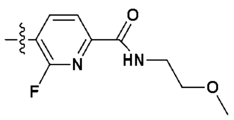, 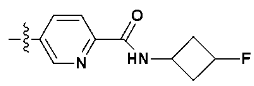, 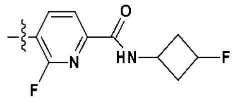,

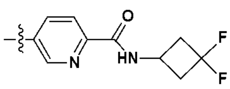, 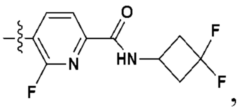, 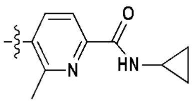, 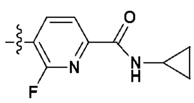, 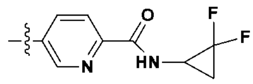,

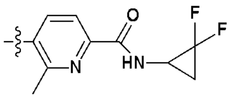, 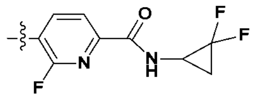, 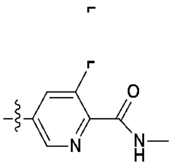, 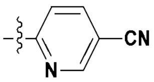, 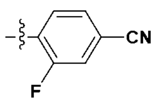,

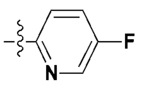, 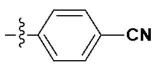, 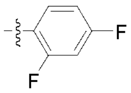, 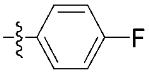, 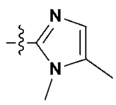, 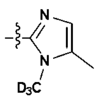, 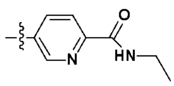,

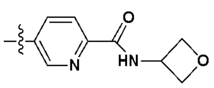, 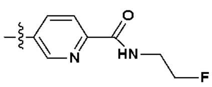, 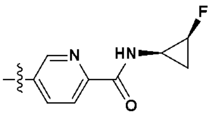, 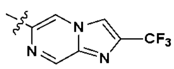, 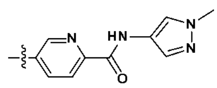,

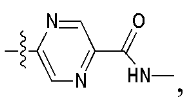, 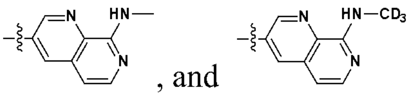, and .

**10.** The compound according to any one of claims 1-9, wherein

$Z_3$ is selected from O, $CH_2$, CHF, and $CF_2$;
preferably, $Z_3$ is selected from O and $CH_2$.

**11.** The compound according to any one of claims 1-10, wherein

$Z_2$ is selected from O, $NR_{b1}$, and $CR_{b2}R_{b3}$; $R_{b1}$ is selected from hydrogen, deuterium, and the following group optionally substituted with 0-3 substituents: $C_{1-4}$ alkyl, 3- to 6-membered cycloalkyl, or 4- to 6-membered heterocycloalkyl; in $R_{b1}$, the substituent is selected from: deuterium, fluorine, -OH, $-NH_2$, -CN, and 3- to 4-membered cycloalkyl; in $R_{b1}$, the 4- to 6-membered heterocycloalkyl contains 1-2 heteroatoms selected from N and O; $R_{b2}$ and $R_{b3}$ are independently selected from hydrogen, deuterium, fluorine, and $C_{1-4}$ alkyl;
preferably, $Z_2$ is selected from O, $NR_{b1}$, and $CR_{b2}R_{b3}$; $R_{b1}$ is selected from hydrogen, deuterium, and the following

group optionally substituted with 0-3 substituents: $C_{1-4}$ alkyl or 3- to 4-membered cycloalkyl; in $R_{b1}$, the substituent is selected from: deuterium, fluorine, -OH, $-NH_2$, -CN, and 3- to 4-membered cycloalkyl; $R_{b2}$ and $R_{b3}$ are independently selected from hydrogen, deuterium, fluorine, and methyl;
more preferably, $Z_2$ is selected from O, $CH_2$, NH, $NCH_3$, $NCD_3$, and *N*-cyclopropyl.

**12.** The compound according to any one of claims 1-11, wherein

when $Z_1$ is selected from a bond, O, $NR_{a1}$, and $CR_{a2}R_{a3}$, $R_{a1}$ is selected from hydrogen, deuterium, and the following group optionally substituted with 0-3 substituents: $C_{1-4}$ alkyl, 3-to 6-membered cycloalkyl, or 4- to 6-membered heterocycloalkyl; in $R_{a1}$, the substituent is selected from: deuterium, fluorine, -OH, $-NH_2$, -CN, and 3-to 4-membered cycloalkyl; in $R_{a1}$, the 4- to 6-membered heterocycloalkyl contains 1-2 heteroatoms selected from N and O; $R_{a2}$ and $R_{a3}$ are independently selected from hydrogen, deuterium, fluorine, and $C_{1-4}$ alkyl;
preferably, when $Z_1$ is selected from a bond, O, $NR_{a1}$, and $CR_{a2}R_{a3}$, $R_{a1}$ is selected from hydrogen, deuterium, and the following group optionally substituted with 0-3 substituents: $C_{1-4}$ alkyl or 3- to 4-membered cycloalkyl; in $R_{a1}$, the substituent is selected from: deuterium, fluorine, -OH, $-NH_2$, -CN, and 3- to 4-membered cycloalkyl; $R_{a2}$ and $R_{a3}$ are independently selected from hydrogen, deuterium, fluorine, and methyl;
more preferably, when $Z_1$ is selected from a bond, O, $NR_{a1}$, and $CR_{a2}R_{a3}$, $Z_1$ is selected from O, $CH_2$, NH, $NCH_3$, $NCD_3$, and *N*-cyclopropyl.

**13.** The compound according to any one of claims 1-12, wherein

$R_{x1}$ and $R_{y1}$ are independently selected from hydrogen, deuterium, halogen, cyano, amino, $C_{1-4}$ alkyl, $C_{1-4}$ deuterated alkyl, $C_{1-4}$ fluoroalkyl, -NH-$C_{1-4}$ alkyl, -N($C_{1-4}$ alkyl)$_2$, -O-$C_{1-4}$ alkyl, -S-$C_{1-4}$ alkyl, 3- to 6-membered cycloalkyl, 3- to 6-membered fluorocycloalkyl, and phenyl;
$R_{x2}$ is selected from hydrogen, deuterium, $C_{1-4}$ alkyl, $C_{1-4}$ deuterated alkyl, $C_{1-4}$ fluoroalkyl, 3-to 6-membered cycloalkyl, 3- to 6-membered fluorocycloalkyl, and phenyl;
$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -NH-$C_{1-4}$ alkyl, and -N($C_{1-4}$ alkyl)$_2$; n4 is selected from 1, 2, 3, and 4;
$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 4-membered cycloalkyl;
preferably, $R_{x1}$ and $R_{y1}$ are independently selected from hydrogen, deuterium, fluorine, chlorine, methyl, deuterated methyl, fluoromethyl, ethyl, fluoroethyl, cyclopropyl, and fluorocyclopropyl;
$R_{x2}$ is selected from hydrogen, deuterium, methyl, deuterated methyl, fluoromethyl, ethyl, fluoroethyl, cyclopropyl, and fluorocyclopropyl;
$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;
$R_8$ is selected from hydrogen, methyl, deuterated methyl, and fluoromethyl; more preferably, the structural fragment

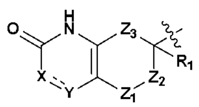

is selected from:

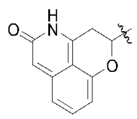,

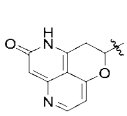, 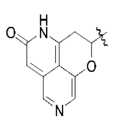, 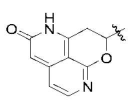, 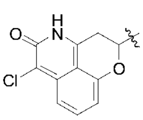, 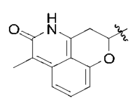, 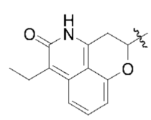, 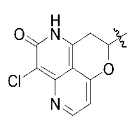,

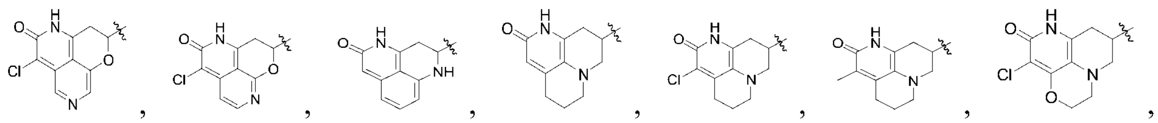
, , , , , , ,

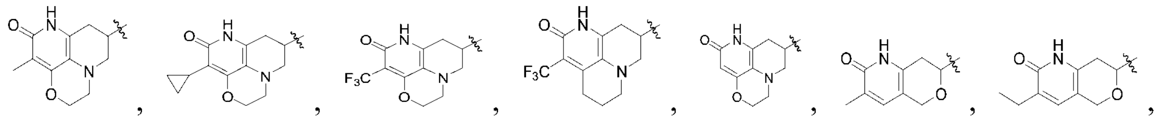
, , , , , , ,

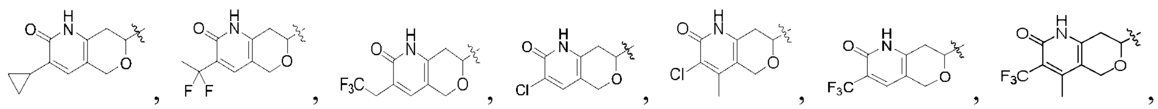
, , , , , , ,

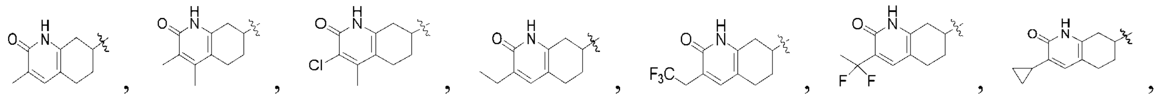
, , , , , , ,

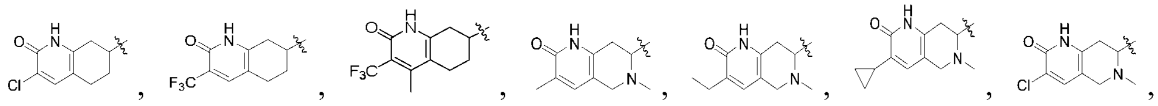
, , , , , , ,

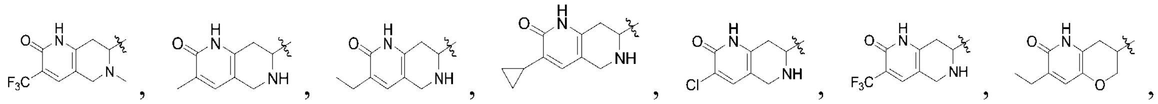
, , , , , , ,

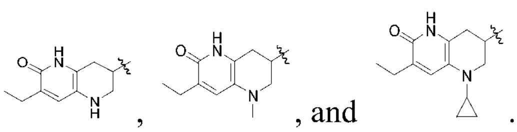
, , and .

**14.** The compound according to any one of claims 1-13, wherein the compound is selected from:

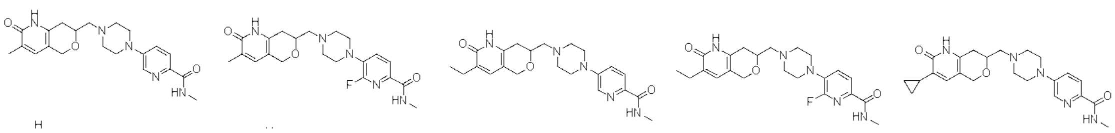

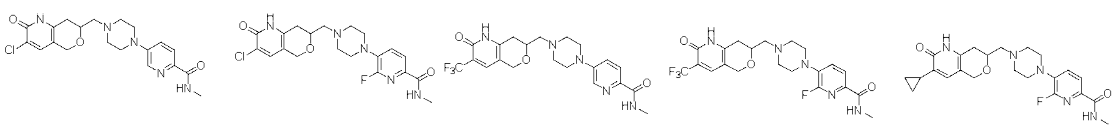

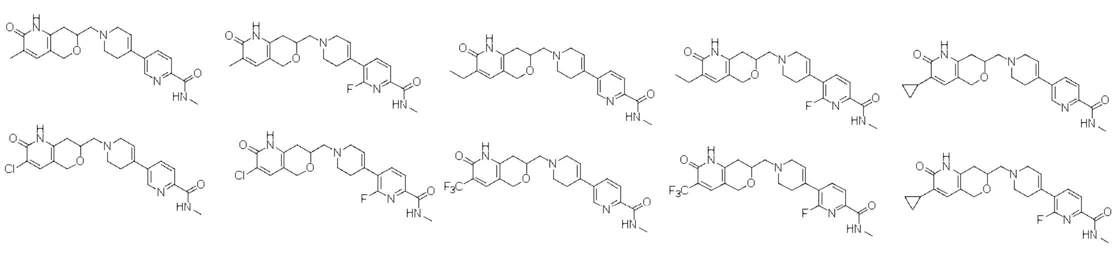

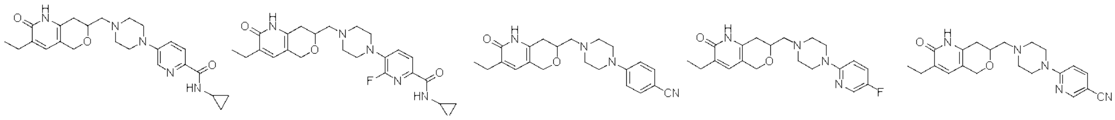

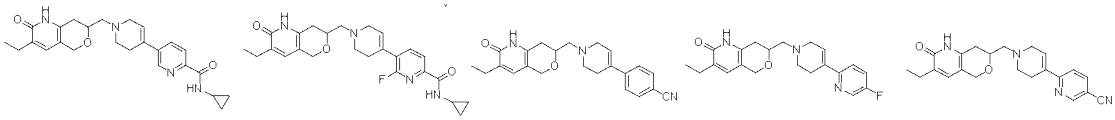

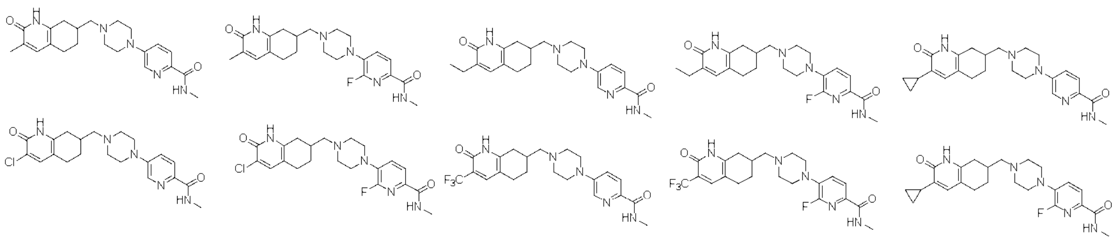

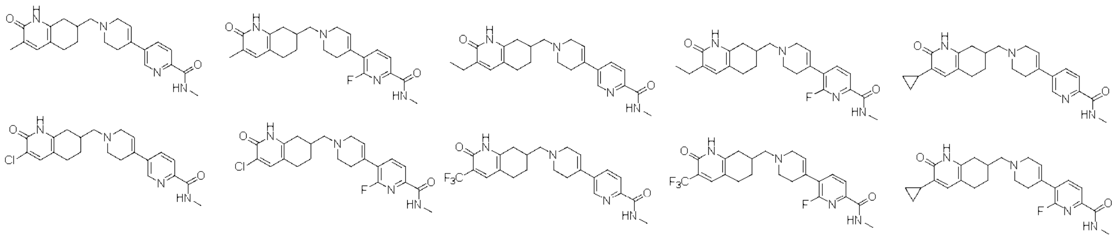

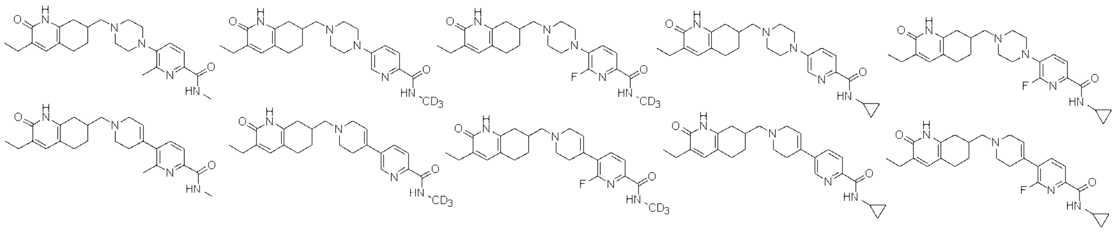

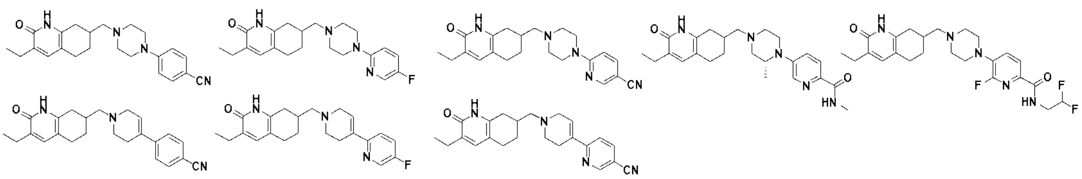 .

**15.** The compound according to any one of claims 1-5, wherein the structural fragment

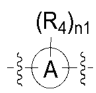

is selected from

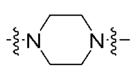 , 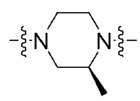 , 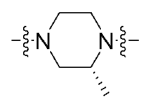 ,

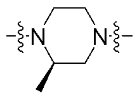, 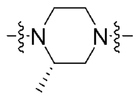, 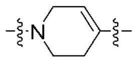, 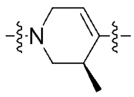, 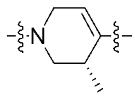, 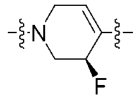, 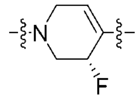,

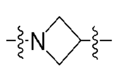, 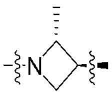, 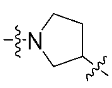, 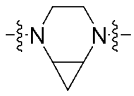, 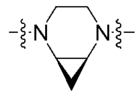, 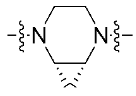, 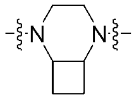, 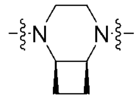,

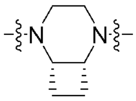, and 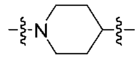.

**16.** The compound according to any one of claims 1-5, 7, or 15, wherein

$R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, 5- to 6-membered heteroarylaminoacyl, $C_{1-4}$ alkoxyaminoacyl, 3- to 6-membered cycloalkyl-substituted $C_{1-4}$ alkoxyaminoacyl, and 3- to 6-membered cycloalkoxyaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5- to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3; preferably, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl,

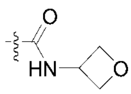,

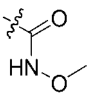, 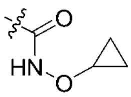, and 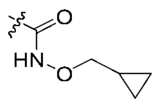;

n2 is selected from 1 and 2.

**17.** The compound according to any one of claims 1-5, 7, 15, or 16, wherein the structural fragment

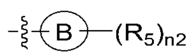

is selected from:

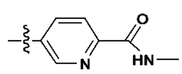, 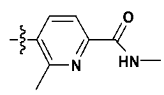,

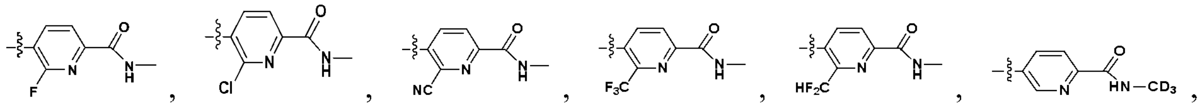

, , , , , ,

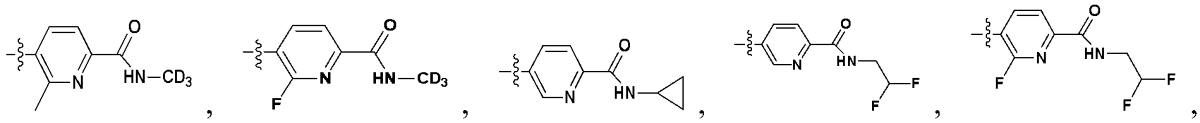

, , , , ,

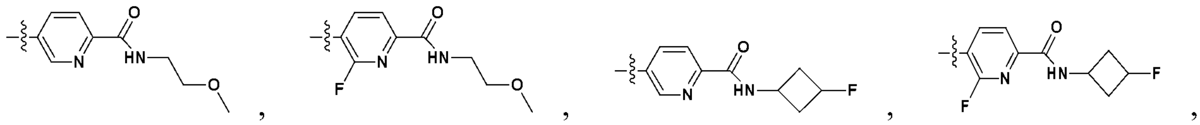

, , , ,

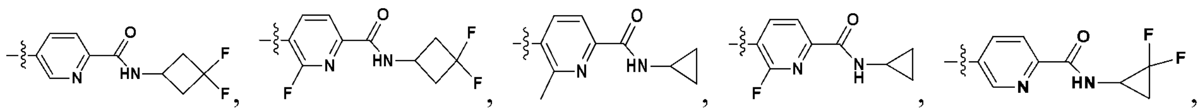

, , , , ,

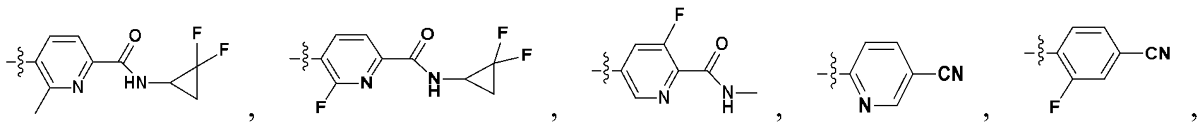

, , , , ,

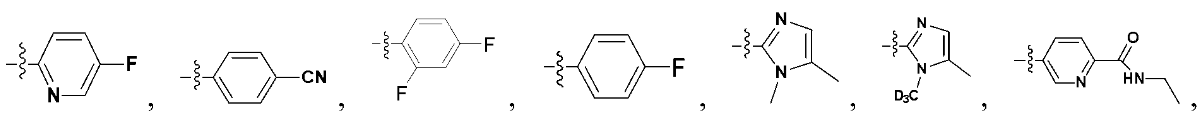

, , , , , , ,

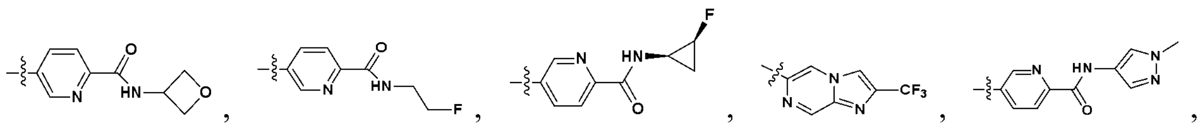

, , , , ,

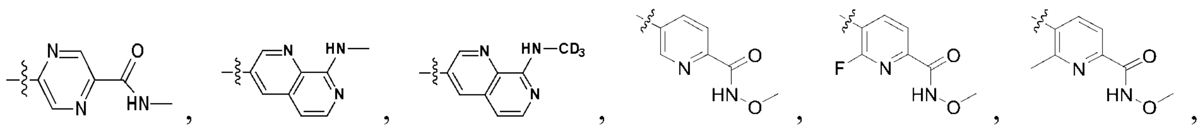

, , , , , ,

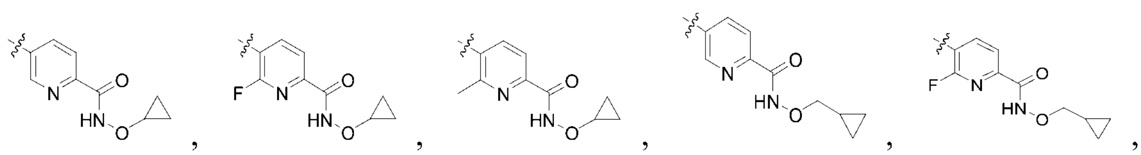

, , , , ,

and

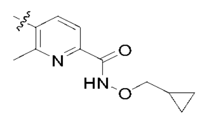

.

**18.** The compound according to any one of claims 1-5, 7, 10-12, or 15-17, wherein

$R_{x1}$ and $R_{y1}$ are independently selected from hydrogen, deuterium, fluorine, chlorine, bromine, methyl, deuterated methyl, fluoromethyl, ethyl, fluoroethyl, cyclopropyl, and fluorocyclopropyl;

$R_{x2}$ is selected from hydrogen, deuterium, methyl, deuterated methyl, fluoromethyl, ethyl, fluoroethyl, cyclopropyl, and fluorocyclopropyl;
$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;
$R_8$ is selected from hydrogen, methyl, deuterated methyl, and fluoromethyl;
preferably, the structural fragment

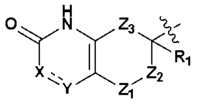

is selected from:

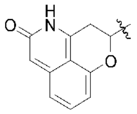, 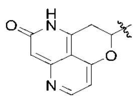,

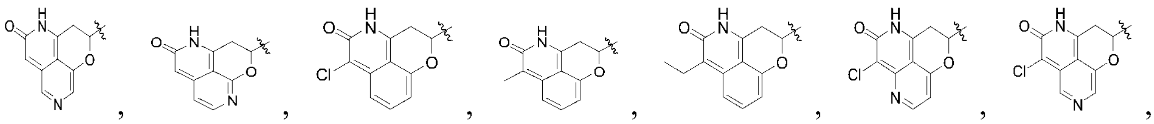
, , , , , , ,

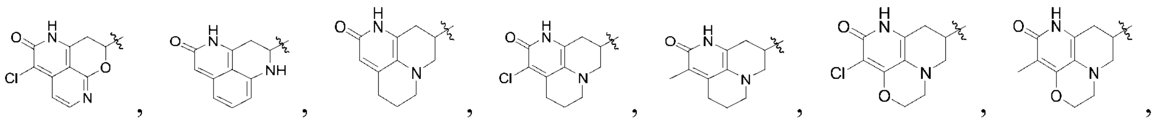
, , , , , , ,

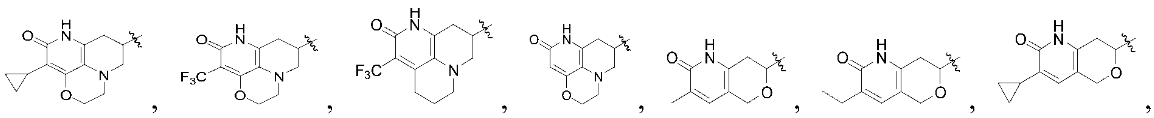
, , , , , , ,

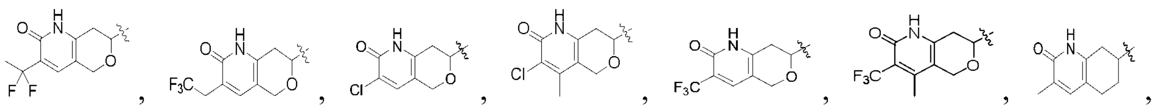
, , , , , , ,

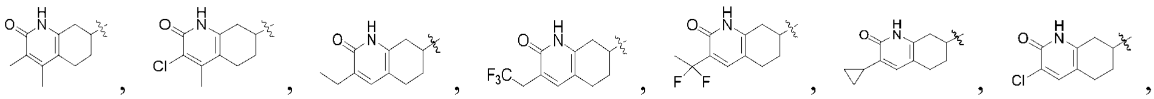
, , , , , , ,

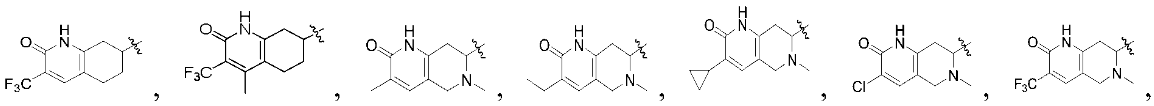
, , , , , , ,

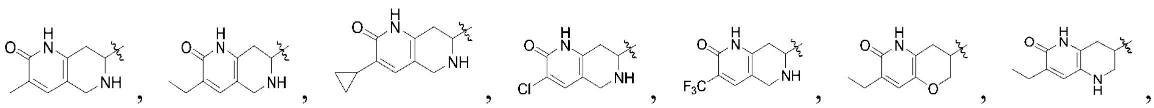
, , , , , , ,

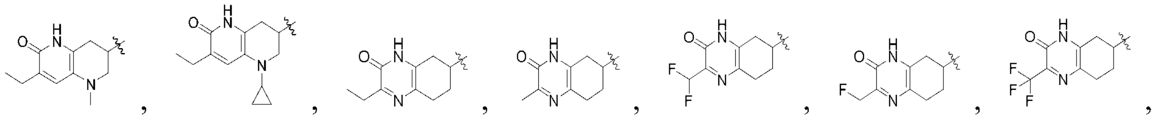
, , , , , , ,

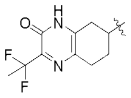, 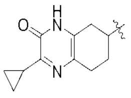, 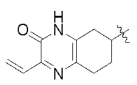, 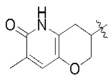, 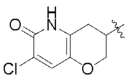, 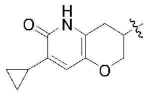, 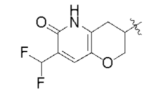,

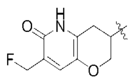, 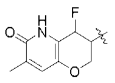, 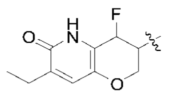, 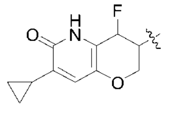, 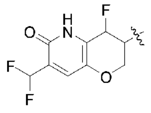, 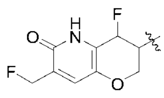,

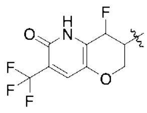, 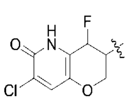, 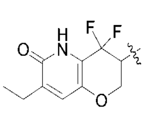, 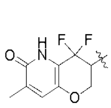, 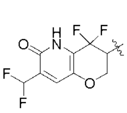, 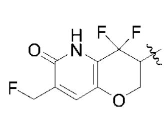, 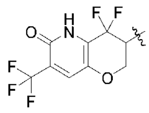,

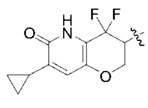, 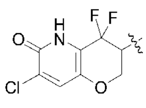, 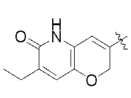, 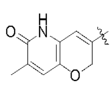, 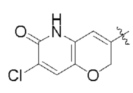, 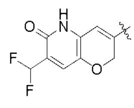, 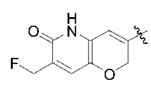,

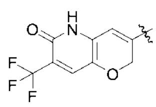, 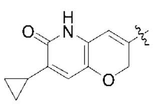, 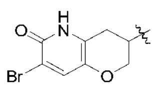, 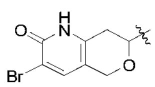, 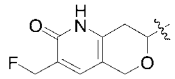, 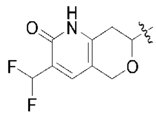,

and

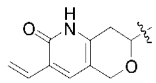.

**19.** The compound according to any one of claims 1-3, 10-12, or 15-18, wherein the structural fragment

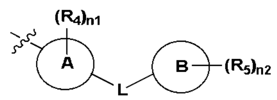

is replaced with:

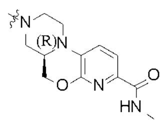, 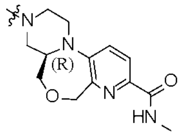,

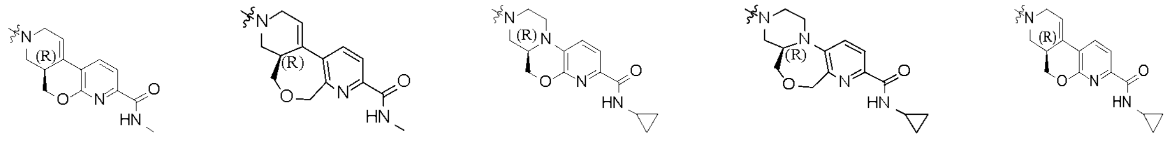
, , , , ,

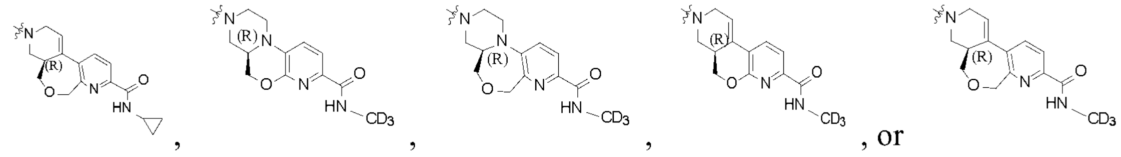
, , , , or .

**20.** The compound according to any one of claims 1-5, 7, 10-12, or 15-19, wherein the compound is selected from:

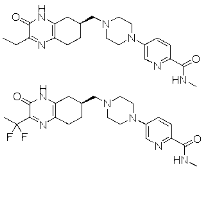 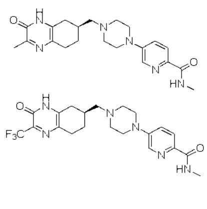 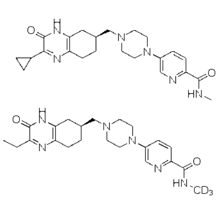 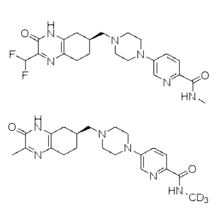 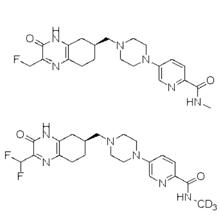

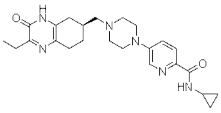 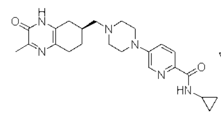 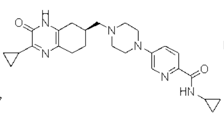 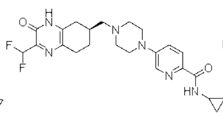 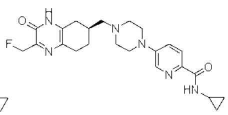

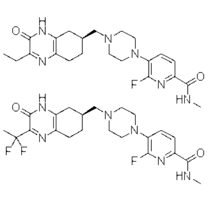 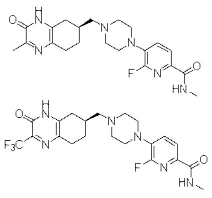 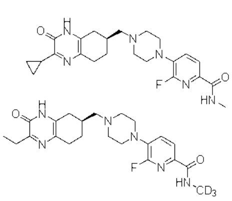 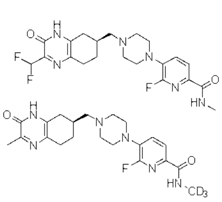 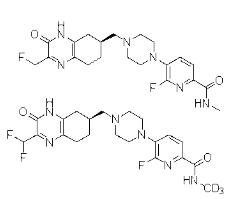

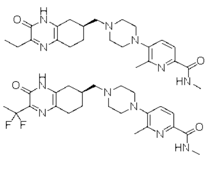 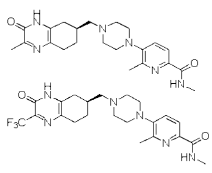 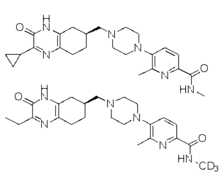 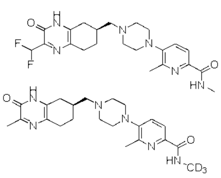 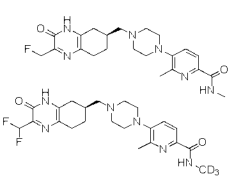

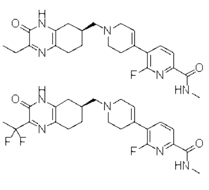 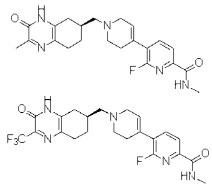 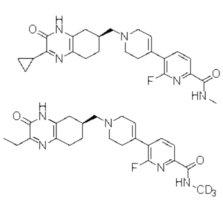 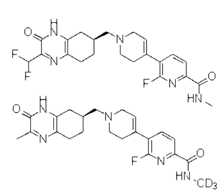 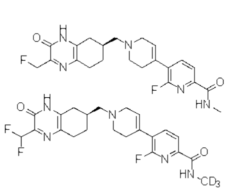

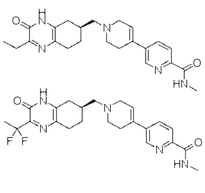 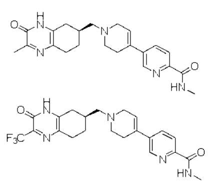 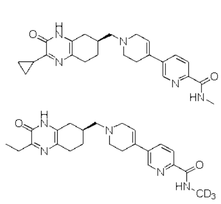 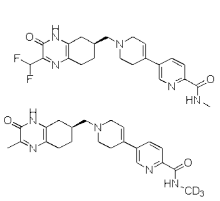 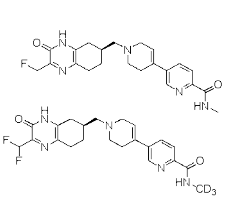

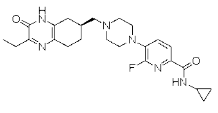 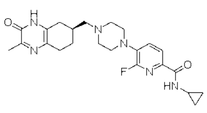 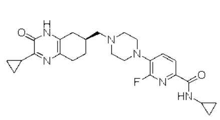 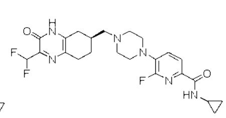 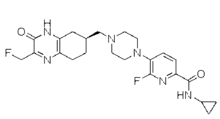

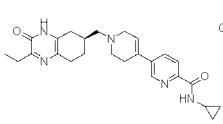 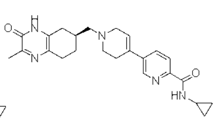 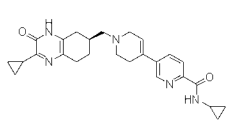 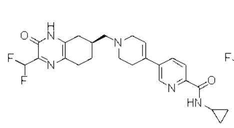 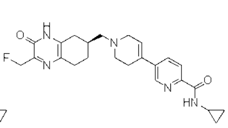

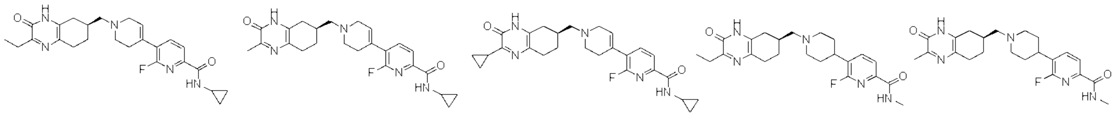

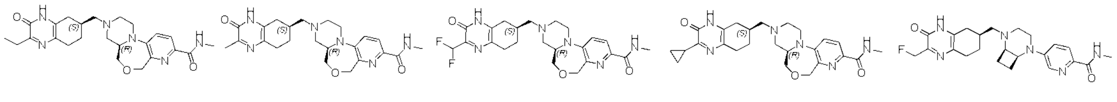

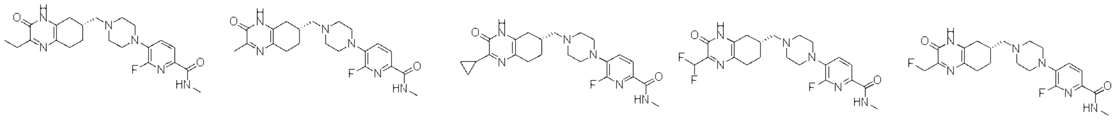

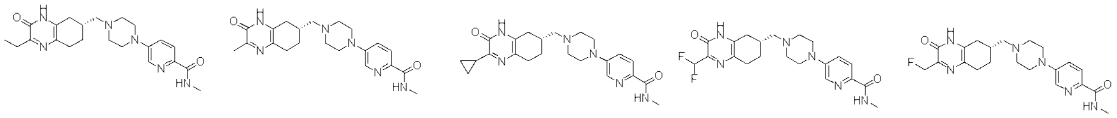

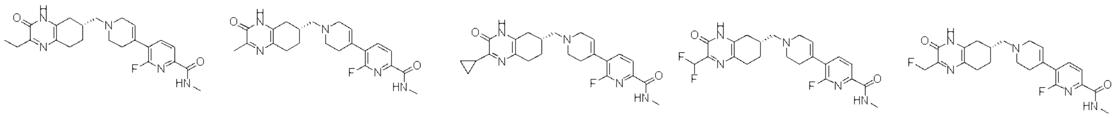

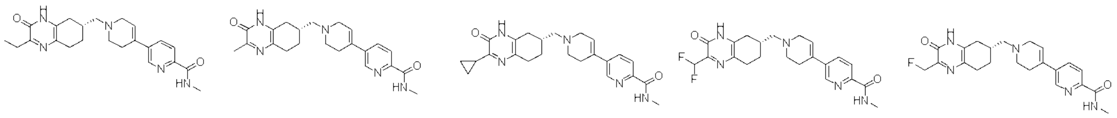

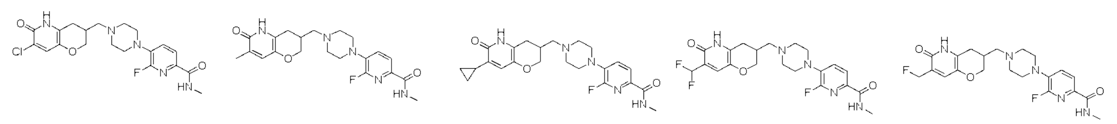

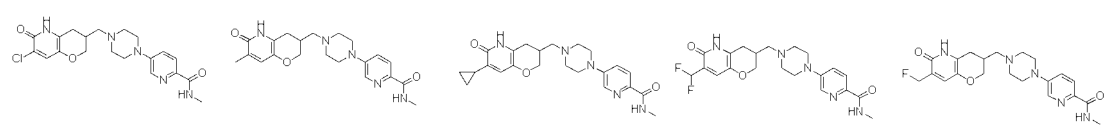

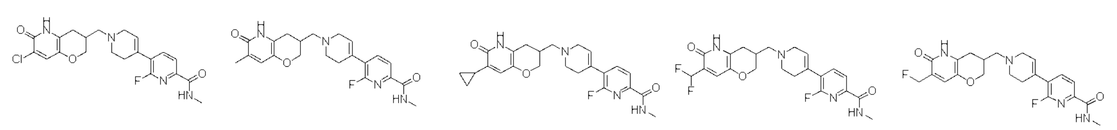

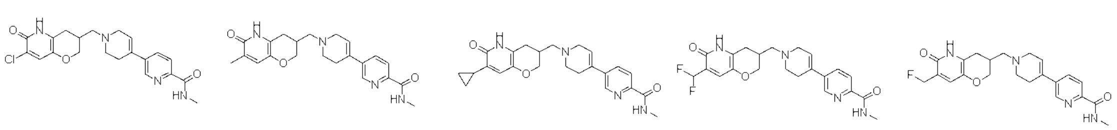

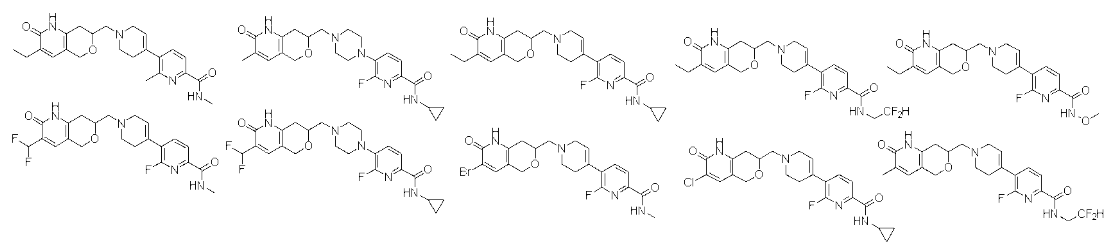

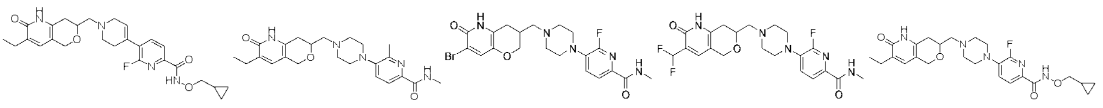

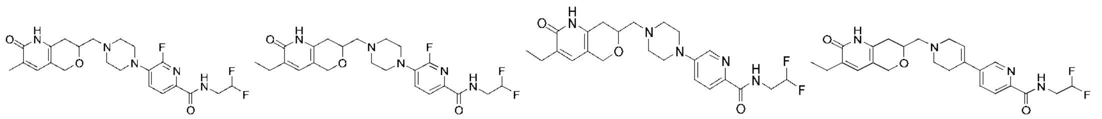

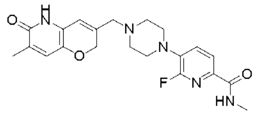.

**21.** The compound according to any one of claims 1-5, wherein

the structural fragment

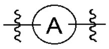

is selected from:

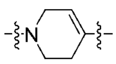, 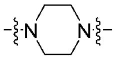, 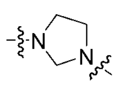, 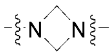,

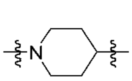, 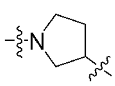, 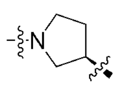, 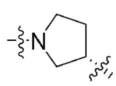, 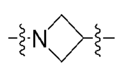, 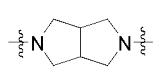, and 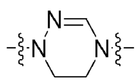;

preferably, the structural fragment

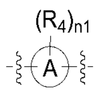

is selected from:

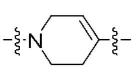, 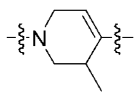,

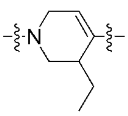, 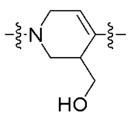, 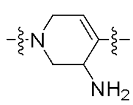, 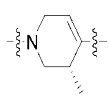, 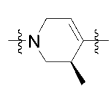, 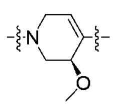, 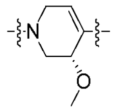, 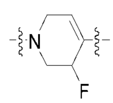,

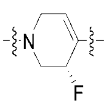, 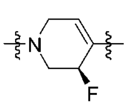, 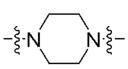, 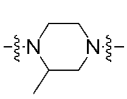, 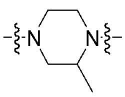, 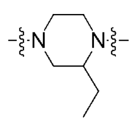, 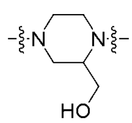,

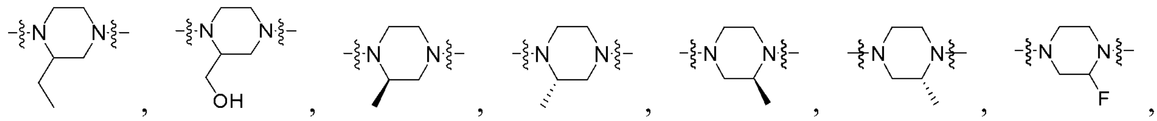

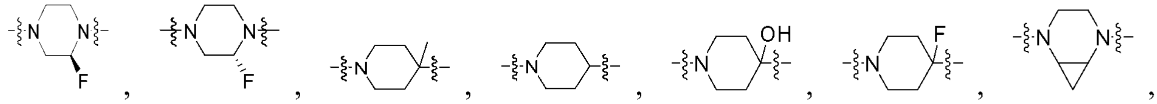

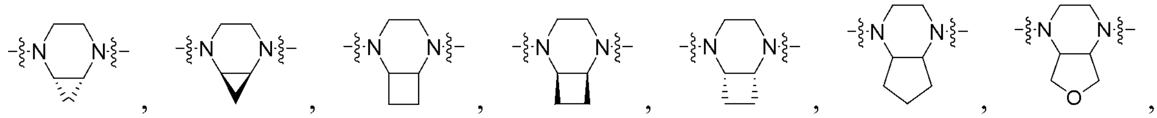

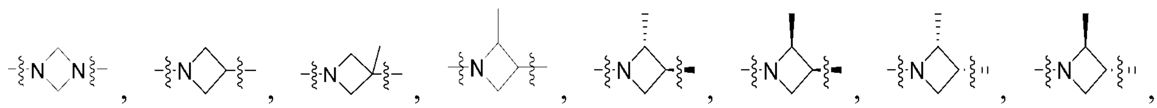

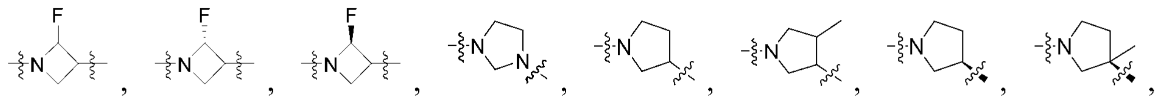

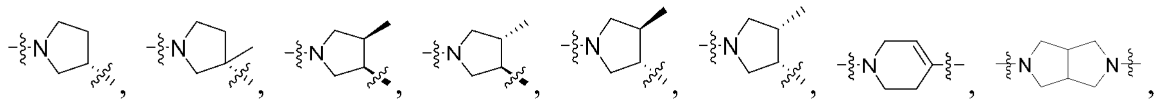

and

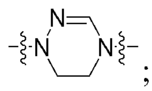;

more preferably, the structural fragment

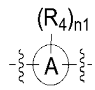

is selected from

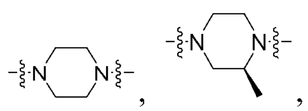

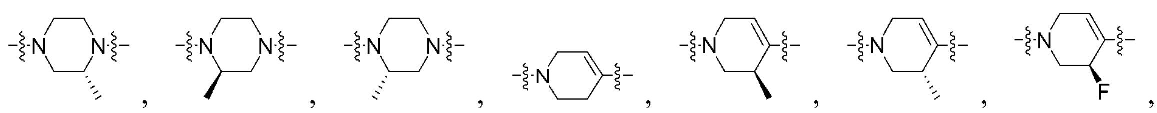

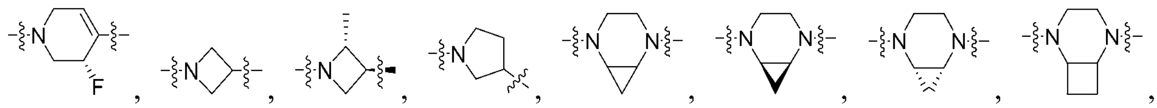

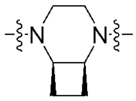, 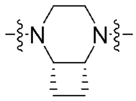, 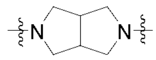, and 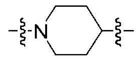.

**22.** The compound according to any one of claims 1-5 or 21, wherein ring B is selected from phenyl, pyridyl, imidazolyl, imidazo[1,2-*a*]pyrazinyl, 1,7-naphthyridinyl, pyridazinyl, pyrazinyl, pyridothiazolyl, pyrazolo[1,5-*a*]pyrimidinyl, benzopyrazolyl, benzimidazolyl, thiazolyl, [1,2,4]triazolo[1,5-*a*]pyridinyl, thienyl, furyl, pyrrolyl, pyrazolyl, pyridazinopyrrolyl, pyridopyrazolyl, pyridooxazolyl, and pyrimidinyl; preferably, ring B is selected from

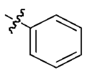, 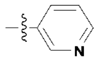, 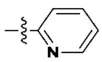, 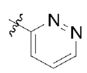, 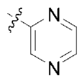, 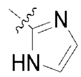, 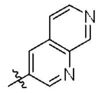,

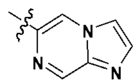, 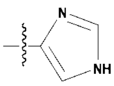, and 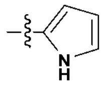.

**23.** The compound according to any one of claims 1-5 or 21-22, wherein

$R_5$ is selected from at least one of hydrogen, fluorine, chlorine, bromine, cyano, amino, $C_{1-4}$ alkyl, $C_{1-4}$ deuterated alkyl, $C_{1-4}$ fluoroalkyl, $C_{1-4}$ alkoxy, $C_{1-4}$ deuterated alkoxy, $C_{1-4}$ fluoroalkoxy, 3- to 6-membered cycloalkyl, $C_{1-4}$ alkylaminoacyl, $C_{1-4}$ deuterated alkylaminoacyl, $C_{1-4}$ fluoroalkylaminoacyl, $C_{1-4}$ alkoxy-substituted $C_{1-4}$ alkylaminoacyl, 3- to 6-membered cycloalkylaminoacyl, 3- to 6-membered fluorocycloalkylaminoacyl, 4- to 6-membered heterocycloalkylaminoacyl, 6-membered arylaminoacyl, 5- to 6-membered heteroarylaminoacyl, $C_{1-4}$ alkoxyaminoacyl, 3- to 6-membered cycloalkyl-substituted $C_{1-4}$ alkoxyaminoacyl, and 3- to 6-membered cycloalkoxyaminoacyl; or two adjacent $R_5$, together with the atoms to which they are attached, form a 5- to 6-membered carbocycle or a 5- to 6-membered heterocycle; in $R_5$, the 4- to 6-membered heterocycloalkylaminoacyl or 5- to 6-membered heteroarylaminoacyl contains 1-2 heteroatoms selected from at least one of N, S, and O; when two adjacent $R_5$, together with the atoms to which they are attached, form a ring, the 5- to 6-membered heterocycle contains 1-2 heteroatoms selected from at least one of N, S, and O; n2 is selected from 1, 2, and 3; preferably, $R_5$ is selected from at least one of hydrogen, fluorine, chlorine, cyano, amino, methyl, deuterated methyl, fluoromethyl, ethyl, deuterated ethyl, fluoroethyl, cyclopropyl, cyclobutyl, methylamino, deuterated methylamino, fluoromethylamino, methylaminoacyl, deuterated methylaminoacyl, fluoromethylaminoacyl, ethylaminoacyl, deuterated ethylaminoacyl, fluoroethylaminoacyl, methoxyethylaminoacyl, cyclopropylaminoacyl, fluorocyclopropylaminoacyl, cyclobutylaminoacyl, fluorocyclobutylaminoacyl,

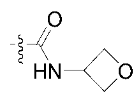,

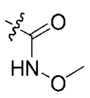, 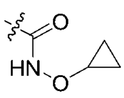, 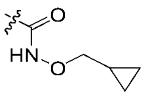, 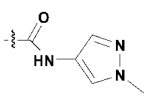, 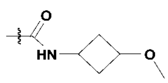, 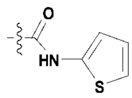, 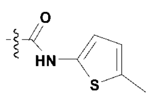,

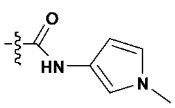, 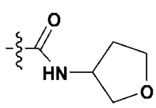, 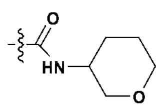, and 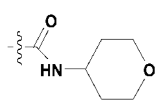;

n2 is selected from 1 and 2.

**24.** The compound according to any one of claims 1-5 or 21-23, wherein the structural fragment

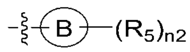

is selected from:

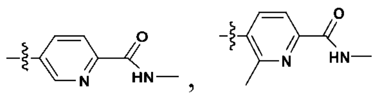 , ,

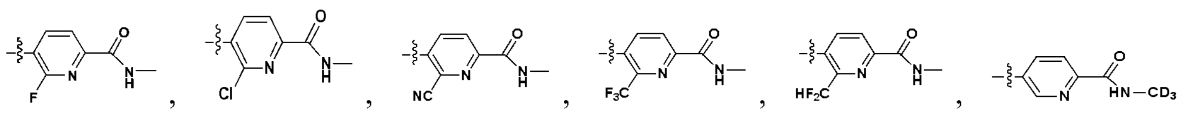 , , , , , ,

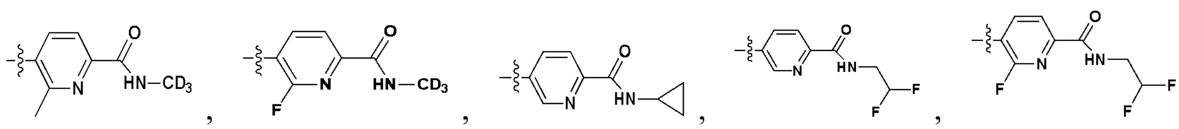 , , , , ,

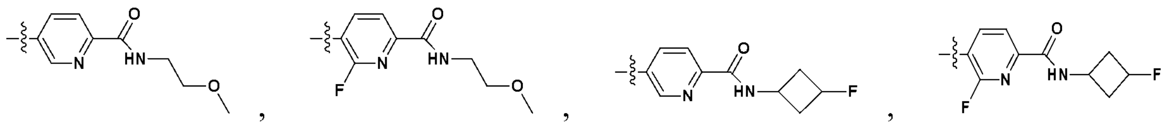 , , , ,

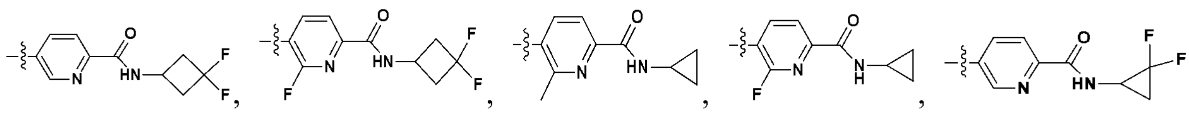 , , , , ,

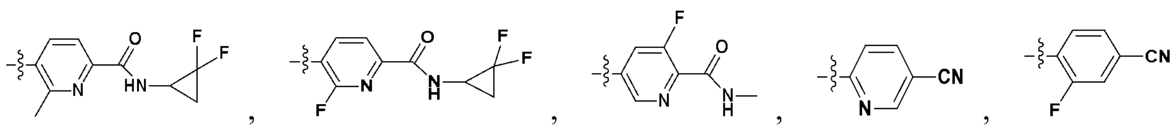 , , , , ,

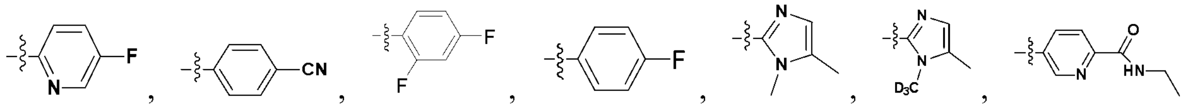 , , , , , , ,

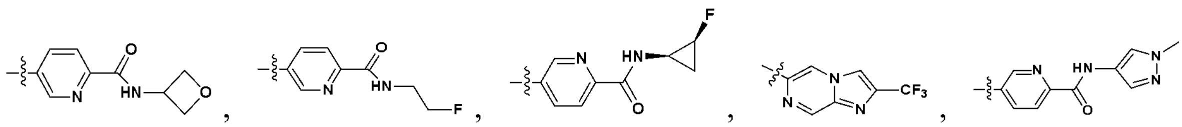 , , , , ,

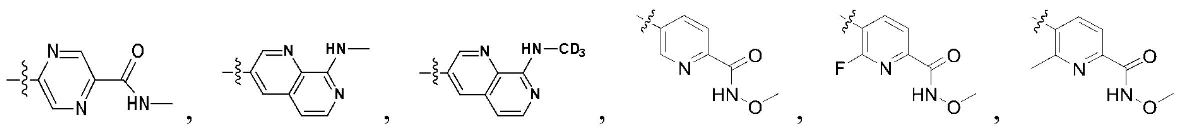 , , , , , ,

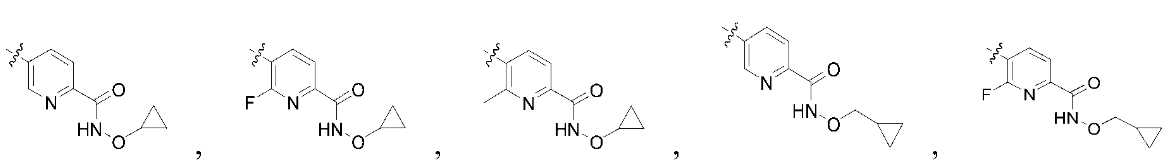 , , , , ,

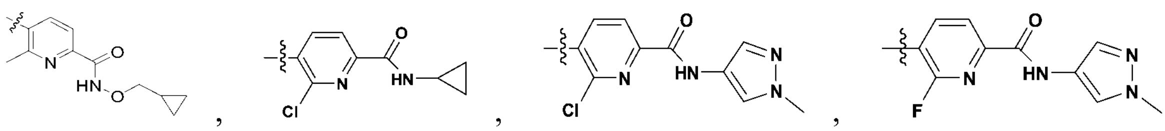 , , , ,

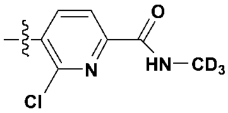, 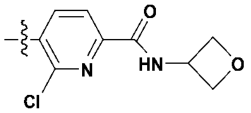, 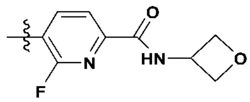, 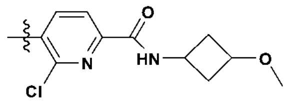,

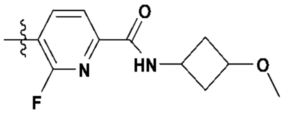, 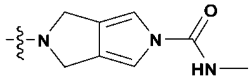, 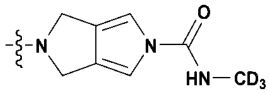, 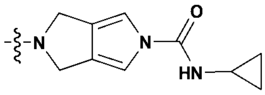,

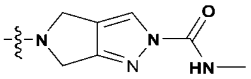, 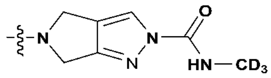, 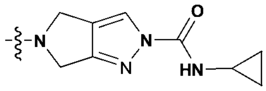, 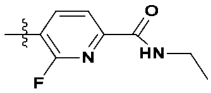,

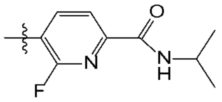, 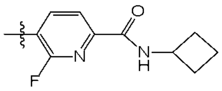, 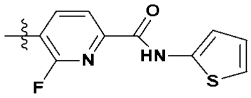, 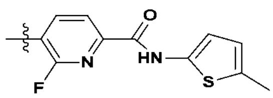,

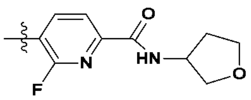, 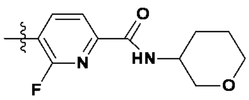, 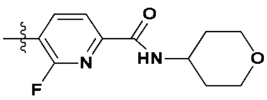, 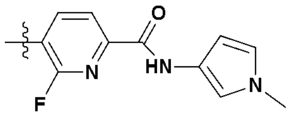,

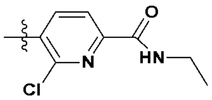, 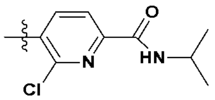, 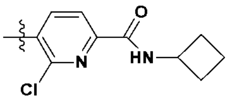, 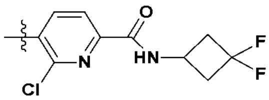,

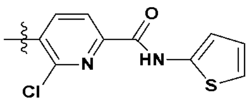, 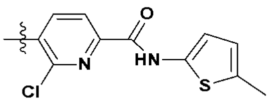, 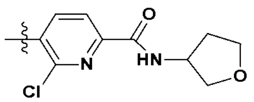, 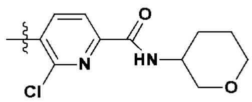,

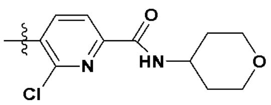, 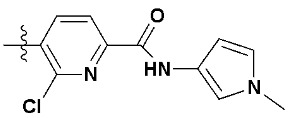, 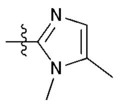, 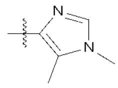, 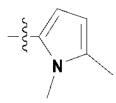, 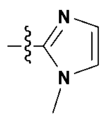,

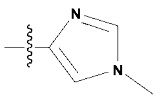, and 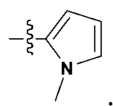.

**25.** The compound according to any one of claims 1-5, 10-12, or 21-24, wherein

$R_{x1}$ and $R_{y1}$ are independently selected from hydrogen, deuterium, halogen, cyano, amino, $C_{1-4}$ alkyl, $C_{1-4}$ deuterated alkyl, $C_{1-4}$ fluoroalkyl, -NH-$C_{1-4}$ alkyl, -N($C_{1-4}$ alkyl)$_2$, -O-$C_{1-4}$ alkyl, -S-$C_{1-4}$ alkyl, 3- to 6-membered cycloalkyl, 3- to 6-membered fluorocycloalkyl, vinyl, and phenyl;

$R_{x2}$ is selected from hydrogen, deuterium, $C_{1-4}$ alkyl, $C_{1-4}$ deuterated alkyl, $C_{1-4}$ fluoroalkyl, 3-to 6-membered cycloalkyl, 3- to 6-membered fluorocycloalkyl, and phenyl;

$R_7$ is selected from at least one of hydrogen, deuterium, halogen, hydroxyl, amino, cyano, $C_{1-4}$ alkyl, $C_{1-4}$ deuterated alkyl, $C_{1-4}$ fluoroalkyl, hydroxyl-substituted $C_{1-4}$ alkyl, $C_{1-4}$ alkoxy, $C_{1-4}$ deuterated alkoxy, $C_{1-4}$ fluoroalkoxy, -NH-$C_{1-4}$ alkyl, and -N($C_{1-4}$ alkyl)$_2$; n4 is selected from 1, 2, 3, and 4;

$R_8$ is selected from hydrogen, deuterium, $C_{1-4}$ alkyl, $C_{1-4}$ fluoroalkyl, hydroxyl-substituted $C_{1-4}$ alkyl, $C_{1-4}$ deuterated alkyl, and 3- to 4-membered cycloalkyl;

preferably, $R_{x1}$ and $R_{y1}$ are independently selected from hydrogen, deuterium, fluorine, chlorine, bromine, methyl, deuterated methyl, fluoromethyl, ethyl, fluoroethyl, cyclopropyl, methoxy, methylthio, vinyl, and fluorocyclopropyl;

$R_{x2}$ is selected from hydrogen, deuterium, methyl, deuterated methyl, fluoromethyl, ethyl, fluoroethyl, cyclopropyl, and fluorocyclopropyl;

$R_7$ is selected from hydrogen, deuterium, fluorine, chlorine, amino, methylamino, deuterated methylamino, fluoromethylamino, methyl, deuterated methyl, fluoromethyl, ethyl, fluoroethyl, cyclopropyl, fluorocyclopropyl, methoxy, deuterated methoxy, and fluoromethoxy; n4 is selected from 1 and 2;

$R_8$ is selected from hydrogen, methyl, deuterated methyl, and fluoromethyl;

more preferably, the structural fragment

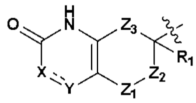

is selected from:

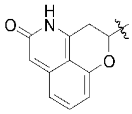,

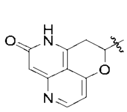, 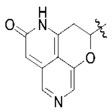, 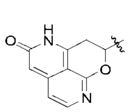, 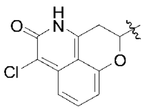, 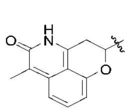, 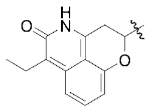, 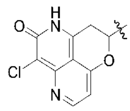,

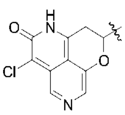, 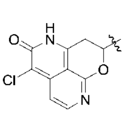, 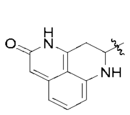, 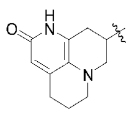, 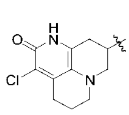, 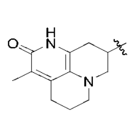, 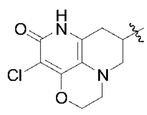,

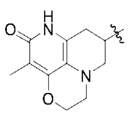, 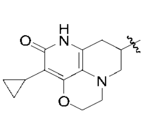, 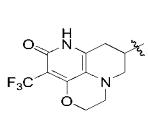, 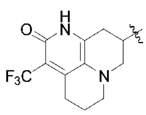, 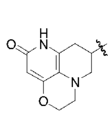, 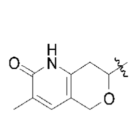, 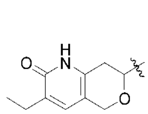,

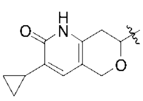, 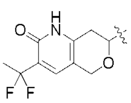, 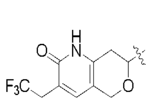, 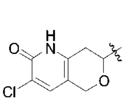, 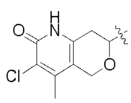, 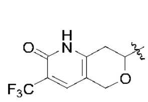, 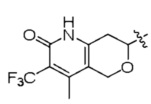,

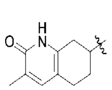, 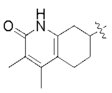, 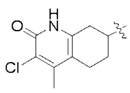, 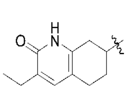, 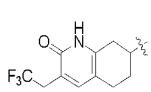, 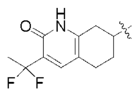, 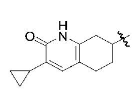,

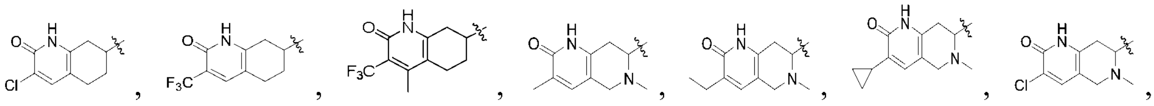

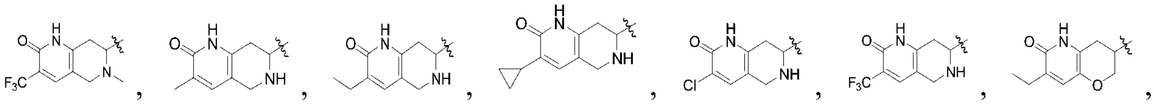

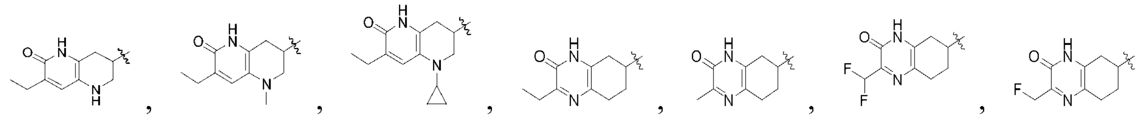, , , , , , ,

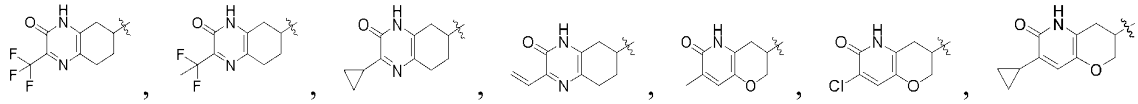, , , , , , ,

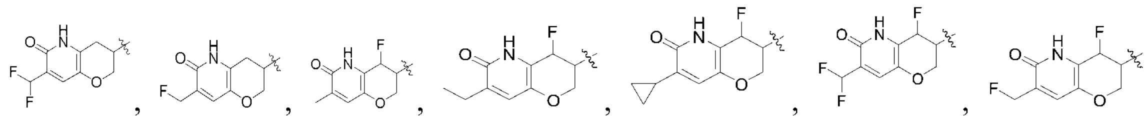, , , , , , ,

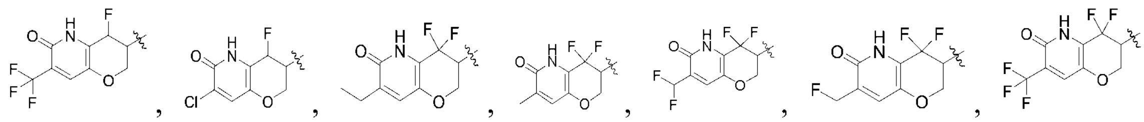, , , , , , ,

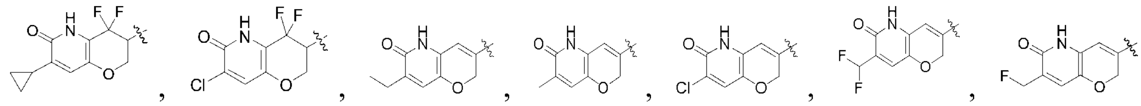, , , , , , ,

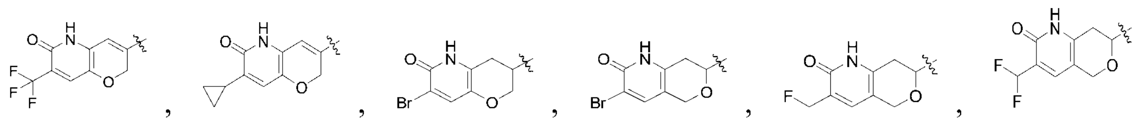, , , , , ,

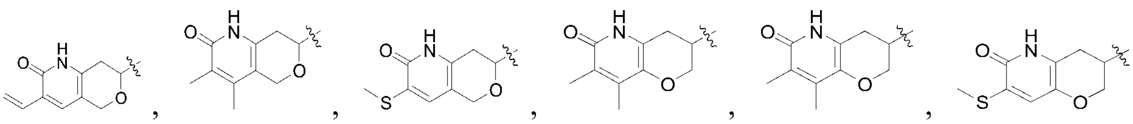, , , , , ,

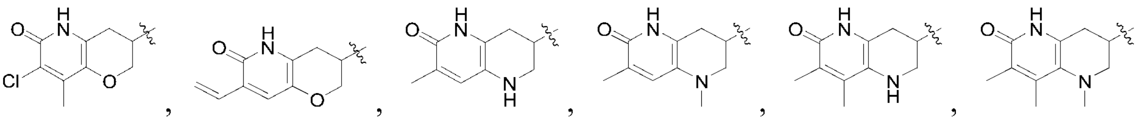, , , , , ,

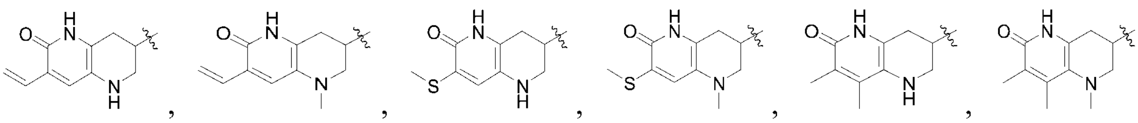, , , , , ,

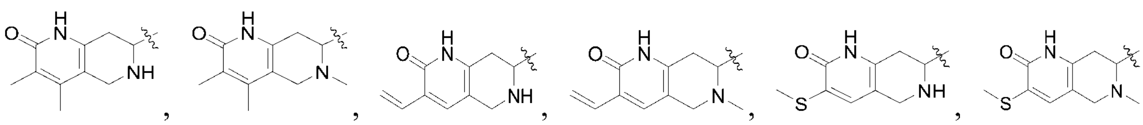, , , , , ,

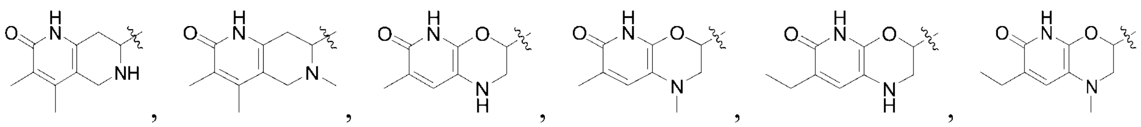, , , , , ,

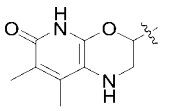, 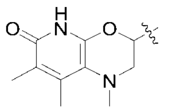, 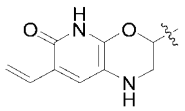, 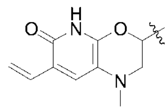, 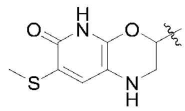,

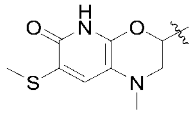, 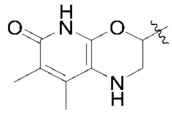, and 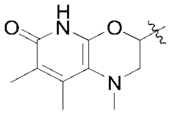.

**26.** The compound according to any one of claims 1-3, 10-12, or 21-25, wherein the structural fragment

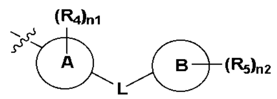

is replaced with:

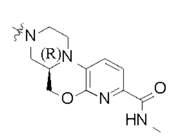, 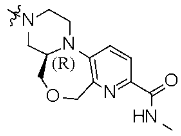,

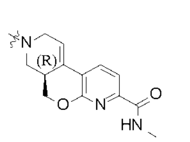, 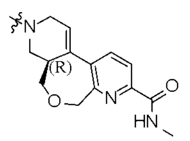, 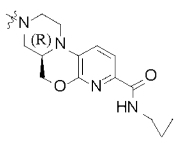, 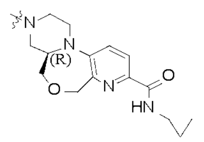, 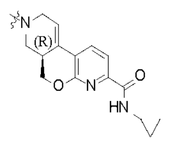,

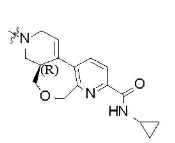, 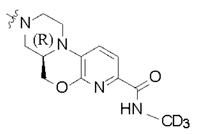, 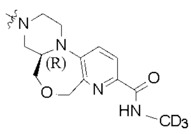, 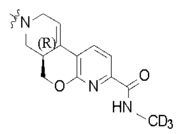, 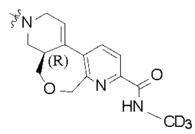,

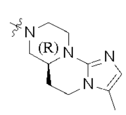, 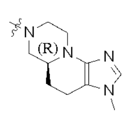, 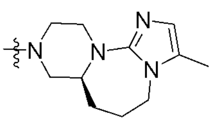, or 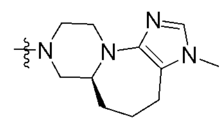.

**27.** The compound according to any one of claims 1-5 or 21-26, wherein the compound is selected from:

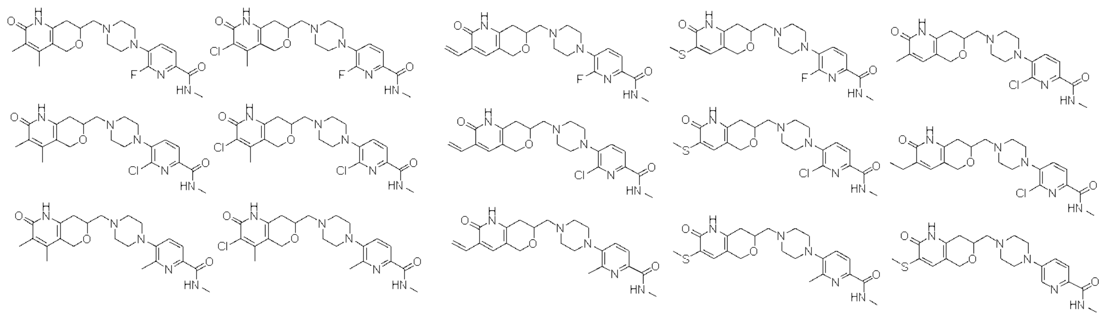

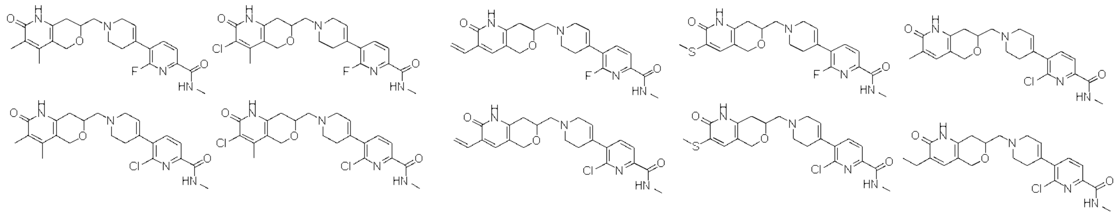

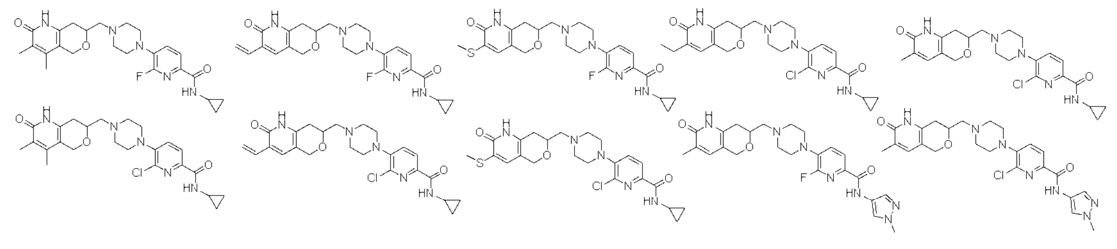

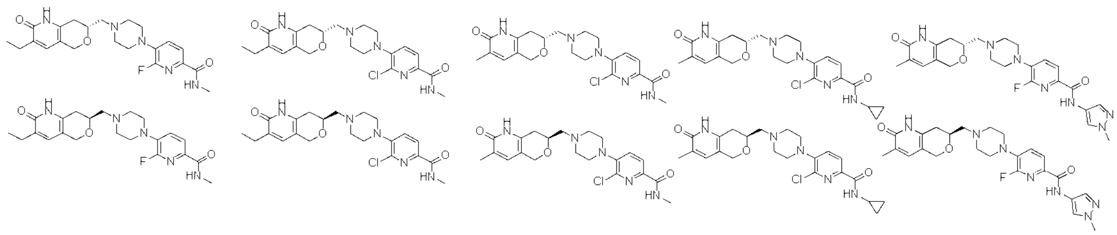

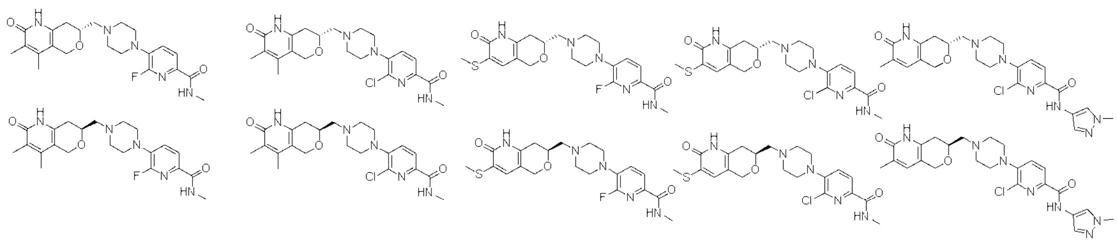

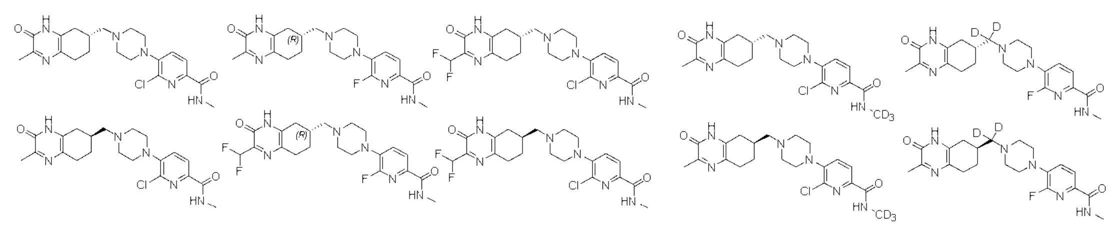

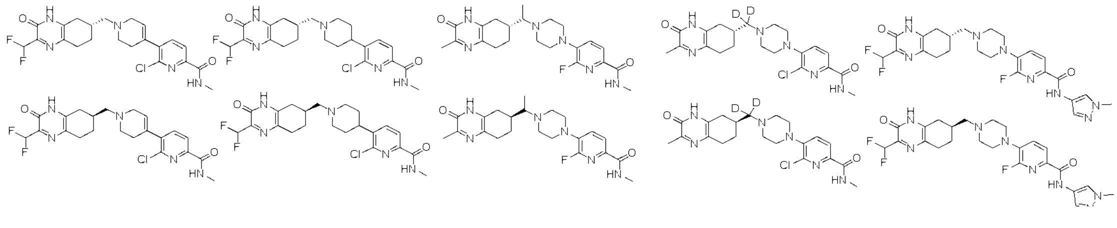

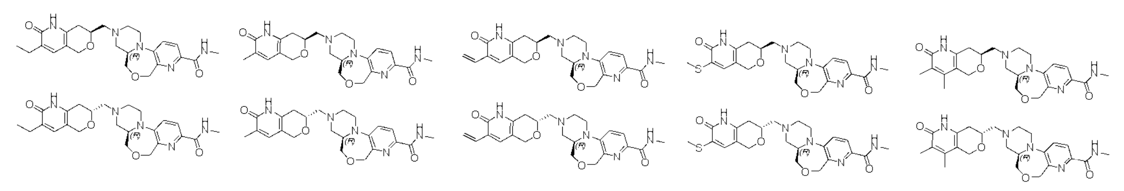

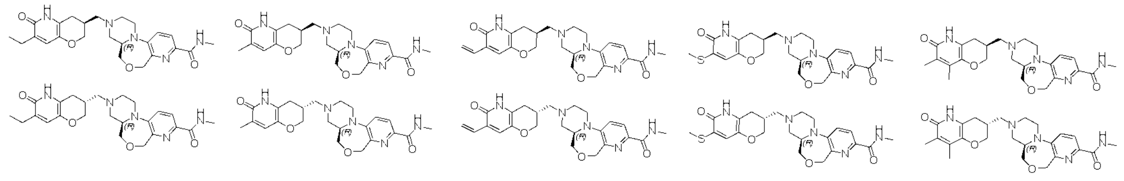

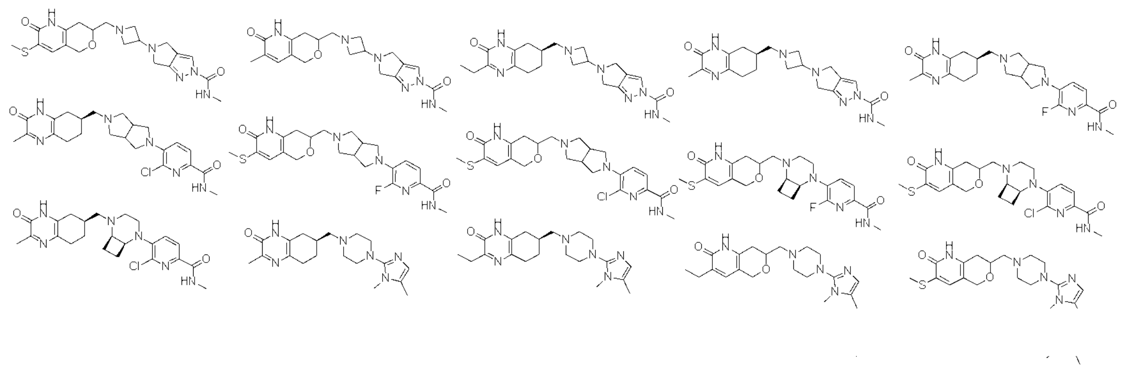

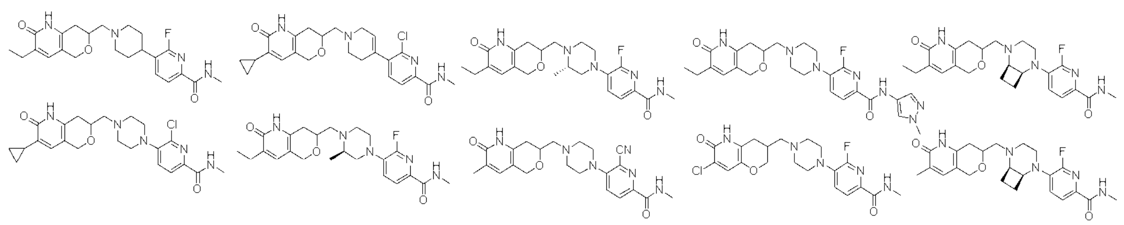

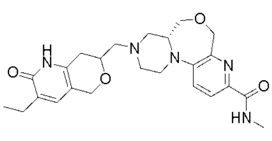,
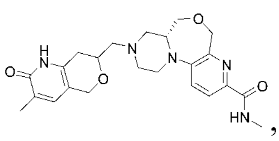,
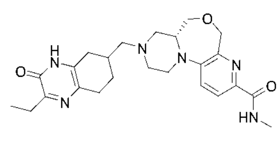,
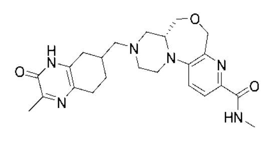

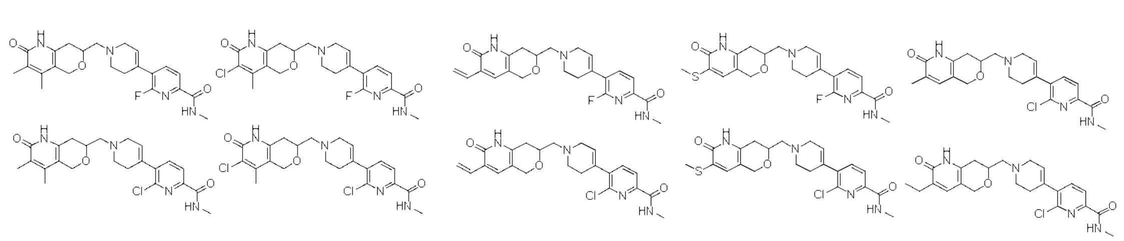

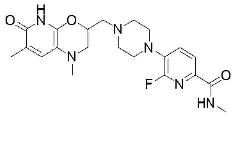,
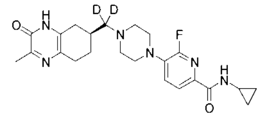,
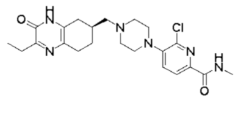,
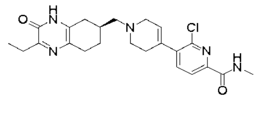,

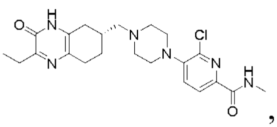, 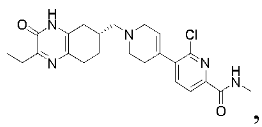, 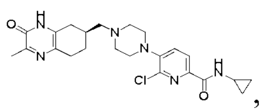, and 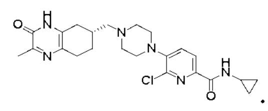.

**28.** A compound or a pharmaceutically acceptable form thereof, wherein the compound is selected from:

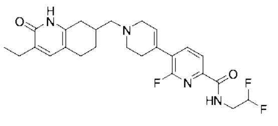, 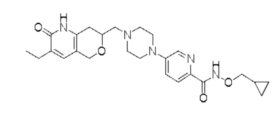, 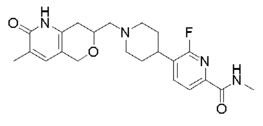, 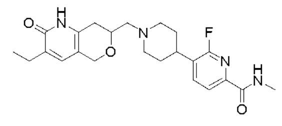,

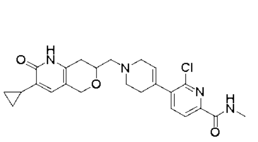, 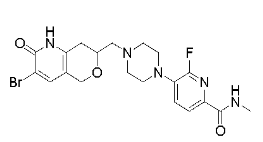, 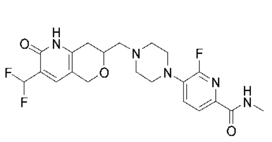, 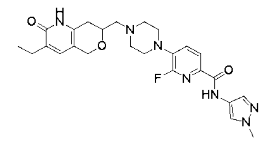,

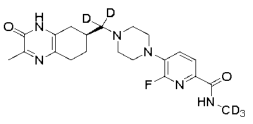, 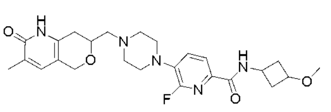, 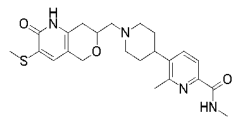, 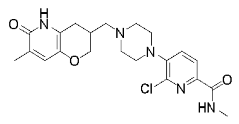,

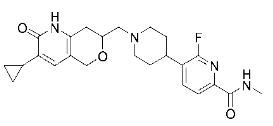, 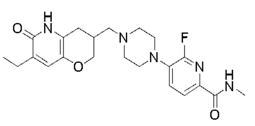, 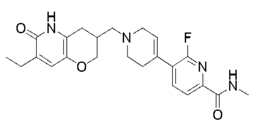, 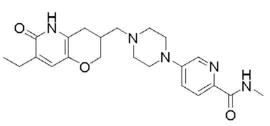,

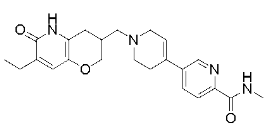, 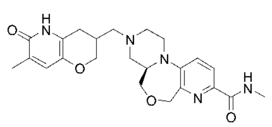, 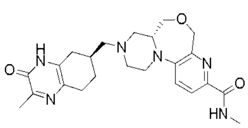, 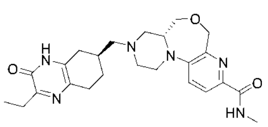,

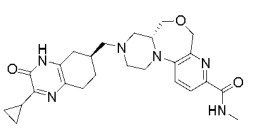, 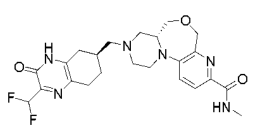, 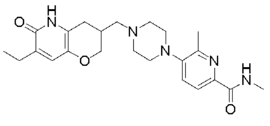, 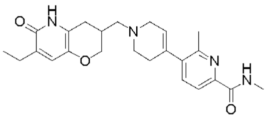,

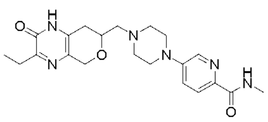, 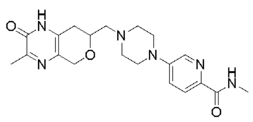, 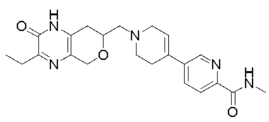, 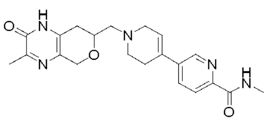,

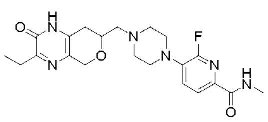, 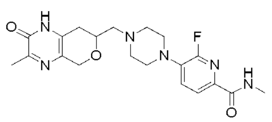, 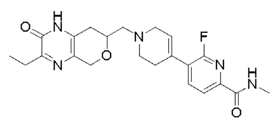, 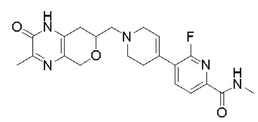,

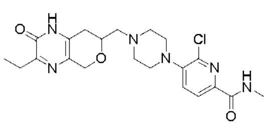, 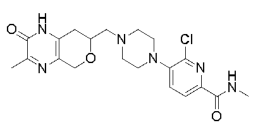, 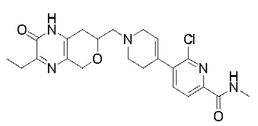, 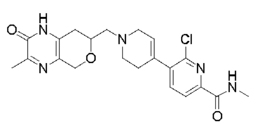,

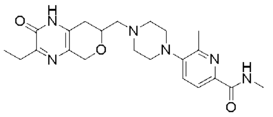, 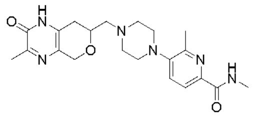, 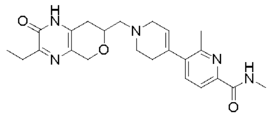, 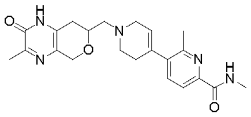,

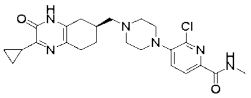, 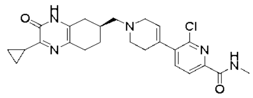, 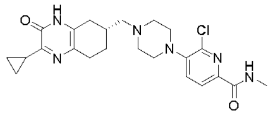, 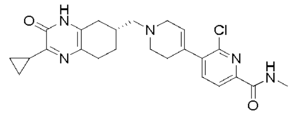,

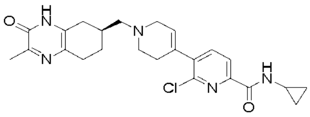, and 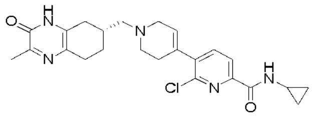.

**29.** A pharmaceutical composition, comprising the compound or the pharmaceutically acceptable salt, ester, stereoisomer, tautomer, polymorph, solvate, nitrogen oxide, isotopically labeled compound, metabolite or prodrug thereof according to any one of claims 1-28 as an active ingredient, in combination with a pharmaceutically acceptable carrier.

**30.** Use of the compound or the pharmaceutically acceptable salt, ester, stereoisomer, tautomer, polymorph, solvate, nitrogen oxide, isotopically labeled compound, metabolite or prodrug thereof according to any one of claims 1-28, or the pharmaceutical composition according to claim 29 in the manufacture of a medicament for preventing and/or treating a PARP1 enzyme-related disease.

**31.** The use according to claim 30, wherein the PARP1 enzyme-related disease is a tumor-related disease.

**32.** The use according to claim 31, wherein the tumor-related disease is deficient in an HR-dependent DNA DSB repair pathway.

**33.** The use according to claim 32, wherein the tumor-related disease comprises one or more cancer cells that have a reduced or absent ability to repair a DNA DSB through HR compared to normal cells.

**34.** The use according to claim 33, wherein the cancer cell has a BRCA1- or BRCA2-deficient phenotype.

**35.** The use according to any one of claims 31-34, wherein the tumor-related disease is breast cancer, ovarian cancer, primary peritoneal cancer, pancreatic cancer, prostate cancer, hematological cancer, gastrointestinal cancer, glioblastoma, or lung cancer.

**36.** Use of the compound or the pharmaceutically acceptable salt, ester, stereoisomer, tautomer, polymorph, solvate, nitrogen oxide, isotopically labeled compound, metabolite or prodrug thereof according to any one of claims 1-28, or the pharmaceutical composition according to claim 29 in the manufacture of a PARP1 inhibitor.

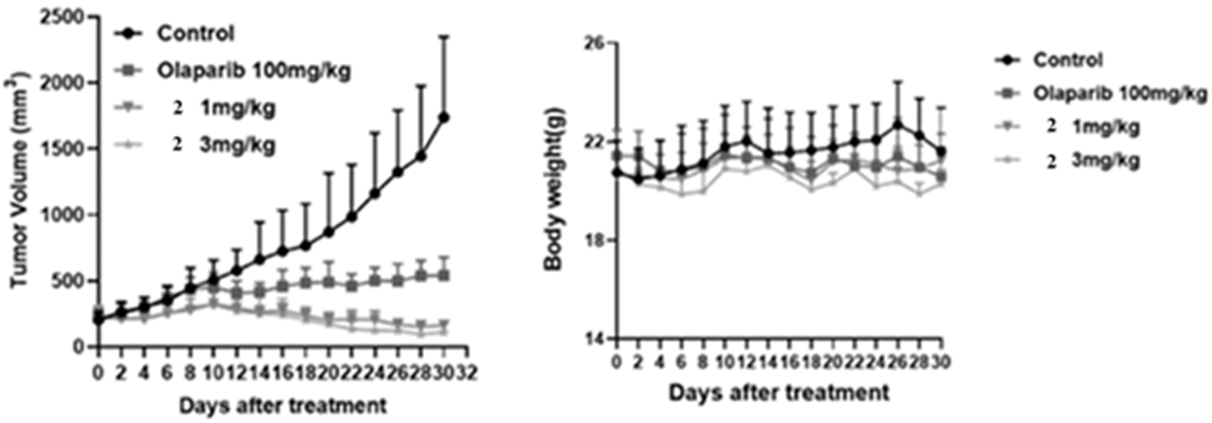
Tumor Volume (mm3)
2500
2000
1500
1000
500
0
Control
Olaparib 100mg/kg
2 1mg/kg
2 3mg/kg
0 2 4 6 8 10 12 14 16 18 20 22 24 26 28 30 32
Days after treatment
Body weight(g)
26
22
18
14
Control
Olaparib 100mg/kg
2 1mg/kg
2 3mg/kg
0 2 4 6 8 10 12 14 16 18 20 22 24 26 28 30
Days after treatment

FIG. 1

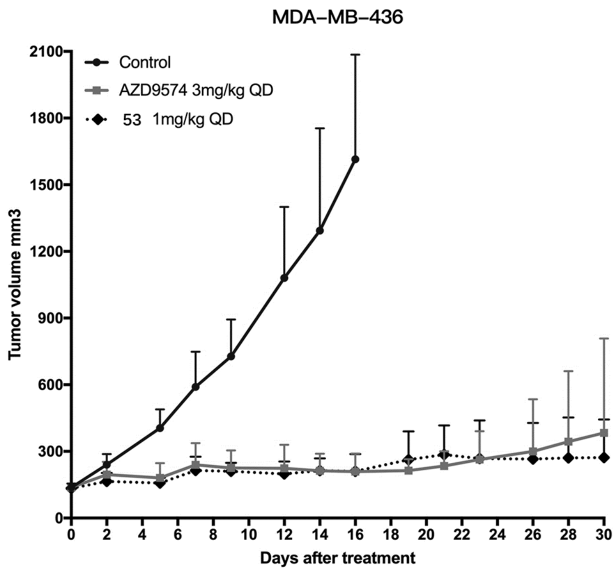
MDA-MB-436
Tumor volume mm3
2100
1800
1500
1200
900
600
300
0
Control
AZD9574 3mg/kg QD
53 1mg/kg QD
0 2 4 6 8 10 12 14 16 18 20 22 24 26 28 30
Days after treatment

FIG. 2

## INTERNATIONAL SEARCH REPORT

International application No.
**PCT/CN2024/121721**

### A. CLASSIFICATION OF SUBJECT MATTER

C07D491/052(2006.01)i; C07D401/14(2006.01)i; C07D487/00(2006.01)i; C07D471/00(2006.01)i; A61K31/435(2006.01)i; A61K31/496(2006.01)i; A61K31/498(2006.01)i; A61P35/00(2006.01)i

According to International Patent Classification (IPC) or to both national classification and IPC

### B. FIELDS SEARCHED

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, DWPI, STN(REGISTRY, CAPLUS, MARPAT), CNKI: 成都赜灵生物医药, 贾涛, 王太津, 李刚, 哌啶, 哌嗪, 吡啶, 癌症, 肿瘤, piperidi? , piperaz? , pyridin? , PARP1, cancer? , tumor? , 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| PA | WO 2024099364 A2 (LAEKNA THERAPEUTICS SHANGHAI CO., LTD.) 16 May 2024 (2024-05-16)<br>see claims 27, 55, and 64-65 | 1-36 |
| A | WO 2023122140 A1 (SYNNOVATION THERAPEUTICS, INC.) 29 June 2023 (2023-06-29)<br>see claims 1-57, and description, table 1 | 1-36 |
| A | WO 2023096915 A1 (SLAP PHARMACEUTICALS LLC) 01 June 2023 (2023-06-01)<br>see claims 1-46 | 1-36 |
| A | WO 2023146957 A1 (XINTHERA, INC.) 03 August 2023 (2023-08-03)<br>see claims 1 and 59, description, table 1, and abstract | 1-36 |
| A | WO 2023051807 A1 (HAISCO PHARMACEUTICAL GROUP CO., LTD.) 06 April 2023 (2023-04-06)<br>see claims 1-15 | 1-36 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 December 2024** | **31 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/121721**

| Patent document cited in search report | Publication date (day/month/year) | Patent family member(s) | Publication date (day/month/year) |
|---|---|---|---|
| WO 2024099364 A2 | 16 May 2024 | WO 2024099364 A3 | 13 June 2024 |
| WO 2023122140 A1 | 29 June 2023 | AU 2022420969 A1 | 13 June 2024 |
| | | PE 20242004 A1 | 03 October 2024 |
| | | KR 20240136978 A | 19 September 2024 |
| | | CO 2024009571 A2 | 29 July 2024 |
| | | MX 2024007673 A | 06 September 2024 |
| | | TW 202332438 A | 16 August 2023 |
| | | CR 20240296 A | 18 September 2024 |
| | | CA 3241875 A1 | 29 June 2023 |
| | | IL 313727 A | 01 August 2024 |
| | | EP 4452963 A1 | 30 October 2024 |
| WO 2023096915 A1 | 01 June 2023 | TW 202340166 A | 16 October 2023 |
| WO 2023146957 A1 | 03 August 2023 | KR 20240139604 A | 23 September 2024 |
| | | AU 2023213731 A1 | 15 August 2024 |
| | | CN 118510767A A | 16 August 2024 |
| WO 2023051807 A1 | 06 April 2023 | CN 118043323 A | 14 May 2024 |
| | | EP 4410791 A1 | 07 August 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021260092 A


### Non-patent literature cited in the description


- **FARMER H** ; **MCCABE N et al.** Targeting the DNA repair defect in BRCA mutant cells as a therapeutic strategy[J. *Nature*, 2005, vol. 434 (7035), 917-921
- **BRYANT, H** ; **SCHULTZ, N** ; **THOMAS, H. et al.** Specific killing of BRCA2-deficient tumours with inhibitors of poly(ADP-ribose) polymerase. *Nature*, 2005, vol. 434, 913-917
- **HARRIS P A** ; **BOLOOR A** ; **CHEUNG M et al.** Discovery of 5-[[4-[(2,3-dimethyl-2H-indazol-6-yl) methylamino]-2-pyrimidinyl]amino]-2-methyl-benzene sulfonamide (Pazopanib), a novel and potent vascular endothelial growth factor receptor inhibitor.[J. *Journal of Medicinal Chemistry*, 2008, vol. 51 (15), 4632
- Remington's Pharmaceutical Sciences. 2005